# EUROPÄISCHE PATENTSCHRIFT

(11) **EP 0 650 960 B1**
(45) Veröffentlichungstag und Bekanntmachung des Hinweises auf die Patenterteilung: **05.03.1997**
(21) Anmeldenummer: 94117017.7
(22) Anmeldetag: 27.10.1994
(51) Int. Cl.: C07D 213/81, C07D 213/82, C07D 217/26, C07D 409/12, C07D 213/89, A61K 31/44, A61K 31/47

(54) **Substituierte heterocyclische Carbonsäureamidester, ihre Herstellung und ihre Verwendung als Arzneimittel**
Substituted heterocyclic carboxylic acid amide esters, their preparation and their us as medicaments
Esters de carboxamide hétérocycliques substitués, leur préparation et leur utilisation comme médicaments

(30) Priorität: 02.11.1993 DE 4337270; 26.09.1994 DE 4434288
(43) Veröffentlichungstag der Anmeldung: 03.05.1995
(73) Patentinhaber: HOECHST AKTIENGESELLSCHAFT, 65929 Frankfurt am Main (DE)
(72) Erfinder: Weidmann, Klaus, Dr., D-61476 Kronberg (DE); Baringhaus, Karl-Heinz, Dr., D-61200 Wölfersheim (DE); Tschank, Georg, Dr., D-55270 Klein-Winternheim (DE); Bickel, Martin, Dr., D-61348 Bad Homburg (DE)

(56) Entgegenhaltungen:
- EP-A- 0 541 042
- EP-A- 0 562 512
- JOURNAL OF ORGANIC CHEMISTRY., Bd.31, Nr.2, 1966, EASTON US Seiten 636 - 638 J.T. SHEEHAN '3-Hydroxypicolinic acid and some of its derivatives.'
- LIEBIGS ANNALEN DER CHEMIE., Nr.1, 1986, WEINHEIM DE Seiten 1 - 20 H. KESSLER ET AL. 'Synthese des Benzylethers von Virginiamycin S1'

## Beschreibung

Die Erfindung betrifft substituierte heterocyclische Carbonsäureamidester, ihre Herstellung und ihre Verwendung zur Hemmung der Kollagenbiosynthese und ihre Verwendung als Arzneimittel zur Behandlung von fibrotischen Erkrankungen.

Verbindungen, die die Enzyme Prolin- und Lysinhydroxylase inhibieren, bewirken eine sehr selektive Hemmung der Kollagenbiosynthese durch Beeinflussung der kollagenspezifischen Hydroxylierungsreaktionen. In deren Verlauf wird proteingebundenes Prolin oder Lysin durch die Enzyme Prolin- bzw. Lysinhydroxylase hydroxyliert. Wird diese Reaktion durch Inhibitoren unterbunden, so entsteht ein nicht funktionsfähiges, unterhydroxyliertes Kollagenmolekül, das von den Zellen nur in geringer Menge in den extrazellulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens.

Inhibitoren der Prolylhydroxylase sind deshalb geeignete Substanzen in der Therapie von Erkrankungen, in denen die Ablagerung von Kollagenen maßgeblich zum Krankheitsbild beiträgt. Hierzu gehören u. a. Fibrosen der Lunge, Leber und Haut (Skleroderma und Vernarbungen nach Verbrennungen, Verletzungen und chirurgischen Eingriffen) sowie die Atherosklerose.

Es ist bekannt, daß das Enzym Prolinhydroxylase durch Pyridin-2,4- und - 2,5-dicarbonsäure effektiv gehemmt wird (K. Majamaa et al., Eur. J. Biochem. 138 (1984) 239-245). Diese Verbindungen sind in der Zellkultur allerdings nur in sehr hohen Konzentrationen als Hemmstoffe wirksam (Tschank, G. et al., Biochem. J. 238 (1987) 625-633).
Auch Prodrugs der Pyridin-2,4(5)-dicarboxylate sind bekannt. Diese sind in den älteren deutschen Anmeldungen P 42 33 124.2, P 42 38 506.7 und P 42 09 424.0 beschrieben.

N-Oxalylglycine als Inhibitoren der Prolyl-4-hydroxylase sind aus J.Med.Chem. 1992, 35, 2652-2658 (Cunliffe et al.) und EP-A-0 457 163 bekannt.

Hydroxyisochinoline- und Hydroxycinnolincarbonsäureglycylamide sind aus Biochem. Soc. Trans. 1991, 19, 812-815 (Franklin et al.) bekannt. 3-Benzyloxypyridin-2-carbonsäure-(L-threonylmethylesteramid), 3-Benzyloxypyridin-2-carbonsäure-(L-threonyl(Fmoc-Phg)tert.butylester)amid, 3-Benzyloxypyridin-2-carbonsäure-(L-threonyl-tert.butylester)amid und 3-Benzyloxypyridin-2-carbonsäure-(D-allothreonylmethylester)amid sind aus Liebigs Ann. Chem., 1986, 1-20, Kessler et al., bekannt.

Weiterhin ist 3-Benzyoxy-pyridin-2-carbonsäure-(glycylethylester)amid in J. Org. Chem. 31, 636-638 (1966) beschrieben.

Überraschenderweise wurde nun gefunden, daß heterocyclische Carbonsäureamide der Formel I mit einem Ether-, einem Thioether- einem Amino-Substituenten oder einem Rest R⁴ in ortho-Position zur Amidfunktion in vivo stark wirksame Inhibitoren der Kollagenbiosynthese sind.

Die Verbindungen sind Ester-Prodrugs der entsprechenden Carbonsäuren der Formel I, in denen B eine Carboxylgruppe bedeutet.

Die Verbindungen der Formel I werden im lebenden Organismus (in vivo) und in Zellkulturen (in vitro) zu Verbindungen der Formel I, in denen B eine Carboxylgruppe oder deren Salze bedeutet, gespalten.

Nach der Applikation der Verbindungen der Formel I bewirken sie die in vivo und in vitro zu beobachtende Hemmung der Kollagenbiosynthese, indem sie Verbindungen der Formel I, in denen B eine Carboxylgruppe oder deren Salze bedeutet, bilden. Diese Verbindungen inhibieren die Prolyl-4-hydroxylase und führen deshalb zu einer Hemmung der Kollagenbiosynthese.

Die erfindungsgemäßen Verbindungen entsprechen der allgemeinen Formel I 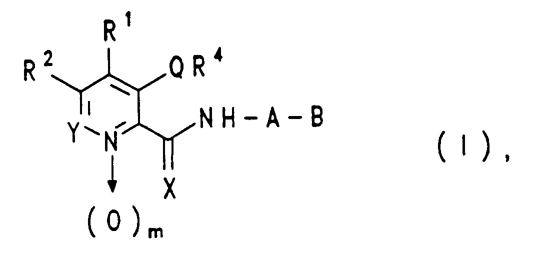 in welcher
- Q: O, S, NR' oder eine Bindung,
- X: O und S,
- Y: C-R³ oder, falls R¹ und R² einen Cyclus bilden,
- Y: N oder CR³ bedeutet,
- m: 0 und 1,
- A: (C₁-C₄)-Alkylen, das gegebenenfalls substituiert ist mit einem oder zwei Substituenten aus der Reihe Halogen, Cyano, Nitro, Trifluormethyl, (C₁-C₆)-Alkyl, (C₁-C₆)-Hydroxyalkyl, (C₁-C₆)-Alkoxy, -O-[CH₂]ₓ-C_{f}H_{(2f+1-g)}Hal_{g}, vorzugsweise (C₁-C₈)-Fluoralkoxy, (C₁-C₈)-Fluoralkenyloxy, (C₁-C₈)-Fluoralkinyloxy, -OCF₂Cl oder -O-CF₂-CHFCl, (C₁-C₆)-Alkylmercapto, (C₁-C₆)-Alkylsulfinyl, (C₁-C₆)-Alkylsulfonyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Phenyl, Benzyl, Phenoxy, Benzyloxy, Anilino, N-Methylanilino, Phenylmercapto, Phenylsulfonyl, Phenylsulfinyl, Sulfamoyl, N-(C₁-C₄)-Alkylsulfamoyl, N,N-Di-(C₁-C₄)-alkylsulfamoyl, oder mit einem substituierten (C₆-C₁₂)-Aryloxy-, (C₇-C₁₁)-Aralkyloxy, (C₆-C₁₂)-Aryl- oder (C₇-C₁₁)-Aralkyl-Rest, der im Arylteil 1, 2, 3, 4 oder 5 gleiche oder verschiedene Substituenten aus der Reihe Halogen, Cyano, Nitro, Trifluormethyl, (C₁-C₆)-Alkyl, (C₁-C₆)-Alkoxy, - O-[CH₂]ₓ-C_{f}H_{(2f+1-g)}Hal_{g}, -OCF₂Cl,-O-CF₂-CHFCl, (C₁-C₆)-Alkylmercapto, (C₁-C₆)-Alkylsulfinyl, (C₁-C₆)-Alkylsulfonyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Sulfamoyl, N-(C₁-C₄)-Alkylsulfamoyl oder N,N-Di-(C₁-C₄)-alkylsulfamoyl trägt, oder
mit den Substituenten R⁵ des α-C-Atoms einer α-Aminosäure, wobei die natürlichen L-Aminosäuren und ihre D-Isomeren Verwendung finden können;
- B: -CO₂G bedeutet, wobei G den Rest eines Alkohols GOH bedeutet,
- R¹, R² und R³: gleich oder verschieden sind und Wasserstoff, Hydroxy, Halogen, Cyano, Trifluormethyl, Nitro, Carboxy, (C₁-C₂₀)-Alkyl, (C₃-C₈)-Cycloalkyl, (C₃-C₈)-Cycloalkyl-(C₁-C₁₂)-alkyl, (C₃-C₈)-Cycloalkoxy, (C₃-C₈)-Cycloalkyl-(C₁-C₁₂)-alkoxy, (C₃-C₈)-Cycloalkyloxy-(C₁-C₁₂)-alkyl, (C₃-C₈)-Cycloalkyloxy-(C₁-C₁₂)-alkoxy, (C₃-C₈)-Cycloalkyl-(C₁-C₈)-alkyl-(C₁-C₆)-alkoxy, (C₃-C₈)-Cycloalkyl-(C₁-C₈)-alkoxy-(C₁-C₆)-alkyl, (C₃-C₈)-Cycloalkyloxy-(C₁-C₈)-alkoxy-(C₁-C₆)-alkyl, (C₃-C₈)-Cycloalkoxy-(C₁-C₈)-alkoxy-(C₁-C₈)-alkoxy, (C₆-C₁₂)-Aryl, (C₇-C₁₆)-Aralkyl, (C₇-C₁₆)-Aralkenyl, (C₇-C₁₆)-Aralkinyl, (C₂-C₂₀)-Alkenyl, (C₂-C₂₀)-Alkinyl, (C₁-C₂₀)-Alkoxy, (C₂-C₂₀)-Alkenyloxy, (C₂-C₂₀)-Alkinyloxy, Retinyloxy, (C₁-C₁₂)-Alkoxy-(C₁-C₁₂)-alkyl, (C₁-C₁₂)-Alkoxy-(C₁-C₁₂)-alkoxy, (C₁-C₁₂)-Alkoxy-(C₁-C₈)-alkoxy-(C₁-C₈)-alkyl, (C₆-C₁₂)-Aryloxy, (C₇-C₁₆)-Aralkyloxy, (C₆-C₁₂)-Aryloxy-(C₁-C₆)-alkoxy, (C₇-C₁₆)-Aralkoxy-(C₁-C₆)-alkoxy, (C₁-C₁₆)-Hydroxyalkyl, (C₆-C₁₆)-Aryloxy-(C₁-C₈)-alkyl, (C₇-C₁₆)-Aralkoxy-(C₁-C₈)-alkyl, (C₆-C₁₂)-Aryloxy-(C₁-C₈)-alkoxy-(C₁-C₆)-alkyl, (C₇-C₁₂)-Aralkyloxy-(C₁-C₈)-alkoxy-(C₁-C₆)-alkyl, (C₂-C₂₀)-Alkenyloxy-(C₁-C₆)-alkyl, (C₂-C₂₀)-Alkinyloxy-(C₁-C₆)-alkyl, Retinyloxy-(C₁-C₆)-alkyl, -O-[CH₂₋]ₓC_{f}H_{(2f+1-g)}F_{g}, -OCF₂Cl, -OCF₂-CHFCl,
(C₁-C₂₀)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₂-C₂₀) Alkenylcarbonyl, (C₂-C₂₀)-Alkinylcarbonyl,
(C₁-C₂₀)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₂₀)-Alkenyloxycarbonyl, Retinyloxycarbonyl, (C₂-C₂₀)-Alkinyloxycarbonyl, (C₆-C₁₂)-Aryloxy-(C₁-C₆)-alkoxycarbonyl, (C₇-C₁₆)-Aralkoxy-(C₁-C₆)-alkoxycarbonyl, (C₃-C₈)-Cycloalkyl-(C₁-C₆)-alkoxycarbonyl, (C₃-C₈)-Cycloalkoxy-(C₁-C₆)-alkoxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₂-C₁₂)-Alkenyloxycarbonyloxy, (C₂-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-Cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₈)-Alkoxy-(C₁-C₁₀)-alkyl)carbamoyl, N-((C₆-C₁₆)-Aryloxy-(C₁-C₁₀)-alkyl)carbamoyl,
N-((C₇-C₁₆)-Aralkyloxy-(C₁-C₁₀)-alkyl)carbamoyl, N-(C₁-C₁₀)-Alkyl-N-((C₁-C₁₀)-alkoxy-(C₁-C₁₀)-alkyl)carbamoyl, N-(C₁-C₁₀)-Alkyl-N-((C₆-C₁₂)-aryloxy-(C₁-C₁₀)-alkyl)carbamoyl, N-(C₁-C₁₀)-Alkyl-N-((C₇-C₁₆)-aralkyloxy-(C₁-C₁₀)-alkyl)carbamoyl, CON(CH₂)ₕ, worin eine CH₂-Gruppe durch O, S, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 7 bedeutet,
einen Carbamoyl-Rest der allgemeinen Formel II 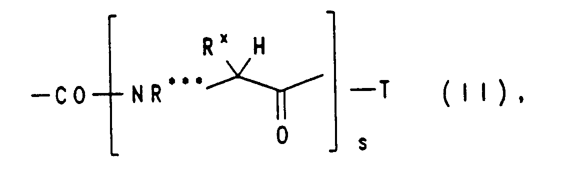 worin
- R^{x}: den Substituenten einer α-Aminosäure bedeutet, zu denen die L-und D-Aminosäuren zählen,
- s: 1, 2, 3, 4 oder 5 und
- T: OH, OR oder NR*R** bedeutet, wobei
- R*, R** und R***: gleich oder verschieden sind und Wasserstof (C₆-C₁₂)-Aryl, (C₇-C₁₁)-Aralkyl, (C₁-C₈)-Alkyl, (C₃-C₈)-Cycloalkyl, (+)-Dehydroabietyl, (C₁-C₈)-Alkoxy-(C₁-C₈)-alkyl, (C₇-C₁₂)-Aralkoxy-(C₁-C₈)-alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, oder
- R* und R**: gemeinsam für -[CH₂]ₕ stehen, worin eine CH₂ Gruppe durch O, S, SO, SO₂, N-Acylamino, N-(C₁-C₁₀)-Alkoxycarbonylimino, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt sein kann und h 3 bis 7 bedeutet,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₂)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy, N-((C₁-C₁₀)-alkyl))carbamoyloxy, N-((C₆-C₁₂)-Aryloxy-(C₁-C₁₀)-alkyl)carbamoyloxy, N-((C₇-C₁₆)-Aralkyloxy-(C₁-C₁₀)-alkyl)carbamoyloxy, N-(C₁-C₁₀)-Akyl-N-((C₁-C₁₀)-alkoxy-(C₁-C₁₀)-alkyl)carbamoyloxy, N-(C₁-C₁₀)-Alkyl-N-((C₆-C₁₂)-aryloxy-(C₁-C₁₀)-alkyl)carbamoyloxy, N-(C₁-C₁₀)-Akyl-N-((C₇-C₁₆)-aralkyloxy-(C₁-C₁₀)-alkyl)carbamoyloxy,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₂)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)-alkyl, N,N-Di(C₁-C₁₀)-alkylamino-(C₁-C₁₀)-alkyl, (C₃-C₈)-Cycloalkylamino-(C₁-C₁₀)-alkyl, (C₁-C₂₀)-Alkylmercapto, (C₁-C₂₀)-Alkylsulfinyl, (C₁-C₂₀)-Alkylsulfonyl, (C₆-C₁₂)-Arylmercapto, (C₆-C₁₂)-Arylsulfinyl, (C₆-C₁₂)-Arylsulfonyl, (C₇-C₁₆)-Aralkylmercapto, (C₇-C₁₆)-Aralkylsulfinyl, (C₇-C₁₆)-Aralkylsulfonyl, (C₁-C₁₂)-Alkylmercapto-(C₁-C₆)-alkyl, (C₁-C₁₂)-Alkylsulfinyl-(C₁-C₆)-alkyl, (C₁-C₁₂)-Alkylsulfonyl-(C₁-C₆)-alkyl, (C₆-C₁₂)-Arylmercapto-(C₁-C₆)-alkyl, (C₆-C₁₂)-Arylsulfinyl-(C₁-C₆)-alkyl, (C₆-C₁₂)-Arylsulfonyl-(C₁-C₆)-alkyl, (C₇-C₁₆)-Aralkylmercapto-(C₁-C₆)-alkyl, (C₇-C₁₆)-Aralkylsulfinyl-(C₁-C₆)-alkyl, (C₇-C₁₆)-Aralkylsulfonyl-(C₁-C₆)-alkyl,
Sulfamoyl, N-(C₁-C₁₀)-Alkylsulfamoyl, N,N-Di(C₁-C₁₀)-alkylsulfamoyl,
(C₃-C₈)-Cycloalkylsulfamoyl,
N-(C₆-C₁₂)-Arylsulfamoyl,
N-(C₇-C₁₆)-Aralkylsulfamoyl,
N-(C₁-C₁₀)-Alkyl-N-(C₆-C₁₂)-arylsulfamoyl,
N-(C₁-C₁₀)-Alkyl-N-(C₇-C₁₆)-aralkylsulfamoyl,
(C₁-C₁₀)-Alkyl-sulfonamido,
N-((C₁-C₁₀)-alkyl)-(C₁-C₁₀)-alkylsulfonamido, (C₇-C₁₆)-Aralkylsulfonamido,
N-((C₁-C₁₀)-alkyl-(C₇-C₁₆)-aralkylsulfonamido,
wobei die Reste, die einen Arylrest enthalten ihrerseits am Aryl substituiert sein können durch 1 bis 5 gleiche oder verschiedene Reste aus der Reihe:
Hydroxy, Halogen, Cyano, Trifluormethyl, Nitro, Carboxy, (C₁-C₁₆)-Alkyl, (C₃-C₈)-Cycloalkyl, (C₃-C₈)-Cycloalkyl-(C₁-C₁₂)-alkyl, (C₃-C₈)-Cycloalkoxy, (C₃-C₈)-Cycloalkyl-(C₁-C₁₂)-alkoxy, (C₃-C₈)-Cycloalkyloxy-(C₁-C₁₂)-alkyl, (C₃-C₈)-Cycloalkyloxy-(C₁-C₁₂)-alkoxy, (C₃-C₈)-Cycloalkyl-(C₁-C₈)-alkyl-(C₁-C₆)-alkoxy, (C₃-C₈)-Cycloalkyl-(C₁-C₈)-alkoxy-(C₁-C₆)-alkyl, (C₃-C₈)-Cycloalkyloxy-(C₁-C₈)-alkoxy-(C₁-C₆)-alkyl, (C₃-C₈)-Cycloalkoxy-(C₁-C₈)-alkoxy-(C₁-C₈)-alkoxy, (C₆-C₁₂)-Aryl, (C₇-C₁₆)-Aralkyl, (C₂-C₁₆)-Alkenyl, (C₂-C₁₂)-Alkinyl, (C₁-C₁₆)-Alkoxy, (C₁-C₁₆)-Alkenyloxy, (C₁-C₁₂)-Alkoxy-(C₁-C₁₂)-alkyl, (C₁-C₁₂)-Alkoxy-(C₁-C₁₂)-alkoxy, (C₁-C₁₂)-Alkoxy-(C₁-C₈)-alkoxy-(C₁-C₈)-alkyl, (C₆-C₁₂)-Aryloxy, (C₇-C₁₆)-Aralkyloxy, (C₆-C₁₂)-Aryloxy-(C₁-C₆)-alkoxy, (C₇-C₁₆)-Aralkoxy-(C₁-C₆)-alkoxy, (C₁-C₈)-Hydroxyalkyl, (C₆-C₁₆)-Aryloxy-(C₁-C₈)-alkyl, (C₇-C₁₆)-Aralkoxy-(C₁-C₈)-alkyl, (C₆-C₁₂)-Aryloxy-(C₁-C₈)-alkoxy-(C₁-C₆)-alkyl, (C₇-C₁₂)-Aralkyloxy-(C₁-C₈)-alkoxy-(C₁-C₆)-alkyl, -O-[CH₂₋]ₓC_{f}H_{(2f+1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₁₂)-Alkenyloxycarbonyl, (C₂-C₁₂)-Alkinyloxycarbonyl, (C₆-C₁₂)-Aryloxy-(C₁-C₆)-alkoxycarbonyl, (C₇-C₁₆)-Aralkoxy-(C₁-C₆)-alkoxycarbonyl, (C₃-C₈)-Cycloalkyl-(C₁-C₆)-alkoxycarbonyl, (C₃-C₈)-Cycloalkoxy-(C₁-C₆)-alkoxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₂-C₁₂)-Alkenyloxycarbonyloxy, (C₂-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Akyl-N-(C₇-C₁₆)-aralkylcarbamoyl, N-((C₁-C₁₆)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl,
N-(C₁-C₁₀)-Akyl-N-((C₁-C₁₀)-alkoxy-(C₁-C₁₀)-alkyl)carbamoyl, N-(C₁-C₁₀)-Alkyl-N-((C₆-C₁₂)-aryloxy-(C₁-C₁₀)-alkyl)carbamoyl, N-(C₁-C₁₀)-Alkyl-N-((C₇-C₁₆)-aralkyloxy-(C₁-C₁₀)-alkyl)carbamoyl, CON(CH₂)ₕ, worin eine CH₂-Gruppe durch O, S, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 7 bedeutet,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₆)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy, N-((C₁-C₁₀)-alkyl))carbamoyloxy, N-((C₆-C₁₂)-Aryloxy-(C₁-C₁₀)-alkyl)-carbamoyloxy, N-((C₇-C₁₆)-Aralkyloxy-(C₁-C₁₀)-alkyl)carbamoyloxy, N-(C₁-C₁₀)-Alkyl-N-((C₁-C₁₀)-alkoxy-(C₁-C₁₀)-alkyl)carbamoyloxy, N-(C₁-C₁₀)-Alkyl-N-((C₆-C₁₂)-aryloxy-(C₁-C₁₀)-alkyl)carbamoyloxy, N-(C₁-C₁₀)-Alkyl-N-((C₇-C₁₆)-aralkyloxy-(C₁-C₁₀)-alkyl)carbamoyloxy,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀]-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₂)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-Alkylamino-(C₁-C₁₀)-alkyl, N,N-Di-(C₁-C₁₀)-alkylamino-(C₁-C₁₀)-alkyl, (C₃-C₈)-Cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl,
R¹ und R² oder R² und R³ eine Kette [CH₂]ₒ bilden, in welcher eine oder zwei CH₂-Gruppen der gesättigten oder mit einer C = C-Doppelbindung ungesättigten Kette gegebenenfalls durch O, S, SO, SO₂ oder NR' ersetzt sind, o = 3,4 oder 5 bedeutet und
R' Wasserstoff, (C₆-C₁₂)-Aryl, (C₁-C₈)-Alkyl, (C₁-C₈)-Alkoxy-(C₁-C₈)-alkyl, (C₇-C₁₂)-Aralkoxy-(C₁-C₈)-alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, wobei
vorzugsweise die Reste R¹ und R² oder R² und R³ zusammen mit dem sie tragenden Pyridin oder Pyridazin einen 5,6,7,8-Tetrahydroisochinolin-, einen 5,6,7,8-Tetrahydrochinolin- oder einen 5,6,7,8-Tetrahydrocinnolin-Ring bilden,
oder
R¹ und R² oder R² und R³ einen carbocylischen oder einen heterocyclischen, 5- oder 6-gliedrigen aromatischen Ring bilden, wobei
vorzugsweise die Reste R¹ und R² oder R² und R³ zusammen mit dem sie tragenden Pyridin oder Pyridazin folgende ggf. substituierte heterocyclischen Ringsysteme bilden:
Thienopyridine,
Furanopyridine,
Pyridopyridine,
Pyrimidinopyridine,
Imidazopyridine,
Thiazolopyridine,
Oxazolopyridine,
Chinolin, Isochinolin und
Cinnolin,
wobei Chinolin, Isochinolin oder Cinnolin vorzugsweise den Formeln 1a, 1b und 1c genügen 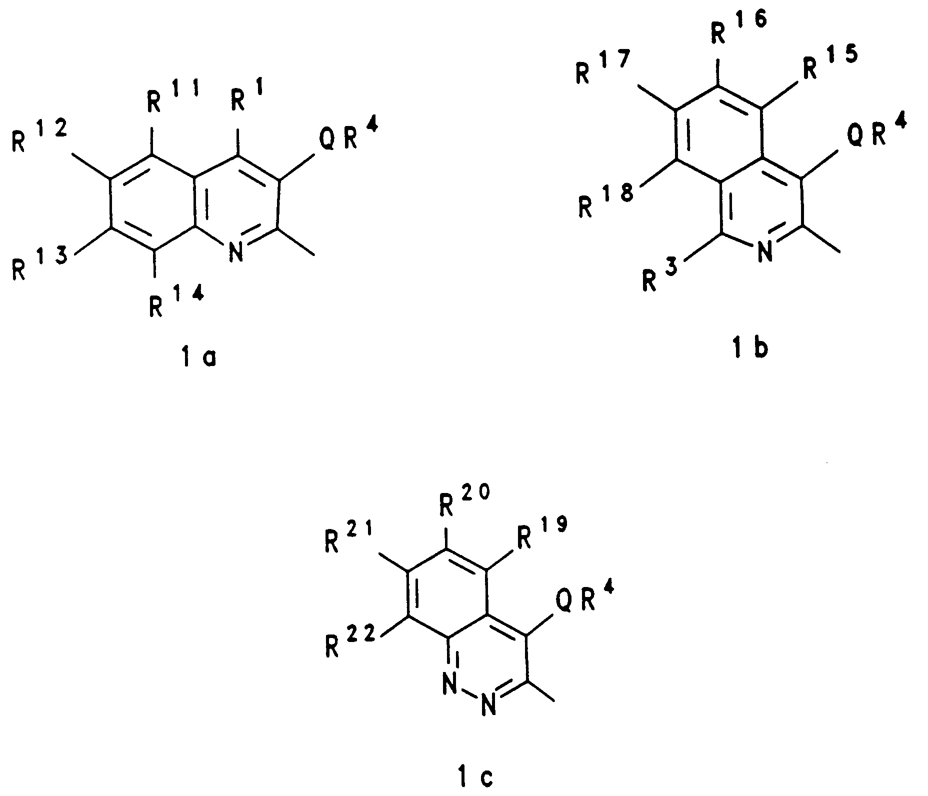
und die Substituenten R¹¹ bis R²² jeweils unabhängig voneinander in der Bedeutung von R¹, R² und R³ stehen,
- R⁴: falls Q eine Bindung ist, Halogen, Nitril und Trifluormethyl bedeutet, oder falls Q O, S oder NR' ist, einen verzweigten oder unverzweigten (C₁-C₂₀)-Alkylrest, einen unsubstituierten, gesättigten Fluoralkylrest der Formel [CH₂]ₓC_{f}H_{(2f+1-g)}F_{g}, einen (C₆-C₁₆)-Arylrest, einen (C₇-C₁₆)-Aralkylrest, einen Heteroarylrest oder einen Heteroaralkylrest bedeutet,
wobei diese Reste substituiert sind mit einem oder mehreren Resten aus der Reihe
Hydroxy, Halogen, Cyano, Trifluormethyl, Nitro, Carboxy, (C₁-C₁₂)-Alkyl, (C₃-C₈)-Cycloalkyl, (C₃-C₈)-Cycloalkyl-(C₁-C₁₂)-alkyl, (C₃-C₈)-Cycloalkoxy, (C₃-C₈)-Cycloalkyl-(C₁-C₁₂)-alkoxy, (C₃-C₈)-Cycloalkyloxy-(C₁-C₁₂)-alkyl, (C₃-C₈)-Cycloalkyloxy-(C₁-C₁₂)-alkoxy, (C₃-C₈)-Cycloalkyl-(C₁-C₈)-alkyl-(C₁-C₆)-alkoxy, (C₃-C₈)-Cycloalkyl-(C₁-C₈)-alkoxy-(C₁-C₆)-alkyl, (C₃-C₈)-Cycloalkyloxy-(C₁-C₈)-alkoxy-(C₁-C₆)-alkyl, (C₃-C₈)-Cycloalkoxy-(C₁-C₈)-alkoxy-(C₁-C₈)-alkoxy, (C₆-C₁₂)-Aryl, (C₇-C₁₆)-Aralkyl, (C₂-C₁₂)-Alkenyl, (C₂-C₁₂)-Alkinyl, (C₁-C₁₂)-Alkoxy, (C₁-C₁₂)-Alkoxy-(C₁-C₁₂)-alkyl, (C₁-C₁₂)-Alkoxy-(C₁-C₁₂)-alkoxy, (C₁-C₁₂)-Alkoxy-(C₁-C₈)-alkoxy-(C₁-C₈)-alkyl, (C₆-C₁₂)-Aryloxy, (C₇-C₁₆)-Aralkyloxy, (C₆-C₁₂)-Aryloxy-(C₁-C₆)-alkoxy, (C₇-C₁₆)-Aralkoxy-(C₁-C₆)-alkoxy, (C₁-C₈)-Hydroxyalkyl, (C₆-C₁₆)-Aryloxy-(C₁-C₈)-alkyl, (C₇-C₁₆)-Aralkoxy-(C₁-C₈)-alkyl, (C₆-C₁₂)-Aryloxy-(C₁-C₈)-alkoxy-(C₁-C₆)-alkyl, (C₇-C₁₂)-Aralkyloxy-(C₁-C₈)-alkoxy-(C₁-C₆)-alkyl, -O-[CH₂₋]ₓC_{f}H_{(2f+1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₂-C₁₂) Alkenylcarbonyl, (C₂-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl, (C₂-C₁₂)-Alkenyloxycarbonyl, (C₂-C₁₂)-Alkinyloxycarbonyl, (C₆-C₁₂)-Aryloxy-(C₁-C₆)-alkoxycarbonyl, (C₇-C₁₆)-Aralkoxy-(C₁-C₆)-alkoxycarbonyl, (C₃-C₈)-Cycloalkyl-(C₁-C₆)-alkoxycarbonyl, (C₃-C₈)-Cycloalkoxy-(C₁-C₆)-alkoxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₂-C₁₂)-Alkenyloxycarbonyloxy, (C₂-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-Cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₀)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl, N-(C₁-C₁₀)-Alkyl-N-((C₁-C₁₀)-alkoxy-(C₁-C₁₀)-alkyl)carbamoyl, N-(C₁-C₁₀)-Alky-N-((C₆-C₁₂)-aryloxy-(C₁-C₁₀)-alkyl)carbamoyl, N-(C₁-C₁₀)-Alkyl-N-((C₇-C₁₆)-aralkyloxy-(C₁-C₁₀)-alkyl)carbamoyl, CON(CH₂)ₕ, worin eine CH₂-Gruppe durch O, S, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 7 bedeutet, oder mit
einem Carbamoyl-Rest der allgemeinen Formel II 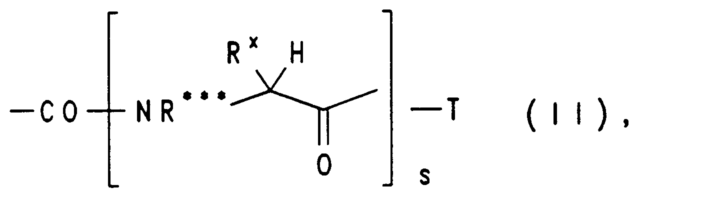 worin
- R^{x}: den Substituenten einer α-Aminosäure bedeutet, zu denen die L-und D-Aminosäuren zählen,
- s: 1, 2, 3, 4 oder 5 und
- T: OH, OR oder NR*R** bedeutet, wobei
- R*, R** und R***: gleich oder verschieden sind und Wasserstoff, (C₆-C₁₂)-Aryl, (C₇-C₁₁)-Aralkyl, (C₁-C₈)-Alkyl, (C₃-C₈)-Cycloalkyl, (+)-Dehydroabietyl, (C₁-C₈)-Alkoxy-(C₁-C₈)-alkyl, (C₇-C₁₂)-Aralkoxy-(C₁-C₈)-alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, oder
- R* und R**: gemeinsam für -[CH₂]ₕ stehen, worin eine CH₂ Gruppe durch O, S, SO, SO₂, N-Acylamino, N-(C₁-C₁₀)-Alkoxycarbonylimino, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt sein kann und h 3 bis 7 bedeutet, oder mit
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₂)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-aralkylcarbamoyloxy, N-((C₁-C₁₀)-alkyl))carbamoyloxy, N-((C₆-C₁₂)-Aryloxy-(C₁-C₁₀)-alkyl)carbamoyloxy,
N-((C₇-C₁₆)-Aralkyloxy-(C₁-C₁₀)-alkyl)carbamoyloxy, N-(C₁-C₁₀)-Alkyl-N-((C₁-C₁₀)-alkoxy-(C₁-C₁₀)-alkyl)carbamoyloxy, N-(C₁-C₁₀)-Alkyl-N-((C₆-C₁₂)-aryloxy-(C₁-C₁₀)-alkyl)carbamoyloxy, N-(C₁-C₁₀)-Alkyl-N-((C₇-C₁₆)-aralkyloxy-(C₁-C₁₀)-alkyl)carbamoyloxy,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₂)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)-alkyl, N,N-Di(C₁-C₁₀)-alkylamino-(C₁-C₁₀)-alkyl, (C₃-C₈)-Cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₂)-Arylmercapto, (C₆-C₂)-Arylsulfinyl, (C₆-C₁₂)-Arylsulfonyl, (C₇-C₁₆)-Aralkylmercapto, (C₇-C₁₆)-Aralkylsulfinyl, (C₇-C₁₆)-Aralkylsulfonyl,
Sulfamoyl, N-(C₁-C₁₀)-Alkylsulfamoyl, N,N-Di-(C₁-C₁₀)-alkylsulfamoyl, (C₃-C₈)-Cycloalkylsulfamoyl,
N-(C₆-C₁₂)-Arylsulfamoyl,
N-(C₇-C₁₆)-Aralkylsulfamoyl,
N-(C₁-C₁₀)-Alkyl-N-(C₆-C₁₂)-arylsulfamoyl,
N-(C₁-C₁₀)-Alkyl-N-(C₇-C₁₆)-aralkylsulfamoyl,
(C₁-C₁₀)-Alkyl-sulfonamido,
N-((C₁-C₁₀)-Alkyl)-(C₁-C₁₀)-alkylsulfonamido, (C₇-C₁₆)-Aralkylsulfonamido, N-((C₁-C₁₀)-Alkyl-(C₇-C₁₆)-aralkylsulfonamido, wobei die Reste, die einen Arylrest enthalten ihrerseits am Aryl substituiert sein können durch 1 bis 5 gleiche oder verschiedene Reste aus der Reihe:
Hydroxy, Halogen, Cyano, Trifluormethyl, Nitro, Carboxy, (C₁-C₁₂)-Alkyl, (C₃-C₈)-Cycloalkyl, (C₃-C₈)-Cycloalkyl-(C₁-C₁₂)-alkyl, (C₃-C₈)-Cycloalkoxy, (C₃-C₈)-Cycloalkyl-(C₁-C₁₂)-alkoxy, (C₃-C₈)-Cycloalkyloxy-(C₁-C₁₂)-alkyl, (C₃-C₈)-Cycloalkyloxy-(C₁-C₁₂)-alkoxy, (C₃-C₈)-Cycloalkyl-(C₁-C₈)-alkyl-(C₁-C₆)-alkoxy, (C₃-C₈)-Cycloalkyl-(C₁-C₈)-alkoxy-(C₁-C₆)-alkyl, (C₃-C₈)-Cycloalkyloxy-(C₁-C₈)-alkoxy-(C₁-C₆)-alkyl, (C₃-C₈)-Cycloalkoxy-(C₁-C₈)-alkoxy-(C₁-C₈)-alkoxy, (C₆-C₁₂)-Aryl, (C₇-C₁₆)-Aralkyl, (C₂-C₁₂)-Alkenyl, (C₂-C₁₂)-Alkinyl, (C₁-C₁₂)-Alkoxy, (C₁-C₁₂)-Alkoxy-(C₁-C₁₂)-alkyl, (C₁-C₁₂)-Alkoxy-(C₁-C₁₂)-alkoxy, (C₁-C₁₂)-Alkoxy-(C₁-C₈)-alkoxy-(C₁-C₈)-alkyl, (C₆-C₁₂)-Aryloxy, (C₇-C₁₆)-Aralkyloxy, (C₆-C₁₂)-Aryloxy-(C₁-C₆)-alkoxy, (C₇-C₁₆)-Aralkoxy-(C₁-C₆)-alkoxy, (C₁-C₈)-Hydroxyalkyl, (C₆-C₁₆)-Aryloxy-(C₁-C₈)-alkyl, (C₇-C₁₆)-Aralkoxy-(C₁-C₈)-alkyl, (C₆-C₁₂)-Aryloxy-(C₁-C₈)-alkoxy-(C₁-C₆)-alkyl, (C₇-C₁₂)-Aralkyloxy-(C₁-C₈)-alkoxy-(C₁-C₆)-alkyl, -O-[CH₂₋]ₓC_{f}H_{(2f+1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₁₂)-Alkenyloxycarbonyl, (C₂-C₁₂)-Alkinyloxycarbonyl, (C₆-C₁₂)-Aryloxy-(C₁-C₆)-alkoxycarbonyl, (C₇-C₁₆)-Aralkoxy-(C₁-C₆)-alkoxycarbonyl, (C₃-C₈)-Cycloalkyl-(C₁-C₆)-alkoxycarbonyl, (C₃-C₈)-Cycloalkoxy-(C₁-C₆)-alkoxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₂-C₁₂)-Alkenyloxycarbonyloxy, (C₂-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₀)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl, N-(C₁-C₁₀)-Alkyl-N-((C₁-C₁₀)-alkoxy-(C₁-C₁₀)-alkyl)carbamoyl, N-(C₁-C₁₀)-Alkyl-N-((C₆-C₁₂)-aryloxy-(C₁-C₁₀)-alkyl)carbamoyl, N-(C₁-C₁₀)-Alkyl-N-((C₇-C₁₆)-aralkyloxy-(C₁-C₁₀)-alkyl)carbamoyl, CON(CH₂)ₕ, worin eine CH₂-Gruppe durch O, S, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 7 bedeutet,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₆)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy, N-((C₁-C₁₀)-alkyl))carbamoyloxy,
N-((C₆-C₁₂)-Aryloxy-(C₁-C₁₀)-alkyl)-carbamoyloxy, N-((C₇-C₁₆)-Aralkyloxy-(C₁-C₁₀)-alkyl)carbamoyloxy, N-(C₁-C₁₀)-Alkyl-N-((C₁-C₁₀)-alkoxy-(C₁-C₁₀)-alkyl)carbamoyloxy, N-(C₁-C₁₀)-Alkyl-N-((C₆-C₁₂)-aryloxy-(C₁-C₁₀)-alkyl)carbamoyloxy, N-(C₁-C₁₀)-Alky-N-((C₇-C₁₆)-aralkyloxy-(C₁-C₁₀)-alkyl)carbamoyloxy,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂)-Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₂)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-Alkylamino-(C₁-C₁₀)-alkyl, N,N-Di-(C₁-C₁₀)-alkylamino-(C₁-C₁₀)-alkyl, (C₃-C₈)-Cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl, und
- R⁴: R'' bedeutet, sofern Q in der Bedeutung von NR' steht, wobei
R' und R'' gleich oder verschieden sind und Wasserstoff, (C₆-C₁₂)-Aryl, (C₇-C₁₁)-Aralkyl, (C₁-C₈)-Alkyl, (C₁-C₈)-Alkoxy-(C₁-C₈)-alkyl, (C₇-C₁₂)-Aralkoxy-(C₁-C₈)-alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, oder
- R' und R'': gemeinsam für -[CH₂]ₕ stehen, worin eine CH₂-Gruppe durch O, S, N-Acylimino oder N-(C₁-C₁₀)-Alkoxycarbonylimino ersetzt sein kann, und
- f: 1 bis 8,
- g: 0,1 bis (2f + 1),
- x: 0 bis 3,
- h: 3 bis 7 bedeuten,
einschließlich der physiologisch wirksamen Salze,
wobei 3-Benzyloxypyridin-2-carbonsäure-(L-threonylmethylester)amid, 3-Benzyloxypyridin-2-carbonsäure-(L-threonyl(Fmoc-Phg)tert. butylester)amid, 3-Benzyloxypyridin-2-carbonsäure-(L-threonyl-tert. butylester)amid und 3-Benzyloxypyridin-2-carbonsäure-(D-allothreonylmethylester)amid ausgenommen sind.

Unter Aryl werden im allgemeinen carbocyclische und heterocyclische aromatische Ringsysteme verstanden. Insbesondere versteht man hierunter Phenyl-, Biphenyl- oder Naphthyl- bzw. unsubstituierte 5- und 6-gliedrige heteroaromatische Ringe mit 1, 2 oder 3 Stickstoff- und/oder Sauerstoff- und/oder Schwefelatomen, wie Pyridyl-, Pyridazyl-, Pyrimidyl-, Pyrazyl-, Imidazolyl-, Triazolyl-, Thienyl-, Oxazolyl, und Thiazolyl-Derivate, und deren benzoannellierte Derivate.

Die Erfindung umfaßt weiterhin Salze der Verbindungen der allgemeinen Formel I.

Die Salzbildung mit basischen Reagenzien kann ein-, zwei- oder dreifach an den aciden Gruppen der Verbindungen der Formel I (d.h. Reste B, R¹, R², R³ und R⁴) erfolgen, insbesondere an dem Rest R².

Zur Anwendung kommende Reagenzien sind beispielsweise Alkoholate, Hydroxide, Carbonate, Hydrogencarbonate, Hydrogenphosphate, Metallorganyle der Alkali- und Erdalkalielemente, der Elemente der 3. und 4. Hauptgruppe des Periodensystems und der Elemente der Übergangsmetalle,

Amine, ggf. 1- bis 3-fach substituiert mit (C₁-C₈)-Hydroxyalkyl, (C₁-C₄)-Alkoxy-(C₁-C₈)-alkyl, Phenyl, Benzyl oder (C₁-C₈)-Alkyl, welches 1- bis 3-fach substituiert sein kann mit Hydroxy oder (C₁-C₄)-Alkoxy,
beispielsweise Tromethan (Tris-Puffer), 2-Aminoethanol, 3-Aminopropanol, Hydroxylamin, Dimethylhydroxylamin, 2-Methoxyethylamin, 3-Ethoxypropylamin, und
basische Aminosäuren und -derivate, wie Aminosäureester, Histidin, Arginin und Lysin und deren Derivate, sowie
Arzneimittel, die eine basische Gruppe enthalten, wie beispielsweise ®Amilorid, ®Verapamil und Betablocker.

Die Erfindung betrifft weiterhin die Verbindungen, gemäß Formel I, zuzüglich 3-Benzyloxypyridin-2-carbonsäure-(L-threonylmethylester)amid, 3-Benzyloxypyridin-2-carbonsäure-(L-threonyl(Fmoc-Phg)tert.butylester)amid, 3-Benzyloxypyridin-2-carbonsäure-(L-threonyl-tert. butylester)amid und 3-Benzyloxypyridin-2-carbonsäure-(D-allothreonylmethylester)amid zur Anwendung als Arzneimittel.

Von großem Interesse sind Verbindungen der Formel I,
in der
- Q: O, S, NR' oder eine Bindung bedeutet,
- X: O,
- Y: CR³ oder
falls R¹ und R² einen Cyclus bilden,
- Y: N oder R³,
- m: 0 oder 1 und
- G: den Rest eines Alkohols GOH
bedeuten.

Sehr wichtig sind Verbindungen der Formel I,
in der
- Q: O, NR' oder einer Bindung,
- X: O und
- G: den Rest eines Alkohols GOH
bedeuten.

Sehr wichtig sind ebenso Verbindungen der Formel I, in der Q = S, X = O und G den Rest eines Alkohols GOH bedeuten.

Von besonderer Bedeutung sind Verbindungen der Formel I, in der
- Q: O, NR' oder eine Bindung bedeutet,
- X: O,
- Y: CR³ oder, falls R¹ und R² einen Cyclus bilden, N oder CR³,
- m: 0 und 1,
- A: (C₁-C₃)-Alkylen, das gegebenenfalls einfach substituiert ist mit Halogen, Cyano, Trifluormethyl, (C₁-C₆)-Alkyl, (C₁-C₆)-Hydroxyalkyl, (C₁-C₆)-Alkoxy, -O-[CH₂]ₓ-C_{f}H_{(2f+1-g)}F_{g}, oder
- A: -CHR⁵- bedeutet, wobei R⁵ einen der Substituenten des α-C-Atoms einer α-Aminosäure bedeutet, insbesondere einer natürlichen L-Aminosäure und ihres D-Isomeren,
- B: -CO₂G bedeutet, wobei
- G: den Rest eines Alkohols GOH bedeutet, worin G einen verzweigten oder unverzweigten oder cyclischen aliphatischen (C₁-C₂₀)-Alkyl-Rest, oder einen verzweigten oder unverzweigten ggf. cyclischen (C₂-C₂₀)-Alkenyl-Rest, einen Retinyl-Rest, einen (C₂-C₂₀)-Alkinyl-Rest oder einen entsprechenden (C₄-C₂₀)-Alkeninyl-Rest bedeutet, wobei die Reste jeweils eine oder mehrere Mehrfachbindungen enthalten können, oder einen (C₆-C₁₆)-Aryl-Rest, einen (C₇-C₁₆)-Aralkyl-Rest oder einen 5- oder 6-gliedrigen, vorzugsweise Stickstoff-enthaltenden Heteroaryl- oder einen 5- oder 6-gliedrigen, vorzugsweise Stickstoff-enthaltenden Heteroaralkyl-Rest bedeutet, wobei die vorstehenden Reste insbesondere einen oder mehrere Substituenten aus der Reihe
Hydroxy, Halogen, Cyano, Trifluormethyl, Nitro, Carboxy, (C₁-C₁₂)-Alkyl, (C₃-C₈)-Cycloalkyl, (C₅-C₈)-Cycloalkenyl, (C₆-C₁₂)-Aryl, (C₇-C₁₆)-Aralkyl, (C₂-C₁₂)-Alkenyl, (C₂-C₁₂)-Alkinyl, (C₁-C₁₂)-Alkoxy, (C₁-C₁₂)-Alkoxy-(C₁-C₁₂)alkyl, (C₁-C₁₂)-Alkoxy-(C₁-C₁₂)alkoxy, (C₆-C₁₂)-Aryloxy, (C₇-C₁₆)-Aralkyloxy, (C₁₋C₈)-Hydroxyalkyl,-O-[CH₂₋]ₓC_{f}H_{(2f+1-g)}F_{g}, - OCF₂Cl, - OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoy, (C₂-C₁₂) Alkenylcarbonyl, (C₂-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₁₂)-Alkenyloxycarbonyl, (C₂-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₂-C₁₂)-Alkenyloxycarbonyloxy, (C₂-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)arylcarbamoyl, N-(C₁-C₁₀)-Alkyl-N-(C₇-C₁₆)-Aralkylcarbamoyl, N-((C₁-C₁₀)-Alkoxy-(C₁-C₁₀)alkyl)carbamoyl, N-((C₆-C₁₂)-Aryloxy-(C₁-C₁₀)alkyl)carbamoyl, N-((C₇-C₁₆)-Aralkyloxy-(C₁-C₁₀)alkyl)carbamoyl, N-(C₁-C₁₀)-Alkyl-N-((C₁-C₁₀)-Alkoxy-(C₁-C₁₀)alkyl)carbamoyl, N-(C₁-C₁₀)-Alkyl-N-((C₆-C₁₆)-Aryloxy-(C₁-C₁₀)alkyl)carbamoyl, N-(C₁-C₁₀)-Alkyl-N-((C₇-C₁₆)Aralkyloxy-(C₁-C₁₀)alkyl)carbamoyl,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₂)-Arylcarbamoyloxy, N-(C₇-C₁₆)-Aralkylcarbamoyloxy, N-(C₁-C₁₀)-Alkyl-N-(C₆-C₁₂)arylcarbamoyloxy, N-(C₁-C₁₀)-Alkyl-N-(C₇-C₁₆)-Aralkylcarbamoyloxy,
N-((C₁-C₁₀)alkyl))carbamoyloxy,
N-((C₆-C₁₂)-Aryloxy-(C₁-C₁₀)alkyl)carbamoyloxy,
N-((C₇-C₁₆)-Aralkyloxy-(C₁-C₁₀)alkyl)carbamoyloxy,
N-(C₁-C₁₀)-Alkyl-N-((C₁-C₁₀)-Alkoxy-(C₁-C₁₀)alkyl)carbamoyloxy,
N-(C₁-C₁₀)-Alkyl-N-((C₆-C₁₂)-Aryloxy-(C₁-C₁₀)alkyl)carbamoyloxy,
N-(C₁-C₁₀)-Alkyl-N-((C₇-C₁₆)-Aralkyloxy-(C₁-C₁₀)alkyl)carbamoyloxy,
Amino, (C₁-C₁₂)-Alkylamino, Di-(C₁-C₁₂)alkylamino, (C₃-C₈)-Cycloalkylamino, (C₂-C₁₂)-Alkenylamino, (C₂-C₁₂) Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)alkyl, (C₆-C₁₂)-Aroylamino-(C₁-C₈)-alkyl, (C₇-C₁₂)-Aralkanoylamino-(C₁-C₈)-alkyl, Amino-(C₁-C₁₀)-alkyl, N-(C₁-C₁₀)alkylamino-(C₁-C₁₀)alkyl, N,N-Di(C₁-C₁₀)alkylamino-(C₁-C₁₀)alkyl, (C₃-C₈)cycloalkylamino-(C₁-C₁₀)alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₂)-Arylsulfonyl, (C₇-C₁₆)-Aralkylmercapto, (C₇-C₁₆)-Aralkylsulfinyl, (C₇-C₁₆)-Aralkylsulfonyl,
Sulfamoyl, N-(C₁-C₁₀)-Alkylsulfamoyl, N,N-Di-(C₁-C₁₀)-alkylsulfamoyl, (C₃-C₈-cycloalkylsulfamoyl,
N-(C₆-C₁₂)-Arylsulfamoyl,
N-(C₇-C₁₆)-Aralkylsulfamoyl,
N-(C₁-C₁₀)-Alkyl-N-(C₆-C₁₂)-Arylsulfamoyl,
N-(C₁-C₁₀)-Alkyl-N-(C₇-C₁₆)-Aralkylsulfamoyl,
(C₁-C₁₀)-Alkyl-sulfonamido,
N-((C₁-C₁₀)alkyl)-(C₁-C₁₀)alkylsulfonamido, (C₇-C₁₆)-Aralkylsulfonamido,
N-((C₁-C₁₀)alkyl-(C₇-C₁₆)-Aralkylsulfonamido, bedeuten, wobei die Reste,
die einen Arylrest enthalten, ihrerseits am Aryl substituiert sein können durch 1 bis 5 gleiche oder verschiedene Reste aus der Reihe:
Hydroxy, Halogen, Cyano, Trifluormethyl, Nitro, Carboxy, (C₁-C₁₂)-Alkyl, (C₃-C₈)-Cycloalkyl, (C₆-C₁₂)-Aryl, (C₇-C₁₆)-Aralkyl, (C₁-C₁₂)-Alkoxy, (C₁-C₁₂)-Alkoxy-(C₁-C₁₂)alkyl, (C₁-C₁₂)-Alkoxy-(C₁-C₁₂)alkoxy, (C₆-C₁₂)-Aryloxy, (C₇-C₁₆)-Aralkyloxy, (C₁-C₈)-Hydroxyalkyl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₁₂)-Alkenyloxycarbonyl, (C₂-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₂-C₁₂)-Alkenyloxycarbonyloxy, (C₂-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-cycloalkylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₂)-arylcarbamoyl, N-(C₁-C₁₀)-Alkyl-N-(C₇-C₁₆)-aralkylcarbamoyl,
N-((C₁-C₁₀)-Alkoxy-(C₁-C₁₀)alkyl)carbamoyl,
N-((C₆-C₁₂)-Aryloxy-(C₁-C₁₀)alkyl)carbamoyl,
N-((C₇-C₁₆)-Aralkyloxy-(C₁-C₁₀)alkyl)carbamoyl,
N-(C₁-C₁₀)-Alkyl-N-((C₁-C₁₀)-Alkoxy-(C₁-C₁₀)alkyl)carbamoyl,
N-(C₁-C₁₀)-Alkyl-N-((C₆-C₁₂)-Aryloxy-(C₁-C₁₀)alkyl)carbamoyl,
N-(C₁-C₁₀)-Alkyl-N-((C₇-C₁₆)Aralkyloxy-(C₁-C₁₀)alkyl)carbamoyl,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₂)-Arylcarbamoyloxy, N-(C₇-C₁₆)-Aralkylcarbamoyloxy, N-(C₁-C₁₀)-Alkyl-N-(C₆-C₁₂)arylcarbamoyloxy, N-(C₁-C₁₀)-Alkyl-N-(C₇-C₁₆)-Aralkylcarbamoyloxy,
N-((C₁-C₁₀)alkyl))carbamoyloxy,
N-((C₆-C₁₂)-Aryloxy-(C₁-C₁₀)alkyl)carbamoyloxy,
N-((C₇-C₁₆)-Aralkyloxy-(C₁-C₁₀)alkyl)carbamoyloxy,
N-(C₁-C₁₀)-Alkyl-N-((C₁-C₁₀)-Alkoxy-(C₁-C₁₀)alkyl)carbamoyloxy,
N-(C₁-C₁₀)-Alkyl-N-((C₆-C₁₂)-Aryloxy-(C₁-C₁₀)alkyl)carbamoyloxy,
N-(C₁-C₁₀)-Alkyl-N-((C₇-C₁₆)-Aralkyloxy-(C₁-C₁₀)alkyl)carbamoyloxy,
Amino, (C₁-C₁₂)-Alkylamino, Di-(C₁-C₁₂)alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)alkyl, (C₆-C₁₂)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)alkylamino-(C₁-C₁₀)alkyl, N,N-Di(C₁-C₁₀)alkylamino-(C₁-C₁₀)alkyl, (C₃-C₈)Cycloalkylamino-(C₁-C₁₀)alkyl,
(C₁-C₁₂)-Alkylmercapto, (C₁-C₁₂)-Alkylsulfinyl, (C₁-C₁₂)-Alkylsulfonyl, (C₆-C₁₂)-Arylmercapto, (C₆-C₁₂)-Arylsulfinyl, (C₆-C₁₂)-Arylsulfonyl, (C₇-C₁₆)-Aralkylmercapto, (C₇-C₁₆)-Aralkylsulfinyl, (C₇-C₁₆)-Aralkylsulfonyl,
- R²: Wasserstoff, (C₁-C₂₀)-Alkyl, (C₂-C₂₀)-Alkenyl, (C₂-C₂₀)-Alkinyl, (C₂-C₂₀)-Alkenyloxy, (C₂-C₂₀)-Alkinyloxy, Retinyloxy, (C₁-C₂₀)-Alkoxy-(C₁-C₃)-alkyl, (C₂-C₂₀)-Alkenyloxy-(C₁-C₃)-alkyl, Retinyloxy-(C₁-C₃)-alkyl, (C₂-C₂₀)-Alkinyloxy-(C₁-C₃)-alkyl, (C₁-C₂₀)-Alkoxy, Halogen, Cyano, Trifluormethyl, (C₁-C₈)-Hydroxyalkyl, (C₁-C₁₀)-Alkanoyl,(C₇-C₁₂)-Aralkanoyl, (C₆-C₁₂)-Aroyl, -O-[CH₂]ₓC_{f}H_{(2f+1-g)}F_{g}, NR'R", (C₁-C₁₀)-Alkylmercapto, (C₁-C₁₀)-Alkylsulfinyl, (C₁-C₁₀)-Alkylsulfonyl, (C₆-C₁₂)-Arylmercapto, (C₆-C₁₂)-Arylsulfinyl), (C₆-C₁₂)-Arylsulfonyl, (C₇-C₁₂)-Aralkylmercapto, (C₇-C₁₂)-Aralkylsulfinyl, (C₇-C₁₂)-Aralkylsulfonyl, (C₆-C₁₂)-Aryloxy, (C₇-C₁₆)-Aralkyloxy, Carboxy, (C₁-C₂₀)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₂₀)-Alkenyloxycarbonyl, Retinyloxycarbonyl, (C₂-C₂₀)-Alkinyloxycarbonyl, (C₃-C₈)-Cycloalkyl-(C₁-C₆)-alkoxycarbonyl, (C₃-C₈)-Cycloalkoxy-(C₁-C₆)-alkoxycarbonyl, (C₆-C₁₂)-Aryloxy-(C₁-C₆)-alkoxycarbonyl, (C₇-C₁₆)-Aralkoxy-(C₁-C₆)-alkoxycarbonyl,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo(C₃-C₈)-alkylcarbamoyl, N-(C₁-C₁₀)-Alkyl-N-(C₃-C₈)-cycloalkylcarbamoyl, N-(C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₂)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl,N-(C₁-C₁₀)-Alkyl-N-((C₁-C₁₀)-alkoxy-(C₁-C₁₀)-alkyl)carbamoyl,
N-(C₁-C₁₀)-Alkyl-N-((C₆-C₁₂)-aryloxy-(C₁-C₁₀)-alkyl)carbamoyl,
N-(C₁-C₁₀)-Alkyl-N-((C₇-C₁₆)-aralkyloxy-(C₁-C₁₀)-alkyl)carbamoyl,
CON(CH₂)ₕ, worin eine CH₂-Gruppe durch O, S, N-(C₁-C₈)-alkylimino,
N-(C₃-C₈)-Cycloalkylimino, N-(C₃-C₈)-Cycloalkyl-(C₁-C₄)-alkylimino,
N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 7 bedeutet,
wobei Aryl in der Weise substiuiert ist wie für R¹ und R³ definiert,
- R¹ und R³: gleich oder verschieden sind und Wasserstoff, Halogen, (C₁-C₁₂)-Alkyl, (C₁-C₁₂)-Alkoxy, -O-[CH₂]ₓ-C_{f}H_{(2f + 1-g)}Hal_{g}, (C₁-C₁₂)-Alkoxy-(C₁-C₁₂)-alkyl, (C₁-C₈)-Alkoxy-(C₁-C₁₂)-alkoxy, (C₁-C₁₂)-Alkoxy-(C₁-C₈)-alkoxy-(C₂-C₆)-alkyl, (C₇-C₁₁)-Aralkyloxy, (C₃-C₈)-Cycloalkyl, (C₃-C₈)-Cycloalkyl-(C₁-C₈)-alkyl, (C₃-C₈)-Cycloalkyloxy, (C₃-C₈)-Cycloalkyl-(C₁-C₈)-alkoxy, (C₃-C₈)-Cycloalkyloxy-(C₁-C₈)-alkyl, (C₃-C₈)-Cycloalkyloxy-(C₁-C₈)-alkoxy, (C₃-C₈)-Cycloalkyl-(C₁-C₆)-alkyl-(C₁-C₆)-alkoxy, (C₃-C₈)-Cycloalkyl-(C₁-C₆)-alkoxy-(C₁-C₆)-alkyl, (C₃-C₈)-Cycloalkoxy-(C₁-C₆)-alkoxy-(C₁-C₆)-alkyl, NR^{Y}R^{Z}, (C₁-C₈)-Alkylmercapto, (C₁-C₈)-Alkylsulfinyl oder (C₁-C₈)-Alkylsulfonyl, (C₆-C₁₂)-Arylmercapto, (C₆-C₁₂)-Arylsulfinyl, (C₆-C₁₂)-Arylsulfonyl, (C₇-C₁₂)-Aralkylmercapto, (C₇-C₁₁)-Aralkylsulfinyl, (C₇-C₁₁)-Aralkylsulfonyl, substituiertes (C₆-C₁₂)-Aryloxy-(C₁-C₆)-alkyl, (C₇-C₁₁)-Aralkoxy-(C₁-C₆)-alkyl, (C₆-C₁₂)-Aryloxy-(C₁-C₆)-alkoxy-(C₁-C₆)-alkyl, (C₇-C₁₁)-Aralkyloxy-(C₁-C₆)-alkoxy-(C₁-C₆)-alkyl, (C₆-C₁₂)-Aryloxy, (C₇-C₁₁)-Aralkyloxy, (C₆-C₁₂)-Aryloxy-(C₁-C₆)-alkoxy oder (C₇-C₁₁)-Aralkoxy-(C₁-C₆)-alkoxy bedeutet, wobei ein aromatischer Rest mit 1, 2, 3, 4 oder 5 gleichen oder verschiedenen Substituenten aus der Reihe Wasserstoff, Halogen, Cyano, Nitro, Trifluormethyl, (C₁-C₁₆)-Alkyl, (C₁-C₁₆)-Alkenyl, (C₁-C₆)-Hydroxyalkyl, (C₁-C₁₆)-Alkoxy, (C₁-C₁₆)-Alkenyloxy, -O-[CH₂]ₓC_{f}H_{(2f+1-g)}F_{g}, -OCF₂Cl, -O-CF₂-CHFCl, (C₁-C₆)-Alkylmercapto, (C₁-C₆)-Alkylsulfinyl, (C₁-C₆)-Alkylsulfonyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkylcarbamoyl, Phenyl, Benzyl, Phenoxy, Benzyloxy, NR^{Y}R^{Z}, Phenylmercapto, Phenylsulfonyl, Phenylsulfinyl, Sulfamoyl, N-(C₁-C₄)-Alkylsulfamoyl oder N,N-Di-(C₁-C₄)-alkylsulfamoyl trägt, oder gegebenenfalls bis zu 3 der vorstehend genannten gleichen oder verschiedenen Substituenten trägt und zwei benachbarte C-Atome des Aralkyloxyrestes gemeinsam eine Kette -[CH₂-] und/oder -CH=CH-CH=CH- tragen, wobei eine CH₂-Gruppe der Kette gegebenenfalls durch O, S, SO, SO₂ oder NR' ersetzt ist,
- R¹ und R² oder R² und R³: eine Kette [CH₂]ₒ bilden, wobei o = 3, 4 oder 5 bedeutet, oder
zusammen mit dem sie tragenden Pyridin oder Pyridazin einen Cinnolin-, einen Chinolin- oder einen Isochinolin-Ring bilden,
- R⁴: , falls Q eine Bindung ist, Fluor, Chlor oder Brom bedeutet, oder falls Q O oder NR' ist, einen verzweigten oder unverzweigten (C₁-C₂₀)-Alkylrest, der bis zu 3 C-C-Mehrfachbindungen enthalten kann, einen unsubstituierten gesättigten Fluoralkylrest der Formel [CH₂]ₓC_{f}H_{(2f + 1-g)}F_{g}, einen (C₆-C₁₆)-Arylrest oder einen (C₇-C₁₆)-Aralkylrest, der in der Alkylkette bis zu 2 C-C-Mehrfachverbindungen enthalten kann, oder einen Heteroarylrest oder einen Heteroarylalkylrest bedeutet, wobei diese Reste substituiert sind mit einem oder mehreren Resten aus der Reihe Hydroxy, Fluor, Chlor, Cyano, Trifluormethyl, Carboxy, (C₁-C₁₂)-Alkyl, (C₃-C₈)-Cycloalkyl, (C₃-C₈)-Cycloalkyl-(C₁-C₁₂)-alkyl, (C₃-C₈)-Cycloalkoxy, (C₃-C₈)-Cycloalkyl-(C₁-C₁₂)-alkoxy, (C₃-C₈)-Cycloalkyloxy-(C₁-C₁₂)-alkyl, (C₃-C₈)-Cycloalkyloxy-(C₁-C₁₂)-alkoxy, (C₃-C₈)-Cycloalkyl-(C₁-C₈)-alkyl-(C₁-C₆)-alkoxy, (C₃-C₈)-Cycloalkoxy-(C₁-C₈)-alkoxy-(C₁-C₈)-alkoxy, (C₆-C₁₂)-Aryl, (C₇-C₁₆)-Aralkyl, (C₂-C₁₂)-Alkenyl, (C₂-C₁₂)-Alkinyl, (C₁-C₁₂)-Alkoxy, (C₁-C₁₂)-Alkoxy-(C₁-C₁₂)-alkoxy, (C₁-C₁₂)-Alkoxy-(C₁-C₈)-alkoxy-(C₁-C₈)-alkyl, (C₆-C₁₂)-Aryloxy, (C₇-C₁₆)-Aralkyloxy, (C₆-C₁₂)-Aryloxy-(C₁-C₆)-alkoxy, (C₇-C₁₆)-Aralkoxy-(C₁-C₆)-alkoxy, (C₁-C₈)-Hydroxyalkyl, -O-[CH₂₋]ₓC_{f}H_{(2f+1-g)}F_{g},
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₁₂)-Alkenyloxycarbonyl, (C₂-C₁₂)-Alkinyloxycarbonyl, (C₃-C₈)-Cycloalkyl-(C₁-C₆)-alkoxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-Cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₀)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl, CON(CH₂)ₕ, worin eine CH₂-Gruppe durch O, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino oder N-(C₇-C₁₆)-Aralkylimino ersetzt ein kann und h 3 bis 6 bedeutet,
wobei die Reste, die einen Arylrest enthalten ihrerseits am Aryl substituiert sein können durch 1 bis 5 gleiche oder verschiedene Reste aus der Reihe:
Hydroxy, Fluor, Chlor, Cyano, Trifluormethyl, Carboxy, (C₁-C₁₂)-Alkyl, (C₃-C₈)-Cycloalkyl, (C₁-C₆)-Alkoxy, (C₃-C₈)-Cycloalkoxy, (C₁-C₁₂)-Alkoxycarbonyl,
N-(C₁-C₆)-Alkylcarbamoyl, N, N-Di-(C₁-C₆)-alkylcarbamoyl, N-(C₃-C₈)-Cycloalkylcarbamoyl, und
- R⁴: R'' bedeutet, sofern Q in der Bedeutung von NR' steht, wobei
R' und R'' gleich oder verschieden sind und Wasserstoff, (C₁-C₈)-Alkyl oder gegebenenfalls mit Fluor, Chlor, (C₁-C₄)-Alkoxy einfach substituiertes (C₇-C₁₁)-Aralkyl bedeuten,
- R^{Y} und R^{Z}: gleich oder verschieden sind und Wasserstoff, (C₆-C₁₂)-Aryl, (C₁-C₁₀)-Alkyl, (C₃-C₁₀)-Cycloalkyl, (C₁-C₈)-Alkoxy-(C₁-C₈)-alkyl, (C₇-C₁₂)-Aralkoxy-(C₁-C₈)-alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, oder
- R^{Y} und R^{Z}: gemeinsam für -[CH₂]ₕ₋ stehen, worin eine CH₂-Gruppe durch O, S, N-(C₁-C₄)-Alkanoylimino oder N-(C₁-C₄)-Alkoxycarbonylimino ersetzt sein kann, und
- f: 1 bis 8,
- g: 0, 1 bis (2f + 1),
- h: 3 bis 6,
- x: 0 bis 3, und
- n: 3 oder 4 ist.

Bevorzugt sind Verbindungen der Formel I, in der
- Q: O, NR' oder eine Bindung bedeutet,
- X: O,
- Y: CR³ oder, falls R¹ und R² einen Cyclus bilden, N oder CR³ bedeuten,
- m: 0
- A: (C₁-C₃)-Alkylen, das gegebenenfalls einfach substituiert ist mit Halogen, Cyano, Trifluormethyl, (C₁-C₆)-Alkyl, (C₁-C₆)-Hydroxyalkyl, (C₁-C₆)-Alkoxy, -O-[CH₂]ₓ-C_{f}H_{(2f+1-g)}F_{g} oder
- A: -CHR⁵- bedeutet, wobei R⁵ einen der Substituenten des α-C-Atoms einer α-Aminosäure bedeutet, insbesondere einer natürlichen L-Aminosäure und ihres D-Isomeren,
- B: CO₂G bedeutet, wobei
- G: einen verzweigten oder unverzweigten oder cyclischen aliphatischen (C₁-C₂₀)-Alkyl-Rest, einen Retinyl-Rest oder
einen verzweigten oder unverzweigten (C₂-C₂₀)-Alkenyl-Rest oder einen (C₂-C₂₀)-Alkinyl-Rest, der jeweils eine oder mehrere C-C-Mehrfachbindungen enthalten kann, oder
einen (C₆-C₁₂)-Aryl-Rest, einen (C₇-C₁₁)-Aralkyl-Rest oder einen Heteroaryl- oder Heteroaralkyl-Rest bedeutet,
wobei die vorstehenden Reste einen oder zwei Substituenten aus der Reihe (C₁-C₈)-Alkyl, (C₃-C₈)-Cycloalkyl, Fluor, Chlor, Hydroxy, (C₁-C₆)-Alkoxy, (C₁-C₆)-Alkoxy-(C₁-C₆)alkoxy, (C₆-C₁₂)-Aryloxy, (C₇-C₁₂)-Aralkyloxy,
(C₁-C₈)-Alkylcarbonyl, (C₃-C₈)-Cycloalkylcarbonyl, (C₆-C₁₂)-Arylcarbonyl, (C₇-C₁₂)-Aralkylcarbonyl,
(C₁-C₈)-Alkoxycarbonyl, (C₁-C₆)-Alkoxy-(C₁-C₆)-alkoxycarbonyl, (C₆-C₁₂)-Aryloxycarbonyl, (C₇-C₁₂)-Aralkoxycarbonyl, (C₃-C₈)-Cycloalkoxycarbonyl,
(C₁-C₂₀)-Alkylcarbonyloxy, (C₃-C₈)-Cycloalkylcarbonyloxy, (C₆-C₁₂)-Arylcarbonyloxy, (C₇-C₁₂)-Aralkylcarbonyloxy,
(C₁-C₈)-Alkoxycarbonyloxy, (C₁-C₆)-Alkoxy-(C₁-C₆)-alkoxycarbonyloxy, (C₆-C₁₂)-Aryloxycarbonyloxy, (C₇-C₁₂)-Aralkyloxycarbonyloxy, (C₃-C₈)-Cycloalkoxycarbonyloxy,
Carbamoyl, N-(C₁-C₈)-Alkylcarbamoyl, N,N-Di-(C₁-C₈)-alkylcarbamoyl, N-(C₃-C₈)-Cycloalkylcarbamoyl,
N-((C₁-C₆)-Alkoxy-(C₁-C₆)alkyl)carbamoyl,
Amino, (C₁-C₆)-Alkylamino, Di-(C₁-C₆)alkylamino, (C₃-C₈)-Cycloalkylamino,N-(C₆-C₁₂)-Arylamino, N-(C₇-C₁₁)-Aralkylamino, N-(C₁-C₅)-Alkyl-(C₆-C₁₂)-Arylamino,
(C₁-C₈)-Alkanoylamino, (C₃-C₈)-Cycloalkanoylamino, (C₆-C₁₂)-Aroylamino, (C₇-C₁₂)-Aralkanoylamino, (C₁-C₈)-Alkanoyl-N-(C₁-C₆)-alkylamino, (C₃-C₈)-Cycloalkanoyl-N-(C₁-C₆)-alkylamino, (C₆-C₁₂)-Aroyl-N-(C₁-C₆)alkylamino, (C₇-C₁₁)-Aralkanoyl-N-(C₁-C₆)-alkylamino tragen können, und
wobei die Reste, die einen Arylrest enthalten, insbesondere substituiert sind mit bis zu 3 Substituenten aus der Reihe
Hydroxy, Fluor, Chlor, Cyano, Trifluormethyl, (C₁-C₆)-Alkyl, (C₃-C₈)-Cycloalkyl, (C₁-C₈)-Alkoxy,
(C₁-C₆)-Alkylcarbonyl, (C₃-C₈)-Cycloalkylcarbonyl,
(C₁-C₆)-Alkoxycarbonyl, (C₃-C₈)-Cycloalkoxycarbonyl,
(C₁-C₆)-Alkylcarbonyloxy, (C₃-C₈)-Cycloalkylcarbonyloxy,
(C₁-C₆)-Alkoxycarbonyloxy, (C₃-C₈)-Cycloalkoxycarbamoyloxy,
Carbamoyl, N-(C₁-C₆)-Alkylcarbamoyl, N,N-Di-(C₁-C₆)-alkylcarbamoyl, N-(C₃-C₈)-Cycloalkylcarbamoyl,
N-((C₁-C₆)-Alkoxy-(C₁-C₆)alkyl)carbamoyl,
N-(C₁-C₆)-Alkyl-N-((C₁-C₆)-Alkoxy-(C₁-C₆)alkyl)carbamoyl,
Carbamoyloxy, N-(C₁-C₆)-Alkylcarbamoyloxy, N,N-Di-(C₁-C₆)-alkylcarbamoyloxy, N-(C₃-C₈)-Cycloalkylcarbamoyloxy,
(C₁-C₆)-Alkanoylamino, (C₃-C₈)-Cycloalkanoylamino,
(C₁-C₆)Alkylmercapto, (C₁-C₆)-Alkylsulfinyl, (C₁-C₆)-Alkylsulfonyl, und
- R²: Wasserstoff, (C₁-C₂₀)-Alkyl, (C₂-C₂₀)-Alkenyl, (C₂-C₂₀)-Alkenyloxy, (C₂-C₂₀)-Alkinyloxy, Retinyloxy, (C₁-C₂₀)-Alkoxy-(C₁-C₃)-alkyl, (C₁-C₂₀)-Alkoxy-(C₁-C₃)-alkyl, (C₂-C₂₀)-Alkenyloxy-(C₁-C₃)-alkyl, Retinyloxy-(C₁-C₃)-alkyl, (C₂-C₂₀)-Alkinyloxy-(C₁-C₃)-alkyl, (C₁-C₂₀)-Alkoxy, Halogen, Cyano, Trifluormethyl, (C₁-C₈)-Hydroxyalkyl, (C₁-C₁₀)-Alkanoyl,(C₇-C₁₂)-Aralkanoyl, (C₆-C₁₂)-Aroyl, -O-[CH₂]ₓC_{f}H_{(2f+1-g)}F_{g}, NR'R'', (C₁-C₁₀)-Alkylmercapto, (C₁-C₁₀)-Alkylsulfinyl, (C₁-C₁₀)-Alkylsulfonyl, (C₆-C₁₂)-Arylmercapto, (C₆-C₁₂)-Arylsulfinyl), (C₆-C₁₂)-Arylsulfonyl, (C₇-C₁₂)-Aralkylmercapto, (C₇-C₁₂)-Aralkylsulfinyl, (C₇-C₁₂)-Aralkylsulfonyl, (C₆-C₁₂)-Aryloxy, (C₇-C₁₆)-Aralkyloxy, Carboxy, (C₁-C₂₀)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl, (C₂-C₂₀)-Alkenyloxycarbonyl, Retinyloxycarbonyl, (C₂-C₂₀)-Alkinyloxycarbonyl, (C₃-C₈)-Cycloalkyl-(C₁-C₆)-alkoxycarbonyl, (C₃-C₈)-Cycloalkoxy-(C₁-C₆)-alkoxycarbonyl, (C₆-C₁₂)-Aryloxy-(C₁-C₆)-alkoxycarbonyl, (C₇-C₁₆)-Aralkoxy-(C₁-C₆)-alkoxycarbonyl, Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo(C₃-C₈)-alkylcarbamoyl, N-(C₁-C₁₀)-Alkyl-N-(C₃-C₈)-cycloalkylcarbamoyl, N-(C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₂)-aralkylcarbamoyl, N-((C₁-C₁₂)-Alkoxy-(C₁-C₁₀)-alkyl)carbamoyl, N-((C₆-C₁₆)-Aryloxy-(C₁-C₁₀)-alkyl)carbamoyl,
N-((C₇-C₁₆)-Aralkyloxy-(C₁-C₁₀)-alkyl)carbamoyl,N-(C₁-C₁₀)-Alkyl-N-((C₁-C₁₀)-alkoxy-(C₁-C₁₀)-alkyl)carbamoyl,
N-(C₁-C₁₀)-Alkyl-N-((C₆-C₁₂)-aryloxy-(C₁-C₁₀)-alkyl)carbamoyl,
N-(C₁-C₁₀)-Alkyl-N-((C₇-C₁₆)-aralkyloxy-(C₁-C₁₀)-alkyl)carbamoyl,
CON(CH₂)ₕ, worin eine CH₂-Gruppe durch O, S, N-(C₁-C₈)-alkylimino,
N-(C₃-C₈)-Cycloalkylimino, N-(C₃-C₈)-Cycloalkyl-(C₁-C₄)-alkylimino,
N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 6 bedeutet,
wobei Aryl in der Weise substiuiert ist wie für R¹ und R³ definiert,
- R¹ und R³: gleich oder verschieden sind und Wasserstoff, Halogen, (C₁-C₁₂)-Alkyl, (C₁-C₁₂)-Alkoxy, -O-[CH₂]ₓ-C_{f}H_{(2f+1-g)}Hal_{g}, (C₁-C₁₂)-Alkoxy-(C₁-C₁₂)-alkyl, (C₁-C₈)-Alkoxy-(C₁-C₁₂)-alkoxy, (C₁-C₁₂)-Alkoxy-(C₁-C₈)-alkoxy-(C₂-C₆)-alkyl, (C₇-C₁₁)-Aralkyloxy, (C₃-C₈)-Cycloalkyl, (C₃-C₈)-Cycloalkyl-(C₁-C₈)-alkyl, (C₃-C₈)-Cycloalkyloxy, (C₃-C₈)-Cycloalkyl-(C₁-C₈)-alkoxy, (C₃-C₈)-Cycloalkyloxy-(C₁-C₈)-alkyl, (C₃-C₈)-Cycloalkyloxy-(C₁-C₈)-alkoxy, (C₃-C₈)-Cycloalkyl-(C₁-C₆)-alkyl-(C₁-C₆)-alkoxy, (C₃-C₈)-Cycloalkyl-(C₁-C₆)-alkoxy-(C₁-C₆)-alkyl, (C₃-C₈)-Cycloalkoxy-(C₁-C₆)-alkoxy-(C₁-C₆)-alkyl, NR^{Y}R^{Z}, (C₁-C₈)-Alkylmercapto, (C₁-C₈)-Alkylsulfinyl oder (C₁-C₈)-Alkylsulfonyl, (C₆-C₁₂)-Arylmercapto, (C₆-C₁₂)-Arylsulfinyl, (C₆-C₁₂)-Arylsulfonyl, (C₇-C₁₂)-Aralkylmercapto, (C₇-C₁₁)-Aralkylsulfinyl, (C₇-C₁₁)-Aralkylsulfonyl, substituiertes (C₆-C₁₂)-Aryloxy-(C₁-C₆)-alkyl, (C₇-C₁₁)-Aralkoxy-(C₁-C₆)-alkyl, (C₆-C₁₂)-Aryloxy-(C₁-C₆)-alkoxy-(C₁-C₆)-alkyl, (C₇-C₁₁)-Aralkyloxy-(C₁-C₆)-alkoxy-(C₁-C₆)-alkyl, (C₆-C₁₂)-Aryloxy, (C₇-C₁₁)-Aralkyloxy, (C₆-C₁₂)-Aryloxy-(C₁-C₆)-alkoxy oder (C₇-C₁₁)-Aralkoxy-(C₁-C₆)-alkoxy bedeutet, wobei ein aromatischer Rest mit 1, 2, 3, 4 oder 5 gleichen oder verschiedenen Substituenten aus der Reihe Wasserstoff, Halogen, Cyano, Nitro, Trifluormethyl, (C₁-C₁₂)-Alkyl, (C₁-C₁₂)-Alkenyl, (C₁-C₆)-Hydroxyalkyl, (C₁-C₁₂)-Alkoxy, (C₁-C₁₂)-Alkenyloxy, -O-[CH₂]ₓC_{f}H_{(2f+1-g)}F_{g}, - OCF₂Cl, -O-CF₂-CHFCl, (C₁-C₆)-Alkylmercapto, (C₁-C₆)-Alkylsulfinyl, (C₁-C₆)-Alkylsulfonyl, (C₁-C₆)-Alkylcarbonyl,(C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkylcarbamoyl, Phenyl, Benzyl, Phenoxy, Benzyloxy, NR^{Y}R^{Z}, Phenylmercapto, Phenylsulfonyl, Phenylsulfinyl, Sulfamoyl, N-(C₁-C₄)-Alkylsulfamoyl oder N,N-Di-(C₁-C₄)-alkylsulfamoyl trägt, oder gegebenenfalls bis zu 3 der vorstehend genannten gleichen oder verschiedenen Substituenten trägt und zwei benachbarte C-Atome des Aralkyloxyrestes gemeinsam eine Kette -[CH₂-] und/oder -CH=CH-CH=CH- tragen, wobei eine CH₂-Gruppe der Kette gegebenenfalls durch O, S, SO, SO₂ oder NR^{Y} ersetzt ist,
- R¹ und R² oder R² und R³: eine Kette [CH₂]ₒ bilden können, wobei o = 3, 4 oder 5 bedeutet, und
- R⁴: sofern Q eine Bindung ist, Chlor bedeutet oder
falls Q O oder NR' ist, einen verzweigten oder unverzweigten (C₁-C₁₀)-Alkylrest, der eine oder zwei C-C-Mehrfachbindungen enthalten kann, oder einen unsubstituierten Fluoralkylrest der Formel -[CH₂]ₓ C_{f}H_{(2f+1-g)}F_{g} oder (C₁-C₈)-Alkoxy-(C₁-C₆)-alkyl, (C₁-C₆)-Alkoxy-(C₁-C₄)-alkoxy-(C₁-C₄)-alkyl oder einen Rest der Formel Z bedeutet,

-[CH₂]ᵥ-[O]_{w}-[CH₂]ₜ-E (Z),

wobei E einen substituierten Phenylrest der Formel F 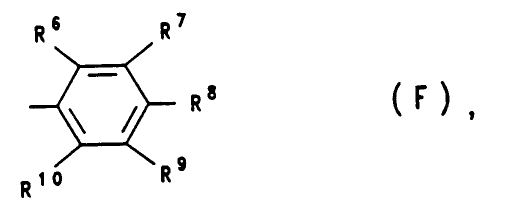 oder einen (C₃-C₈)-Cycloalkylrest bedeutet, wobei
v = 0, 1, 2, 3, 4, 5, 6, w = 0,1 und t = 0, 1, 2, 3 bedeutet mit der Einschränkung, daß v ungleich 0 ist, falls w = 1 ist, und
R⁶, R⁷, R⁸, R⁹ und R¹⁰ gleich oder verschieden sind und Wasserstoff, Halogen, Cyano, Nitro, Trifluormethyl, (C₁-C₆)-Alkyl, (C₃-C₈-Cycloalkyl, (C₁-C₆)-Alkoxy, -O-[CH₂₋]ₓC_{f}H_{(2f+1-g)}F_{g}, -OCF₂Cl, -O-CF₂-CHFCl, (C₁-C₆)-Alkylmercapto, (C₁-C₆)-Hydroxyalkyl, (C₁-C₆)-Alkoxy-(C₁-C₆)-alkoxy, (C₁-C₆)-Alkoxy-(C₁-C₆)-alkyl, (C₁-C₆)-Alkylsulfinyl, (C₁-C₆)-Alkylsulfonyl, (C₁-C₆)-Alkylcarbonyl, (C₁-C₈)-Alkoxycarbonyl, Carbamoyl, N-(C₁-C₈)-Alkylcarbamoyl, N,N-Di-(C₁-C₈)-alkylcarbamoyl, gegebenenfalls mit Fluor, Chlor, Brom, Trifluormethyl und (C₁-C₆)-Alkoxy-substituiertes (C₇-C₁₁)-Aralkylcarbamoyl, N-(C₃-C₈)-Cycloalkylcarbamoyl, N-(C₃-C₈)-Cycloalkyl-(C₁-C₄)-alkylcarbamoyl, (C₁-C₆)-Alkylcarbonyloxy, Phenyl, Benzyl, Phenoxy, Benzyloxy, NR^{Y}R^{Z}, wie Amino, Anilino, N-Methylanilino, Phenylmercapto, Phenylsulfonyl, Phenylsulfinyl, Sulfamoyl, N-(C₁-C₈)-Alkylsulfamoyl oder N,N-Di-(C₁-C₈)-alkylsulfamoyl bedeuten, oder zwei benachbarte Substituenten gemeinsam eine Kette -[CH₂₋]ₙ oder -CH=CH-CH=CH- bedeuten, wobei eine CH₂-Gruppe der Kette gegebenenfalls durch O, S, SO, SO₂ oder NR^{Y} ersetzt ist und wobei ein Heteroarylrest 1, 2 oder 3 Substituenten und ein Cycloalkylrest einen Substituenten aus der vorstehenden Reihe tragen kann, und
- R⁴: R'' bedeutet, sofern Q in der Bedeutung von NR' steht, wobei
- R': Wasserstoff und Methyl und
- R'': Benzyl bedeuten, und
falls R¹ und/oder R³ in der Bedeutung von (C₆-C₁₂)-Aryloxy, (C₇-C₁₁)-Aralkyloxy, (C₆-C₁₂)-Aryloxy-(C₁-C₆)-alkoxy, (C₇-C₁₁)-Aralkoxy -(C₁-C₆)-alkoxy oder einem entsprechenden endständige Cycloalkyl-Gruppen enthaltenden Rest stehen, so bedeutet dieser Rest vorzugsweise einen Rest der Formel D

OZ (D), oder

falls R¹ und/oder R³ in der Bedeutung von (C₇-C₁₁)-Aralkyl, (C₆-C₁₂)-Aryloxy-(C₁-C₆)-alkyl, (C₇-C₁₁)-Aralkoxy-(C₁-C₆)-alkyl oder einem entsprechenden endständige Cycloalkyl-Gruppen enthaltenden Rest stehen, so bedeutet dieser Rest vorzugsweise einen Rest der Formel Z,
- R^{Y} und R^{Z}: sind gleich oder verschieden sind und Wasserstoff, (C₆-C₁₂)-Aryl, (C₁-C₁₀)-Alkyl, (C₃-C₁₀)-Cycloalkyl, (C₁-C₈)-Alkoxy-(C₁-C₈)-alkyl, (C₇-C₁₂)-Aralkoxy-(C₁-C₈)-alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, oder
- R^{Y} und R^{Z}: stehen gemeinsam für -[CH₂]ₕ₋, worin eine CH₂-Gruppe durch O, S, N-(C₁-C₄)-Alkanoylimino oder N-(C₁-C₄)-Alkoxycarbonylimino ersetzt sein kann, und
- f: 1 bis 8,
- g: 0, 1 bis (2f + 1),
- h: 3 bis 6,
- x: 0 bis 3, und
- n: 3 oder 4 ist.

Besonders bevorzugt sind Verbindungen der Formel I, in der
- Q: O,
- X: O,
- Y: CR³ und zusätzlich N, falls R¹ und R² einen Cyclus bilden,
- m: 0,
- A: -CHR⁵-, wobei R⁵ den Substituenten des α-C-Atoms einer α-Aminosäure, insbesondere einer natürlichen L-Aminosäure oder ihr
D-Isomeres bedeutet,
- B: CO₂G bedeutet, wobei
- G: für einen verzweigten oder unverzweigten oder cyclischen aliphatischen (C₁-C₁₈)-Alkyl-Rest, einen (C₃-C₈)-Cycloalkyl-(C₁-C₈)-alkylrest,
einen verzweigten oder unverzweigten (C₂-C₁₈)-Alkenylrest, wie z.B. einen Geranyl- oder Farnesyl-Rest oder einen Retinyl-Rest, oder (C₂-C₁₈)-Alkinylrest, einen Phenyl-, Benzyl-, Phenethyl-, Phenylpropyl- oder Phenylbutylrest steht,
wobei die vorstehenden Reste einen Substituenten aus der Reihe Hydroxy, (C₁-C₄)-Alkoxy, Acyloxy, (C₁-C₆)-Alkylcarbonyloxy, (C₃-C₈)-Cycloalkylcarbonyloxy, Benzoyloxy, (C₇-C₁₆)-Phenylalkylcarbonyloxy, (C₃-C₈)-Cycloalkoxycarbonyloxy enthalten,
- R²: Wasserstoff, Brom, Chlor, Cyano, (C₁-C₁₈)-Alkyl, (C₁-C₈)-Alkoxy, (C₁-C₁₈)-Alkoxymethyl, (C₂-C₁₈)-Alkenyloxymethyl, (C₂-C₁₈)-Alkinyloxymethyl,Carbamoyl, N-(C₁-C₁₀)-Alkylcarbamoyl, N-((C₁-C₁₂)-Alkoxy-(C₁-C₄)-alkyl)carbamoyl, N, N-Di-(C₁-C₈)-alkylcarbamoyl, N-(C₃-C₈)-Cycloalkylcarbamoyl, N-(C₆-C₁₂)-Phenylcarbamoyl, N-(C₇-C₁₂)-Phenylalkylcarbamoyl, N-(C₁-C₆)-Alkyl-N-(C₆-C₁₂)phenylcarbamoyl, N-(C₁-C₆)-Alkyl-N-(C₇-C₁₂)-phenylalkylcarbamoyl, N-((C₁-C₆)-Alkoxy-(C₁-C₆)-alkyl)carbamoyl, Carboxy, (C₁-C₂₀)-Alkoxycarbonyl, (C₂-C₂₀)-Alkenyloxycarbonyl, Retinyloxycarbonyl, (C₃-C₈)-Cycloalkoxycarbonyl, (C₃-C₈)-Cycloalkyl-(C₁-C₆)-alkoxycarbonyl, (C₃-C₈)-Cycloalkoxy-(C₁-C₆)-alkoxycarbonyl, Phenyl-(C₁-C₆)-alkoxycarbonyl, Phenoxy-(C₁-C₆)-alkoxycarbonyl, Benzyloxy-(C₁-C₆)-alkoxycarbonyl, wobei ein Phenylrest in der Weise substituiert ist wie für R¹ und R³ definiert, und einer der Reste
- R¹ oder R³: Wasserstoff und der andere einen Rest aus der Reihe Wasserstoff, Fluor, Chlor, (C₁-C₈)-Alkyl, (C₁-C₁₀)-Alkoxy, (C₅-C₆)-Cycloalkyl, (C₅-C₆)-Cycloalkyl-(C₁-C₆)-alkyl, (C₅-C₆)-Cycloalkyloxy, (C₅-C₆)-Cycloalkyl-(C₁-C₆)-alkoxy, (C₅-C₆)-Cycloalkyloxy-(C₁-C₆)-alkyl, (C₅-C₆)-Cycloalkyloxy-(C₁-C₆)-alkoxy, (C₅-C₆)-Cycloalkyl-(C₁-C₄)-alkyl-(C₁-C₄)-alkoxy, (C₅-C₆)-Cycloalkyl-(C₁-C₄)-alkoxy-(C₁-C₂)-alkyl, (C₅-C₆)-Cycloalkoxy-(C₁-C₄)-alkoxy-(C₁-C₂)-alkyl, -O-[CH₂]ₓ-C_{f}H_{(2f+1-g)}F_{g}, (C₁-C₆)-Alkoxy-(C₁-C₆)-alkyl, (C₁-C₆)-Alkoxy-(C₁-C₆)-alkoxy, (C₁-C₆)-Alkoxy-(C₁-C₄)-alkoxy-(C₁-C₂)-alkyl, substituiertes (C₆-C₁₂)-Phenoxy, (C₇-C₁₁)-Phenylalkyloxy, (C₆-C₁₂)-Phenoxy-(C₁-C₆)-alkoxy oder (C₇-C₁₁)-Phenylalkoxy-(C₁-C₆)-alkoxy, Phenoxy-(C₁-C₄)-alkyl, (C₇-C₁₁)-Phenylalkyloxy-(C₁-C₄)-alkyl, Phenoxy-(C₁-C₄)-alkoxy-(C₁-C₂)-alkyl, (C₇-C₁₁)-Phenylalkyloxy-(C₁-C₄)-alkoxy-(C₁-C₂)-alkyl bedeutet, wobei ein aromatischer Rest mit 1, 2 oder 3 gleichen oder verschiedenen Substituenten aus der Reihe Fluor, Chlor, Cyano, Trifluormethyl, (C₁-C₁₂)-Alkyl, (C₂-C₁₂)-Alkenyl, (C₂-C₁₂)-Alkenyloxy, (C₁-C₁₂)-Alkoxy, substituiert ist,
- R¹ und R²: mit dem sie tragenden Pyridin einen 5,6,7,8-Tetrahydroisochinolin-Ring bilden,
- R⁴: einen verzweigten oder unverzweigten (C₁-C₁₀)-Alkylrest, (C₁-C₄)-Alkoxy-(C₁-C₄)-alkyl oder einen Rest der Formel Z bedeutet,

-[CH₂]ᵥ-[O]_{w}-[CH₂]ₜ-E (Z),

wobei E einen substituierten Phenylrest der Formel F 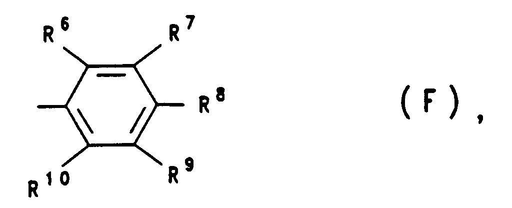 oder einen (C₃-C₈)-Cycloalkylrest bedeutet, wobei
v = 0, 1, 2, 3, w = 0, und t = 0, 1 sein kann und
worin R⁶, R⁷, R⁸, R⁹ und R¹⁰ gleich oder verschieden sind und Wasserstoff, Fluor, Chlor, Cyano, Trifluormethyl, (C₁-C₆)-Alkyl, (C₁-C₆)-Alkoxy, -O-[CH₂₋]ₓC_{f}H_{(2f+1-g)}F_{g}, N-(C₁-C₈)-Alkylcarbamoyl, N,N-Di-(C₁-C₆)-alkylcarbamoyl, N-(C₃-C₆)-Cycloalkylcarbamoyl, N-(+)-Dehydroabietylaminocarbonyl, gegebenenfalls mit Fluor, Chlor, Trifluormethyl und (C₁-C₆)-Alkoxy substituiertes (C₇-C₁₁)-Phenylalkylcarbamoyl bedeuten, oder wobei
R⁶ und R⁷ oder R⁷ und R⁸ zusammen mit dem sie tragenden Phenylring Naphthalin-Derivate bilden.

Falls R¹ oder R³ in der Bedeutung von (C₆-C₁₂)-Phenoxy, (C₇-C₁₁)-Phenylalkyloxy, (C₆-C₁₂)-Phenoxy-(C₁-C₆)-alkoxy, (C₇-C₁₁)-Phenylalkoxy-(C₁-C₆)-alkoxy, (C₅-C₆)-Cycloalkyloxy, (C₅-C₆)-Cycloalkyl-(C₁-C₆)-alkoxy, (C₅-C₆)-Cycloalkoxy-(C₁-C₆)-alkoxy oder (C₅-C₆)-Cycloalkyl-(C₁-C₄)-alkyl-(C₁-C₄)-alkoxy steht, so bedeutet dieser Rest im speziellen einen Rest der Formel D

OZ (D), oder

falls R¹ oder R³ in der Bedeutung von Phenyl, Phenoxy-(C₁-C₆)-alkyl, (C₇-C₁₁)-Phenylalkyl, (C₇-C₁₁)-Phenylalkyloxy-(C₁-C₄)-alkyl, (C₅-C₆)-Cycloalkyl, (C₅-C₆)-Cycloalkyl-(C₁-C₆)-alkyl, (C₅-C₆)-Cycloalkoxy-(C₁-C₄)-alkyl, (C₅-C₆)-Cycloalkyl-(C₁-C₄)-alkoxy-(C₁-C₂)-alkyl, (C₅-C₆)-Cycloalkoxy-(C₁-C₄)-alkoxy-(C₁-C₂)-alkyl, steht, so bedeutet dieser Rest im speziellen einen Rest der Formel Z
worin in beiden Fällen
v = 1, 2, 3 und 4, w = 0 und t = 0 oder
v = 1, 2, 3 und 4, w = 1 und t = 0 oder
v = 1, 2, 3 und 4, w = 1, t = 1 und
f = 1 bis 4
g = 0,1 bis (2f + 1)
x = o und 1 bedeutet.

Ganz besonders bevorzugt sind Verbindungen der Formel I, in der
- Q: O,
- X: O,
- Y: CR³,
- m: 0,
- A: eine -CH₂-Gruppe bedeutet, die mit einer Methylgruppe substituiert sein kann,
- B: -CO₂G, wobei
- G: für einen verzweigten oder unverzweigten oder cyclischen aliphatischen (C₁-C₁₈)-Alkyl-Rest, einen (C₃-C₈)-Cycloalkyl-(C₁-C₄)-alkylrest, einen verzweigten oder unverzweigten (C₂-C₁₈)-Alkenylrest, steht, wobei die vorstehenden Reste einen Substituenten aus der Reihe Hydroxy, (C₁-C₄)-Alkoxy, Acyloxy, (C₁-C₆)-Alkylcarbonyloxy, (C₃-C₈)-Cycloalkylcarbonyloxy, Benzoyloxy, (C₇-C₁₆)-Phenylalkylcarbonyloxy, (C₃-C₈)-Cycloalkoxycarbonyloxy enthalten können, oder
- G: für einen Phenyl-, Benzyl-, Phenethyl-, Phenylpropyl- oder Phenylbutylrest steht,
- R²: Wasserstoff, (C₁-C₈)-Alkoxy, (C₁-C₁₆)-Alkoxymethyl, (C₂-C₁₆)-Alkenyloxymethyl, Retinyloxymethyl, N-(C₁-C₁₀)-Alkylcarbamoyl, N-((C₁-C₁₂)-Alkoxy-(C₁-C₃)-alkyl)carbamoyl, N, N-Di-(C₁-C₈)-alkylcarbamoyl, N-(C₅-C₆)-Cycloalkylcarbamoyl, N-Phenylcarbamoyl, N-Phenyl-(C₁-C₄)-alkylcarbamoyl, Carboxy, (C₁-C₁₆)-Alkoxycarbonyl, (C₂-C₁₆)-Alkenyloxycarbonyl, Retinyloxycarbonyl, (C₅-C₆)-Cycloalkoxycarbonyl, (C₅-C₆)-Cycloalkyl-(C₁-C₆)-alkoxycarbonyl, Phenyl-(C₁-C₆)-alkoxycarbonyl, wobei ein Phenylrest in der Weise substituiert ist wie für R¹ und R³ definiert, und einer der Reste
- R¹ oder R³: Wasserstoff und der andere Rest einen Rest aus der Reihe Wasserstoff, (C₁-C₁₀)-Alkoxy, (C₅-C₆)-Cycloalkyloxy, (C₅-C₆)-Cycloalkyl-(C₁-C₂)-alkoxy, -O-[CH₂]ₓ-C_{f}H_{(2f+1-g)}F_{g}, (C₁-C₄)-Alkoxy-(C₁-C₄)-alkoxy, substituiertes (C₆-C₁₂)-Phenoxy, (C₇-C₁₁)-Phenylalkyloxy, (C₆-C₁₂)-Phenoxy-(C₁-C₄)-alkoxy oder (C₇-C₁₁)-Phenylalkoxy-(C₁-C₄)-alkoxy bedeutet, wobei ein aromatischer Rest mit 1, 2 oder 3 gleichen oder verschiedenen Substituenten aus der Reihe Fluor, Chlor, Cyano, Trifluormethyl, (C₁-C₁₀)-Alkyl, (C₁-C₁₀)-Alkoxy, (C₁-C₁₀)-Alkenyloxy substituiert ist und
- R⁴: einen verzweigten oder unverzweigten (C₁-C₈)-Alkylrest oder einen Rest der Formel Z bedeutet,

-[CH₂]ᵥ-[O]_{w}-[CH₂]ₜ-E (Z),

wobei E einen substituierten Phenylrest der Formel F 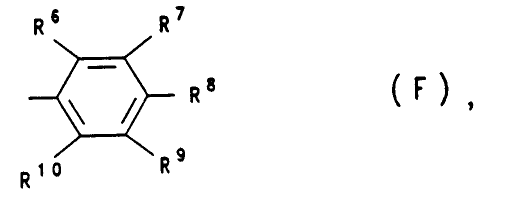 oder einen (C₃-C₈)-Cycloalkylrest bedeutet, wobei
v = 0, 1, 2, 3, w = 0, und t = 0, 1 sein kann und
worin R⁶, R⁷, R⁸, R⁹ und R¹⁰ gleich oder verschieden sind und Wasserstoff, Fluor, Chlor, Cyano, Trifluormethyl, (C₁-C₆)-Alkyl, (C₁-C₆)-Alkoxy, -O-[CH₂₋]ₓC_{f}H_{(2f+1-g)}F_{g}, N-(C₁-C₈)-Alkylcarbamoyl, N,N-Di-(C₁-C₆)-alkylcarbamoyl, N-(C₃-C₆)-Cycloalkylcarbamoyl, N-(+)-Dehydroabietylaminocarbonyl substituierten Benzylrest und f = 1 bis 4, g = 0,1 bis (2f + 1) und x = 0 und 1 bedeuten.

Im Besonderen bevorzugt sind Verbindungen der Formel I, in der
- Q: O,
- X: O,
- Y: CR³,
- m: 0,
- B: -CO₂G, wobei
- G: für einen verzweigten oder unverzweigten aliphatischen (C₁-C₁₆)-Alkyl-Rest, einen 2-Cyclohexylethyl-Rest, einen (C₁-C₄)-Alkoxy-(C₁-C₂)-alkyl-Rest, einen verzweigten oder unverzweigten (C₂-C₁₀)-Alkenylrest, einen Phenyl-, Benzyl-, Phenethyl-, Phenylpropyl- oder Phenylbutylrest steht,
- A: eine -CH₂-Gruppe bedeutet,
- R¹: Wasserstoff, (C₁-C₆)-Alkoxy, -O-[CH₂]ₓ-C_{f}H_{(2f+1-g)}F_{g},
- R²: Wasserstoff, N-(C₁-C₁₀)-Alkylcarbamoyl, N-((C₁-C₁₂)-Alkoxy-(C₁-C₃)-alkyl)carbamoyl, N, N-Di-(C₁-C₈)-alkylcarbamoyl, N-(C₅-C₆]-Cycloalkylcarbamoyl, N-Phenylcarbamoyl, N-Phenyl-(C₁-C₄)-alkylcarbamoyl, Carboxy, (C₁-C₁₆)-Alkoxycarbonyl, (C₂-C₁₆)-Alkenyloxycarbonyl, Retinyloxycarbonyl, (C₅-C₆)-Cycloalkoxycarbonyl, (C₅-C₆)-Cycloalkyl-(C₁-C₆)-alkoxycarbonyl, Phenyl-(C₁-C₆)-alkoxycarbonyl, wobei ein Phenylrest mit 1 oder 2 gleichen oder verschiedenen Substituenten aus der Reihe Fluor, Chlor, Cyano, Trifluormethyl, (C₁-C₁₀)-Alkyl, (C₁-C₁₀)-Alkenyloxy, (C₁-C₁₀)-Alkoxy, substituiert ist und
- R³: Wasserstoff, (C₁-C₅)-Alkoxy, (C₅-C₆)-Cycloalkyl-(C₁-C₂)-alkoxy, wobei einer der Substituenten R¹ und R³ Wasserstoff bedeutet,
- R⁴: einen verzweigten oder unverzweigten (C₁-C₆)-Alkylrest, oder einen 2-Phenylethyl-Rest, oder einen mit 1 oder 2 Resten aus der Reihe Fluor, Chlor, Cyano, Trifluormethyl, (C₁-C₆)-Alkyl, (C₁-C₆)-Alkoxy, -O-[CH₂₋]ₓC_{f}H_{(2f+1-g)}F_{g}, N-(C₁-C₈)-Alkylcarbamoyl, N,N-Di-(C₁-C₆)-alkylcarbamoyl, N-(C₃-C₆)-Cycloalkylcarbamoyl, N-(+)-Dehydroabietylaminocarbonyl substituierten Benzylrest bedeutet und f = 1 bis 4, g = 0,1 bis (2f + 1) und x = 1 bedeuten.

In höchstem Maße bevorzugt sind Verbindungen der Formel I, in der
- Q: O,
- X: O,
- Y: CR³,
- m: 0,
- A: eine -CH₂-Gruppe bedeutet,
- B: -CO₂G, wobei
- G: für einen verzweigten oder unverzweigten aliphatischen (C₁-C₁₆)-Alkyl-Rest oder einen Benzyl-Rest steht,
- R¹: Wasserstoff,
- R²: Wasserstoff, N-(C₁-C₁₀)-Alkylcarbamoyl, N-((C₁-C₁₂)-Alkoxy-(C₁-C₃)-alkyl)carbamoyl, N-Cyclohexylcarbamoyl, N-Phenylcarbamoyl, N-(Phenyl-(C₁-C₂)alkyl)carbamoyl, wobei in den beiden letzten Fällen der Phenylrest einen Fluor-, (C₁-C₁₀)-Alkyl- oder (C₁-C₁₀)-Alkoxy-Substituenten tragen kann, Carboxy, (C₁-C₁₆)-Alkoxycarbonyl, (C₂-C₁₆)-Alkenyloxycarbonyl, Retinyloxycarbonyl, (C₅-C₆)-Cycloalkoxycarbonyl, Benzyloxycarbonyl,
- R³: Wasserstoff, (C₁-C₅)-Alkoxy, 2-(Cyclohexyl)ethyloxy, wobei einer der Substituenten R² und R³ Wasserstoff bedeutet,
- R⁴: einen verzweigten oder unverzweigten (C₁-C₄)-Alkylrest oder einen mit Fluor, Chlor, Trifluormethyl, (C₁-C₄)-Alkyl oder (C₁-C₃)-Alkoxy einfach substituierten Benzylrest bedeuten.

In höchstem Maße bevorzugt sind weiterhin Verbindungen der Formel I, in der
- Q: S,
- X: O,
- Y: CR³,
- m: 0,
- A: eine -CH₂-Gruppe bedeutet,
- B: -CO₂G, wobei
- G: für einen verzweigten oder unverzweigten aliphatischen (C₁-C₁₆)-Alkyl-Rest, oder einen Benzyl-Rest steht,
- R¹: Wasserstoff,
- R²: Wasserstoff, N-(C₁-C₁₀)-Alkylcarbamoyl, N-((C₁-C₁₂)-Alkoxy-(C₁-C₃)alkyl)carbamoyl, N-Cyclohexylcarbamoyl, N-Phenylcarbamoyl, N-(Phenyl-(C₁-C₂)alkyl)carbamoyl, wobei in den beiden letzten Fällen der Phenylrest einen Fluor-, (C₁-C₁₀)-Alkyl- oder (C₁-C₁₀)-Alkoxy-Substituenten tragen kann, Carboxy, (C₁-C₁₆)-Alkoxycarbonyl, (C₂-C₁₆)-Alkenyloxycarbonyl, Retinyloxycarbonyl, (C₅-C₆)-Cycloalkoxycarbonyl, Benzyloxycarbonyl,
- R³: Wasserstoff, (C₁-C₅)-Alkoxy, 2-(Cyclohexyl)ethyloxy, wobei einer der Substituenten R² und R³ Wasserstoff bedeutet, und
- R⁴: einen verzweigten oder unverzweigten (C₁-C₄)-Alkylrest oder einen mit Fluor, Chlor, Trifluormethyl, (C₁-C₄)-Alkyl oder (C₁-C₃)-Alkoxy einfach substituierten Benzylrest bedeuten.

In höchstem Maße bevorzugt sind weiterhin die Verbindungen der Formel I, in denen
- Q: S,
- X: O,
- Y: CR³,
- m: 0,
- A: eine -CH₂-Gruppe bedeutet,
- B: -CO₂G, wobei
- G: für einen verzweigten oder unverzweigten aliphatischen (C₁-C₁₆)-Alkyl-Rest, oder einen Benzyl-Rest steht,
- R¹: Wasserstoff,
- R²: Carboxy oder (C₁-C₁₆)-Alkoxycarbonyl,
- R³: Wasserstoff und
- R⁴: einen verzweigten oder unverzweigten (C₁-C₄)-Alkylrest bedeutet.

In höchstem Maße bevorzugt sind weiterhin die Verbindungen der Formel I, in denen
- Q: O,
- X: O,
- Y: CR³, wobei R³ Wasserstoff bedeutet,
- m: O,
- A: eine -CH₂-Gruppe,
- B: -CO₂G, wobei
- G: für einen verzweigten oder unverzweigten aliphatischen (C₁-C₁₆)-Alkyl-Rest oder einen Benzyl-Rest steht,
- R¹ und R²: zusammen mit dem sie tragenden Pyridin einen Isochinolin-Ring mit unsubstituiertem Benzoteil bilden und
- R⁴: Methyl bedeutet.

In höchstem Maße bevorzugt sind weiterhin die Verbindungen der Formel I, in denen
- Q: O,
- X: O,
- Y: CR³,
- m: O,
- A: eine -CH₂-Gruppe,
- B: -CO₂G, wobei
- G: für einen verzweigten oder unverzweigten aliphatischen (C₁-C₁₆)-Alkyl-Rest oder einen Benzyl-Rest steht,
- R¹: Wasserstoff,
- R² und R³: zusammen mit dem sie tragenden Pyridin einen Chinolin-Ring mit unsubstituiertem Benzoteil bilden und
- R⁴: Methyl bedeutet.

Die Erfindung betrifft die Verwendung von Verbindungen der allgemeinen Formel I sowie die physiologisch verträglichen Salze zur Inhibierung der Kollagenbiosynthese.

Die Erfindung betrifft die Verwendung von Verbindungen der allgemeinen Formel I sowie die physiologisch verträglichen Salze zur Hemmung der Prolyl-4-hydroxylase in vivo.

Weiterhin betrifft die Erfindung die Verwendung von Verbindungen der allgemeinen Formel I sowie die physiologisch verträglichen Salze zur Herstellung eines Arzneimittels gegen fibrotische Erkrankungen.

Weiterhin betrifft die Erfindung die Verwendung von Verbindungen der allgemeinen Formel I sowie die physiologisch verträglichen Salze zur Herstellung eines Arzneimittels gegen fibrotische Erkrankungen der Leber, der Lunge und der Haut.

Schließlich betrifft die Erfindung die Verbindungen der allgemeinen Formel I zur Verwendung als Arzneimittel.

Insbesondere betrifft die Erfindung die Verbindungen der Formel I zur Anwendung als Fibrosuppressiva.

Weiterhin betrifft die Erfindung ein Verfahren zur Herstellung von Verbindungen der allgemeinen Formel I.

Die Herstellung der Verbindungen der Formel I, in denen A einen substituierten Alkylen-Teil, B CO₂G, Y = CR³ und m = 0 und 1 bedeuten erfolgt, indem
i1.) Pyridin-2-carbonsäuren der Formel II (R²³ = H) mit den Aminoestern der Formel III zu den Amidestern der Formel I umgesetzt werden, oder
i2.) Pyridin-2-carbonsäureester der Formel II (R²³ = (C₁-C₁₆)-Alkyl) unter den Bedingungen der Aminolyse zu den Verbindungen der Formel I umgesetzt werden; oder
ii) die Verbindungen der Formel IV mit dem Alkohol GOH verestert werden; oder
iii) die Verbindungen der Formel V mit R⁴X alkyliert werden und ggf.
iv) die Verbindungen der Formel I, sofern Q = O und NR' gilt, in ihre Pyridin-N-oxide (Formel I') übergeführt werden.

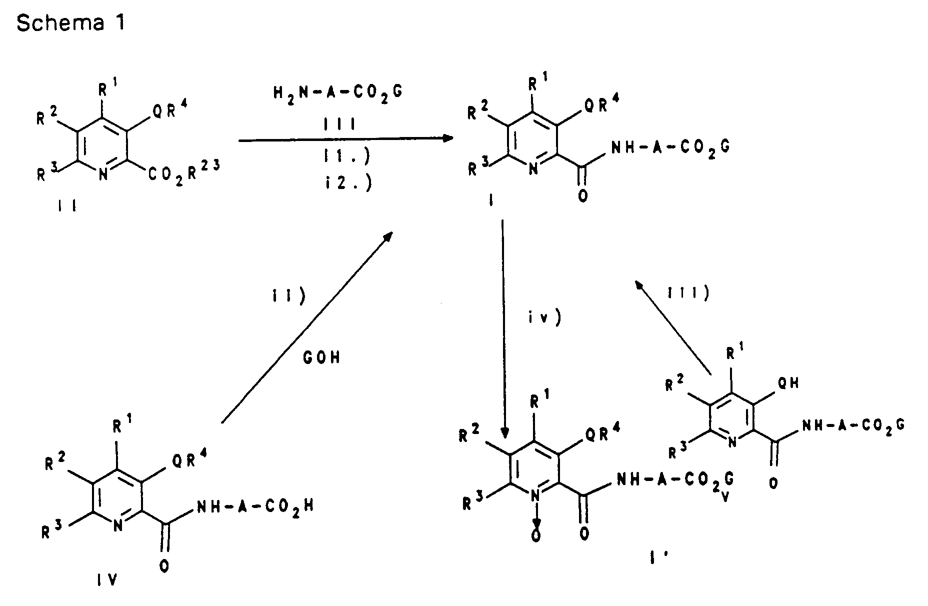

R²³ = H, (C₁-C₁₆)-Alkyl,
X = Abgangsgruppe insbesondere: Halogen, OSO₂Me, OSO₂Phenyl u.a.

Geeignete Verfahren zur Amidbildung (Umsetzung i1) sind die Methoden der Carboxylaktivierung und die aus der Peptidchemie bekannten Kondensationsreaktionen.

An Reagenzien zur Carbonsäureaktivierung können die dem Fachmann bekannten Substanzen, wie Thionylchlorid, Oxalylchlorid, Pivaloylchlorid, Chlorameisensäureester-Derivate oder N,N'-Carbonyldimidazol Verwendung finden. Die aktivierten Derivate der Verbindungen der Formel II werden nach Herstellung in situ mit den Amidderivaten der Formel III umgesetzt.

Ein geeignetes Kondensationsmittel ist beispielsweise die Kombination von N,N'-Dicyclohexylcarbodiimid/N-Hydroxy-1H-benzotriazol und N-Ethylmorpholin.

Geeignete Lösungsmittel sind Dichlormethan, Tetrachlormethan, Butylacetat, Ethylacetat, Toluol, Tetrahydrofuran, Dimethoxyethan, 1,4-Dioxan, Acetonitril, N,N-Dimethylformamid, N,N-Dimethylacetamid, Dimethylsulfoxid, Nitromethan und/oder Pyridin.

Die Verbindungen der Formel I, in denen R¹ und R³ Wasserstoff und R² einen Carboxy-, einen Carbamoyl- oder einen Estersubstituenten bedeutet, wurden, wie in den Schemata 1, 2 und 3 skizziert, hergestellt.

Schema 2 veranschaulicht die Herstellung der Verbindungen der Formel II, in denen R² einen Carbonsäure-Substituenten oder dessen Derivat und R¹ und R³ Wasserstoff bedeuten.

Die 3-substituierten 5-Carboxypyridin-2-carbonsäureester der Formel XI und ihre Isomere der Formel XII werden aus den Pyridin-2,5-dicarbonsäurediestern der Formel VII hergestellt.

Die Oxidation der Pyridin-2,5-dicarboxylate der Formel VII ist in J. Chem. Soc. Perkin Trans. 2, 1978, 34-38 und in J. Org. Chem. 25 (1960) 565 bis 568 (M. L. Peterson) beschrieben.

Die Halogenierung (Chlorierung) der Pyridin-N-oxide der Formel VIII mit Thionylchlorid und die Reaktion des 3-Chlorpyridin-2,5-dicarbonsäurediesters (Formel IX) mit Alkoholaten (Q = O, S) kann in Analogie zu dem in der Patentschrift CH 658 651 (LONZA) beschriebenen Verfahren hergestellt werden, wobei M = Metallion, ein- oder zweiwertig, bevorzugt aus der ersten und zweiten Hauptgruppe des Periodensystems, bedeutet.

Analog der bekannten Literatur (CA: Vol. 68, 1968, 68 840 h) werden aus den substituierten Pyridin-2,5-dicarbonsäurediestern der Formel Xb unter Verseifungsbedingungen die Monoester der Formel XII hergestellt.

Ein weiteres Verfahren zur Herstellung der Verbindungen der Formel XII aus den Diestern der Formel Xb ist die selektive Verseifung mit Cu-II-salzen, J. Delarge in Pharmaceutica Acta Helvetiae 44, 637-643, 1969.

Die so erhaltenen Verbindungen der Formel XII werden mit den Aminoestern der Formel III zu den Verbindungen der Formel IV umgesetzt (Schema 2).

Aus substituierten Pyridin-2,5-dicarbonsäuren der Formel Xa (siehe CA: Vol. 68, 1968, 68840 h) können unter Veresterungsbedingungen die Pyridin-2-carbonsäureester-5-carboxylate der Formel XI hergestellt werden. Geeignete Bedingungen sind z.B. die Veresterung mit Methanol in Gegenwart von Schwefelsäure, wobei die Reaktionszeit so zu wählen ist, daß die vollständige Veresterung zum Diesterprodukt nur untergeordnet stattfindet, bzw. die Diesterprodukte als Nebenprodukte abgetrennt werden können.

Die Verbindungen der Formel XI werden mit Aminen oder Alkoholen in die 5-Carbonsäure-Derivate der Formel XIV überführt (Schema 3).

Diese werden sodann zu den Verbindungen der Formel II (R²³ = H) verseift, die anschließend analog Schema 1 umgesetzt werden.

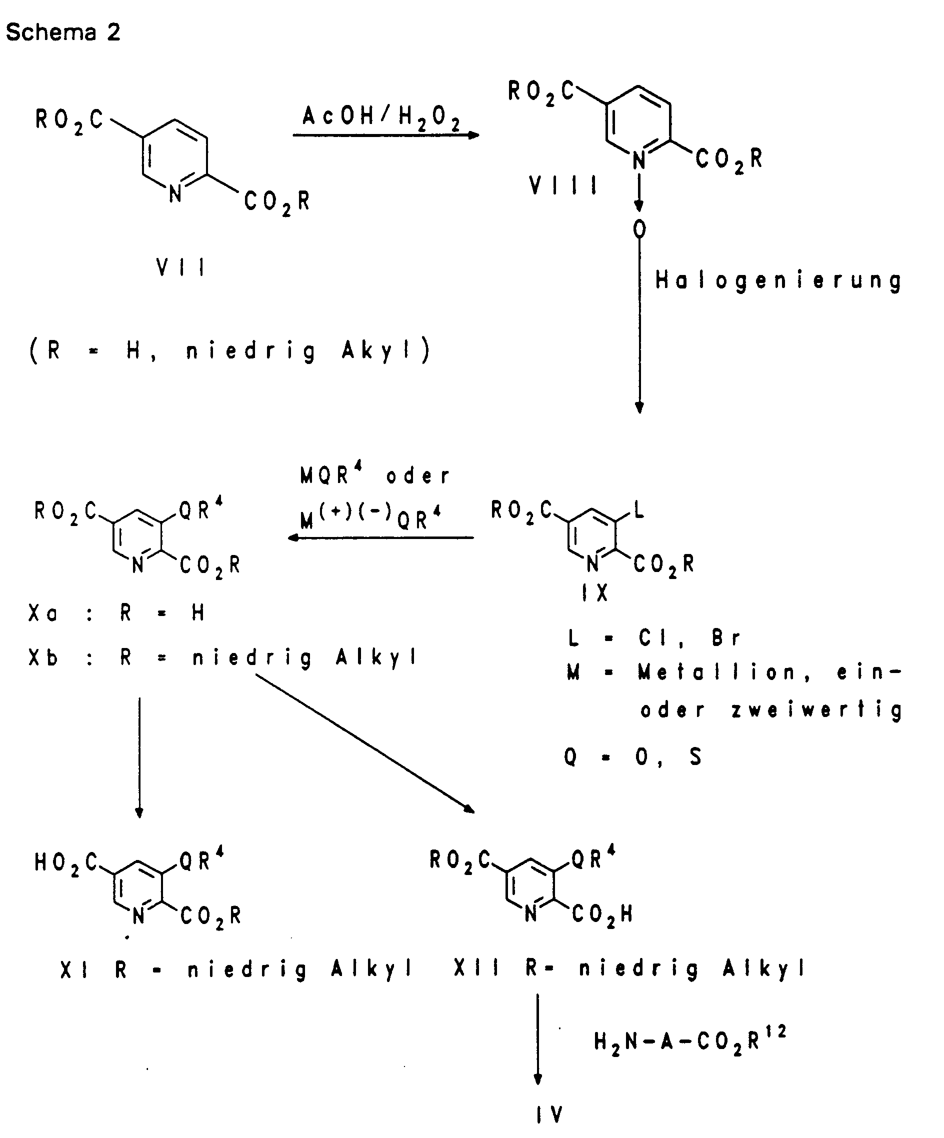

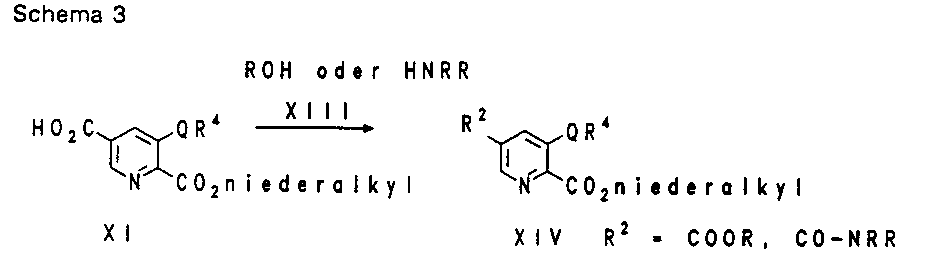

Zur Herstellung von in 4-Position substituierten Derivaten (R¹) können die aus EP-A-0 304 732, EP-A-0 321 385 und EP-A-0 208 452 bekannten 2-Hydroxymethylpyridine der Formel VIa als Zwischenprodukte Verwendung finden.

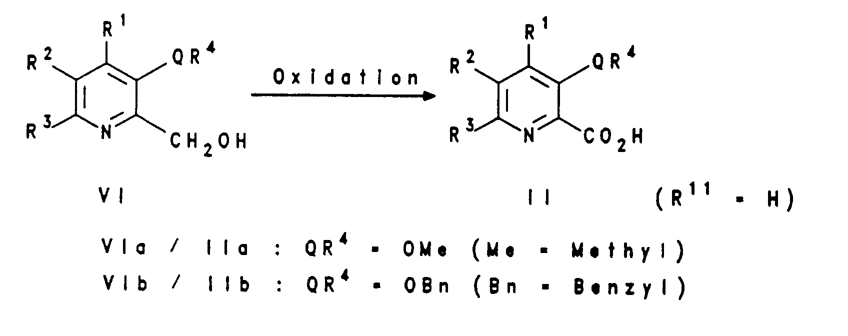

Wie dort beschrieben, wurden in analoger Weise auch die 3-O-Benzylderivate der Formel VIb erhalten.

Die Verbindungen der Formeln VIa und VIb wurden mit einem Oxidationsmittel, vorzugsweise mit KMnO₄ in wäßrigem alkalischen Milieu, zu den Pyridin-2-carbonsäurederivaten der Formel II umgesetzt.

Die Herstellung von substituierten Pyridin-2-carbonsäuren ist beispielsweise aus DE-A-353 046 und für 3-(3-Chlorphenoxy)pyridin-2-carbonsäure und 3-(3-Methylphenoxy)pyridin-2-carbonsäure aus J. Med. Chem. 1975, 18, S. 1-8, Villani et al.; für 3,5-Diethoxypyridin-2-carbonsäure aus J. Med. Chem. 1974, 17, S. 172-181, French et al., sowie für 3-Methylthio- und 3-Benzylthiopyridin-2-carbonsäure aus J. Med. Chem. 1974, 17, S. 1065-1071, Blank et al. und für 3-Methoxypyridin-2,5-dicarbonsäure aus CH-PS 658 651 bekannt.

Die erfindungsgemäßen Verbindungen der Formel I besitzen wertvolle pharmakologische Eigenschaften und zeigen insbesondere antifibrotische Wirksamkeit.

Die antifibrotische Wirkung kann im Modell der Tetrachlorkohlenstoff-induzierten Leberfibrose bestimmt werden. Dazu werden Ratten mit CCl₄ (1 ml/kg) - gelöst in Olivenöl - zweimal wöchentlich behandelt. Die Prüfsubstanz wird täglich, gegebenenfalls sogar zweimal täglich per os oder intraperitoneal - gelöst in einem geeigneten verträglichen Lösungsmittel - verabreicht. Das Ausmaß der Leberfibrose wird histologisch bestimmt und der Anteil Kollagen in der Leber per Hydroxyprolinbestimmung - wie bei Kivirikko et al. (Anal. Biochem. 19, 249 f. (1967)) beschrieben - analysiert. Die Aktivität der Fibrogenese kann durch radioimmunologische Bestimmung von Kollagenfragmenten und Prokollagenpeptiden im Serum bestimmt werden. Die erfindungsgemäßen Verbindungen sind in diesem Modell in Konzentration 1 bis 100 mg/kg wirksam.

Die Aktivität der Fibrogenese kann durch radioimmunologische Bestimmung des N-terminalen Propeptids des Kollagen Typ-III oder der N- bzw. C-terminalen Quervernetzungsdomäne des Kollagen-Typ-IV (7s-Kollagen bzw. Typ-IV-Kollagen NC₁) im Serum bestimmt werden.

Zu diesem Zweck wurden die Hydroxyprolin-, Prokollagen-III-Peptid-, 7s-Kollagen- und Typ-IV-Kollagen-NC-Konzentrationen in der Leber von
a) unbehandelten Ratten (Kontrolle)
b) Ratten, denen Tetrachlorkohlenstoff verabreicht wurden (CCl₄-Kontrolle)
c) Ratten, denen zunächst CCl₄ und anschließend eine erfindungsgemäße Verbindung verabreicht wurde
gemessen (diese Testmethode wird beschrieben von Rouiller, C., experimental toxic injury of the liver; in The Liver, C. Rouiller, Vol. 2, 5. 335 bis 476, New York, Academic Press, 1964).

Weiterhin kann eine Wirksamkeit der erfindungsgemäßen Verbindungen in folgenden Systemen nachgewiesen werden.

### Hemmung der hepatischen Prolyl-4-hydroxylase in vivo:

Dieses Modell dient zum Nachweis der akuten Hemmung der Prolyl-4-hydroxylase in vivo. Dazu werden Ratten beiderlei Geschlechts (gesund bzw. mit induzierter Leberfibrose) die Prüfusubstanz bzw. das entsprechende Vehikel appliziert (intraperitoneal, intravenös, per os) und nach Substanzgabe ¹⁴C-L-Prolin (250 µCi/kg Körpergewicht) intraperitoneal verabreicht. Danach erfolgt erneut eine intraperitoneale Applikation von ¹⁴C-L-Prolin (250 µCi/kg Körpergewicht). Schließlich werden die Tiere unter Pentobarbitalnarkose entblutet und die Leber entnommen. Die Aufreinigung des hepatischen Kollagens durch Pepsinverdau und fraktionierte Ammoniumsulfatfällung erfolgte entsprechend publizierten Protokollen (Ref. 1, 2). Das gereinigte Leberkollagen wurde hydrolysiert und der Gehalt an ¹⁴C-Hydroxyprolin und ¹⁴C-Prolin durch Aminosäureanalyse mittels Ionenaustauschchromatografie bestimmt. Eine Hemmung der Prolyl-4-hydroxylase ergibt sich aus einer Absenkung des Quotienten ¹⁴C-Hydroxyprolin/[¹⁴C-Hydroxyprolin + ¹⁴C-Prolin]. Als Referenzsubstanz wird 2,2'-Dipyridyl verwendet. (1: Chojkier, M. 1986. Hepatocyte collagen production in vivo in normal rats. J. Clin. Invest. 78: 333-339 und 2: Ogata I., et al. 1991. Minor contribution of hepatocytes of hepatocytes to collagen production in normal and early fibrotic livers. Hepatology 14: 361-367).

### Hemmung der Prolyl-4-hydroxylase in Zellkulturen:

Für die Testung von Prolyl-4-hydroxylasehemmstoffen in Zellkulturen werden folgende Zelltypen verwendet:
Normale humane Hautfibrolasten (Normal human fibrolasts, NHDF), Rattenleber-Epithelzellen (rat liver epithelial cells, Ref. 1) und primäre Fettspeicherzellen aus der Rattenleber (fat storing cells, Ref. 2). Dazu werden die Zellen in Gegenwart von Hemmstoffen kultiviert. Gleichzeitig wird das in dieser Zeit neu synthetisierte Kollagen durch 4-³H-L-Prolin und ¹⁴C-Prolin metabolisch markiert. Der Einfluß der Testsubstanzen auf den Hydroxylierungsgrad des Kollagens wird anschließend entsprechend der Methode von Chojkier et al (Ref. 3) bestimmt. Als Referenzsubstanz wird 2,2'-Dipyridyl eingesetzt. (1.: Schrode, W., Mecke, D., Gebhard, R. 1990. Induction of glutamine synthetase in periportal hepatocytes by co-cultivation with a liver epithelial cell line. Eur. J. Cell. Biol. 53: 35-41, 2. Blomhoff, R., Berg T. 1990. Isolation and cultivation of rat liver stellate cells. Methods Enzymol. 190: 59-71 und 3.: Chojkier, M. Peterkofsky, B. Bateman,, J. 1980. A new method for determining the extent of proline hydroxylation by measuring changes in the ration of [4-³H]:[¹⁴C] proline in collagenase digests. Anal. Biochem. 108: 385-393).

Die Verbindungen der Formel I können als Medikamente in Form von pharmazeutischer Präparate Verwendung finden, welche sie gegebenenfalls mit verträglichen pharmazeutischen Trägern enthalten. Die Verbindungen können als Heilmittel, z.B. in Form pharmazeutischer Präparate Verwendung finden, welche diese Verbindungen in Mischung mit einem für die enterale, perkutane oder parenterale Applikation geeigneten pharmazeutischen, organischen oder anorganischen Träger, wie z.B. Wasser, Gummi arabicum, Gelatine, Milchzucker, Stärke, Magnesiumstearat, Talk, pflanzliche Öle, Polyalkylenglykole, Vaseline usw. enthalten.

Sie können zu diesem Zweck oral in Dosen von 0,1 bis 25 mg/kg/Tag, vorzugsweise 1 bis 5 mg/kg/Tag oder parenteral in Dosen von 0,01 bis 5 mg/kg/Tag, vorzugsweise 0,01 bis 2,5 mg/kg/Tag, insbesondere 0,5 bis 1,0 mg/kg/Tag, appliziert werden. Die Dosierung kann in schweren Fällen auch erhöht werden. In vielen Fällen genügen jedoch auch geringere Dosen. Diese Angaben beziehen sich auf einen Erwachsenen von etwa 75 kg Gewicht.

Unter den im folgenden beschriebenen Beispielen werden die erfindungsgemäßen Verbindungen der Formel I als substituierte heterocyclische Carbonsäure(aminosäureester)amide, vorzugsweise als Pyridin-2-carbonsäure (glycylester)amide bezeichnet.
Unter dieser Bezeichnungsweise werden z.B. substituierte Pyridin-2-carbonsäure-N-((alkoxycarbonyl)methyl)amide verstanden.
Die Klassifizierung als substituierte N-(Pyridyl-2-carbonyl)glycine ist eine weitere Möglichkeit.

### Beispiel 1

3-Methoxy-4-(2,2,2-trifluorethyloxy)pyridin-2-carbonsäure-(glycylethylester)amid
a) 2-Methyl-3-methoxy-4-chlorpyridin-N-oxid
   11,2 g (80,5 mmol) 3-Methoxy-2-methyl-4(1H)-pyridon wurden in 100 ml Phosphoroxychlorid 10 Stunden rückfließend erhitzt. Anschließend engte man ein, versetzte 2ml mit je 30 ml Toluol, engte wiederum ein, nahm den Rückstand in 150 ml Wasser auf, brachte mit K₂CO₃ auf pH 11, extrahierte mit Dichlormethan, wusch die organische Phase mit Wasser, trocknete und befreite vom Lösungsmittel.
   Aus dem hellbraunen Öl (9 g) wurden mit m-Clorperbenzoesäure in Dichlormethan unter Standardbedingungen 8 g des Produktes erhalten, Fp. 88-89 °C (aus Petrolether).
b) 2-Methyl-3-methoxy-4-(2,2,2-trifluorethoxy)pyridin-N-oxid
   Zu 20 ml Trifluorethanol gab man bei -20 °C unter Rühren und Stickstoffatomsphäre portionsweise 6,7 g Kalium-tert.Butylat. Nach Erwärmung auf 0°C wurden portionsweise 5,2 g (30 mmol) 2-Methyl-3-methoxy-4-chlorpyridin-N-oxid hinzugegeben. Man erwärmte 3 Stunden unter Rückfluß, ließ auf Raumtemperatur abkühlen, gab weitere 3,45 g Kalium-tert.Butylat zu und erwärmte 2 Stunden unter Rückfluß. Nach dem Abkühlen gab man 40 ml Wasser zum Reaktionsgemisch, extrahierte mit Dichlormethan, trocknete über MgSO₄ und befreite im Vakuum vom Lösungsmittel. Das erhaltene ölige Produkt wurde weiter umgesetzt.
c) 3-Methoxy-4-(2,2,2-trifluorethoxy)-2-hydroxymethyl-pyridin
   8 g (33,8 mmol) der vorstehenden Verbindung wurden in 16 ml Eisessig gelöst und unter Rühren bei 80 °C mit 24 ml Acetanhydrid versetzt. Man erhitzte 2 Stunden auf 110 °C, kühlte sodann auf 80 °C ab und tropfte 40 ml Methanol zur Reaktionsmischung. Anschließend wurde im Vakuum eingeengt, der ölige Rückstand zu 75 ml 2 N methanolischer NaOH hinzugegeben und 30 Minuten gerührt. Nach Behandeln mit Aktivkohle und Filtration wurde im Vakuum eingeengt, der Rückstand mit 50 ml Wasser versetzt, mit Dichlormethan extrahiert, getrocknet (MgSO₄), eingeengt und der Rückstand mit Diisopropylether behandelt. Man erhielt 3,9 g des Produktes in Form farbloser Kristalle, Fp. 107-108 °C.
d) 3-Methoxy-4-(2,2,2-trifluorethyloxy)pyridin-2-carbonsäure
   0,8 g (3,3 mmol) des vorstehenden Alkohols wurden in einer Lösung aus 0,3 g Kaliumhydroxid und 25 ml Wasser gelöst und unter Rühren bei 100 °C, portionsweise 1,6 g Kaliumpermanganat zugegeben. Nach der Entfärbung wurde heiß vom gebildeten Braunstein abgesaugt, zweimal mit heißem Wasser gewaschen, i. Vak. auf 1/3 des Volumens eingeengt, mit conc. wäßriger Salzsäure auf pH 1 gestellt, i. Vak. eingeengt, der Rückstand mit wasserfreiem Ethanol behandelt und von Ungelösten abfiltriert. Aus dem Filtrat erhielt man 0,73 g Produkt, Fp. 157 °C.
e) Zur Herstellung der Titelverbindung wurden 0,58 g (2,3 mmol) der vorstehenden Carbonsäure in 100 ml wasserfreiem Tetrahydrofuran suspendiert, bei 20 °C unter Rühren mit 322 mg (2,3 mmol) Glycinethylester-Hydrochlorid, 0,64 ml (5 mmol) N-Ethylmorpholin, 350 mg (2,6 mmol) 1-Hydroxy-1H-benzotriazol, 537 mg (2,6 mmol) N,N'-Dicyclohexylcarbodiimid versetzt und 48 h bei 20 °C gerührt. Dann wurde vom Ungelöstem abfiltriert, i.Vak. eingeengt, der Rückstand in Ethylacetat aufgenommen, vom Ungelöstem abfiltriert, das Filtrat mit 100 ml gesättigter wäßriger Na-bicarbonat-Lösung gerührt, die organische Phase getrocknet, i.Vak. eingeengt und der Rückstand mit Diisopropylether zur Kristallisation gebracht. Man erhielt 0,45 g des farblosen kristallinen Produkts, Fp. 80-82 °C.

### Beispiel 2

4-Chlor-3-methoxypyridin-2-carbonsäure-(glycylethylester)amid
a) 4-Chlor-2-hydroxymethyl-3-methoxy-pyridin
   30 g (173 mmol) 4-Chlor-3-methoxy-2-methylpyridin-N-oxid (vgl. Beispiel 1a) wurden in 100 ml Eisessig gelöst, bei 80 °C unter Rühren tropfenweise mit 150 ml Acetanhydrid versetzt und 2 h bei 110 °C gerührt. Dann wurde auf 80 °C abgekühlt, 200 ml Methanol zugetropft, 15 min. zum Sieden erhitzt, nach Abkühlen i. Vak. eingeengt, der Rückstand in Methanol aufgenommen und in 300 ml 1,5 N methanolische Natronlauge einfließen lassen, 30 min. bei 20 °C gerührt, i.Vak. eingeengt, der Rückstand in Wasser aufgenomen, dreimal mit Dichlormethan extrahiert, die organische Phase getrocknet, eingeengt und der Rückstand mit Petrolether zur Kristallisation gebracht. Man erhielt 23 g Produkt, Fp. 64 - 66 °C.
b) 4-Chlor-3-methxoypyridin-2-carbonsäure
   8.65 g (50 mmol) des vorstehenden Alkohols wurden in einer Mischung aus 0,8 g Kaliumhydroxid und 60 ml Wasser gelöst und bei 60 °C unter Rühren portionsweise mit Kaliumpermanganat versetzt bis keine Entfärbung mehr zu sehen ist (12 g, 75 mmol). Nach 1 h bei 60 °C wurde vom Braunstein abgesaugt, mit heißem Wasser nachgewaschen, das Filtrat i.Vak. auf 200 ml eingeengt und unter Kühlung mit wäßriger konz. HCl auf pH 1 gestellt. Nach Anreiben kristallisiert unter Kühlung das Produkt aus. Aus der Mutterlauge kann durch Behandeln mit Petrolether weiteres Produkt gewonnen werden, Gesamtmenge 4,2 g, Fp. 116 - 117 °C (unter Gasentwicklung).
c) Zur Herstellung der Titelverbindung wurden 4,7 g (25 mmol) der vorstehenden Carbonsäure in 200 ml wasserfreiem Dichlormethan suspendiert und bei 20 °C unter Rühren nacheinander mit 3,5 g (25 mmol) Glycinethylester-Hydrochlorid, 6,4 ml (50 mmol) N-Ethylmorpholin, 3,8 g (28 mmol) 1-Hydroxy-(1H)-benztriazol und 5,15 (25 mmol) N,N'-Dicyclohexylcarbodiimid versetzt und 20 h bei 20 °C gerührt. Dann wurde vom Ungelösten abfiltriert, die organische Phase mit gesättigter, wäßriger Natriumcarbonat-Lösung geschüttelt, getrocknet, i.Vak. eingeengt, der Rückstand (6 g Öl) mit Ethylacetat an Kieselgel chromatographiert und 5,4 g öliges Produkt erhalten.

### Beispiel 3

4-Butyloxy-3-methoxypyridin-2-carbonsäure-(glycylethylester)amid

### Beispiel 4

3,4-Dimethoxypyridin-2-carbonsäure-(glycylethylester)amid

### Beispiel 5

3-Ethoxy-4-(3-methoxybenzyloxy)pyridin-2-carbonsäure-(glycylethylester)amid

### Beispiel 6

4-Hexyloxy-3-methoxypyridin-2-carbonsäure-(glycylethylester)amid

### Beispiel 7

3-Methoxy-4-(3-methylbutyloxy)pyridin-2-carbonsäure-(glycylethylester)amid

### Beispiel 8

4-(4-Fluorbenzyloxy)-3-methoxypyridin-2-carbonsäure-(glycylethylester)amid

### Beispiel 9

3-Methoxy-4-(4-trifluormethylbenzyloxy)pyridin-2-carbonsäure-(glycylethylester)amid

### Beispiel 10

3-Methoxy-4-(2,2,3,3,3-pentafluorpropyloxy)pyridin-2-carbonsäure-(glycylethylester)amid

### Beispiel 11

4-(2,2,3,3,4,4,4-Heptafluorbutyloxy)-3-methoxypyridin-2-carbonsäure-(glycylethylester)amid

### Beispiel 12

4-(3-Methoxybenzyloxy)-3-methoxypyridin-2-carbonsäure-(glycylethylester)amid

### Beispiel 13

3-Ethyloxy-4-(2,2,2-trifluorethyloxy)pyridin-2-carbonsäure-(glycylethylester)amid

### Beispiel 14

4-Butyloxy-3-ethyloxypyridin-2-carbonsäure-(glycylethylester)amid

### Beispiel 15

3-Methoxy-4-((2-phenoxyethyl)oxy)pyridin-2-carbonsäure-(glycylethylester)amid

### Beispiel 16

3-Ethyloxy-4-benzyloxypyridin-2-carbonsäure-(glycylethylester)amid

### Beispiel 17

3,6-Dimethoxypyridin-2-carbonsäure-(glycylethylester)amid
a) 3,6-Dimethoxy-2-methylpyridin-N-oxid
   1,15 g (50 mmol) Natrium wurden in 100 ml wasserfreiem Methanol gelöst und unter Rühren bei 20 °C 7,4 g (40 mmol) 3-Methoxy-2-methyl-6-nitropyridin-N-oxid hinzugegeben. Dann wurde 3 h zum Rückfluß erhitzt, nach dem Abkühlen i. Vak. eingeengt, der Rückstand in Wasser aufgenommen, mit Dichlormethan extrahiert, die organische Phase getrocknet, eingeengt und der Rückstand mit Diisopropylether zur Kristallisation gebracht. Man erhielt 7 g Produkt, Fp. 63-65 °C.
b) 3,6-Dimethoxy-2-hydroxymethylpyridin
   7 g (41,4 mmol) der vorstehenden Verbindung wurden analog Beispiel 1c) mit Eisessig/Acetanhydrid umgesetzt und das erhaltene Acetat mit 1,5 N methanolischer Natronlauge verseift. Man erhielt 5,6 g öliges Produkt, das unter c) weiter umgesetzt wurde.
c) 3,6-Dimethoxypyridin-2-carbonsäure
   5,6 g (33 mmol) der vorstehenden Verbindung und 2,4 g Kaliumhydroxid wurden in 150 ml Wasser gelöst und bei 60 °C unter Rühren portionsweise mit 15 g (100 mmol) Kaliumpermanganat versetzt. Dann wurde vom gebildeten Braunstein abgesaugt, dieser zweimal mit heißem Wasser gewaschen, die vereinigte Wasserphase auf 100 ml eingeengt, unter Eiskühlung mit conc. wäßriger Salzsäure auf pH 1 gebracht, i.Vak. eingeengt, der Rückstand mit Ethylacetat und Ethanol behandelt, vom Ungelöstem abfiltriert und i. Vak. eingeengt. Der Rückstand wurde mit Diethylether zur Kristallisation gebracht. Man erhielt 4 g Produkt, Fp. 131-132 °C (unter Gasentwicklung).
d) Zur Herstellung der Titelverbindung wurden 2,2 g (12 mmol) der vorstehenden Carbonsäure in 300 ml wasserfreiem Dichlormethan suspendiert, unter Rühren mit 1,68 g (12 mmol) Glycinethylester-Hydrochlorid, 3,25 ml (25 mmol) N-Ethylmorpholin, 1,62 g (12 mmol) 1-Hydroxy-(1H)-benztriazol und 5,2 g (12 mmol) N-Cyclohexyl-N'-(2-morpholinethyl)-carbodiimid-methyl-p-toluolsulfonat versetzt und 20 h bei 20 °C gerührt. Dann wurde von wenig Ungelöstem abfiltriert, einmal mit Wasser, dann mit gesättigter wäßriger Na-bicarbonat-Lösung geschüttelt, die organische Phase getrocknet, i.Vak. eingeengt und der Rückstand mit Diisopropylether zur Kristallisation gebracht. Man erhielt 2 g Produkt, Fp. 93-95 °C.

### Beispiel 18

3,5-Diethoxypyridin-2-carbonsäure-(glycylethylester)amid

### Beispiel 19

3-Methoxy-6-(3-methylbutyloxy)pyridin-2-carbonsäure-(glycylethylester)amid
Fp: 60-62°C (aus Diisopropylether)

### Beispiel 20

3-Benzyloxy-4-(3-ethyloxypropyloxy)pyridin-2-carbonsäure-(glycylethylester)amid

### Beispiel 21

3-Benzyloxy-4-hexyloxypyridin-2-carbonsäure-(glycylethylester)amid

### Beispiel 22

6-(2-Butoxyethyloxy)-3-methoxypyridin-2-carbonsäure-(glycylethylester)amid

### Beispiel 23

6-Butyloxy-3-methoxypyridin-2-carbonsäure-(glycylethylester)amid

### Beispiel 24

3-Ethyloxy-6-methylpyridin-2-carbonsäure-(glycylethylester)amid

### Beispiel 25

6-Benzyloxy-3-methoxypyridin-2-carbonsäure-(glycylethylester)amid

### Beispiel 26

3-Benzyloxypyridin-2-carbonsäure-(glycylethylester)amid

### Beispiel 27

3-Methoxypyridin-2-carbonsäure-(glycylethylester)amid
Fp. 141-142°C (unter Gasentwicklung, aus Diethylether)

Dieser Ethylester wurde durch katalytische Hydrierung von 4-Chlor-3-methoxypyridin-2-carbonsäure-(glycylethylester)amid (siehe Beispiel 2c) erhalten, welches aus 4-Chlor-3-methoxypyridin-2-carbonsäure (Fp. 119-120 °C, aus 4-Chlor-3-methoxy-2-methylpyridin-N-oxid durch Reaktion mit Acetanhydrid/Eisessig und nachfolgender Oxidation des 2-Hydroxymethylpyridin-Derivates) (siehe Beispiel 2a, b) und Glycinethylester-Hydrochlorid erhalten wurde.

### Beispiel 28

3-Ethoxypyridin-2-carbonsäure-(glycylethylester)amid

### Beispiel 29

3-Propyloxypyridin-2-carbonsäure-(glycylethylester)amid

### Beispiel 30

3-Butyloxypyridin-2-carbonsäure-(glycylethylester)amid
a) 3-n-Butyloxypyridin-2-carbonsäure
   9,8 g (70 mmol) 3-Hydroxypyridin-2-carbonsäure wurden in 150 ml N,N-Dimethylacetamid bei 20 °C unter Rühren portionsweise mit 6 g (150 mmol) NaH (60 %, in Mineralöl) versetzt. Nach 30 min. tropfte man 15 ml (140 mmol) Butylbromid zu und erwärmte 2,5 h zwischen 95 °C und 125 °C. Nach dem Abkühlen wurde i. Vak. eingeengt, mit wäßriger Na-Bicarbonat-Lösung behandelt, mit Dichlormethan extrahiert, nach dem Trocknen der Rückstand chromatographisch mit Ethylacetat an Kieselgel gereinigt. Die so erhaltenen 13 g öliges Produkt wurden in 250 ml 1,5 N methanolische Natronlauge eingetragen, 30 min bei 20 °C gerührt, i. Vak. eingeengt, in 200 ml Wasser aufgenommen, mit Dichlormethan extrahiert, die wäßrige Phase mit conc. wäßriger Salzsäure auf pH 1 gebracht, i. Vak. eingeengt, der Rückstand mit Ethylacetat, sodann mit wasserfreiem Ethanol behandelt. Die erhaltenen Lösungen wurden eingeengt und der Rückstand mit Aceton zur Kristallisation gebracht. Man erhielt 9,3 g Produkt (Fp. 93-95 °C), das laut ¹H-NMR noch ca. 20 % 3-Hydroxypyridin-2-carbonsäure enthielt.
b) 4 g (20 mmol) des obigen Produkts wurden in 200 ml wasserfreiem Tetrahydrofuran und 100 ml wasserfreiem Acetonitril bei 20 °C unter Rühren mit 2,8 g (20 mmol) Glycinethylester-Hydrochlorid, 5,2 ml (40 mmol) N-Ethylmorpholin, 2,7 g (20 mmol) 1-Hydroxy-1H-benztriazol und 3,0 ml (20 mmol) N,N'-Diisopropylcarbodiimid versetzt und 20 h bei 20 °C gerührt. Nach Aufarbeitung (Behandlung mit Na-Bicarbonat-Lösung, Abtrennung von ausgefallenem Diisopropylharnstoff wurde nach Chromatographie an Kieselgel (Ethylacetat/n-Heptan 1:1; dann reines Ethylacetat) 3,5 g öliges Produkt erhalten, das noch N,N'-Diisopropylharnstoff enthielt.

### Beispiel 31

3-(4-Chlorbenzyloxy)pyridin-2-carbonsäure-(glycylethylester)amid
a) 3-(4-Chlorbenzyloxy)pyridin-2-carbonsäure-(4-chlorbenzyl)ester
   8,4 g (60 mmol) 3-Hydroxypyridin-2-carbonsäure wurden analog Beispiel 30a) in N,N-Dimethylacetamid mit 5,2g (ca. 130 mmol, 60 %) Natriumhydrid und 19,3 g (120 mmol) 4-Chlorbenzylchlorid alkyliert (3 h, 110 °C). Nach Einengen i.Vak., Extrahieren mit Na-bicarbonat-Lösung wurde der Rückstand mit Heptan/Ethylacetat (1:1) an Kieselgel gereinigt und aus entsprechenden Fraktionen 14,8 g des Produkts mit Diisopropylether zur Kristallisation gebracht, Fp. 92-94 °C.
b) 3-(4-Chlorbenzyloxy)pyridin-2-carbonsäure
   9,7 g (25 mmol) des vorstehenden Esters wurden mit 200 ml 1,5 N methanolischer Natronlauge verseift (24 h, 20 °C). Nach Aufarbeitung (Einengen, Aufnahme des Rückstands in Wasser, Extrahieren mit Dichlormethan und Ansäuern) wurden 6,5 g Produkt erhalten, Fp. 144 °C (aus Wasser, Zersetzung)
c) Zur Herstellung der Titelverbindung wurden 3,2 g (12 mmol) der vorstehenden Pyridin-2-carbonsäure analog Beispiel 17 d) mit 1,7 g (12 mmol) Glycinethylester-Hydrochlorid, 1,62 (12 mmol) 1-Hydroxy-(1H)-benztriazol, 3,3 ml (25 mmol) N-Ethylmorpholin und 5,2 g (12 mmol) N-Cyclohexyl-N'-(2-morpholinoethyl)-carbodiimid-methyl-p-toluolsulfonat) umgesetzt. Nach Aufarbeitung wurden 3,0 g des Produkts mit Diisopropylether zur Kristallisation gebracht, Fp. 106-108 °C.

### Beispiel 32

3-(3-Methoxybenzyloxy)pyridin-2-carbonsäure-(glycylethylester)amid
a) 3-(3-Methoxybenzyloxy)pyridin-2-carbonsäure-(3-methoxybenzyl)ester
   Analog Beispiel 38a) wurden aus 8,4 g (60 mmol) 3-Hydroxypyridin-2-carbonsäure und 3-Methoxybenzylchlorid nach Chromatographie an Kieselgel 10 g des Produkts als farbloses Öl erhalten, die weiter umgesetzt wurden.
b) 3-(3-Methoxybenzyloxy)pyridin-2-carbonsäure
   10 g des vorstehenden Esters wurden in 300 ml 1,5 N methanolischer Natronlauge verseift. Man erhielt 7,5 g Produkt, Fp. 147 °C (Zersetzung, aus wäßriger Salzsäure)
c) Zur Herstellung der Titelverbindung wurden 3,2 g (12 mmol) der vorstehenden Carbonsäure analog Beispiel 31c) umgesetzt. Man isolierte 3,6 g öliges Rohprodukt, das laut ¹H-NMR-Spektrum noch N-Ethylmorpholin enthielt. Hieraus wurde die reine Substanz erhalten, Fp. 135-137 °C (aus Diisopropylether/Ethylacetat).

### Beispiel 33

3-(2-Phenylethyloxy)pyridin-2-carbonsäure-(glycylethylester)amid
a) 3-((2-Phenylethyloxy)pyridin-2-carbonsäure
   Analog Beispiel 30 a) wurden 8,4 g (60 mmol) 3-Hydroxypyridin-2-carbonsäure mit NaH/2-Phenylethylbromid in N,N-Dimethylacetatamid alkyliert. Die nach säulenchromatographischer Reinigung erhaltenen 10 g öliges Produkts wurden analog Beispiel 30a) mit methanolischer Natronlauge verseift. Man erhielt 3 g Produkt (Fp. 145 °C (unter Schäumen, aus Aceton), das laut ¹H-NMR-Spektrum ca. 25 % 3-Hydroxypicolinsäure enthält.
b) Zur Herstellung der Titelverbindung wurden analog Beispiel 30b) 2,9 g der vorstehenden Verbindung mit Glycinethylester-Hydrochlorid, N-Ethylmorpholin, 1-Hydroxy-1H-benztriazol und N,N-Dicyclohexylcarbodiimid umgesetzt. Nach Aufarbeitung wurde das Rohprodukt mit Ethylacetat an Kieselgel chromatographiert. Als Nebenprodukt wurden zunächst 3-Hydroxy-pyridin-2-carbonsäure-(glycylethylester)amid eluiert und aus entsprechenden Fraktionen mit Petrolether zur Kristallisation gebracht, 1,1 g (Fp. 86-88 °C, starke Fluoreszenz im UV-Licht). Dann wurde aus entsprechenden Fraktionen das Produkt mit Diisopropylether zur Kristallisation gebracht und 1,7 g des Produkts Fp. 73-75 °C erhalten.

### Beispiel 34

3-(4-Trifluormethylbenzyloxy)pyridin-2-carbonsäure-(glycylethylester)amid,
Fp. 107-109°C

### Beispiel 35

3-(4-(2-Propyl)benzyloxy)pyridin-2-carbonsäure-(glycylethylester)amid, farbloses Öl

### Beispiel 36

3-(4-Fluorbenzyloxy)pyridin-2-carbonsäure-(glycylethylester)amid
Fp: 97-99°C (aus Diisopropylether)

### Beispiel 37

3-(4-(2-(4-Methoxyphenyl)ethylamino)carbonyl)benzyloxy)pyridin-2-carbonsäure(glycylethylester)amid
Fp. 141-143°C (aus Diethylether/Ethylacetat (9:1)).

Die folgenden Beispiele Nr. 38-64 wurden analog hergestellt.

### Beispiel 38

3-(2,4-Dichlorbenzyloxy)pyridin-2-carbonsäure-(glycylethylester)amid

### Beispiel 39

3-(3-Fluorbenzyoxy)pyridin-2-carbonsäure-(glycylethylester)amid

### Beispiel 40

3-(3-Chorbenzyloxy)pyridin-2-carbonsäure-(glycylethylester)amid

### Beispiel 41

3-(3,4-Dichlorbenzyloxy)pyridin-2-carbonsäure-(glycylethylester)amid

### Beispiel 42

3-(3-Trifluormethybenzyloxy)pyridin-2-carbonsäure-(glycylethylester)amid

### Beispiel 43

3-(4-Trifluormethoxybenzyloxy)pyridin-2-carbonsäure-(glycylethylester)amid

### Beispiel 44

3-(3-Ethoxybenzyloxy)pyridin-2-carbonsäure-(glycylethylester)amid

### Beispiel 45

3-(4-Cyanobenzyloxy)pyridin-2-carbonsäure-(glycylethylester)amid

### Beispiel 46

3-((2-Pyridylmethyl)oxy)pyridin-2-carbonsäure-(glycylethylester)amid-Hydrochlorid

### Beispiel 47

3-((3-Pyridylmethyl)oxy)pyridin-2-carbonsäure-(glycylethylester)amid-Hydrochlorid

### Beispiel 48

3-((4-Pyridylmethyl)oxy)pyridin-2-carbonsäure-(glycylethylester)amid-Hydrochlorid

### Beispiel 49

3-(2-Thienylmethyl)oxy)pyridin-2-carbonsäure-(glycylethylester)amid

### Beispiel 50

3-(3,5-Dimethoxybenzyloxy)pyridin-2-carbonsäure-(glycylethylester)amid

### Beispiel 51

3-Cyclohexyloxypyridin-2-carbonsäure-(glycylethylester)amid

### Beispiel 52

3-(3-Phenylpropyloxy)pyridin-2-carbonsäure-(glycylethylester)amid

### Beispiel 53

3-(4-Phenylbutyloxy)pyridin-2-carbonsäure-(glycylethylester)amid

### Beispiel 54

3-(((4-Methoxy-2-pyridyl)methyl)oxy)pyridin-2-carbonsäure-(glycylethylester)amid

### Beispiel 55

3-(((4-Ethoxy-2-pyridyl)methyl)oxy)pyridin-2-carbonsäure-(glycylethylester)amid

### Beispiel 56

3-Methylthiopyridin-2-carbonsäure-(glycylethylester)amid

### Beispiel 57

3-Benzylthiopyridin-2-carbonsäure-(glycylethylester)amid

### Beispiel 58

3-(3-Chlorphenoxy)pyridin-2-carbonsäure-(glycylethylester)amid

### Beispiel 59

3-(3-Methoxyphenoxy)pyridin-2-carbonsäure-(glycylethylester)amid

### Beispiel 60

3-Phenoxypyridin-2-carbonsäure-(glycylethylester)amid

### Beispiel 61

3-Butyloxypyridin-2-carbonsäure-L-(alanylethylester)amid

### Beispiel 62

3-Butyloxypyridin-2-carbonsäure-D-(alanylethylester)amid

### Beispiel 63

3-Benzyloxypyridin-2-carbonsäure-β-(alanylethylester)amid

### Beispiel 64

3-(3-Methylbutyloxy)pyridin-2-carbonsäure-L-(leucylethylester)amid

### Beispiel 65

4-Methoxyisochinolin-3-carbonsäure-(glycylmethylester)amid
a) 1,2-Dihydro-4-hydroxy-1-oxoisochinolin-3-carbonsäuremethylester, wurde wie beschrieben hergestellt (M. Suzuki et al., Synthesis 1978, 461).
b) 1,2-Dihydro-4-methoxy-1-oxoisochinolin-3-carbonsäuremethylester mit Trimethylsilyldiazomethan in Methanol/Acetonitril aus a), Fp. 177 bis 179°C (Ethylacetat/Heptan).
c) 1-Chlor-4-methoxyisochinolin-3-carbonsäuremethylester mit Phosphoroxychlorid aus b), Fp. 108°C (Ethylacetat).
d) 4-Methoxyisochinolin-3-carbonsäuremethylester mit Wasserstoff/Pd/C aus c), Fp. 129°C (aus Methyl-tert.butylether).
e) 4-Methoxyisochinolin-3-carbonsäure durch Verseifung von d), Fp. 185 bis 189°C (aus wäßriger Salzsäure).
f) Die Titelverbindung wurde aus der obigen Verbindung und Glycinmethylester-Hydrochlorid mit DCC, HOBT, THF, NEM erhalten; ölige Substanz, ¹H-NMR (CDCl₃): δ = 4,33 (d, CH₂-Glycin).

Die Beispiele 66 bis 76 wurden analog aus den entsprechenden Isochinolin-3-carbonsäuren bzw. den 5,6,7,8-Tetrahydro-Derivaten erhalten:

### Beispiel 66

4-Ethoxyisochinolin-3-carbonsäure-(glycylethylester)amid

### Beispiel 67

4-Propyloxychinolin-3-carbonsäure-(glycylethylester)amid

### Beispiel 68

4-(3-Methybutyoxy)isochinolin-3-carbonsäure-(glycylethylester)amid

### Beispiel 69

4-Methoxy-5,6,7,8-tetrahydroisochinolin-3-carbonsäure-(glycylethylester)amid

### Beispiel 70

4-(3-Methylbutyloxy)-5,6,7,8-tetrahydroisochinolin-3-carbonsäure-(glycylethylester)amid

### Beispiel 71

4-Ethoxy-5,6,7,8-tetrahydroisochinolin-3-carbonsäure-(glycylethylester)amid

### Beispiel 72

4-Benzyloxy-5,6,7,8-tetrahydroisochinolin-3-carbonsäure-(glycylethylester)amid

### Beispiel 73

4-Benzyloxyisochinolin-3-carbonsäure-(glycylethylester)amid

### Beispiel 74

4-(3-Methoxybenzyloxy)-5,6,7,8-tetrahydroisochinolin-3-carbonsäure-(glycylethylester)amid

### Beispiel 75

7-Brom-4-methoxyisochinolin-3-carbonsäure-(glycylethylester)amid

### Beispiel 76

7-Methoxy-4-methoxyisochinolin-3-carbonsäure-(glycylethylester)amid

### Beispiel 77

3-Methoxy-6-((3-methylbutyloxy)methyl)pyridin-2-carbonsäure-(glycylethylester)amid

### Beispiel 78

3-Methoxy-6-((cyclohexyloxy)methyl)pyridin-2-carbonsäure-(glycylethylester)amid

### Beispiel 79

3-Methoxy-6-benzyloxymethylpyridin-2-carbonsäure-(glycylethylester)amid

### Beispiel 80

5-Carboxy-3-methoxypyridin-2-carbonsäure-(glycylethylester)amid

### Beispiel 81

5-Methoxycarbonyl-3-methoxypyridin-2-carbonsäure-(glycylbenzylester)amid
a) 5-Methoxycarbonylpyridin-2-carbonsäure-1-oxid
   12 g (60 mMol) Pyridin-2,5-dicarbonsäure-dimethylester wurden in 30 ml Eisessig suspendiert und bei 20°C unter Rühren mit 13 ml Wasserstoffperoxid (35 %) versetzt. Unter Rühren wurde sodann auf 100°C (Innentemperatur) erwärmt, wobei sich bei 50°C eine klare Lösung bildete. Nachdem 90 Minuten bei 100°C gerührt worden war, ließ man auf 20°C abkühlen, saugte die kristalline Fällung ab, wusch mit Wasser und nach dem Trocknen erhielt man 7,5 g Produkt, Fp. 160°C (Zers.)
b) 3-Chlorpyridin-2,5-dicarbonsäure-dimethylester
   Es wurden 17 ml Thionylchlorid, 35 ml wasserfreies Chloroform und 1,5 ml N,N-Dimethylformamid unter Rühren auf 60°C erwärmt und bei dieser Temperatur portionsweise mit 7,5 g des vorstehenden Produkts versetzt. Dann wurde weitere 60 Minuten bei 60°C gerührt, das Lösungsmittel und überschüssiges Reagenz nach dem Abkühlen im Vakuum abdestilliert, der Rückstand mit Dichlormethan versetzt, der N,N-Dimethylformamid x HCl-Komplex abgesaugt und mit Dichlormethan gewaschen. Zur Mutterlauge gab man unter Kühlung ca. 15 ml Triethylamin und 10 ml Methanol und rührte 30 Minuten Nach dem Eindampfen im Vakuum wurde der Rückstand in 50 ml Wasser gelöst, 3 x mit Dichlormethan extrahiert, die organische Phase getrocknet, eingeengt und der Rückstand mit n-Heptan und n-Heptan:Ethylacetat (3:1) an Kieselgel chromatographiert. Aus entsprechenden Fraktionen wurden 5,3 g Produkt mit Petrolether zur Kristallisation gebracht, Fp. 36 bis 38°C.
c) 3-Methoxypyridin-2,5-dicarbonsäure
   53 g (0,231 mol) des vorstehenden Diesters wurden in 500 ml Methanol gelöst und unter Rühren bei 20°C mit 150 ml (0,81 mol) Natriummethylat-Lösung (30 % in Methanol) versetzt, wobei die Temperatur auf 30°C anstieg. Man erhitzte 4,5 Stunden unter Rückfluß, versetzte bei 20°C mit 300 ml Wasser und rührte 30 Minuten bei 35°C. Das überschüssige Methanol wurde im Vakuum abdestilliert, die wäßrige Phase unter Kühlung mit halbkonzentrierter wäßriger Salzsäure auf pH 2 gebracht, das farblose kristalline Produkt abgesaugt und getrocknet. Man erhielt 49 g, Fp. 185°C (Gasentwicklung); 255°C (Zers.)
d) 3-Methoxypyrindin-2,5-dicarbonsäuredimethylester, vgl. Beispiel 90 a)
e) 5-Methoxycarbonyl-3-methoxypyridin-2-carbonsäure
   Die Verbindung wurde im Gemisch mit dem isomeren Monomethylester (vgl. Beispiel 90 a)) aus 3,4 g (15 mmol) des vorstehenden Diesters durch Verseifen mit verdünnter methanolischer Natronlauge (0,54 g NaOH (13,5 mmol) erhalten. Man erhielt neben 0,8 g unumgesetzten Diester 1,8 g Monoester-Gemisch, Fp. 152°C.
f) 1,8 g des vorstehenden Gemischs wurden analog Beispel 90 b) mit 2,9 g (8,6 mmol) Glycinbenzylestertosylat in Gegenwart von N-Ethylmorpholin, 1-Hydroxy-1H-benztriazol und CMC kondensiert. Nach Aufarbeitung wurden 2,3 g öliges Gemisch mit Dichlormethan (bis zu 2 % Methanol-Zusatz) an Kieselgel chromatographiert. Man erhielt 0,82 g Produkt, Fp. 108°C. Desweiteren wurden 0,6 g des öligen Isomeren isoliert.

### Beispiel 82

5-(3-Pentyloxy)carbonyl-3-methoxypyridin-2-carbonsäure-(glycylethylester)amid

### Beispiel 83

5-Cyclohexyloxycarbonyl-3-methoxypyridin-2-carbonsäure-(glycylethylester)amid

### Beispiel 84

5-(n-Butylaminocarbonyl)-3-methoxypyridin-2-carbonsäure-(glycylethylester)amid

### Beispiel 85

5-(2-Methyl-2-butylaminocarbonyl)-3-methoxypyridin-2-carbonsäure-(glycylethylester)amid

### Beispiel 86

5-(Cyclohexylaminocarbonyl)-3-methoxypyridin-2-carbonsäure-(glycylethylester)amid
a) 5-(Cyclohexylaminocarbonyl)-3-methoxypyridin-2-carbonsäure
   Analog Beispiel 90 b) erhielt man das Produkt aus 5-Carboxy-3-methoxypyridin-2-carbonsäure und Cyclohexylamin,
   Fp. 155°C (bei 80°C sintern, aus wäßriger Salzsäure).
b) Die Titelverbindung wurde analog Beispiel 90 c) aus der vorstehenden Verbindung erhalten, Fp. 187 bis 188°C (aus Diethylether)

### Beispiel 87

5-(Cyclohexylaminocarbonyl)-3-ethyloxypyridin-2-carbonsäure-(glycylethylester)amid

### Beispiel 88

5-((2-Phenylethyl)aminocarbonyl)-3-methoxypyridin-2-carbonsäure-(glycylethylester)amid

### Beispiel 89

5-((+)-Dehydroabietylaminocarbonyl)-3-methoxypyridin-2-carbonsäure-(glycylethylester)amid
a) 5-((+)-Dehydroabietylaminocarbonyl)-3-methoxypyridin-2-carbonsäure
   Analog Beispiel 90 a) erhielt man das harzige Produkt aus 5-Carboxy-3-methoxypyridin-2-carbonsäuremethylester und (+)-Dehydroabietylamin.
b) Die Titelverbindung wurde analog Beispiel 90 c) nach Verseifung aus der vorstehenden Verbindung erhalten, Fp. ab 150°C unter Schäumen, sintern bei 120°C, aus Diethylether).

### Beispiel 90

5-((2-(4-Fluorphenyl)ethyl)aminocarbonyl)-3-methoxypyridin-2-carbonsäure-(glycylethylester)amid
a) 5-Carboxy-3-methoxypyridin-2-carbonsäuremethylester
   10 g (50,7 mmol) der 3-Methoxypyridin-2,5-dicarbonsäure (Beispiel 81c) wurden in 150 ml wasserfreiem Methanol suspendiert, mit 2 ml konzentrierter Schwefelsäure versetzt und 3 Stunden rückfließend erhitzt. Dann wurde die Hälfte des Methanols im Vakuum abdestilliert, der Rückstand in 400 ml Eiswasser eingetragen, der kristalline Rückstand abgesaugt, mit Wasser gewaschen, der Rückstand in 150 ml gesättigter wäßriger Na-bicarbonat-Lösung gelöst, zweimal mit je 80 ml Dichlormethan extrahiert, die Bicarbonat-Phase unter Kühlung mit halbkonzentrierter wäßriger Salzsäure auf pH 1 gebracht, das ausgefallene Produkt abgesaugt und getrocknet. Man erhielt 5 g farblose, kristalline Substanz, Fp. 196 bis 197°C. Aus der Dichlormethan-Phase wurden 1,7 g Dimethylester erhalten, Fp. 53 bis 55°C (aus Petrolether).
b) 5-(((2-(4-Fluorphenyl)ethyl)amino)carbonyl)-3-methoxypyridin-2-carbonsäure
   3,2 g 5-Carboxy-3-methoxypyridin-2-carbonsäuremethylester wurden in 300 ml wasserfreiem Dichlormethan suspendiert, unter Rühren bei 20°C nacheinander mit 2,0 ml (15 mmol) 2-(4-Fluorphenyl)ethylamin, 1,95 ml (15 mmol) N-Ethylmorpholin, 2,2 g (16,5 mmol) 1-Hydroxy-1H-benztriazol und 6,35 g (15 mmol) N-Cyclohexyl-N'-(2-morpholinoethyl)-carbodiimid-methyl-p-toluolsulfonat (CMC) versetzt und 24 Stunden gerührt. Dann wurde vom Ungelösten abfiltriert, die organische Phase je 3x mit wäßriger Na-bicarbonat-Lösung, mit 1N wäßriger Salzsäure und mit Wasser extrahiert, die organische Phase getrocknet und eingeengt. Man erhielt 3,7 g (Fp. 168 bis 169°C) Methylester, der in 150 ml 1,5N methanolische NaOH eingetragen wurde. Nach 30 Minuten wurde eingeengt, in 100 ml Wasser gelöst und mit konz. wäßriger Salzsäure auf pH 1 gebracht, die kristalline Fällung abgesaugt, mit Wasser gewaschen und getrocknet. Man erhielt 3,4 g Produkt, Fp. 110°C (unter Schäumen, sintern bei 75°C).
c) Analog Beispiel 90 a) wurden 3,2 g (10 mmol) der vorstehenden Verbindung mit 1,4 g(10 mmol) Glycinethylester-Hydrochlorid, N-Ethylmorpholin, 1-Hydroxy-1H-benztriazol, und CMC umgesetzt. Nach analoger Aufarbeitung wurden 2,8 g des farblos kristallinen Produkts mit Diisopropylether zur Kristallisation gebracht, Fp. 170 bis 171 °C.

### Beispiel 91

5-((2-(4-Methoxyphenyl)ethyl)aminocarbonyl)-3-ethyloxypyridin-2-carbonsäure-(glycylethylester)amid

### Beispiel 92

5-Chor-3-ethyloxypyridin-2-carbonsäure-(glycylethylester)amid

### Beispiel 93

5-Chlor-3-ethyloxypyridin-2-carbonsäure-(glycylethylester)amid

### Beispiel 94

5-Cyclohexyloxymethyl-3-methoxypyridin-2-carbonsäure-(glycylethylester)amid

### Beispiel 95

5-(3-Methylbutyl)oxymethyl-3-methoxypyridin-2-carbonsäure-(glycylethylester)amid

### Beispiel 96

5-Benzyloxymethyl-3-ethyloxypyridin-2-carbonsäure-(glycylethylester)amid

### Beispiel 97

3-((Cyclohexyl)methyloxy)pyridin-2-carbonsäure-(glycylethylester)amid

### Beispiel 98

3-((2-Cyclohexyl)ethyloxy)pyridin-2-carbonsäure-(glycylethylester)amid

### Beispiel 99

3-((3-Cyclohexyl)propyloxy)pyridin-2-carbonsäure-(glycylethylester)amid

### Beispiel 100

3-(3-Methylbutyloxy)pyridin-2-carbonsäure-(glycylethylester)amid

### Beispiel 101

3-Hexyloxypyridin-2-carbonsäure-(glycylethylester)amid

### Beispiel 102

3-(4-Ethylbenzyloxy)pyridin-2-carbonsäure-(glycylethylester)amid

### Beispiel 103

3-(4-Propylbenzyloxy)pyridin-2-carbonsäure-(glycylethylester)amid

### Beispiel 104

3-(4-Butylbenzyloxy)pyridin-2-carbonsäure-(glycylethylester)amid

### Beispiel 105

3-(4-tert.-Butylbenzyloxy)pyridin-2-carbonsäure-(glycylethylester)amid

### Beispiel 106

6-(3-Methoxybenzyloxy)-3-ethyloxypyridin-2-carbonsäure-(glycylethylester)amid

### Beispiel 107

3-(4-Trifluormethylbenzyloxy)pyridin-2-carbonsäure-(glycylmethylester)amid

### Beispiel 108

3-(4-(2-Propyl)benzyloxy)pyridin-2-carbonsäure-(glycylmethylester)amid

### Beispiel 109

3-(3-Fluorbenzyloxy)pyridin-2-carbonsäure-(glycylmethylester)amid

### Beispiel 110

3-(4-Fluorbenzyloxy)pyridin-2-carbonsäure-(glycylmethylester)amid

### Beispiel 111

3-(2,4-Dichlorbenzyloxy)pyridin-2-carbonsäure-(glycylmethylester)amid

### Beispiel 112

3-(4-(2,2,2-Trifluorethyloxy)benzyloxy)pyridin-2-carbonsäure-(glycylmethylester)amid

### Beispiel 113

3-(4-Chlorbenzyoxy)pyridin-2-carbonsäure-(glycylbutylester)amid

### Beispiel 114

3-(3,4-Dichorbenzyloxy)pyridin-2-carbonsäure-(glycylbutylester)amid

### Beispiel 115

3-(3-Trifluormethybenzyloxy)pyridin-2-carbonsäure-(glycylbutylester)amid

### Beispiel 116

3-(4-Trifluormethylbenzyloxy)pyridin-2-carbonsäure-(glycylbutylester)amid

### Beispiel 117

3-(4-(2-Propyl)benzyloxy)pyridin-2-carbonsäure-(glycylbutylester)amid

### Beispiel 118

3-(4-Fluorbenzyloxy)pyridin-2-carbonsäure-(glycylbutylester)amid

### Beispiel 119

3-(4-(2,2,2-Trifluorethyloxy)benzyloxy)pyridin-2-carbonsäure-(glycylbutylester)amid

### Beispiel 120

3-(4-Trifluormethylbenzyloxy)pyridin-2-carbonsäure-(glycyl-3-pentylester)amid

### Beispiel 121

3-(4-(2-Propyl)benzyloxy)pyridin-2-carbonsäure-(glycyl-3-pentylester)amid

### Beispiel 122

3-(3-Fluorbenzyloxy)pyridin-2-carbonsäure-(glycyl(2-ethylbutyl)ester)amid

### Beispiel 123

3-(4-Fluorbenzyoxy)pyridin-2-carbonsäure-(glycyl(2-ethylbutyl)ester)amid

### Beispiel 124

3-(4-(2,2,2-Trifluorethyloxy)benzyloxy)pyridin-2-carbonsäure-(glycyl(3-methylbutyl)ester)amid

### Beispiel 125

3-(Fluorbenzyloxy)pyridin-2-carbonsäure-(glycyl(3-methylbutyl)ester)amid

### Beispiel 126

3-(3-Trifluormethylbenzyloxy)pyridin-2-carbonsäure-(glycylcyclohexylester)amid

### Beispiel 127

3-(4-Trifluormethylbenzyloxy)pyridin-2-carbonsäure-(glycylbenzylester)amid

### Beispiel 128

3-(4-(2-Propyl)benzyloxy)pyridin-2-carbonsäure-(glycylbenzylester)amid

### Beispiel 129

3-(3-Fluorbenzyloxy)pyridin-2-carbonsäure-(glycylbenzylester)amid

### Beispiel 130

3-(4-Fluorbenzyloxy)pyridin-2-carbonsäure-(glycylbenzylester)amid

### Beispiel 131

3-(2,4-Dichlorbenzyloxy)pyridin-2-carbonsäure-(glycylbenzylester)amid

### Beispiel 132

3-(4-(2,2,2-Trifluorethyloxy)benzyloxy)pyridin-2-carbonsäure-(glycylbenzylester)amid

### Beispiel 133

3-(Fluorbenzyloxy)pyridin-2-carbonsäure-(glycylhexylester)amid

### Beispiel 134

3-(4-Chlorbenzyloxy)pyridin-2-carbonsäure-(glycyloctylester)amid

### Beispiel 135

3-(3-Trifluormethylbenzyloxy)pyridin-2-carbonsäure-(glycylhexylester)amid

### Beispiel 136

3-(4-Trifluormethylbenzyloxy)pyridin-2-carbonsäure-(glycyl-(2-ethoxyethyl)ester)amid

### Beispiel 137

3-(4-(2-Propyl)benzyloxy)pyridin-2-carbonsäure-(glycyl-(2-ethoxyethyl)ester)amid

### Beispiel 138

3-(4-Fluorbenzyloxy)pyridin-2-carbonsäure-(glycyl-(2-butoxyethyl)ester)amid

### Beispiel 139

3-(4-Fluorbenzyloxy)pyridin-2-carbonsäure-(glycyl(methylcyclohexyl)ester)amid

### Beispiel 140

3-(4-(2,2,2-Trifluorethyloxy)benzyloxy)pyridin-2-carbonsäure-(glycyl(methylcyclohexyl)ester)amid

### Beispiel 141

3-(Fluorbenzyloxy)pyridin-2-carbonsäure-(glycyl-2-propylester)amid

### Beispiel 142

3-(3,4-Dichlorbenzyloxy)pyridin-2-carbonsäure-(glycyl-2-propylester)amid

### Beispiel 143

3-(4-Trifluormethylbenzyloxy)pyridin-2-carbonsäure-(glycyl-2-propylester)amid

### Beispiel 144

3-Benzyloxypyridin-2-carbonsäure-(glycylbenzylester)amid

### Beispiel 145

3-Benzyloxypyridin-2-carbonsäure-(glycylhexylester)amid

### Beispiel 146

3-Methoxypyridin-2-carbonsäure-N-(((hexadecyloxy)carbonyl)methyl)amid-Hydrochlorid

5,7 g (30 mmol) 4-Chlor-3-methoxypyridin-2-carbonsäure und 14,2 g (30 mmol) Glycinhexadecylester-tosylat (Fp. ca. 90°C, hergestellt aus Glycin, 1-Hexadecanol, p-Toluolsulfonsäure am Wasserabscheider mit Toluol) wurden in 300 ml Dichlormethan analog zu Beispiel 90 b) und c) mit 7,7 (60 mmol) N-Ethylmorpholin, 4,5 g (33 mmol) 1-Hydroxy-1H-benzotriazol und 12,8 g (30 mmol) N-Cyclohexyl-N'-(2-morpholinoethyl)-carbodiimid-methyl-p-toluolsulfonat (CMC) versetzt und 24 h gerührt.
Dann wurde vom Ungelösten abgesaugt, das Filtrat mit wäßriger Na-bicarbonat-Lösung, mit Wasser und mit wäßriger Salzsäure ausgeschüttelt, die organische Phase eingeengt, der Rückstand (14 g) in 500 ml Tetrahydrofuran/Methanol (1:1) gelöst, mit Pd/C (10 %) versetzt und an der Schüttelente hydriert. Nach beendeter Wasserstoff-Aufnahme wurde vom Katalysator abgesaugt, das Filtrat eingeengt und der Rückstand mit Ethylacetat an Kieselgel chromatographiert. Entsprechende Fraktionen wurden eingeengt und der Rückstand mit Diisopropylether zur Kristallisation gebracht. Man erhielt 2,1 g der Titelverbindung als farblose Substanz, Fp. 63-65°C.

### Beisiel 147

3-Methoxypyridin-2-carbonsäure-N-(((1-octyloxy)carbonyl)methyl)amid

2,5 g (13 mmol) 3-Methoxypyridin-2-carbonsäure-Hydrochlorid (Fp. 170°C u. Zers. (aus Ethylacetat)), 5,5 ml (45 mmol) N-Ethylmorpholin, 2 g (15 mmol) 1-Hydroxy-1H-benzotriazol, 4,7 g (13 mmol) Glycinoctylestertosylat (am Wasserabscheider mit Toluol aus Glycin, Octanol und p-Tos OH hergestellt) und 6,3 g (15 mmol) CMC (vgl. Beispel 146) wurden 48 h in 350 wasserfreiem Dichlormethan gerührt. Nach analoger Aufarbeitung wie in Beispiel 146) wurde das Rohprodukt mit Dichlormethan (im Verlauf bis zu 2,5 % Methanol-Zusatz) an Kieselgel chromatographiert. Man erhielt 3,6 g der farblosen, öligen Titelverbindung, ¹H-NMR (CDCl₃): δ = 4,26 (d, CH₂-Glycin).

### Beispiel 148

3-Methoxypyridin-2-carbonsäure-N-(((1-hexyloxy)carbonyl)methyl)amid

### Beispiel 149

3-Methoxypyridin-2-carbonsäure-N-(((1-butyloxy)carbonyl)methyl)amid

Die Titelverbindung wurde analog Beispel 147 mit Glycin-1-butylester-tosylat erhalten, Fp. 60-62°C (aus Dichlormethan).

### Beispiel 150

3-Methoxypyridin-2-carbonsäure-N-(((2-nonyloxy)carbonyl)methyl)amid-Racemat

2,5 g (10 mmol) 3-Methoxypyridin-2-carbonsäure-N-(carboxymethyl)amid-Hydrochlorid (Fp. 157°C unter Gasentwicklung) wurden in 100 ml wasserfreiem Tetrahydrofuran suspendiert, mit 1,6 ml (12 mmol) Triethylamin, sodann unter Rühren tropfenweise mit 2,4 g Pivaloylchlorid, gelöst in wenig Tetrahydrofuran, versetzt (Temperaturanstieg auf 35-40°C).
Nach 30 min wurde i. Vak. eingeengt, der rötlich gefärbte Rückstand in 100 ml wasserfreiem Tetrahydrofuran aufgenommen, mit 1,6 ml Triethylamin versetzt, und sodann bei 20°C 30 ml einer Lösung von Na-2-nonanolat in 2-Nonanol (hergestellt aus 30 ml 2-Nonanol und 0,8 g (20 mmol) NaH) hinzugegeben. Nach 1 h wurde i. Vak. eingeengt, der Rückstand mit Dichlormethan versetzt, mit 2N wäßriger Ammoniumchlorid-Lösung ausgeschüttelt, die organische Phase getrocknet, i. Vak, eingeengt und der Rückstand (9 g) mit Ethylacetat an Kieselgel chromatographiert. Man erhielt 1,1 g der öligen farblosen Titelverbindung, ¹H-NMR (DMSO): δ = 3,95 (d, CH₂-Glycin).

### Beispiel 151

3-Methoxypyridin-2-carbonsäure-N-(((4-heptyloxyl)carbonyl)methyl)amid

2,5 g (10 mmol) 3-Methoxypyridin-2-carbonsäure-N-(carboxymethyl)amid-Hydrochlorid wurden wie in Beispiel 150 behandelt und sodann bei 20°C mit 140 ml einer Lösung von Na-4-heptanolat in 4-Heptanol (hergestellt aus 140 ml 4-Heptanol und 0,6 g (25 mmol) Natrium, Ultraschallbad) versetzt. Nach 30 min wurde 1 h auf 70-80°C erhitzt, nach dem Abkühlen i. Vak. eingeengt, der Rückstand in Wasser aufgenommen, mit Dichlormethan extrahiert, die organische Phase i. Vak. eingeengt und an der Ölpumpe getrocknet. Das ölige Rohprodukt kristallisiert nach ca. 15 h, Fp. 75-78°C.

### Beispiel 152

3-Benzyloxypyridin-2-carbonsäure-N-(((1-octyloxy)carbonyl)methyl)amid

1,1 g (5 mmol) 3-Benzyloxypyridin-2-carbonsäure wurden analog Beispiel 147 mit Glycin-1-octylester-tosylat kondensiert. Man erhielt ohne Säulenchromatographie 1,3 g der Titelverbindung als hellbraunes Öl, ¹H-NMR (DMSO): δ = 5,24 (s, CH₂-Benzyl).

### Beispiel 153

3-Benzyloxypyridin-2-carbonsäure-N-(((1-butyloxy)carbonyl)methyl)amid

Analog Beispiel 152 wurde mit Glycin-1 butylester-tosylat die Titelverbindung erhalten. Nach Extrahieren der organischen Phase mit gesättigter wäßriger Na-bicarbonat-Lösung, mit 1 N Salzsäure und mit Wasser, Trocknen und Einengen wurde der Rückstand mit Ether/Petrolether zur Kristallisation gebracht, Fp. 55-58°C.

### Beispiel 154

5-(((3-(1-Butyloxy)propyl)amino)carbonyl)-3-methoxypyridin-2-carbonsäure-N-((benzyloxycarbonyl)methyl)amid
a) 5-(((3-(1-Butyloxy)propyl)amino)carbonyl)-3-methoxypyridin-2-carbonsäuremethylester
   Zu 2,1 g (10 mmol) 5-Carboxy-3-methoxypyridin-2-carbonsäuremethylester in 100 ml wasserfreiem Tetrahydrofuran wurden unter Rühren bei 10°C 1,7 ml Oxalylchlorid (20 mmol), sowie 2 Tropfen N,N-Dimethylformamid, gelöst in Tetrahydrofuran, hinzugetropft, die Reaktionsmischung 30 min bei 10°C und 1 h bei 20°C gerührt. Dann wurde eingeengt, der Rückstand in Dichlormethan gelöst bei 0°C mit 6,8 ml (50 mmol) Triethylamin und sodann mit 1,3 g (1,5 ml, 10 mmol) 3-Butoxypropylamin, gelöst in Dichlormethan, versetzt.
   Nach 30 min ließ man auf Raumtemperatur erwärmen, extrahierte mit Wasser, Na-bicarbonat-Lösung und wäßriger 1N HCl, trocknete die organische Phase, engte ein und brachte den Rückstand mit Diethylether/Petrolether (3:1) zur Kristallisation. Man erhielt 2,3 g Produkt Fp. 51-53°C.
b) Die vorstehende Substanz wurde nach Standard-Verfahren verseift und 1,5 g (5 mmol) der amorphen, an der Ölpumpe getrockneten, 5-((3-(1-Butyloxy)propyl)amino)carbonyl)-3-methoxypyridin-2-carbonsäure mit Glycinbenzylester-Tosylat, N-Ethylmorpholin, 1-Hydroxy-1H-benzotriazol und CMC (wie beschrieben) umgesetzt.
   1,42 g der Titelverbindung wurden mit Aceton zur Kristallisation gebracht, Fp. 97-99°C

### Beispiel 155

5-(((3-(1-Butyloxy)propyl)amino)carbonyl)-3-methoxypyridin-2-carbonsäure-N-(((1-butyloxy)carbonyl)methyl)amid

Die Titelverbindung wurde analog dem vorstehenden Beispiel mit Glycin-1-butylester-Tosylat (Fp. 80-82°C (aus Toluol)) erhalten,
Fp. 115-117°C (aus Diisopropylether).

### Beispiel 156

5-(((3-Lauryloxy)propyl)amino)carbonyl)-3-methoxypyridin-2-carbonsäure-N-(((benzyloxy)carbonyl)methyl)amid

Analog Beispiel 154 wurde die Titelverbindung mit 3-Lauryloxypropylamin erhalten, Fp. ab 109-111°C (aus Diisopropylether)

### Beispiel 157

5-(((2-Methoxyethyl)amino)carbonyl)-3-methoxypyridin-2-carbonsäure-N-((benzoxylcarbonyl)methyl)amid

Die Titelverbindung wurde analog Beispiel 154 mit 2-Methoxyethylamin hergestellt.
a) 5-(((2-Methoxyethyl)amino)carbonyl)-3-methoxypyridin-2-carbonsäure
   Fp. 160-161°C (unter Gasentwicklung, aus Ethylacetat)
b) Die Titelverbindung wurde mit Diisopropylether zur Kristallisation gebracht, Fp. 129-131°C.

Die folgenden Beispiele wurden analog zu den Beispielen Nr. 146-157 hergestellt.

### Beispiel 158

3-Methoxypyridin-2-carbonsäure-N-(((2-propyloxy)carbonyl)methyl)amid

### Beispiel 159

3-Methoxypyridin-2-carbonsäure-N-(((1-propyloxy)carbonyl)methyl)amid

### Beispiel 160

3-Methoxypyridin-2-carbonsäure-N-(((1-pentyloxy)carbonyl)methyl)amid

### Beispiel 161

3-Methoxypyridin-2-carbonsäure-N-(((3-pentyloxy)carbonyl)methyl)amid

### Beispiel 162

3-Methoxypyridin-2-carbonsäure-N-(((1-decyloxy)carbonyl)methyl)amid

### Beispiel 163

3-Methoxypyridin-2-carbonsäure-N-(((1-dodecyloxy)carbonyl)methyl)amid

### Beispiel 164

3-Methoxypyridin-2-carbonsäure-N-(((1-geranyloxy)carbonyl)methyl)amid

### Beispiel 165

3-Benzyloxypyridin-2-carbonsäure-N-(((2-propyloxy)carbonyl)methyl)amid

### Beispiel 166

3-Benzyloxypyridin-2-carbonsäure-N-(((3-pentyloxy)carbonyl)methyl)amid

### Beispiel 167

3-Benzyloxypyridin-2-carbonsäure-N-(((-1-pentyloxy)carbonyl)methyl)amid

### Beispiel 168

3-Benzyloxypyridin-2-carbonsäure-N-(((1-dodecyloxy)carbonyl)methyl)amid

### Beispiel 169

3-Benzyloxypyridin-2-carbonsäure-N-(((1-geranyloxy)carbonyl)methyl)amid

### Beispiel 170

5-(((3-(1-Butyloxy)propyl)amino)carbonyl)-3-methoxypyridin-2-carbonsäure-N-(((1-hexyloxy)carbonyl)methyl)amid

### Beispiel 171

5-(((3-(1-Butyloxy)propyl)amino)carbonyl)-3-methoxypyridin-2-carbonsäure-N-(((hex-3-en-1-yloxy)carbonyl)methyl)amid

### Beispiel 172

5-(((3-(1-Butyloxy)propyl)amino)carbonyl)-3-methoxypyridin-2-carbonsäure-N-(((1-octyloxy)carbonyl)methyl)amid

### Beispiel 173

5-(((3-(1-Butyloxy)propyl)amino)carbonyl)-3-methoxypyridin-2-carbonsäure-N-(((1-decyloxy)carbonyl)methyl)amid

### Beispiel 174

5-(((3-(1-Butyloxy)propyl)amino)carbonyl)-3-methoxypyridin-2-carbonsäure-N-(((1-dodecyloxy)carbonyl)methyl)amid

### Beispiel 175

5-(((3-(1-Butyloxy)propyl)amino)carbonyl)-3-methoxypyridin-2-carbonsäure-N-(((3-methyl-1-butyloxy)carbonyl)methyl)amid

### Beispiel 176

5-(((3-(1-Butyloxy)propyl)amino)carbonyl)-3-methoxypyridin-2-carbonsäure-N-(((1-geranyloxy)carbonyl)methyl)amid

### Beispiel 177

5-(((4-(1-Butyloxy)phenyl)amino)carbonyl)-3-chlorpyridin-2-carbonsäure-N-((ethyloxycarbonyl)methyl)amid
a) 5-Carboxy-3-chlorpyridin-2-carbonsäuremethylester wurde analog Beispiel 90a) hergestellt, Fp. 182-184°C (aus wäßriger Salzsäure).
b) 5-(((4-(1-Butyloxy)phenyl)amino)carbonyl)-3-chlorpyridin-2-carbonsäuremethylester wurde aus der obigen Verbindung mit Oxalylchlorid und 4-(1-Butyloxy)anilin erhalten, Fp. 121-123°C (aus Diethylether).
c) 5-(((4-(1-Butyloxy)phenyl)amino)carbonyl)-3-chlorpyiridin-2-carbonsäure durch Verseifung des Produkts aus b), Fp. 163-164°C (aus wäßriger Salzsäure).
d) Die Titelverbindung wurde analog Beispiel 90 b) aus der vorstehenden Substanz durch Kondensation (N-Ethylmorpholin, 1-Hydroxy-1H-benzotriazol, CMC) mit Glycinethylester-Hydrochlorid erhalten, Fp. 177-179°C (aus Ethanol).

### Beispiel 178

3-(4-Chlorbenzyloxy)pyridin-2-carbonsäure-N-(((ethyloxy)carbonyl)methyl)amid-1-oxid

0,7 g (2 mmol) der Titelverbindung aus Beispiel 31 wurden in Dichlormethan gelöst und mit 1,41 g 3-Chlorperbenzoesäure umgesetzt. Nach 1 h Rühren bei 20°C wurde Ammoniak eingeleitet bis keine Fällung mehr auftrat, abfiltriert, das Filtrat eingeengt und der ölige Rückstand mit Diethylether zur Kristallisation gebracht, Fp. 70-72°C.

### Beispiel 179

5-Methoxycarbonyl-3-(2-methyl-1-propyloxy)-pyridin-2-carbonsäure-(glycylbenzylester)amid
a) 3-(2-Methyl-1-propyloxy)-pyridin-2,5-dicarbonsäure
   Analog Beispiel 81 c) wurden 3,5 g (146 mmol) Natrium in 350 ml 2-Methyl-1-propanol (iso-Butylalkohol) gelöst und unter Rühren bei 20°C mit 13,7 g (55 mmol) 3-Chlorpyridin-2-carbonsäureethylester-5-carbonsäuremethylester (analog zu Beispiel 81 b) hergestellt) versetzt. Dann wurde 90 Minuten bei 80°C gerührt, nach dem Abkühlen im Vakuum eingeengt, der Rückstand in 200 ml 1 N methanolische NaOH aufgenommen und bei 20°C gerührt. Nach 15 Minuten trübte sich die Lösung. Man setzte Wasser hinzu bis eine klare Lösung entstand, rührte 1 Stunde, engte im Vakuum ein, säuerte die wäßrige Lösung mit wäßriger Salzsäure an, saugte das kristalline Produkt ab, wusch, trocknete und erhielt 10,6 g Dicarbonsäure, Fp. 192°C (Zers.).
b) 3-(2-Methyl-1-propyloxy)-pyridin-2,5-dicarbonsäuredimethylester
   Das ölige Produkt wurde aus der vorstehenden Dicarbonsäure unter Veresterungsbedingungen (Methanol/Schwefelsäure) und nach der Aufarbeitung (Waschen mit Wasser, Extraktion mit Ethylacetat) erhalten.
c) 3,2 g (12 mmol) des vorstehenden Diesters wurden in 25 ml Methanol mit 0,48 g (12 mmol) NaOH, gelöst in 50 ml Methanol, versetzt und 90 Minuten bei 65°C gerührt. Dann wurde unter Kühlung mit verdünnter wäßriger Salzsäure angesäuert und im Vakuum vom Methanol befreit. 2,5 g (10 mmol) des so erhaltenen Monoester-Gemischs wurden analog zu Beispiel 90 b) in 250 ml Dichlormethan mit 3,4 g (10 mmol) Glycinbenzylester-Tosylat, 1,4 g (10 mmol) 1-Hydroxy-1H-benztriazol, 2,6 ml (20 mmol) N-Ethylmorpholin und 4,3 g (10 mmol) CMC 24 Stunden bei 20°C gerührt.
   Dann wurde vom Ungelösten abgesaugt, das Filtrat mit wäßriger Na-bicarbonat-Lösung, mit verdünnter Salzsäure und Waser extrahiert, die organische Phase getrocknet, eingeengt und der Rückstand mt n-Heptan/Ethylacetat (1:1) an Kieselgel chromatographiert. Aus entsprechenden Fraktionen wurden 0,8 g der farblosen Titelverbindung erhalten, Fp. 103 bis 105°C. Desweiteren werden 1,1 g des isomeren harzigen Produkts erhalten.

Die Beispiele 180-228 wurden analog hergestellt:

### Beispiel 180

5-Ethoxycarbonyl-3-(2-methyl-1-propyloxy)pyridin-2-carbonsäure(glycylethylester)amid

### Beispiel 181

5-Methoxycarbonyl-3-(3-methyl-1-butyloxy)pyridin-2-carbonsäure(glycylethylester)amid

### Beispiel 182

5-Ethoxycarbonyl-3-ethoxypyridin-2-carbonsäure(glycylethylester)amid

### Beispiel 183

5-Ethoxycarbonyl-3-(1-propyloxy)pyridin-2-carbonsäure(glycylethylester)amid

### Beispiel 184

5-Ethoxycarbonyl-3-(2-propyloxy)pyridin-2-carbonsäure(glycylethylester)amid

### Beispiel 185

3-Benzyloxy-5-ethoxycarbonylpyridin-2-carbonsäure(glycylethylester)amid

### Beispiel 186

3-(4-Chlorbenzyloxy)-5-ethoxycarbonylpyridin-2-carbonsäure(glycylethylester)amid

### Beispiel 187

5-Ethoxycarbonyl-3-(4-fluorbenzyloxy)pyridin-2-carbonsäure(glycyl-1-butylester)amid

### Beispiel 188

5-Ethoxycarbonyl-3-(4-(trifluormethyl)benzyloxy)pyridin-2-carbonsäure(glycyl-1-butylester)amid

### Beispiel 189

5-Ethoxycarbonyl-3-(4-(trifluormethoxy)benzyloxy)pyridin-2-carbonsäure(glycyl-1-butylester)amid

### Beispiel 190

5-Ethoxycarbonyl-3-(4-(2-propyl)benzyloxy)pyridin-2-carbonsäure(glycyl-1-butylester)amid

### Beispiel 191

3-(4-Ethoxybenzyloxy)-5-ethoxycarbonylpyridin-2-carbonsäure(glycyl-1-butylester)amid

### Beispiel 192

5-Ethoxycarbonyl-3-(3,4-dimethoxybenzyloxy)pyridin-2-carbonsäure(glycyl-1-butylester)amid

### Beispiel 193

5-Ethoxycarbonyl-3-(2-(4-fluorphenyl)ethyloxy)pyridin-2-carbonsäureglycyl(1-butylester)amid

### Beispiel 194

5-Ethoxycarbonyl-3-(2,2,2-trifluorethyloxy)pyridin-2-carbonsäureglycyl(1-butylester)amid

### Beispiel 195

5-Carboxy-3-(3-methyl-1-butyloxy)pyridin-2-carbonsäure(glycyl-1-octylester)amid

### Beispiel 196

5-Carboxy-3-ethoxypyridin-2-carbonsäure(glycyl-1-octylester)amid

### Beispiel 197

5-Carboxy-3-propyloxypyridin-2-carbonsäure(glycyl-1-octylester)amid

### Beispiel 198

5-Carboxy-3-(2-propyloxy)pyridin-2-carbonsäure(glycyl-1-octylester)amid

### Beispiel 199

3-Benzyloxy-5-carboxypyridin-2-carbonsäure(glycyl-1-octylester)amid

### Beispiel 200

5-Carboxy-3-(4-chlorbenzyloxy)pyridin-2-carbonsäure(glycyl-1-octylester)amid

### Beispiel 201

5-Carboxy-3-(4-fluorbenzyloxy)pyridin-2-carbonsäure(glycyl-1-octylester)amid

### Beispiel 202

5-Carboxy-3-((4-trifluormethyl)benzyloxy)pyridin-2-carbonsäure(glycyl-1-octylester)amid

### Beispiel 203

5-Carboxy-3-((4-trifluormethoxy)benzyloxy)pyridin-2-carbonsäure(glycyl-1-octylester)amid

### Beispiel 204

5-Carboxy-3-(4-(2-propyl)benzyloxy)pyridin-2-carbonsäure(glycyl-1-octylester)amid

### Beispiel 205

5-Carboxy-3-(naphthyl-2-methyloxy)pyridin-2-carbonsäure-(glycyl-1-butylester)amid

### Beispiel 206

5-Carboxy-3-(naphthyl-1-methyloxy)pyridin-2-carbonsäure-(glycyl-1-butylester)amid

### Beispiel 207

5-(3-Pentyloxy)carbonyl-3-propyloxypyridin-2-carbonsäure(glycyl-1-octylester)amid

### Beispiel 208

5-(3-Pentyloxy)carbonyl-3-(2-propyloxy)pyridin-2-carbonsäure(glycyl-1-octylester)amid

### Beispiel 209

3-Benzyloxy-5-(3-pentyloxy)carbonylpyridin-2-carbonsäure(glycyl-1-octylester)amid

### Beispiel 210

3-(4-Fluorbenzyloxy)-5-(3-pentyloxy)carbonylpyridin-2-carbonsäure-(glycyl-1-butylester)amid

### Beispiel 211

5-(4-Heptyloxy)carbonyl-3-methoxypyridin-2-carbonsäure-(glycyl-1-butylester)amid

### Beispiel 212

3-Benzyloxy-5-(4-heptyloxy)carbonylpyridin-2-carbonsäure(glycyl-1-octylester)amid

### Beispiel 213

3-(4-Chlorbenzyloxy)-5-(4-heptyloxy)carbonylpyridin-2-carbonsäure(glycyl-1-octylester)amid

### Beispiel 214

3-(4-Fluorbenzyloxy)-5-(4-heptyloxy)pyridin-2-carbonsäure(glycyl-1-hexylester)amid

### Beispiel 215

5-(4-Heptyloxy)carbonyl-3-(4-(2-propyl)benzyloxy)pyridin-2-carbonsäure(glycyl-1-butylester)amid

### Beispiel 216

3-Benzyloxy-5-((5-nonyloxy)carbonyl)pyridin-2-carbonsäure(glycyl-1-hexylester)amid

### Beispiel 217

3-(4-Fluorbenzyloxy)-5-(5-nonyloxy)carbonylpyridin-2-carbonsäure(glycyl-1-hexylester)amid

### Beispiel 218

5-(5-Nonyloxy)carbonyl-3-(4-(2-propyl)benzyloxy)pyridin-2-carbonsäure(glycyl-1-butylester)amid

### Beispiel 219

5-Geranyloxycarbonyl-3-(2-methyl-1-propyloxy)pyridin-2-carbonsäure(glycyl-1-butylester)amid

### Beispiel 220

3-Benzyloxy-5-(geranyloxycarbonyl)pyridin-2-carbonsäure(glycyl-1-butylester)amid

### Beispiel 221

3-(4-Fluorbenzyloxy)-5-(geranyloxycarbonyl)pyridin-2-carbonsäure(glycylethylester)amid

### Beispiel 222

5-Geranyloxycarbonyl-3-(3-methoxybenzyloxy)pyridin-2-carbonsäure(glycylethylester)amid

### Beispiel 223

3-Benzyloxy-5-(farnesyloxycarbonyl)pyridin-2-carbonsäure(glycylethylester)amid

### Beispiel 224

5-Farnesyloxycarbonyl-3-(4-fluorbenzyloxy)pyridin-2-carbonsäure(glycyletylester)amid

### Beispiel 225

3-Methoxy-5-(retinyloxycarbonyl)pyridin-2-carbonsäure(glycylethylester)amid

### Beispiel 226

3-Ethoxy-5-(retinyloxycarbonyl)pyridin-2-carbonsäure(glycylethylester)amid

### Beispiel 227

3-Benzyloxy-5-(retinyloxycarbonyl)pyridin-2-carbonsäure(glycylethylester)amid

### Beispiel 228

3-(4-Fluorbenzyloxy)-5-(retinyloxycarbonyl)pyridin-2-carbonsäure(glycyl-1-butylester)amid

### Beispiel 229

5-(((4-n-Butyloxyphenyl)amino)carbonyl)-3-methoxypyridin-2-carbonsäure(glycylethylester)amid
a) 5-(((4-n-Butyloxyphenyl)amino)carbonyl)-3-methoxypyridin-2-carbonsäuremethylester
   3,2 g (15 mmol) 5-Carboxy-3-methoxypyridin-2-carbonsäuremethylester (vgl. Beispiel 90 a)) wurden analog Beispiel 90 b) mit 2,5 g (15 mmol) 4-n-Butoxyanilin und den dort beschriebenen Reagenzien zur Reaktion gebracht. 3,9 g Produkt wurden mit Diethylether zur Kristallisation gebracht (Fp. 138-141°C).
b) 5-(((4-n-Butyloxyphenyl)amino)carbonyl)-3-methoxypyridin-2-carbonsäure
   3,2 g des vorstehenden Esters wurden mit in 100 ml 1,5 N methanolischer Natronlauge bei 20°C verseift. Man erhielt 2,7 g Produkt aus wäßriger Salzsäure, Fp. 128-130°C, Sintern ab 120°C).
c) 5-(((4-n-Butyloxyphenyl)amino)carbonyl)-3-methoxypyridin-2-carbonsäure-N-(ethoxycarbonylmethyl)amid
   Die Titelverbindung wurde wie folgt hergestellt:
   2,7 g (7,8 mmol) der vorstehenden Pyridin-2-carbonsäure wurden in 500 ml wasserfreiem Dichlormethan mit 1,1 g (7,8 mmol) Glycinethylester-Hydrochlorid, 3,0 ml (23,4 mmol) N-Ethylmorpholin, 1,2 g (8,6 mmol) 1-Hydroxy-1H-benztriazol und 3,3 g (7,8 mmol) CMC 24 h bei 20°C gerührt. Dann wurde vom Ungelösten abfiltriert, die org. Phase nacheinander mit je 200 ml Wasser, wäßriger Na-bicarbonat-Lösung, 1N wäßriger Salzsäure und Wasser extrahiert, über Mg-sulfat getrocknet, i. Vak. eingeengt und der Rückstand mit Diethylether zur Kristallisation gebracht. Man erhielt 2,4 g Produkt, Fp. 193-195°C.

### Beispiel 230

5-(((4-(1-Hexyloxy)phenyl)amino)carbonyl)-3-methoxypyridin-2-carbonsäure-((N-ethoxycarbonyl)methyl)amid
a) 5-(((4-n-Hexyloxyphenyl)amino)carbonyl)-3-methoxypyridin-2-carbonsäuremethylester wurde aus 5-Carboxy-3-methoxypyridin-2-carbonsäuremethylester und 4-Hexyloxyanilin hergestellt, Fp. 118-119°C (aus Diethylether).
b) 5-(((4-n-Hexyloxyphenyl)amino)carbonyl)-3-methoxypyridin-2-carbonsäure, 160-162°C, sintern bei 148°C (aus wäßriger Salzsäure/Tetrahydrofuran)
c) Die Titelverbindung wurde in Analogie zu Beispiel 231 c) aus 4,2 g der vorstehenden Verbindung erhalten. 4,0 g Produkt wurden mit Ethylacetat zur Kristallisation gebracht, Fp. 157-159°C.

### Beispiel 231

5-(((4-n-Decylphenyl)amino)carbonyl)-3-methoxypyridin-2-carbonsäure-(N-ethoxycarbonylmethyl)amid wurde aus 5-(((-4-n-Decylphenyl)amino)carbonyl)-3-methoxypyridin-2-carbonsäure (Fp. 160°C, Zers.; aus wäßriger Salzsäure/THF) und Glycinethylester-Hyrochlorid hergestellt, Fp. 155-157°C (aus Diisopropylether).

Die Beispiele Nr. 232 bis 240 wurden analog den Beispielen 229 bis 231 hergestellt.

### Beispiel 232

5-(((4-Ethoxyphenyl)amino)carbonyl-3-methoxypyridin-2-carbonsäure(glycyl-1-octylester)amid

### Beispiel 233

5-(((4-Ethoxyphenyl)amino)carbonyl)-3-benzyloxypyridin-2-carbonsäure(glycyl-1-octylester)amid

### Beispiel 234

5-(((4-n-Butyloxyphenyl)amino)carbonyl)-3-(4-fluorbenzyloxy)pyridin-2-carbonsäure(glycyl-1-butylester)amid

### Beispiel 235

5-(((4-n-Butyloxyphenyl)amino)carbonyl)-3-benzyloxypyridin-3-carbonsäure(glycyl-1-butylester)amid

### Beispiel 236

5-(((4-(1-Hexyloxy)phenyl)amino)carbonyl)-3-methoxypyridin-2-carbonsäure(glycyl-1-octylester)amid

### Beispiel 237

5-(((4-n-Decyloxyphenyl)amino)carbonyl)-3-methoxypyridin-2-carbonsäure-N-(((1-butyloxy)carbonyl)methyl)amid

### Beispiel 238

5-(((4-Geranyloxyphenyl)amino)carbonyl)-3-methoxypyridin-2-carbonsäure-N-((-1-hexyloxy)carbonyl)methyl)amid

### Beispiel 239

5-(((4-n-Octyloxyphenyl)amino)carbonyl)-3-methoxypyridin-2-carbonsäure-N-((-1-butyloxy)carbonyl)methyl)amid

### Beispiel 240

5-(((4-n-Octyloxyphenyl)amino)carbonyl)-3-methoxypyridin-2-carbonsäure-N-(((1-hexyloxy)carbonyl)methyl)amid

### Beispiel 241

5-Farnesyloxycarbonyl-3-methoxypyridin-2-carbonsäure(glycyl-1-butylester)amid

### Beispiel 242

5-Geranyloxycarbonyl-3-methoxypyridin-2-carbonsäure(glycylethylester)amid

### Beispiel 243

5-(Farnesyloxymethyl)-3-methoxypyridin-2-carbonsäure(glycylethylester)amid

### Beispiel 244

5-(Geranyloxymethyl)-3-methoxypyridin-2-carbonsäure(glycyl-1-butylester)amid

### Beispiel 245

5-Retinyloxymethyl-3-methoxypyridin-2-carbonsäure(glycyl-1-butylester)amid

### Beispiel 246

5-Retinyloxymethyl-3-(2-propyloxy)pyridin-2-carbonsäure(glycyl-1-butylester)amid

### Beispiel 247

5-(1-Butoxymethyl)-3-methoxypyridin-2-carbonsäure-N-(((1-octyloxy)carbonyl)methyl)amid

### Beispiel 248

5-(n-Hexyloxymethyl)-3-methoxypyridin-2-carbonsäure-N-(((1-octyloxy)carbonyl)methyl)amid

### Beispiel 249

5-(n-Octyloxymethyl)-3-methoxypyrdin-2-carbonsäure-N-(((1-hexyloxy)carbonyl)methyl)amid

### Beispiel 250

5-((1-Hex-3-enyloxy)methyl)-3-methoxypyridin-2-carbonsäure-N-(((1-octyloxy)carbonyl)methyl)amid

### Beispiel 251

5-(n-Decyloxymethyl)-3-methoxypyridin-2-carbonsäure-N-(((1-butyloxy)carbonyl)methyl)amid

### Beispiel 252

5-(n-Dodecyloxymethyl)-3-methoxypyridin-2-carbonsäure-N-(((1-butyloxy)carbonyl)methyl)amid

### Beispiel 253

3-(4-((+)-Dehydroabietylamino)carbonyl)benzyloxy)pyridin-2-carbonsäure-N-((ethylcarbonyl)methyl)amid
Fp. ca. 80°C (amorphe Substanz, aus Ethylacetat)

### Beispiel 254

N-(3-Benzyloxypyridyl-2-carbonyl)alaninethylester-Racemat
¹H-NMR (CDCl₃): δ = 5.13 (s, CH₂).

### Beispiel 255

N-(3-Benzyloxypyridyl-2-carbonyl)-L-phenylalanin-tert.-butylester
¹H-NMR (CDCl₃): δ = 5.12 (s, CH₂).

### Beispiel 256

N-(3-Benzyloxypyridyl-2-carbonyl)glycinmethylester,
Fp. 81-82°C (aus Ethylacetat).

### Beispiel 257

5-((1-Butyloxy)carbonyl)-3-methoxypyridin-2-carbonsäure-N-((tert.butyloxycarbonyl)methyl)amid
a) 3-Methoxypyridin-2,5-dicarbonsäure-di-(1-butyl)ester
   5,0 g 3-Methoxypyridin-2,5-dicarbonsäure-dimethylester (vgl. Beispiel 90 a)) wurden in 100 ml 1-Butanol gelöst, mit 1,5 ml konz. Schwefelsäure versetzt und 2 h zum Sieden erhitzt, wobei ein Teil des Lösungsmittels abdestilliert wurde. Nach dem Abkühlen wurde i.Vak. eingeengt, der Rückstand in Dichlormethan aufgenommen, mit gesättigter wäßriger Na-bicarbonat-Lösung extrahiert, die organische Phase getrocknet und eingeengt, 6 g öliges Rohprodukt.
b) Bis[5-((1-butyloxy)carbonyl)-3-methoxypyridin-2-carbonsäure]-Cu-II-komplex
   6 g (20 mmol) des vorstehenden öligen Produkts wurden, in 10 ml Methanol gelöst, zu einer Lösung von 4,8 g (20 mmol) Cu-II-nitrat x 3 H₂O in 100 ml Methanol gegeben und 4 h zum Sieden erhitzt. Dann wurde auf 0-5°C abgekühlt, die kristalline Fällung abgesaugt und mit Diethylether gewaschen. Man erhielt 4,2 g blau-grünliches Produkt, Fp. 267°C (unter Zersetzung).
c) 5-((1-Butyloxy)carbonyl)-3-methoxypyridin-2-carbonsäure
   4 g des vorstehenden Cu-Komplexes wurden in 75 ml 1,4-Dioxan suspendiert. Unter Rühren leitete man 30 min H₂S-Gas ein, saugte den ausgefallenen Niederschlag (CuS) über Kieselgur ab, wusch mit 1,4-Dioxan nach (weiteres H₂S-Einleiten ergab keine weitere Fällung) und engte das Filtrat i.Vak. ein. Der Rückstand wurde mit Petrolether zur Kristallisation gebracht, Fp. 96-98°C.
d) Die Titelverbindung wurde erhalten, indem 0,76 g (3 mmol) der vorstehenden Pyridincarbonsäure mit 0,52 g (3 mmol) Glycin-tert.butylester-Hydrochlorid, 1,2 ml (9 mmol) N-Ethylmorpholin, 0,45 g (3,3 mmol) 1-Hydroxy-1H-benzotriazol und 1,3 g (3 mmol) CMC kondensiert wurden. Man erhielt 0,8 g farblos kristallines Produkt, Fp. 50-52°C (aus Petrolether).

### Beispiel 258

5-((1-Butyloxy)carbonyl)-3-methoxypyridin-2-carbonsäure-N-(((1-butyloxy)carbonyl)methyl)amid

Die Titelverbindung wurde aus der in Beispiel 257c) beschriebenen Pyridin-2-carbonsäure durch Kondensation mit Glycin-(1-butyl)ester-Tosylat hergestellt, Fp. 80-81°C (aus Petrolether).

### Beispiel 259

5-((1-Hexyloxy)carbonyl)-3-methoxypyridin-2-carbonsäure-N-(((1-butyloxy)carbonyl)methyl)amid
a) Bis[5-((1-hexyloxycarbonyl)-3-methoxypyridin-2-carbonsäure]-Cu-II-Komplex
   6,6 g (18 mmol) 3-Methoxypyridin-2,5-dicarbonsäure-di-(1-hexyl)-ester (analog Bsp. 257 a) durch sauer-katalysierte Umesterung mit 1-Hexanol erhalten) wurden analog Bsp. 257 b) umgesetzt. Man erhielt 4,6 g Cu-II-Komplex, Fp. 265°C (Zers., mit Diethylether gewaschen).
b) 5-((1-Hexyloxy)carbonyl)-3-methoxypyridin-2-carbonsäure wurde analog Beispiel 257 c) aus dem vorstehenden Cu-II-Komplex erhalten; 3,4 g, Fp. 108-110°C (aus Petrolether).
c) Die Titelverbindung wurde aus 0,71 g (2,5 mmol) der vorstehenden Säure und 0,76 g (2,5 mmol) Glycin-1-butylester-Tosylat mit N-Ethyl-morpholin, 1-Hydroxy-1H-benzotriazol und CMC erhalten. Man isolierte 0,81 g Produkt, Fp. 53-55°C (aus Petrolether).

Die Beispiele 260 bis 287 wurden analog den Beispielen 257 bis 260 erhalten.

### Beispiel 260

5-((1-Butyloxy)carbonyl)-3-methoxypyridin-2-carbonsäure-N-((ethyloxycarbonyl)methyl)amid

### Beispiel 261

5-((1-Hexyloxy)carbonyl)-3-methoxypyridin-2-carbonsäure-N-((ethyloxycarbonyl)methyl)amid

### Beispiel 262

3-Methoxy-5-((1-pentyloxy)carbonyl)pyridin-2-carbonsäure-N-((ethyloxycarbonyl)methyl)amid

### Beispiel 263

5-((1-Heptyloxy)carbonyl)-3-methoxypyridin-2-carbonsäure-N-((ethyloxycarbonyl)methyl)amid

### Beispiel 264

3-Methoxy-5-((1-octyloxy)carbonyl)pyridin-2-carbonsäure-N-((ethyloxycarbonyl)methyl)amid

### Beispiel 265

5-(Ethyloxycarbonyl)-3-methoxypyridin-2-carbonsäure-N-(((1-butyloxy)carbonyl)methyl)amid

### Beispiel 266

5-(Ethyloxycarbonyl)-3-methoxypyridin-2-carbonsäure-N-((ethyloxycarbonyl)methyl)amid

### Beispiel 267

3-Methoxy-5-((1-propyloxy)carbonyl)pyridin-2-carbonsäure-N-((ethyloxycarbonyl)methyl)amid

### Beispiel 268

3-Methoxy-5-((1-pentyloxy)carbonyl)pyridin-2-carbonsäure-N-(((1-butyloxy)carbonyl)methyl)amid

### Beispiel 269

5-((1-Heptyloxy)carbonyl)-3-methoxypyridin-2-carbonsäure-N-(((1-butyloxy)carbonyl)methyl)amid

### Beispiel 270

3-Methoxy-5-((1-octyloxy)carbonyl)ypyridin-2-carbonsäure-N-(((1-butyloxy)carbonyl)methyl)amid

### Beispiel 271

5-(Ethyloxycarbonyl)-3-methoxypyridin-2-carbonsäure-N-(((1-hexyloxy)carbonyl)methyl)amid

### Beispiel 272

5-((1-Butyloxycarbonyl)-3-methoxypyridin-2-carbonsäure-N-(((1-hexyloxy)carbonyl)methyl)amid

### Beispiel 273

5-((1-Hexyloxycarbonyl)-3-methoxypyridin-2-carbonsäure-N-(((1-hexyloxy)carbonyl)methyl)amid

### Beispiel 274

3-Methoxy-5-((1-pentyloxy)carbonyl)pyridin-2-carbonsäure-N-(((1-hexyloxy)carbonyl)methyl)amid

### Beispiel 275

5-((1-Heptyloxy)carbonyl)-3-methoxypyridin-2-carbonsäure-N-(((1-hexyloxy)carbonyl)methyl)amid

### Beispiel 276

3-Methoxy-5-((1-octyloxy)carbonyl)pyridin-2-carbonsäure-N-(((1-hexyloxy)carbonyl)methyl)amid

### Beispiel 277

5-((1-Butyloxy)carbonyl)-3-methoxypyridin-2-carbonsäure-N-(((1-octyloxy)carbonyl)methyl)amid

### Beispiel 278

5-((1-Hexyloxy)carbonyl)-3-methoxypyridin-2-carbonsäure-N-(((1-octyloxy)carbonyl)methyl)amid

### Beispiel 279

3-Methoxy-5-((1-pentyloxy)carbonyl)pyridin-2-carbonsäure-N-(((1-octyloxy)carbonyl)methyl)amid

### Beispiel 280

5-((1-Heptyloxy)carbonyl)-3-methoxypyridin-2-carbonsäure-N-(((1-octyloxy)carbonyl)methyl)amid

### Beisiel 281

3-Methoxy-5-((1-octyloxy)carbonyl)pyridin-2-carbonsäure-N-(((1-octyloxy)carbonyl)methyl)amid

### Beispiel 282

5-(Ethyloxycarbonyl)-3-(2-propyloxy)pyridin-2-carbonsäure-N-(((1-butyloxy)carbonyl)methyl)amid

### Beispiel 283

5-((1-Butyloxy)carbonyl)-3-(2-propyloxy)pyridin-2-carbonsäure-N-(((1-butyloxy)carbonyl)methyl)amid

### Beispiel 284

5-((1-Hexyloxy)carbonyl)-3-(2-propyloxy)pyridin-2-carbonsäure-N-(((1-butyloxy)carbonyl)methyl)amid

### Beispiel 285

5-((1-Octyloxy)carbonyl)-3-(2-propyloxy)pyridin-2-carbonsäure-N-(((1-propyloxy)carbonyl)methyl)amid

### Beispiel 286

5-((1-Octyloxy)carbonyl)-3-(2-propyloxy)pyridin-2-carbonsäure-N-(((1-butyloxy)carbonyl)methyl)amid

### Beispiel 287

5-((1-Octyloxy)carbonyl)-3-(2-propyloxy)pyridin-2-carbonsäure-N-(((1-hexyloxy)carbonyl)methyl)amid

### Beispiel 288

5-Methoxycarbonyl-3-(methylthio)pyridin-2-carbonsäure-N-(((1-butyloxy)carbonyl)methyl)amid
a) 3-(Methylthio)pyridin-2,5-dicarbonsäure
   4,6 g (12 mmol) 3-Chlorpyridin-2,5-dicarbonsäuredibenzylester wurden unter Rühren bei 20°C in 30 ml Dimethylsulfoxid gelöst und mit 5,0 g (70 mmol) Natriumthiomethanolat versetzt, wobei sich die Temperatur auf 80°C erhöhte. Man erhitzte 1 h auf 140°C, kühlte das Reaktionsgemisch ab, versetzte mit Wasser, trennte die ölige Schicht ab, versetzte die wäßrige DMSO-Phase mit konz. Salzsäure (pH 1) und saugte das ausgefallene Produkt ab. Man erhielt 2,8 g gelbes kristallines Produkt, Fp. 223°C (unter Zersetzung).
b) 3-(Methylthio)pyridin-2,5-dicarbonsäuredimethylester
   2,8 g der vorstehenden Verbindung wurden in 150 ml Methanol mit 50 ml 1,4-Dioxan, 40 ml Tetrahydrofuran und 0,5 konz. Schwefelsäure versetzt und 2 h zum Rückfluß erhitzt, wobei Lösung eintrat. Nach dem Abkühlen wurde i. Vak. eingeengt, der Rückstand mit 100 ml wäßriger Na-bicarbonat-Lösung versetzt, mit Dichlormethan extrahiert, die org. Phase getrocknet und eingeengt. Man erhielt 1,4 g des gelben, kristallinen Produkts, Fp. 103-105°C.
c) 5-Methoxycarbonyl-3-(methylthio)pyridin-2-carbonsäure-Cu-II-Komplex 1,3 g des vorstehenden 3-(Methylthio)pyridin-2,5-dicarbonsäuredimethylesters wurden analog Beispiel 257 b) umgesetzt. Man erhielt 1,3 g grünlich-kristallines Produkt, Fp. > 330°C.
d) 5-Methoxycarbonyl-3-(methylthio)pyridin-2-carbonsäure
   1,3 g der vorstehenden Verbindung wurden analog Beispiel 257 c) umgesetzt, 0,72 g Produkt, Fp. 183-185°C.
e) Die Titelverbindung wurde erhalten, indem 0,68 g (3 mmol) der vorstehenden Pyridincarbonsäure mit 0,91 g (3 mmol) Glycin-1-butylester-Tosylat kondensiert wurden (1-Hydroxy-1H-benzotriazol, N-Ethylmorpholin, CMC). Man erhielt 0,57 g blaßgelbes Produkt, Fp. 47-49°C (aus Petrolether).

### Beispiel 289

3-Methoxychinolin-2-carbonsäure-N-((methoxycarbonyl)methyl)amid
a) 2-Acetyl-3-hydroxychinolin, bekannt aus D.W. Bayne et al., J. Chem. Soc. Chem. Comm. 1975, 782, Fp. 106°C (aus wäßriger Salzsäure).
b) 2-Acetyl-3-methoxychinolin mit Kaliumcarbonat/Methyljodid in Aceton aus a), öliges Rohprodukt.
c) 3-Methoxychinolin-2-carbonsäure mit Kaliumhypochlorit in Wasser/Dioxan aus b), Fp. 123°C (aus Methyl-tert.butylether).
d) Die Titelverbindung wurde mit DCC, HOBT, THF, NEM und Glycinmethylester-Hydrochlorid aus c) erhalten, ¹H-NMR (DMSO): δ = 4,08 (d, CH₂-Glycin).

## Patentansprüche

1. Verbindungen der allgemeinen Formel I 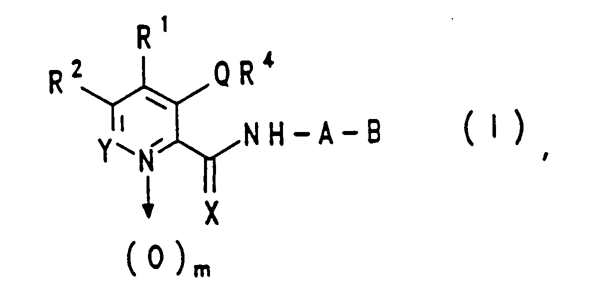 in welcher
Q O, S, NR' oder eine Bindung,
X O und S,
Y C-R³ oder,
falls R¹ und R² einen Cyclus bilden,
Y N oder CR³ bedeutet,
m 0 und 1,
A (C₁-C₄)-Alkylen, das gegebenenfalls substituiert ist mit einem oder zwei Substituenten aus der Reihe Halogen, Cyano, Nitro, Trifluormethyl, (C₁-C₆)-Alkyl, (C₁-C₆)-Hydroxyalkyl, (C₁-C₆)-Alkoxy, -O-[CH₂]ₓ-C_{f}H_{(2f+1-g)}Hal_{g}, vorzugsweise (C₁-C₈)-Fluoralkoxy, (C₁-C₈)-Fluoralkenyloxy, (C₁-C₈)-Fluoralkinyloxy, -OCF₂Cl oder -O-CF₂-CHFCl, (C₁-C₆)-Alkylmercapto, (C₁-C₆)-Alkylsulfinyl, (C₁-C₆)-Alkylsulfonyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Phenyl, Benzyl, Phenoxy, Benzyloxy, Anilino, N-Methylanilino, Phenylmercapto, Phenylsulfonyl, Phenylsulfinyl, Sulfamoyl, N-(C₁-C₄)-Alkylsulfamoyl, N,N-Di-(C₁-C₄)-alkylsulfamoyl, oder
mit einem substituierten (C₆-C₁₂)-Aryloxy-, (C₇-C₁₁)-Aralkyloxy, (C₆-C₁₂)-Aryl- oder (C₇-C₁₁)-Aralkyl-Rest, der im Arylteil 1, 2, 3, 4 oder 5 gleiche oder verschiedene Substituenten aus der Reihe Halogen, Cyano, Nitro, Trifluormethyl, (C₁-C₆)-Alkyl, (C₁-C₆)-Alkoxy, -O-[CH₂]ₓ-C_{f}H_{(2f+1-g)}Hal_{g}, -OCF₂Cl,-O-CF₂-CHFCl, (C₁-C₆)-Alkylmercapto, (C₁-C₆)-Alkylsulfinyl, (C₁-C₆)-Alkylsulfonyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Sulfamoyl, N-(C₁-C₄)-Alkylsulfamoyl oder N,N-Di-(C₁-C₄)-alkylsulfamoyl trägt, oder
mit den Substituenten R⁵ des α-C-Atoms einer α-Aminosäure, wobei die natürlichen L-Aminosäuren und ihre D-Isomeren Verwendung finden können;
B -CO₂G bedeutet, wobei G den Rest eines Alkohols GOH bedeutet,
R¹, R² und R³ gleich oder verschieden sind und Wasserstoff, Hydroxy, Halogen, Cyano, Trifluormethyl, Nitro, Carboxy, (C₁-C₂₀)-Alkyl, (C₃-C₈)-Cycloalkyl, (C₃-C₈)-Cycloalkyl-(C₁-C₁₂)-alkyl, (C₃-C₈)-Cycloalkoxy, (C₃-C₈)-Cycloalkyl-(C₁-C₁₂)-alkoxy, (C₃-C₈)-Cycloalkyloxy-(C₁-C₁₂)-alkyl, (C₃-C₈)-Cycloalkyloxy-(C₁-C₁₂)-alkoxy, (C₃-C₈)-Cycloalkyl-(C₁-C₈)-alkyl-(C₁-C₆)-alkoxy, (C₃-C₈)-Cycloalkyl-(C₁-C₈)-alkoxy-(C₁-C₆)-alkyl, (C₃-C₈)-Cycloalkyloxy-(C₁-C₈)-alkoxy-(C₁-C₆)-alkyl, (C₃-C₈)-Cycloalkoxy-(C₁-C₈)-alkoxy-(C₁-C₈)-alkoxy, (C₆-C₁₂)-Aryl, (C₇-C₁₆)-Aralkyl, (C₇-C₁₆)-Aralkenyl, (C₇-C₁₆)-Aralkinyl, (C₂-C₂₀)-Alkenyl, (C₂-C₂₀)-Alkinyl, (C₁-C₂₀)-Alkoxy, (C₂-C₂₀)-Alkenyloxy, (C₂-C₂₀)-Alkinyloxy, Retinyloxy, (C₁-C₂₀)-Alkoxy-(C₁-C₁₂)-alkyl, (C₁-C₁₂)-Alkoxy-(C₁-C₁₂)-alkoxy, (C₁-C₁₂)-Alkoxy-(C₁-C₈)-alkoxy-(C₁-C₈)-alkyl, (C₆-C₁₂)-Aryloxy, (C₇-C₁₆)-Aralkyloxy, (C₆-C₁₂)-Aryloxy-(C₁-C₆)-alkoxy, (C₇-C₁₆)-Aralkoxy-(C₁-C₆)-alkoxy, (C₁-C₁₆)-Hydroxyalkyl, (C₆-C₁₆)-Aryloxy-(C₁-C₈)-alkyl, (C₇-C₁₆)-Aralkoxy-(C₁-C₈)-alkyl, (C₆-C₁₂)-Aryloxy-(C₁-C₈)-alkoxy-(C₁-C₆)-alkyl, (C₇-C₁₂)-Aralkyloxy-(C₁-C₈)-alkoxy-(C₁-C₆)-alkyl, (C₂-C₂₀)-Alkenyloxy-(C₁-C₆)-alkyl, (C₂-C₂₀)-Alkinyloxy-(C₁-C₆)-alkyl, Retinyloxy-(C₁-C₆)-alkyl, -O-[CH₂₋]ₓC_{f}H_{(2f+1-g)}F_{g}, -OCF₂Cl, -OCF₂-CHFCl,
(C₁-C₂₀)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₂-C₂₀) Alkenylcarbonyl, (C₂-C₂₀)-Alkinylcarbonyl,
(C₁-C₂₀)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₂₀)-Alkenyloxycarbonyl, Retinyloxycarbonyl, (C₂-C₂₀)-Alkinyloxycarbonyl, (C₆-C₁₂)-Aryloxy-(C₁-C₆)-alkoxycarbonyl, (C₇-C₁₆)-Aralkoxy-(C₁-C₆)-alkoxycarbonyl, (C₃-C₈)-Cycloalkyl-(C₁-C₆)-alkoxycarbonyl, (C₃-C₈)-Cycloalkoxy-(C₁-C₆)-alkoxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₂-C₁₂)-Alkenyloxycarbonyloxy, (C₂-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-Cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₈)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl, N-(C₁-C₁₀)-Alkyl-N-((C₁-C₁₀)-alkoxy-(C₁-C₁₀)-alkyl)carbamoyl, N-(C₁-C₁₀)-Alkyl-N-((C₆-C₁₂)-aryloxy-(C₁-C₁₀)-alkyl)carbamoyl, N-(C₁-C₁₀)-Alkyl-N-((C₇-C₁₆)-aralkyloxy-(C₁-C₁₀)-alkyl)carbamoyl, CON(CH₂)ₕ, worin eine CH₂-Gruppe durch O, S, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 7 bedeutet,
einen Carbamoyl-Rest der allgemeinen Formel II 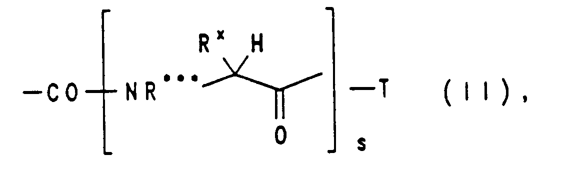 worin
R^{x} den Substituenten einer α-Aminosäure bedeutet, zu denen die L-und D-Aminosäuren zählen,
s 1, 2, 3, 4 oder 5 und
T OH, OR oder NR*R** bedeutet, wobei
R*, R** und R*** gleich oder verschieden sind und Wasserstoff (C₆-C₁₂)-Aryl, (C₇-C₁₁)-Aralkyl, (C₁-C₈)-Alkyl, (C₃-C₈)-Cycloalkyl, (+)-Dehydroabietyl, (C₁-C₈)-Alkoxy-(C₁-C₈)-alkyl, (C₇-C₁₂)-Aralkoxy-(C₁-C₈)-alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, oder
R* und R** gemeinsam für -[CH₂]ₕ stehen, worin eine CH₂ Gruppe durch O, S, SO, SO₂, N-Acylamino, N-(C₁-C₁₀)-Alkoxycarbonylimino, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt sein kann und h 3 bis 7 bedeutet,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₂)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy, N-((C₁-C₁₀)-alkyl))carbamoyloxy, N-((C₆-C₁₂)-Aryloxy-(C₁-C₁₀)-alkyl)carbamoyloxy, N-((C₇-C₁₆)-Aralkyloxy-(C₁-C₁₀)-alkyl)carbamoyloxy, N-(C₁-C₁₀)-Alkyl-N-((C₁-C₁₀)-alkoxy-(C₁-C₁₀)-alkyl)carbamoyloxy, N-(C₁-C₁₀)-Alkyl-N-((C₆-C₁₂)-aryloxy-(C₁-C₁₀)-alkyl)carbamoyloxy, N-(C₁-C₁₀)-Alkyl-N-((C₇-C₁₆)-aralkyloxy-(C₁-C₁₀)-alkyl)carbamoyloxy,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₂)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)-alkyl, N,N-Di(C₁-C₁₀)-alkylamino-(C₁-C₁₀)-alkyl, (C₃-C₈)-Cycloalkylamino-(C₁-C₁₀)-alkyl, (C₁-C₂₀)-Alkylmercapto, (C₁-C₂₀)-Alkylsulfinyl, (C₁-C₂₀)-Alkylsulfonyl, (C₆-C₁₂)-Arylmercapto, (C₆-C₁₂)-Arylsulfinyl, (C₆-C₁₂)-Arylsulfonyl, (C₇-C₁₆)-Aralkylmercapto, (C₇-C₁₆)-Aralkylsulfinyl, (C₇-C₁₆)-Aralkylsulfonyl, (C₁-C₁₂)-Alkylmercapto-(C₁-C₆)-alkyl, (C₁-C₁₂)-Alkylsulfinyl-(C₁-C₆)-alkyl, (C₁-C₁₂)-Alkylsulfonyl-(C₁-C₆)-alkyl, (C₆-C₁₂)-Arylmercapto-(C₁-C₆)-alkyl, (C₆-C₁₂)-Arylsulfinyl-(C₁-C₆)-alkyl, (C₆-C₁₂)-Arylsulfonyl-(C₁-C₆)-alkyl, (C₇-C₁₆)-Aralkylmercapto-(C₁-C₆)-alkyl, (C₇-C₁₆)-Aralkylsulfinyl-(C₁-C₆)-alkyl, (C₇-C₁₆)-Aralkylsulfonyl-(C₁-C₆)-alkyl,
Sulfamoyl, N-(C₁-C₁₀)-Alkylsulfamoyl, N,N-Di-(C₁-C₁₀)-alkylsulfamoyl, (C₃-C₈)-Cycloalkylsulfamoyl,
N-(C₆-C₁₂)-Arylsulfamoyl,
N-(C₇-C₁₆)-Aralkylsulfamoyl,
N-(C₁-C₁₀)-Alkyl-N-(C₆-C₁₂)-arylsulfamoyl,
N-(C₁-C₁₀)-Alkyl-N-(C₇-C₁₆)-aralkylsulfamoyl,
(C₁-C₁₀)-Alkyl-sulfonamido,
N-((C₁-C₁₀)-alkyl)-(C₁-C₁₀)-alkylsulfonamido, (C₇-C₁₆)-Aralkylsulfonamido,
N-((C₁-C₁₀)-alkyl-(C₇-C₁₆)-aralkylsulfonamido,
wobei die Reste, die einen Arylrest enthalten ihrerseits am Aryl substituiert sein können durch 1 bis 5 gleiche oder verschiedene Reste aus der Reihe:
Hydroxy, Halogen, Cyano, Trifluormethyl, Nitro, Carboxy, (C₁-C₁₆)-Alkyl, (C₃-C₈)-Cycloalkyl, (C₃-C₈)-Cycloalkyl-(C₁-C₁₂)-alkyl, (C₃-C₈)-Cycloalkoxy, (C₃-C₈)-Cycloalkyl-(C₁-C₁₂)-alkoxy, (C₃-C₈)-Cycloalkyloxy-(C₁-C₁₂)-alkyl, (C₃-C₈)-Cycloalkyloxy-(C₁-C₁₂)-alkoxy, (C₃-C₈)-Cycloalkyl-(C₁-C₈)-alkyl-(C₁-C₆)-alkoxy, (C₃-C₈)-Cycloalkyl-(C₁-C₈)-alkoxy-(C₁-C₆)-alkyl, (C₃-C₈)-Cycloalkyloxy-(C₁-C₈)-alkoxy-(C₁-C₆)-alkyl, (C₃-C₈)-Cycloalkoxy-(C₁-C₈)-alkoxy-(C₁-C₈)-alkoxy, (C₆-C₁₂)-Aryl, (C₇-C₁₆)-Aralkyl, (C₂-C₁₆)-Alkenyl, (C₂-C₁₂)-Alkinyl, (C₁-C₁₆)-Alkoxy, (C₁-C₁₆)-Alkenyloxy, (C₁-C₁₂)-Alkoxy-(C₁-C₁₂)-alkyl, (C₁-C₁₂)-Alkoxy-(C₁-C₁₂)-alkoxy, (C₁-C₁₂)-Alkoxy-(C₁-C₈)-alkoxy-(C₁-C₈)-alkyl, (C₆-C₁₂)-Aryloxy, (C₇-C₁₆)-Aralkyloxy, (C₆-C₁₂)-Aryloxy-(C₁-C₆)-alkoxy, (C₇-C₁₆)-Aralkoxy-(C₁-C₆)-alkoxy, (C₁-C₈)-Hydroxyalkyl, (C₆-C₁₆)-Aryloxy-(C₁-C₈)-alkyl, (C₇-C₁₆)-Aralkoxy-(C₁-C₈)-alkyl, (C₆-C₁₂)-Aryloxy-(C₁-C₈)-alkoxy-(C₁-C₆)-alkyl, (C₇-C₁₂)-Aralkyloxy-(C₁-C₈)-alkoxy-(C₁-C₆)-alkyl, -O-[CH₂₋]ₓC_{f}H_{(2f+1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₁₂)-Alkenyloxycarbonyl, (C₂-C₁₂)-Alkinyloxycarbonyl, (C₆-C₁₂)-Aryloxy-(C₁-C₆)-alkoxycarbonyl, (C₇-C₁₆)-Aralkoxy-(C₁-C₆)-alkoxycarbonyl, (C₃-C₈)-Cycloalkyl-(C₁-C₆)-alkoxycarbonyl, (C₃-C₈)-Cycloalkoxy-(C₁-C₆)-alkoxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₂-C₁₂)-Alkenyloxycarbonyloxy, (C₂-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₆)-Alkoxy-(C₁-C₁₀)-alkyl)carbamoyl, N-((C₆-C₁₆)-Aryloxy-(C₁-C₁₀)-alkyl)carbamoyl,
N-((C₇-C₁₆)-Aralkyloxy-(C₁-C₁₀)-alkyl)carbamoyl,
N-(C₁-C₁₀)-Alkyl-N-((C₁-C₁₀)-alkoxy-(C₁-C₁₀)-alkyl)carbamoyl,
N-(C₁-C₁₀)-Alkyl-N-((C₆-C₁₂)-aryloxy-(C₁-C₁₀)-alkyl)carbamoyl,
N-(C₁-C₁₀)-Alkyl-N-((C₇-C₁₆)-aralkyloxy-(C₁-C₁₀)-alkyl)carbamoyl,
CON(CH₂)ₕ, worin eine CH₂-Gruppe durch O, S, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 7 bedeutet,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₆)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy, N-((C₁-C₁₀)-alkyl))carbamoyloxy,
N-((C₆-C₁₂)-Aryloxy-(C₁-C₁₀)-alkyl)-carbamoyloxy,
N-((C₇-C₁₆)-Aralkyloxy-(C₁-C₁₀)-alkyl)carbamoyloxy,
N-(C₁-C₁₀)-Alkyl-N-((C₁-C₁₀)-alkoxy-(C₁-C₁₀)-alkyl)carbamoyloxy,
N-(C₁-C₁₀)-Alkyl-N-((C₆-C₁₂)-aryloxy-(C₁-C₁₀)-alkyl)carbamoyloxy,
N-(C₁-C₁₀)-Alkyl-N-((C₇-C₁₆)-aralkyloxy-(C₁-C₁₀)-alkyl)carbamoyloxy,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₂)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-Alkylamino-(C₁-C₁₀)-alkyl, N,N-Di-(C₁-C₁₀)-alkylamino-(C₁-C₁₀)-alkyl, (C₃-C₈)-Cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl,
R¹ und R² oder R² und R³ eine Kette [CH₂]ₒ bilden, in welcher eine oder zwei CH₂-Gruppen der gesättigten oder mit einer C = C-Doppelbindung ungesättigten Kette gegebenenfalls durch O, S, SO, SO₂ oder NR' ersetzt sind, o = 3, 4 oder 5 bedeutet und
R' Wasserstoff, (C₆-C₁₂)-Aryl, (C₁-C₈)-Alkyl, (C₁-C₈)-Alkoxy-(C₁-C₈)-alkyl, (C₇-C₁₂)-Aralkoxy-(C₁-C₈)-alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, wobei
vorzugsweise die Reste R¹ und R² oder R² und R³ zusammen mit dem sie tragenden Pyridin oder Pyridazin einen 5, 6, 7, 8-Tetrahydroisochinolin-, einen 5, 6, 7, 8-Tetrahydrochinolin- oder einen 5, 6, 7, 8-Tetrahydrocinnolin-Ring bilden,
oder
R¹ und R² oder R² und R³ einen carbocylischen oder einen heterocyclischen, 5- oder 6-gliedrigen aromatischen Ring bilden, wobei
vorzugsweise die Reste R¹ und R² oder R² und R³ zusammen mit dem sie tragenden Pyridin oder Pyridazin folgende ggf. substituierte heterocyclischen Ringsysteme bilden:
Thienopyridine`
Furanopyridine,
Pyridopyridine,
Pyrimidinopyridine,
Imidazopyridine,
Thiazolopyridine,
Oxazolopyridine,
Chinon, Isochinolin und
Cinnolin,
wobei Chinolin, Isochinolin oder Cinnolin vorzugsweise den Formeln 1a, 1b und 1c genügen 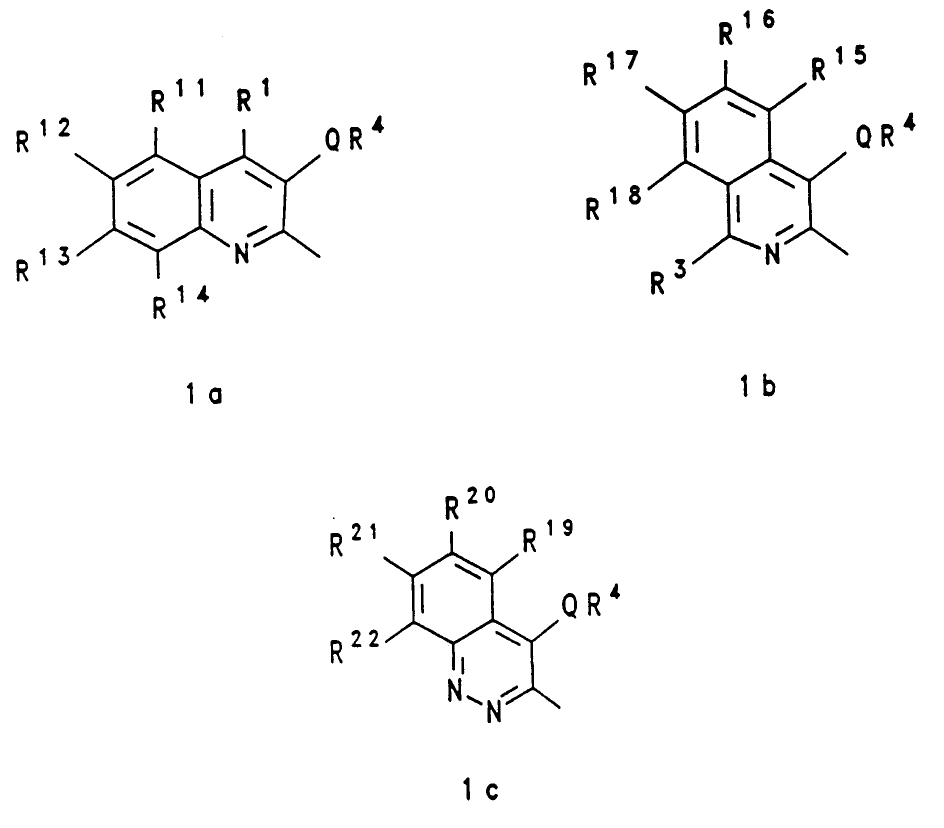 und die Substituenten R¹¹ bis R²² jeweils unabhängig voneinander in der Bedeutung von R¹, R² und R³ stehen,
R⁴ falls Q eine Bindung ist, Halogen, Nitril und Trifluormethyl bedeutet, oder falls Q O oder S ist, einen verzweigten oder unverzweigten (C₁-C₂₀)-Alkylrest, einen unsubstituierten, gesättigten Fluoralkylrest der Formel [CH₂]ₓC_{f}H_{(2f+1-g)}F_{g}, einen (C₆-C₁₆)-Arylrest, einen (C₇-C₁₆)-Aralkylrest, einen Heteroarylrest oder einen Heteroaralkylrest bedeutet,
wobei diese Reste substituiert sind mit einem oder mehreren Resten aus der Reihe
Hydroxy, Halogen, Cyano, Trifluormethyl, Nitro, Carboxy, (C₁-C₁₂)-Alkyl, (C₃-C₈)-Cycloalkyl, (C₃-C₈)-Cycloalkyl-(C₁-C₁₂)-alkyl, (C₃-C₈)-Cycloalkoxy, (C₃-C₈)-Cycloalkyl-(C₁-C₁₂)-alkoxy, (C₃-C₈)-Cycloalkyloxy-(C₁-C₁₂)-alkyl, (C₃-C₈)-Cycloalkyloxy-(C₁-C₁₂)-alkoxy, (C₃-C₈)-Cycloalkyl-(C₁-C₈)-alkyl-(C₁-C₆)-alkoxy, (C₃-C₈)-Cycloalkyl-(C₁-C₈)-alkoxy-(C₁-C₆)-alkyl, (C₃-C₈)-Cycloalkyloxy-(C₁-C₈)-alkoxy-(C₁-C₆)-alkyl, (C₃-C₈)-Cycloalkoxy-(C₁-C₈)-alkoxy-(C₁-C₈)-alkoxy, (C₆-C₁₂)-Aryl, (C₇-C₁₆)-Aralkyl, (C₂-C₁₂)-Alkenyl, (C₂-C₁₂)-Alkinyl, (C₁-C₁₂)-Alkoxy, (C₁-C₁₂)-Alkoxy-(C₁-C₁₂)-alkyl, (C₁-C₁₂)-Alkoxy-(C₁-C₁₂)-alkoxy, (C₁-C₁₂)-Alkoxy-(C₁-C₈)-alkoxy-(C₁-C₈)-alkyl, (C₆-C₁₂)-Aryloxy, (C₇-C₁₆)-Aralkyloxy, (C₆-C₁₂)-Aryloxy-(C₁-C₆)-alkoxy, (C₇-C₁₆)-Aralkoxy-(C₁-C₆)-alkoxy, (C₁-C₈)-Hydroxyalkyl, (C₆-C₁₆)-Aryloxy-(C₁-C₈)-alkyl, (C₇-C₁₆)-Aralkoxy-(C₁-C₈)-alkyl, (C₆-C₁₂)-Aryloxy-(C₁-C₈)-alkoxy-(C₁-C₆)-alkyl, (C₇-C₁₂)-Aralkyloxy-(C₁-C₈)-alkoxy-(C₁-C₆)-alkyl, -O-[CH₂₋]ₓC_{f}H_{(2f+1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₂-C₁₂) Alkenylcarbonyl, (C₂-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₁₂)-Alkenyloxycarbonyl, (C₂-C₁₂)-Alkinyloxycarbonyl, (C₆-C₁₂)-Aryloxy-(C₁-C₆)-alkoxycarbonyl, (C₇-C₁₆)-Aralkoxy-(C₁-C₆)-alkoxycarbonyl, (C₃-C₈)-Cycloalkyl-(C₁-C₆)-alkoxycarbonyl, (C₃-C₈)-Cycloalkoxy-(C₁-C₆)-alkoxycarbonyl, (C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₂-C₁₂)-Alkenyloxycarbonyloxy, (C₂-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-Cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₀)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl, N-(C₁-C₁₀)-Alkyl-N-((C₁-C₁₀)-alkoxy-(C₁-C₁₀)-alkyl)carbamoyl,
N-(C₁-C₁₀)-Alkyl-N-((C₆-C₁₂)-aryloxy-(C₁-C₁₀)-alkyl)carbamoyl,
N-(C₁-C₁₀)-Alkyl-N-((C₇-C₁₆)-aralkyloxy-(C₁-C₁₀)-alkyl)carbamoyl,
CON(CH₂)ₕ, worin eine CH₂-Gruppe durch O, S, N-(C₁-C₈)-Alkylimino,
N-(C₃-C₈)-Cycloalkylimino, N-(C₃-C₈)-Cycloalkyl-(C₁-C₄)-alkylimino,
N-(C₆-C₁₂)-Arylimino, N-(C₇-C₁₆)-Aralkylimino oder N-(C₁-C₄)-Alkoxy-
(C₁-C₆)-alkylimino ersetzt ein kann und h 3 bis 7 bedeutet, oder mit
einem Carbamoyl-Rest der allgemeinen Formel II 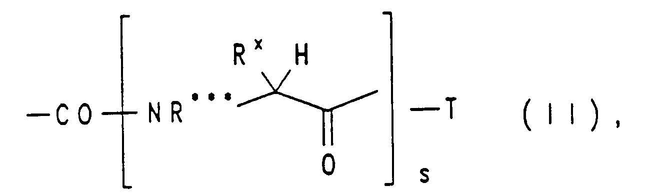 worin
R^{x} den Substituenten einer α-Aminosäure bedeutet, zu denen die L-und D-Aminosäuren zählen,
s 1, 2, 3, 4 oder 5 und
T OH, OR oder NR*R** bedeutet, wobei
R*, R** und R*** gleich oder verschieden sind und Wasserstoff, (C₆-C₁₂)-Aryl, (C₇-C₁₁)-Aralkyl, (C₁-C₈)-Alkyl, (C₃-C₈)-Cycloalkyl, (+)-Dehydroabietyl, (C₁-C₈)-Alkoxy-(C₁-C₈)-alkyl, (C₇-C₁₂)-Aralkoxy-(C₁-C₈)-alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, oder
R* und R** gemeinsam für -[CH₂]ₕ stehen, worin eine CH₂ Gruppe durch O, S, SO, SO₂, N-Acylamino, N-(C₁-C₁₀)-Alkoxycarbonylimino, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt sein kann und h 3 bis 7 bedeutet, oder mit
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₂)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-aralkylcarbamoyloxy, N-((C₁-C₁₀)-alkyl))carbamoyloxy, N-((C₆-C₁₂)-Aryloxy-(C₁-C₁₀)-alkyl)carbamoyloxy,
N-((C₇-C₁₆)-Aralkyloxy-(C₁-C₁₀)-alkyl)carbamoyloxy,
N-(C₁-C₁₀)-Alkyl-N-((C₁-C₁₀)-alkoxy-(C₁-C₁₀)-alkyl)carbamoyloxy,
N-(C₁-C₁₀)-Alkyl-N-((C₆-C₁₂)-aryloxy-(C₁-C₁₀)-alkyl)carbamoyloxy,
N-(C₁-C₁₀)-Alkyl-N-((C₇-C₁₆)-aralkyloxy-(C₁-C₁₀)-alkyl)carbamoyloxy,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₂)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)-alkyl, N,N-Di(C₁-C₁₀)-alkylamino-(C₁-C₁₀)-alkyl, (C₃-C₈)-Cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₂)-Arylmercapto, (C₆-C₂)-Arylsulfinyl, (C₆-C₁₂)-Arylsulfonyl, (C₇-C₁₆)-Aralkylmercapto, (C₇-C₁₆)-Aralkylsulfinyl, (C₇-C₁₆)-Aralkylsulfonyl,
Sulfamoyl, N-(C₁-C₁₀)-Alkylsulfamoyl, N,N-Di-(C₁-C₁₀)-alkylsulfamoyl,
(C₃-C₈)-Cycloalkylsulfamoyl,
N-(C₆-C₁₂)-Arylsulfamoyl,
N-(C₇-C₁₆)-Aralkylsulfamoyl,
N-(C₁-C₁₀)-Alkyl-N-(C₆-C₁₂)-arylsulfamoyl, N-(C₁-C₁₀)-Alkyl-N-(C₇-C₁₆)-aralkylsulfamoyl, (C₁-C₁₀)-Alkyl-sulfonamido, N-((C₁-C₁₀)-Alkyl)-(C₁-C₁₀)-alkylsulfonamido, (C₇-C₁₆)-Aralkylsulfonamido, N-((C₁-C₁₀)-Alkyl-(C₇-C₁₆)-aralkylsulfonamido,
wobei die Reste, die einen Arylrest enthalten ihrerseits am Aryl substituiert sein können durch 1 bis 5 gleiche oder verschiedene Reste aus der Reihe:
Hydroxy, Halogen, Cyano, Trifluormethyl, Nitro, Carboxy, (C₁-C₁₂)-Alkyl, (C₃-C₈)-Cycloalkyl, (C₃-C₈)-Cycloalkyl-(C₁-C₁₂)-alkyl, (C₃-C₈)-Cycloalkoxy, (C₃-C₈)-Cycloalkyl-(C₁-C₁₂)-alkoxy, (C₃-C₈)-Cycloalkyloxy-(C₁-C₁₂)-alkyl, (C₃-C₈)-Cycloalkyloxy-(C₁-C₁₂)-alkoxy, (C₃-C₈)-Cycloalkyl-(C₁-C₈)-alkyl-(C₁-C₆)-alkoxy, (C₃-C₈)-Cycloalkyl-(C₁-C₈)-alkoxy-(C₁-C₆)-alkyl, (C₃-C₈)-Cycloalkyloxy-(C₁-C₈)-alkoxy-(C₁-C₆)-alkyl, (C₃-C₈)-Cycloalkoxy-(C₁-C₈)-alkoxy-(C₁-C₈)-alkoxy, (C₆-C₁₂)-Aryl, (C₇-C₁₆)-Aralkyl, (C₂-C₁₂)-Alkenyl, (C₂-C₁₂)-Alkinyl, (C₁-C₁₂)-Alkoxy, (C₁-C₁₂)-Alkoxy-(C₁-C₁₂)-alkyl, (C₁-C₁₂)-Alkoxy-(C₁-C₁₂)-alkoxy, (C₁-C₁₂)-Alkoxy-(C₁-C₈)-alkoxy-(C₁-C₈)-alkyl, (C₆-C₁₂)-Aryloxy, (C₇-C₁₆)-Aralkyloxy, (C₆-C₁₂)-Aryloxy-(C₁-C₆)-alkoxy, (C₇-C₁₆)-Aralkoxy-(C₁-C₆)-alkoxy, (C₁-C₈)-Hydroxyalkyl, (C₆-C₁₆)-Aryloxy-(C₁-C₈)-alkyl, (C₇-C₁₆)-Aralkoxy-(C₁-C₈)-alkyl, (C₆-C₁₂)-Aryloxy-(C₁-C₈)-alkoxy-(C₁-C₆)-alkyl, (C₇-C₁₂)-Aralkyloxy-(C₁-C₈)-alkoxy-(C₁-C₆)-alkyl, -O-[CH₂₋]ₓC_{f}H_{(2f+1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₁₂)-Alkenyloxycarbonyl, (C₂-C₁₂)-Alkinyloxycarbonyl, (C₆-C₁₂)-Aryloxy-(C₁-C₆)-alkoxycarbonyl, (C₇-C₁₆)-Aralkoxy-(C₁-C₆)-alkoxycarbonyl, (C₃-C₈)-Cycloalkyl-(C₁-C₆)-alkoxycarbonyl, (C₃-C₈)-Cycloalkoxy-(C₁-C₆)-alkoxycarbonyl,
(C₁-C₁₂)-Alkylcarbonyloxy, (C₃-C₈)-Cycloalkylcarbonyloxy,
(C₆-C₁₂)-Arylcarbonyloxy, (C₇-C₁₆)-Aralkylcarbonyloxy, Cinnamoyloxy,
(C₂-C₁₂)-Alkenylcarbonyloxy, (C₂-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₂-C₁₂)-Alkenyloxycarbonyloxy, (C₂-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₀)-Alkoxy-(C₁-C₁₀)-alkyl)carbamoyl, N-((C₆-C₁₆)-Aryloxy-(C₁-C₁₀)-alkyl)carbamoyl,
N-((C₇-C₁₆)-Aralkyloxy-(C₁-C₁₀)-alkyl)carbamoyl,
N-(C₁-C₁₀)-Alkyl-N-((C₁-C₁₀)-alkoxy-(C₁-C₁₀)-alkyl)carbamoyl,
N-(C₁-C₁₀)-Alkyl-N-((C₆-C₁₂)-aryloxy-(C₁-C₁₀)-alkyl)carbamoyl,
N-(C₁-C₁₀)-Alkyl-N-((C₇-C₁₆)-aralkyloxy-(C₁-C₁₀)-alkyl)carbamoyl,
CON(CH₂)ₕ, worin eine CH₂-Gruppe durch O, S, N-(C₁-C₈)-Alkylimino,
N-(C₃-C₈)-Cycloalkylimino, N-(C₃-C₈)-Cycloalkyl-(C₁-C₄)-alkylimino,
N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 7 bedeutet,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₆)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy, N-((C₁-C₁₀)-alkyl))carbamoyloxy,
N-((C₆-C₁₂)-Aryloxy-(C₁-C₁₀)-alkyl)-carbamoyloxy,
N-((C₇-C₁₆)-Aralkyloxy-(C₁-C₁₀)-alkyl)carbamoyloxy,
N-(C₁-C₁₀)-Alkyl-N-((C₁-C₁₀)-alkoxy-(C₁-C₁₀)-alkyl)carbamoyloxy,
N-(C₁-C₁₀)-Alkyl-N-((C₆-C₁₂)-aryloxy-(C₁-C₁₀)-alkyl)carbamoyloxy,
N-(C₁-C₁₀)-Alkyl-N-((C₇-C₁₆)-aralkyloxy-(C₁-C₁₀)-alkyl)carbamoyloxy,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂)-Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino,
(C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino,
(C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₂)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-Alkylamino-(C₁-C₁₀)-alkyl, N,N-Di-(C₁-C₁₀)-alkylamino-(C₁-C₁₀)-alkyl, (C₃-C₈)-Cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl, und
R⁴ R'' bedeutet, sofern Q in der Bedeutung von NR' steht, wobei R' und R'' gleich oder verschieden sind und Wasserstoff, (C₆-C₁₂)-Aryl, (C₇-C₁₁)-Aralkyl, (C₁-C₈)-Alkyl, (C₁-C₈)-Alkoxy-(C₁-C₈)-alkyl, (C₇-C₁₂)-Aralkoxy-(C₁-C₈)-alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, oder
R' und R'' gemeinsam für -[CH₂]ₕ stehen, worin eine CH₂-Gruppe durch O, S, N-Acylimino oder N-(C₁-C₁₀)-Alkoxycarbonylimino ersetzt sein kann, und
f 1 bis 8,
g 0,1 bis (2f + 1),
x 0 bis 3,
h 3 bis 7 bedeuten,
einschließlich der physiologisch wirksamen Salze,
wobei 3-Benzyloxypyridin-2-carbonsäure-(L-threonylmethylester)amid, 3-Benzyloxypyridin-2-carbonsäure-(L-threonyl(Fmoc-Phg)tert. butylester)amid, 3-Benzyloxypyridin-2-carbonsäure-(L-threonyl-tert. butylester)amid und 3-Benzyloxypyridin-2-carbonsäure-(D-allothreonylmethylester)amid, und 3-Benzyloxypicolinylglycin ethyl ester ausgenommen sind.

2. Verbindungen der Formel I, gemäß Anspruch 1,
in der
Q O, S, NR' oder eine Bindung bedeutet,
X O,
Y CR³ oder,
falls R¹ und R² einen Cyclus bilden,
Y N oder CR³,
m 0 oder 1 und
G den Rest eines Alkohols GOH
bedeuten und 3-Benzyloxypicolinylglycin ethyl ester ausgenommen ist.

3. Verbindungen der Formel I, gemäß den Ansprüchen 1 und 2,
in der
Q O, NR' oder eine Bindung,
X O und G den Rest eines Alkohols GOH bedeuten und 3-Benzyloxypicolinylglycin ethyl ester ausgenommen ist.

4. Verbindungen der Formel I, gemäß den Ansprüchen 1 und 2, in der
Q S,
X O,
m 0 oder 1 und G den Rest eines Alkohols GOH bedeuten.

5. Verbindungen der Formel I, gemäß den Ansprüchen 1, 2 und 4, in der
Q S,
X O,
m 0 und G den Rest eines Alkohols GOH bedeuten.

6. Verbindungen der Formel I gemäß den Ansprüchen 1 bis 3, in der
Q O, NR' oder eine Bindung bedeutet,
X O,
Y CR³ oder, falls R¹ und R² einen Cyclus bilden, N oder CR³,
m 0 und 1,
A (C₁-C₃)-Alkylen, das gegebenenfalls einfach substituiert ist mit Halogen, Cyano, Trifluormethyl, (C₁-C₆)-Alkyl, (C₁-C₆)-Hydroxyalkyl, (C₁-C₆)-Alkoxy, -O-[CH₂]ₓ-C_{f}H_{(2f+1-g)}F_{g} oder
A -CHR⁵- bedeutet, wobei R⁵ einen der Substituenten des α-C-Atoms einer α-Aminosäure bedeutet, insbesondere einer natürlichen L-Aminosäure und ihres D-Isomeren,
B CO₂G bedeutet, wobei
G den Rest eines Alkohols GOH bedeutet, worin G einen verzweigten oder unverzweigten oder cyclischen aliphatischen (C₁-C₂₀)-Alkyl-Rest, oder einen verzweigten oder unverzweigten ggf. cyclischen (C₂-C₂₀)-Alkenyl-Rest, einen Retinyl-Rest, einen (C₂-C₂₀)-Alkinyl-Rest oder einen entsprechenden (C₄-C₂₀)-Alkeninyl-Rest bedeutet, wobei die Reste jeweils eine oder mehrere Mehrfachbindungen enthalten können, oder einen (C₆-C₁₆)-Aryl-Rest, einen (C₇-C₁₆)-Aralkyl-Rest oder einen 5-oder 6-gliedrigen, vorzugsweise Stickstoff-enthaltenden Heteroaryl- oder einen 5- oder 6-gliedrigen, vorzugsweise Stickstoff-enthaltenden Heteroaralkyl-Rest bedeutet, wobei die vorstehenden Reste insbesondere einen oder mehrere Substituenten aus der Reihe
Hydroxy, Halogen, Cyano, Trifluormethyl, Nitro, Carboxy, (C₁-C₁₂)-Alkyl, (C₃-C₈)-Cycloalkyl, (C₅-C₈)-Cycloalkenyl), (C₆-C₁₂)-Aryl, (C₇-C₁₆)-Aralkyl, (C₂-C₁₂)-Alkenyl, (C₂-C₁₂)-Alkinyl, (C₁-C₁₂)-Alkoxy, (C₁-C₁₂)-Alkoxy-(C₁-C₁₂)alkyl, (C₁-C₁₂)-Alkoxy-(C₁-C₁₂)alkoxy, (C₆-C₁₂)-Aryloxy, (C₇-C₁₆)-Aralkyloxy, (C₁-C₈)-Hydroxyalkyl,-O-[CH₂₋]ₓC_{f}H_{(2f+1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoy, (C₂-C₁₂) Alkenylcarbonyl, (C₂-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₁₂)-Alkenyloxycarbonyl, (C₂-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₂-C₁₂)-Alkenyloxycarbonyloxy, (C₂-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)arylcarbamoyl, N-(C₁-C₁₀)-Alkyl-N-(C₇-C₁₆)-Aralkylcarbamoyl,
N-((C₁-C₁₀)-Alkoxy-(C₁-C₁₀)alkyl)carbamoyl,
N-((C₆-C₁₂)-Aryloxy-(C₁-C₁₀)alkyl)carbamoyl,
N-((C₇-C₁₆)-Aralkyloxy-(C₁-C₁₀)alkyl)carbamoyl,
N-(C₁-C₁₀)-Alkyl-N-((C₁-C₁₀)-Alkoxy-(C₁-C₁₀)alkyl)carbamoyl,
N-(C₁-C₁₀)-Alkyl-N-((C₆-C₁₆)-Aryloxy-(C₁-C₁₀)alkyl)carbamoyl,
N-(C₁-C₁₀)-Akyl-N-((C₇-C₁₆)Aralkyloxy-(C₁-C₁₀)alkyl)carbamoyl,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₂)-Arylcarbamoyloxy, N-(C₇-C₁₆)-Aralkylcarbamoyloxy, N-(C₁-C₁₀)-Alkyl-N-(C₆-C₁₂)arylcarbamoyloxy, N-(C₁-C₁₀)-Alkyl-N-(C₇-C₁₆)-Aralkylcarbamoyloxy, N-((C₁-C₁₀)alkyl))carbamoyloxy,
N-((C₆-C₁₂)-Aryloxy-(C₁-C₁₀)alkyl)carbamoyloxy,
N-((C₇-C₁₆)-Aralkyloxy-(C₁-C₁₀)alkyl)carbamoyloxy,
N-(C₁-C₁₀)-Akyl-N-((C₁-C₁₀)-Alkoxy-(C₁-C₁₀)alkyl)carbamoyloxy,
N-(C₁-C₁₀)-Alkyl-N-((C₆-C₁₂)-Aryloxy-(C₁-C₁₀)alkyl)carbamoyloxy,
N-(C₁-C₁₀)-Alkyl-N-((C₇-C₁₆)-Aralkyloxy-(C₁-C₁₀)alkyl)carbamoyloxy,
Amino, (C₁-C₁₂)-Alkylamino, Di-(C₁-C₁₂)alkylamino, (C₃-C₈)-Cycloalkylamino, (C₂-C₁₂)-Alkenylamino, (C₂-C₁₂) Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)alkyl, (C₆-C₁₂)-Aroylamino-(C₁-C₈)-alkyl, (C₇-C₁₂)-Aralkanoylamino-(C₁-C₈)-alkyl, Amino-(C₁-C₁₀)-alkyl, N-(C₁-C₁₀)alkylamino-(C₁-C₁₀)alkyl, N,N-Di(C₁-C₁₀)alkylamino-(C₁-C₁₀)alkyl, (C₃-C₈)cycloalkylamino-(C₁-C₁₀)alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₂)-Arylsulfonyl, (C₇-C₁₆)-Aralkylmercapto, (C₇-C₁₆)-Aralkylsulfinyl, (C₇-C₁₆)-Aralkylsulfonyl,
Sulfamoyl, N-(C₁-C₁₀)-Alkylsulfamoyl, N,N-Di-(C₁-C₁₀)-alkylsulfamoyl,
(C₃-C₈-cycloalkylsulfamoyl,
N-(C₆-C₁₂)-Arylsulfamoyl,
N-(C₇-C₁₆)-Aralkylsulfamoyl,
N-(C₁-C₁₀)-Alkyl-N-(C₆-C₁₂)-Arylsulfamoyl,
N-(C₁-C₁₀)-Alkyl-N-(C₇-C₁₆)-Aralkylsulfamoyl,
(C₁-C₁₀)-Alkyl-sulfonamido,
N-((C₁-C₁₀)alkyl)-(C₁-C₁₀)alkylsulfonamido, (C₇-C₁₆)-Aralkylsulfonamido,
N-((C₁-C₁₀)alkyl-(C₇-C₁₆)-Aralkylsulfonamido, bedeuten, wobei die Reste, die einen Arylrest enthalten, ihrerseits am Aryl substituiert sein können durch 1 bis 5 gleiche oder verschiedene Reste aus der Reihe:
Hydroxy, Halogen, Cyano, Trifluormethyl, Nitro, Carboxy, (C₁-C₁₂)-Alkyl, (C₃-C₈)-Cycloalkyl, (C₆-C₁₂)-Aryl, (C₇-C₁₆)-Aralkyl, (C₁-C₁₂)-Alkoxy, (C₁-C₁₂)-Alkoxy-(C₁-C₁₂)alkyl, (C₁-C₁₂)-Alkoxy-(C₁-C₁₂)alkoxy, (C₆-C₁₂)-Aryloxy, (C₇-C₁₆)-Aralkyloxy, (C₁-C₈)-Hydroxyalkyl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₁₂)-Alkenyloxycarbonyl, (C₂-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₂-C₁₂)-Alkenyloxycarbonyloxy, (C₂-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-cycloalkylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₂)-arylcarbamoyl, N-(C₁-C₁₀)-Alkyl-N-(C₇-C₁₆)-aralkylcarbamoyl,
N-((C₁-C₁₀)-Alkoxy-(C₁-C₁₀)alkyl)carbamoyl,
N-((C₆-C₁₂)-Aryloxy-(C₁-C₁₀)alkyl)carbamoyl,
N-((C₇-C₁₆)-Aralkyloxy-(C₁-C₁₀)alkyl)carbamoyl,
N-(C₁-C₁₀)-Alkyl-N-((C₁-C₁₀)-Alkoxy-(C₁-C₁₀)alkyl)carbamoyl,
N-(C₁-C₁₀)-Alkyl-N-((C₆-C₁₂)-Aryloxy-(C₁-C₁₀)alkyl)carbamoyl,
N-(C₁-C₁₀)-Alkyl-N-((C₇-C₁₆)Aralkyloxy-(C₁-C₁₀)alkyl)carbamoyl,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₂)-Arylcarbamoyloxy, N-(C₇-C₁₆)-Aralkylcarbamoyloxy, N-(C₁-C₁₀)-Alkyl-N-(C₆-C₁₂)arylcarbamoyloxy, N-(C₁-C₁₀)-Alkyl-N-(C₇-C₁₆)-Aralkylcarbamoyloxy,
N-((C₁-C₁₀)alkyl))carbamoyloxy,
N-((C₆-C₁₂)-Aryloxy-(C₁-C₁₀)alkyl)carbamoyloxy,
N-((C₇-C₁₆)-Aralkyloxy-(C₁-C₁₀)alkyl)carbamoyloxy,
N-(C₁-C₁₀)-Alkyl-N-((C₁-C₁₀)-Alkoxy-(C₁-C₁₀)alkyl)carbamoyloxy,
N-(C₁-C₁₀)-Alkyl-N-((C₆-C₁₂)-Aryloxy-(C₁-C₁₀)alkyl)carbamoyloxy,
N-(C₁-C₁₀)-Alkyl-N-((C₇-C₁₆)-Aralkyloxy-(C₁-C₁₀)alkyl)carbamoyloxy,
Amino, (C₁-C₁₂)-Alkylamino, Di-(C₁-C₁₂)alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)alkyl, (C₆-C₁₂)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)alkylamino-(C₁-C₁₀)alkyl, N,N-Di(C₁-C₁₀)alkylamino-(C₁-C₁₀)alkyl, (C₃-C₈)Cycloalkylamino-(C₁-C₁₀)alkyl,
(C₁-C₁₂)-Alkylmercapto, (C₁-C₁₂)-Alkylsulfinyl, (C₁-C₁₂)-Alkylsulfonyl, (C₆-C₁₂)-Arylmercapto, (C₆-C₁₂)-Arylsulfinyl, (C₆-C₁₂)-Arylsulfonyl, (C₇-C₁₆)-Aralkylmercapto, (C₇-C₁₆)-Aralkylsulfinyl, (C₇-C₁₆)-Aralkylsulfonyl,
R² Wasserstoff, (C₁-C₂₀)-Alkyl, (C₂-C₂₀)-Alkenyl, (C₂-C₂₀)-Alkinyl, (C₁-C₂₀)-Alkoxy, (C₂-C₂₀)-Alkenyloxy, (C₂-C₂₀)-Alkinyloxy, Retinyloxy, (C₁-C₂₀)-Alkoxy-(C₁-C₃)-alkyl, (C₂-C₂₀)-Alkenyloxy-(C₁-C₃)-alkyl, Retinyloxy-(C₁-C₃)-alkyl, (C₂-C₂₀)-Alkinyloxy-(C₁-C₃)-alkyl, Halogen, Cyano, Trifluormethyl, (C₁-C₈)-Hydroxyalkyl, (C₁-C₂₀)-Alkanoyl,(C₇-C₁₆)-Aralkanoyl, (C₆-C₁₂)-Aroyl, (C₆-C₁₂)-Aryl, (C₇-C₁₆)-Aralkyl, -O-[CH₂]ₓC_{f}H_{(2f+1-g)}F_{g}, NR'R'', (C₁-C₁₀)-Alkylmercapto, (C₁-C₁₀)-Alkylsulfinyl, (C₁-C₁₀)-Alkylsulfonyl, (C₆-C₁₂)-Arylmercapto, (C₆-C₁₂)-Arylsulfinyl), (C₆-C₁₂)-Arylsulfonyl, (C₇-C₁₂)-Aralkylmercapto, (C₇-C₁₂)-Aralkylsulfinyl, (C₇-C₁₂)-Aralkylsulfonyl, (C₆-C₁₂)-Aryloxy, (C₇-C₁₆)-Aralkyloxy, Carboxy, (C₁-C₂₀)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₂₀)-Alkenyloxycarbonyl, Retinyloxycarbonyl, (C₂-C₂₀)-Alkinyloxycarbonyl, (C₃-C₈)-Cycloalkyl-(C₁-C₆)-alkoxycarbonyl, (C₃-C₈)-Cycloalkoxy-(C₁-C₆)-alkoxycarbonyl, (C₆-C₁₂)-Aryloxy-(C₁-C₆)-alkoxycarbonyl, (C₇-C₁₆)-Aralkoxy-(C₁-C₆)-alkoxycarbonyl,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo(C₃-C₈)-alkylcarbamoyl, N-(C₁-C₁₀)-Alkyl-N-(C₃-C₈)-cycloalkylcarbamoyl, N-(C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₂)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl,N-(C₁-C₁₀)-Alkyl-N-((C₁-C₁₀)-alkoxy-(C₁-C₁₀)-alkyl)carbamoyl,
N-(C₁-C₁₀)-Alkyl-N-((C₆-C₁₂)-aryloxy-(C₁-C₁₀)-alkyl)carbamoyl,
N-(C₁-C₁₀)-Alkyl-N-((C₇-C₁₆)-aralkyloxy-(C₁-C₁₀)-alkyl)carbamoyl,
CON(CH₂)ₕ, worin eine CH₂-Gruppe durch O, S, N-(C₁-C₈)-alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 7 bedeutet,
wobei Aryl in der Weise substiuiert ist wie für R¹ und R³ definiert,
R¹ und R³ gleich oder verschieden sind und Wasserstoff, Halogen, (C₁-C₁₂)-Alkyl, (C₁-C₁₂)-Alkoxy, -O-[CH₂]ₓ-C_{f}H_{(2f+1-g)}Hal_{g}, (C₁-C₁₂)-Alkoxy-(C₁-C₁₂)-alkyl, (C₁-C₈)-Alkoxy-(C₁-C₁₂)-alkoxy, (C₁-C₁₂)-Alkoxy-(C₁-C₈)-alkoxy-(C₂-C₆)-alkyl, (C₇-C₁₁)-Aralkyloxy, (C₃-C₈)-Cycloalkyl, (C₃-C₈)-Cycloalkyl-(C₁-C₈)-alkyl, (C₃-C₈)-Cycloalkyloxy, (C₃-C₈)-Cycloalkyl-(C₁-C₈)-alkoxy, (C₃-C₈)-Cycloalkyloxy-(C₁-C₈)-alkyl, (C₃-C₈)-Cycloalkyloxy-(C₁-C₈)-alkoxy, (C₃-C₈)-Cycloalkyl-(C₁-C₆)-alkyl-(C₁-C₆)-alkoxy, (C₃-C₈)-Cycloalkyl-(C₁-C₆)-alkoxy-(C₁-C₆)-alkyl, (C₃-C₈)-Cycloalkoxy-(C₁-C₆)-alkoxy-(C₁-C₆)-alkyl, NR^{Y}R^{Z}, (C₁-C₈)-Alkylmercapto, (C₁-C₈)-Alkylsulfinyl oder (C₁-C₈)-Alkylsulfonyl, (C₆-C₁₂)-Arylmercapto, (C₆-C₁₂)-Arylsulfinyl, (C₆-C₁₂)-Arylsulfonyl, (C₇-C₁₂)-Aralkylmercapto, (C₇-C₁₁)-Aralkylsulfinyl, (C₇-C₁₁)-Aralkylsulfonyl, substituiertes (C₆-C₁₂)-Aryloxy-(C₁-C₆)-alkyl, (C₇-C₁₁)-Aralkoxy-(C₁-C₆)-alkyl, (C₆-C₁₂)-Aryloxy-(C₁-C₆)-alkoxy-(C₁-C₆)-alkyl, (C₇-C₁₁)-Aralkyloxy-(C₁-C₆)-alkoxy-(C₁-C₆)-alkyl, (C₆-C₁₂)-Aryloxy, (C₇-C₁₁)-Aralkyloxy, (C₆-C₁₂)-Aryloxy-(C₁-C₆)-alkoxy oder (C₇-C₁₁)-Aralkoxy-(C₁-C₆)-alkoxy bedeutet, wobei ein aromatischer Rest mit 1, 2, 3, 4 oder 5 gleichen oder verschiedenen Substituenten aus der Reihe Wasserstoff, Halogen, Cyano, Nitro, Trifluormethyl, (C₁-C₁₆)-Alkyl, (C₁-C₁₆)-Alkenyl, (C₁-C₆)-Hydroxyalkyl, (C₁-C₁₆)-Alkoxy, (C₁-C₁₆)-Alkenyloxy, -O-[CH₂]ₓC_{f}H_{(2f+1-g)}F_{g}, -OCF₂Cl, -O-CF₂-CHFCl, (C₁-C₆)-Alkylmercapto, (C₁-C₆)-Alkylsulfinyl, (C₁-C₆)-Alkylsulfonyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkylcarbamoyl, Phenyl, Benzyl, Phenoxy, Benzyloxy, NR^{Y}R^{Z}, Phenylmercapto, Phenylsulfonyl, Phenylsulfinyl, Sulfamoyl N-(C₁-C₄)-Alkylsulfamoyl oder N,N-Di-(C₁-C₄)-alkylsulfamoyl trägt, oder gegebenenfalls bis zu 3 der vorstehend genannten gleichen oder verschiedenen Substituenten trägt und zwei benachbarte C-Atome des Aralkyloxyrestes gemeinsam eine Kette -[CH₂-] und/oder -CH=CH-CH=CH- tragen, wobei eine CH₂-Gruppe der Kette gegebenenfalls durch O, S, SO, SO₂ oder NR' ersetzt ist,
R¹ und R² oder R² und R³ eine Kette [CH₂]ₒ bilden, wobei o = 3, 4 oder 5 bedeutet, oder
zusammen mit dem sie tragenden Pyridin oder Pyridazin einen Cinnolin-, einen Chinolin- oder einen Isochinolin-Ring bilden,
R⁴ , falls Q eine Bindung ist, Fluor, Chlor oder Brom bedeutet, oder falls Q O ist, einen verzweigten oder unverzweigten (C₁-C₂₀)-Alkylrest, der bis zu 3 C-C-Mehrfachbindungen enthalten kann, einen unsubstituierten gesättigten Fluoralkylrest der Formel [CH₂]ₓC_{f}H_{(2f+1-g)}F_{g}, einen (C₆-C₁₆)-Arylrest oder einen (C₇-C₁₆)-Aralkylrest, der in der Alkylkette bis zu 2 C-C-Mehrfachverbindungen enthalten kann, oder einen Heteroarylrest oder einen Heteroarylalkylrest bedeutet, wobei diese Reste substituiert sind mit einem oder mehreren Resten aus der Reihe Hydroxy, Fluor, Chlor, Cyano, Trifluormethyl, Carboxy, (C₁-C₁₂)-Alkyl, (C₃-C₈)-Cycloalkyl, (C₃-C₈)-Cycloalkyl-(C₁-C₁₂)-alkyl, (C₃-C₈)-Cycloalkoxy, (C₃-C₈)-Cycloalkyl-(C₁-C₁₂)-alkoxy, (C₃-C₈)-Cycloalkyloxy-(C₁-C₁₂)-alkyl, (C₃-C₈)-Cycloalkyloxy-(C₁-C₁₂)-alkoxy, (C₃-C₈)-Cycloalkyl-(C₁-C₈)-alkyl-(C₁-C₆)-alkoxy, (C₃-C₈)-Cycloalkoxy-(C₁-C₈)-alkoxy-(C₁-C₈)-alkoxy, (C₆-C₁₂)-Aryl, (C₇-C₁₆)-Aralkyl, (C₂-C₁₂)-Alkenyl, (C₂-C₁₂)-Alkinyl, (C₁-C₁₂)-Alkoxy, (C₁-C₁₂)-Alkoxy-(C₁-C₁₂)-alkoxy, (C₁-C₁₂)-Alkoxy-(C₁-C₈)-alkoxy-(C₁-C₈)-alkyl, (C₆-C₁₂)-Aryloxy, (C₇-C₁₆)-Aralkyloxy, (C₆-C₁₂)-Aryloxy-(C₁-C₆)-alkoxy, (C₇-C₁₆)-Aralkoxy-(C₁-C₆)-alkoxy, (C₁-C₈)-Hydroxyalkyl, -O-[CH₂₋]ₓC_{f}H_{(2f+1-g)}F_{g}, (C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₁₂)-Alkenyloxycarbonyl, (C₂-C₁₂)-Alkinyloxycarbonyl, (C₃-C₈)-Cycloalkyl-(C₁-C₆)-alkoxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-Cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₀)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl, CON(CH₂)ₕ, worin eine CH₂-Gruppe durch O, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino oder N-(C₇-C₁₆)-Aralkylimino ersetzt ein kann und h 3 bis 6 bedeutet,
wobei die Reste, die einen Arylrest enthalten ihrerseits am Aryl substituiert sein können durch 1 bis 5 gleiche oder verschiedene Reste aus der Reihe:
Hydroxy, Fluor, Chlor, Cyano, Trifluormethyl, Carboxy, (C₁-C₁₂)-Alkyl, (C₃-C₈)-Cycloalkyl, (C₁-C₆)-Alkoxy, (C₃-C₈)-Cycloalkoxy, (C₁-C₁₂)-Alkoxycarbonyl,
N-(C₁-C₆)-Alkylcarbamoyl, N, N-Di-(C₁-C₆)-alkylcarbamoyl, N-(C₃-C₈)-Cycloalkylcarbamoyl, und
R⁴ R'' bedeutet, sofern Q in der Bedeutung von NR' steht, wobei R' und R'' gleich oder verschieden sind und Wasserstoff, (C₁-C₈)-Alkyl oder gegebenenfalls mit Fluor, Chlor, (C₁-C₄)-Alkoxy einfach substituiertes (C₇-C₁₁)-Aralkyl bedeuten,
R^{Y} und R^{Z} gleich oder verschieden sind und Wasserstoff, (C₆-C₁₂)-Aryl, (C₁-C₁₀)-Alkyl, (C₃-C₁₀)-Cycloalkyl, (C₁-C₈)-Alkoxy-(C₁-C₈)-alkyl, (C₇-C₁₂)-Aralkoxy-(C₁-C₈)-alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, oder
R^{Y} und R^{Z} gemeinsam für -[CH₂]ₕ₋ stehen, worin eine CH₂-Gruppe durch O, S, N-(C₁-C₄)-Alkanoylimino oder N-(C₁-C₄)-Alkoxycarbonylimino ersetzt sein kann, und
f 1 bis 8,
g 0, 1 bis (2f + 1),
h 3 bis 6,
x 0 bis 3, und
n 3 oder 4 ist, einschließlich der physiologisch wirksamen Salze, wobei 3-Benzyloxypyridin-2-carbonsäure-(L-threonylmethylester)amid, 3-Benzyloxypyridin-2-carbonsäure-(L-threonyl(Fmoc-Phg)tert. butylester)amid, 3-Benzyloxypyridin-2-carbonsäure-(L-threonyl-tert. butylester)amid, 3-Benzyloxypyridin-2-carbonsäure-(D-allothreonylmethylester)amid und 3-Benzyloxypicolinylglycin ethyl ester ausgenommen sind.

7. Verbindungen der Formel I gemäß den Ansprüchen 1 bis 3 und 6, in der
Q O, NR' oder eine Bindung bedeutet,
X O,
Y CR³ oder, falls R¹ und R² einen Cyclus bilden, N oder CR³ bedeuten,
m 0
A (C₁-C₃)-Alkylen, das gegebenenfalls einfach substituiert ist mit Halogen, Cyano, Trifluormethyl, (C₁-C₆)-Alkyl, (C₁-C₆)-Hydroxyalkyl, (C₁-C₆)-Alkoxy, -O-[CH₂]ₓ-C_{f}H_{(2f+1-g)}F_{g} oder
A -CHR⁵- bedeutet, wobei R⁵ einen der Substituenten des α-C-Atoms einer α-Aminosäure bedeutet, insbesondere einer natürlichen L-Aminosäure und ihres D-Isomeren,
B -CO₂G bedeutet, wobei
G einen verzweigten oder unverzweigten oder cyclischen aliphatischen (C₁-C₂₀)-Alkyl-Rest, einen Retinyl-Rest oder
einen verzweigten oder unverzweigten (C₂-C₂₀)-Alkenyl-Rest oder einen (C₂-C₂₀)-Alkinyl-Rest, der jeweils eine oder mehrere C-C-Mehrfachbindungen enthalten kann, oder
einen (C₆-C₁₂)-Aryl-Rest, einen (C₇-C₁₁)-Aralkyl-Rest oder einen Heteroaryl- oder Heteroaralkyl-Rest bedeutet,
wobei die vorstehenden Reste einen oder zwei Substituenten aus der Reihe (C₁-C₈)-Alkyl, (C₃-C₈)-Cycloalkyl, Fluor, Chlor, Hydroxy, (C₁-C₆)-Alkoxy, (C₁-C₆)-Alkoxy-(C₁-C₆)alkoxy, (C₆-C₁₂)-Aryloxy, (C₇-C₁₂)-Aralkyloxy,
(C₁-C₈)-Alkylcarbonyl, (C₃-C₈)-Cycloalkylcarbonyl, (C₆-C₁₂)-Arylcarbonyl, (C₇-C₁₂)-Aralkylcarbonyl,
(C₁-C₈)-Alkoxycarbonyl, (C₁-C₆)-Alkoxy-(C₁-C₆)-alkoxycarbonyl, (C₆-C₁₂)-Aryloxycarbonyl, (C₇-C₁₂)-Aralkoxycarbonyl, (C₃-C₈)-Cycloalkoxycarbonyl,
(C₁-C₂₀)-Alkylcarbonyloxy, (C₃-C₈)-Cycloalkylcarbonyloxy, (C₆-C₁₂)-Arylcarbonyloxy, (C₇-C₁₂)-Aralkylcarbonyloxy,
(C₁-C₈)-Alkoxycarbonyloxy, (C₁-C₆)-Alkoxy-(C₁-C₆)-alkoxycarbonyloxy, (C₆-C₁₂)-Aryloxycarbonyloxy, (C₇-C₁₂)-Aralkyloxycarbonyloxy, (C₃-C₈)-Cycloalkoxycarbonyloxy,
Carbamoyl, N-(C₁-C₈)-Alkylcarbamoyl, N,N-Di-(C₁-C₈)-alkylcarbamoyl, N-(C₃-C₈)-Cycloalkylcarbamoyl,
N-((C₁-C₆)-Alkoxy-(C₁-C₆)alkyl)carbamoyl,
Amino, (C₁-C₆)-Alkylamino, Di-(C₁-C₆)alkylamino, (C₃-C₈)-Cycloalkylamino,N-(C₆-C₁₂)-Arylamino, N-(C₇-C₁₁)-Aralkylamino, N-(C₁-C₅)-Alkyl-(C₆-C₁₂)-Arylamino,
(C₁-C₈)-Alkanoylamino, (C₃-C₈)-Cycloalkanoylamino, (C₆-C₁₂)-Aroylamino, (C₇-C₁₂)-Aralkanoylamino, (C₁-C₈)-Alkanoyl-N-(C₁-C₆)-alkylamino, (C₃-C₈)-Cycloalkanoyl-N-(C₁-C₆)-alkylamino, (C₆-C₁₂)-Aroyl-N-(C₁-C₆)alkylamino, (C₇-C₁₁)-Aralkanoyl-N-(C₁-C₆)-alkylamino tragen können, und
wobei die Reste, die einen Arylrest enthalten, insbesondere substituiert sind mit bis zu 3 Substituenten aus der Reihe
Hydroxy, Fluor, Chlor, Cyano, Trifluormethyl, (C₁-C₆)-Alkyl, (C₃-C₈)-Cycloalkyl, (C₁-C₈)-Alkoxy,
(C₁-C₆)-Alkylcarbonyl, (C₃-C₈)-Cycloalkylcarbonyl,
(C₁-C₆)-Alkoxycarbonyl, (C₃-C₈)-Cycloalkoxycarbonyl,
(C₁-C₆)-Alkylcarbonyloxy, (C₃-C₈)-Cycloalkylcarbonyloxy,
(C₁-C₆)-Alkoxycarbonyloxy, (C₃-C₈)-Cycloalkoxycarbamoyloxy,
Carbamoyl, N-(C₁-C₆)-Alkylcarbamoyl, N,N-Di-(C₁-C₆)-alkylcarbamoyl,
N-(C₃-C₈)-Cycloalkylcarbamoyl,
N-((C₁-C₆)-Alkoxy-(C₁-C₆)alkyl)carbamoyl,
N-(C₁-C₆)-Alkyl-N-((C₁-C₆)-Alkoxy-(C₁-C₆)alkyl)carbamoyl,
Carbamoyloxy, N-(C₁-C₆)-Alkylcarbamoyloxy, N,N-Di-(C₁-C₆)-alkylcarbamoyloxy, N-(C₃-C₈)-Cycloalkylcarbamoyloxy,
(C₁-C₆)-Alkanoylamino, (C₃-C₈)-Cycloalkanoylamino,
(C₁-C₆)Alkylmercapto, (C₁-C₆)-Alkylsulfinyl, (C₁-C₆)-Alkylsulfonyl, und
R² Wasserstoff, (C₁-C₂₀)-Alkyl, (C₂-C₂₀)-Alkenyl, (C₂-C₂₀)-Alkenyloxy, (C₂-C₂₀)-Alkinyloxy, Retinyloxy, (C₁-C₂₀)-Alkoxy-(C₁-C₃)-alkyl, (C₁-C₂₀)-Alkoxy-(C₁-C₃)-alkyl, (C₂-C₂₀)-Alkenyloxy-(C₁-C₃)-alkyl, Retinyloxy-(C₁-C₃)-alkyl, (C₂-C₂₀)-Alkinyloxy-(C₁-C₃)-alkyl, (C₁-C₂₀)-Alkoxy, Halogen, Cyano, Trifluormethyl, (C₁-C₁₆)-Hydroxyalkyl, (C₁-C₂₀)-Alkanoyl,(C₇-C₁₂)-Aralkanoyl, (C₆-C₁₂)-Aroyl, -O-[CH₂]ₓC_{f}H_{(2f+1-g)}F_{g}, NR'R'', (C₁-C₁₀)-Alkylmercapto, (C₁-C₁₀)-Alkylsulfinyl, (C₁-C₁₀)-Alkylsulfonyl, (C₆-C₁₂)-Arylmercapto, (C₆-C₁₂)-Arylsulfinyl), (C₆-C₁₂)-Arylsulfonyl, (C₇-C₁₂)-Aralkylmercapto, (C₇-C₁₂)-Aralkylsulfinyl, (C₇-C₁₂)-Aralkylsulfonyl, (C₆-C₁₂)-Aryloxy, (C₇-C₁₆)-Aralkyloxy, Carboxy, (C₁-C₂₀)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl, (C₂-C₂₀)-Alkenyloxycarbonyl, Retinyloxycarbonyl, (C₂-C₂₀)-Alkinyloxycarbonyl, (C₃-C₈)-Cycloalkyl-(C₁-C₆)-alkoxycarbonyl, (C₃-C₈)-Cycloalkoxy-(C₁-C₆)-alkoxycarbonyl, (C₆-C₁₂)-Aryloxy-(C₁-C₆)-alkoxycarbonyl, (C₇-C₁₆)-Aralkoxy-(C₁-C₆)-alkoxycarbonyl, Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo(C₃-C₈)-alkylcarbamoyl, N-(C₁-C₁₀)-Alkyl-N-(C₃-C₈)-cycloalkylcarbamoyl, N-(C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₂)-aralkylcarbamoyl, N-((C₁-C₁₂)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl,N-(C₁-C₁₀)-Alkyl-N-((C₁-C₁₀)-alkoxy-(C₁-C₁₀)-alkyl)carbamoyl,
N-(C₁-C₁₀)-Alkyl-N-((C₆-C₁₂)-aryloxy-(C₁-C₁₀)-alkyl)carbamoyl,
N-(C₁-C₁₀)-Alkyl-N-((C₇-C₁₆)-aralkyloxy-(C₁-C₁₀)-alkyl)carbamoyl,
CON(CH₂)ₕ, worin eine CH₂-Gruppe durch O, S, N-(C₁-C₈)-alkylimino,
N-(C₃-C₈)-Cycloalkylimino, N-(C₃-C₈)-Cycloalkyl-(C₁-C₄)-alkylimino,
N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 6 bedeutet,
wobei Aryl in der Weise substiuiert ist wie für R¹ und R³ definiert,
R¹ und R³ gleich oder verschieden sind und Wasserstoff, Halogen, (C₁-C₁₂)-Alkyl, (C₁-C₁₂)-Alkoxy, -O-[CH₂]ₓ-C_{f}H_{(2f+1-g)}Hal_{g}, (C₁-C₁₂)-Alkoxy-(C₁-C₁₂)-alkyl, (C₁-C₈)-Alkoxy-(C₁-C₁₂)-alkoxy, (C₁-C₁₂)-Alkoxy-(C₁-C₈)-alkoxy-(C₂-C₆)-alkyl, (C₇-C₁₁)-Aralkyloxy, (C₃-C₈)-Cycloalkyl, (C₃-C₈)-Cycloalkyl-(C₁-C₈)-alkyl, (C₃-C₈)-Cycloalkyloxy, (C₃-C₈)-Cycloalkyl-(C₁-C₈)-alkoxy, (C₃-C₈)-Cycloalkyloxy-(C₁-C₈)-alkyl, (C₃-C₈)-Cycloalkyloxy-(C₁-C₈)-alkoxy, (C₃-C₈)-Cycloalkyl-(C₁-C₆)-alkyl-(C₁-C₆)-alkoxy, (C₃-C₈)-Cycloalkyl-(C₁-C₆)-alkoxy-(C₁-C₆)-alkyl, (C₃-C₈)-Cycloalkoxy-(C₁-C₆)-alkoxy-(C₁-C₆)-alkyl, NR^{Y}R^{Z}, (C₁-C₈)-Alkylmercapto, (C₁-C₈)-Alkylsulfinyl oder (C₁-C₈)-Alkylsulfonyl, (C₆-C₁₂)-Arylmercapto, (C₆-C₁₂)-Arylsulfinyl, (C₆-C₁₂)-Arylsulfonyl, (C₇-C₁₂)-Aralkylmercapto, (C₇-C₁₁)-Aralkylsulfinyl, (C₇-C₁₁)-Aralkylsulfonyl, substituiertes (C₆-C₁₂)-Aryloxy-(C₁-C₆)-alkyl, (C₇-C₁₁)-Aralkoxy-(C₁-C₆)-alkyl, (C₆-C₁₂)-Aryloxy-(C₁-C₆)-alkoxy-(C₁-C₆)-alkyl, (C₇-C₁₁)-Aralkyloxy-(C₁-C₆)-alkoxy-(C₁-C₆)-alkyl, (C₆-C₁₂)-Aryloxy, (C₇-C₁₁)-Aralkyloxy, (C₆-C₁₂)-Aryloxy-(C₁-C₆)-alkoxy oder (C₇-C₁₁)-Aralkoxy-(C₁-C₆)-alkoxy bedeutet, wobei ein aromatischer Rest mit 1, 2, 3, 4 oder 5 gleichen oder verschiedenen Substituenten aus der Reihe Wasserstoff, Halogen, Cyano, Nitro, Trifluormethyl, (C₁-C₁₂)-Alkyl, (C₁-C₁₂)-Alkenyl, (C₁-C₆)-Hydroxyalkyl, (C₁-C₁₂)-Alkoxy, (C₁-C₁₂)-Alkenyloxy, -O-[CH₂]ₓC_{f}H_{(2f+1-g)}F_{g}, -OCF₂Cl, -O-CF₂-CHFCl, (C₁-C₆)-Alkylmercapto, (C₁-C₆)-Alkylsulfinyl, (C₁-C₆)-Alkylsulfonyl, (C₁-C₆)-Alkylcarbonyl,(C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkylcarbamoyl, Phenyl, Benzyl, Phenoxy, Benzyloxy, NR^{Y}R^{Z}, Phenylmercapto, Phenylsulfonyl, Phenylsulfinyl, Sulfamoyl, N-(C₁-C₄)-Alkylsulfamoyl oder N,N-Di-(C₁-C₄)-alkylsulfamoyl trägt, oder gegebenenfalls bis zu 3 der vorstehend genannten gleichen oder verschiedenen Substituenten trägt und zwei benachbarte C-Atome des Aralkyloxyrestes gemeinsam eine Kette -[CH₂-] und/oder -CH=CH-CH=CH- tragen, wobei eine CH₂-Gruppe der Kette gegebenenfalls durch O, S, SO, SO₂ oder NR^{Y} ersetzt ist,
R¹ und R² oder R² und R³ eine Kette [CH₂]ₒ bilden können, wobei o = 3, 4 oder 5 bedeutet, und
R⁴ sofern Q eine Bindung ist, Chlor bedeutet oder
falls Q O ist, einen verzweigten oder unverzweigten (C₁-C₁₀)-Alkylrest, der eine oder zwei C-C-Mehrfachbindungen enthalten kann, oder einen unsubstituierten Fluoralkylrest der Formel -[CH₂]ₓC_{f}H_{(2f+1-g)}F_{g} oder (C₁-C₈)-Alkoxy-(C₁-C₆)-alkyl, (C₁-C₆)-Alkoxy-(C₁-C₄)-alkoxy-(C₁-C₄)-alkyl oder einen Rest der Formel Z bedeutet,
-[CH₂]ᵥ-[O]_{w}-[CH₂]ₜ-E (Z),
wobei E einen substituierten Phenylrest der Formel F 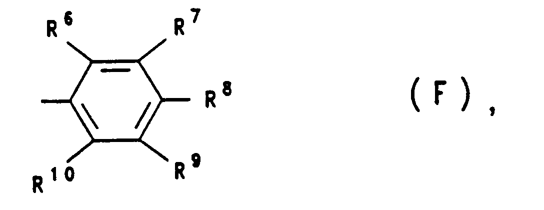 oder einen (C₃-C₈)-Cycloalkylrest bedeutet, wobei
v = 0, 1, 2, 3, 4, 5, 6, w = 0,1 und t = 0, 1, 2, 3 bedeutet mit der Einschränkung, daß v ungleich 0 ist, falls w = 1 ist, und
R⁶, R⁷, R⁸, R⁹ und R¹⁰ gleich oder verschieden sind und Wasserstoff, Halogen, Cyano, Nitro, Trifluormethyl, (C₁-C₆)-Alkyl, (C₃-C₈-Cycloalkyl, (C₁-C₆)-Alkoxy, -O-[CH₂₋]ₓC_{f}H_{(2f+1-g)}F_{g}, -OCF₂Cl, -O-CF₂-CHFCl, (C₁-C₆)-Alkylmercapto, (C₁-C₆)-Hydroxyalkyl, (C₁-C₆)-Alkoxy-(C₁-C₆)-alkoxy, (C₁-C₆)-Alkoxy-(C₁-C₆)-alkyl, (C₁-C₆)-Alkylsulfinyl, (C₁-C₆)-Alkylsulfonyl, (C₁-C₆)-Alkylcarbonyl, (C₁-C₈)-Alkoxycarbonyl, Carbamoyl, N-(C₁-C₈)-Alkylcarbamoyl, N,N-Di-(C₁-C₈)-alkylcarbamoyl, gegebenenfalls mit Fluor, Chlor, Brom, Trifluormethyl und (C₁-C₆)-Alkoxy-substituiertes (C₇-C₁₁)-Aralkylcarbamoyl, N-(C₃-C₈)-Cycloalkylcarbamoyl, N-(C₃-C₈)-Cycloalkyl-(C₁-C₄)-alkylcarbamoyl, (C₁-C₆)-Alkylcarbonyloxy, Phenyl, Benzyl, Phenoxy, Benzyloxy, NR^{Y}R^{Z}, wie Amino, Anilino, N-Methylanilino, Phenylmercapto, Phenylsulfonyl, Phenylsulfinyl, Sulfamoyl, N-(C₁-C₈)-Alkylsulfamoyl oder N,N-Di-(C₁-C₈)-alkylsulfamoyl bedeuten, oder zwei benachbarte Substituenten gemeinsam eine Kette -[CH₂₋]ₙ oder - CH=CH-CH=CH- bedeuten, wobei eine CH₂-Gruppe der Kette gegebenenfalls durch O, S, SO, SO₂ oder NR^{Y} ersetzt ist und wobei ein Heteroarylrest 1, 2 oder 3 Substituenten und ein Cycloalkylrest einen Substituenten aus der vorstehenden Reihe tragen kann, und
R⁴ R'' bedeutet, sofern Q in der Bedeutung von NR' steht, wobei
R' Wasserstoff und Methyl und
R'' Benzyl bedeuten, und
sofern R¹ und/oder R³ in der Bedeutung von (C₆-C₁₂)-Aryloxy, (C₇-C₁₁)-Aralkyloxy, (C₆-C₁₂)-Aryloxy-(C₁-C₆)-alkoxy, (C₇-C₁₁)-Aralkoxy -(C₁-C₆)-alkoxy oder einem entsprechenden endständige Cycloalkyl-Gruppen enthaltenden Rest stehen, dieser Rest vorzugsweise für einen Rest der Formel D steht,
OZ (D), oder
sofern R¹ und/oder R³ in der Bedeutung von (C₇-C₁₁)-Aralkyl, (C₆-C₁₂)-Aryloxy-(C₁-C₆)-alkyl, (C₇-C₁₁)-Aralkoxy-(C₁-C₆)-alkyl oder einem entsprechenden endständige Cycloalkyl-Gruppen enthaltenden Rest stehen, dieser Rest vorzugsweise für einen Rest der Formel Z steht,
R^{Y} und R^{Z} gleich oder verschieden sind und Wasserstoff, (C₆-C₁₂)-Aryl, (C₁-C₁₀)-Alkyl, (C₃-C₁₀)-Cycloalkyl, (C₁-C₈)-Alkoxy-(C₁-C₈)-alkyl, (C₇-C₁₂)-Aralkoxy-(C₁-C₈)-alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, oder
R^{Y} und R^{Z} gemeinsam für -[CH₂]ₕ₋ stehen, worin eine CH₂-Gruppe durch O, S, N-(C₁-C₄)-Alkanoylimino oder N-(C₁-C₄)-Alkoxycarbonylimino ersetzt sein kann, und
f 1 bis 8,
g 0, 1 bis (2f + 1),
h 3 bis 6,
x 0 bis 3, und
n 3 oder 4 ist,
einschließlich der physiologisch wirksamen Salze,
wobei 3-Benzyloxypyridin-2-carbonsäure-(L-threonylmethylester)amid, 3-Benzyloxypyridin-2-carbonsäure-(L-threonyl(Fmoc-Phg)tert. butylester)amid, 3-Benzyloxypyridin-2-carbonsäure-(L-threonyl-tert. butylester)amid, 3-Benzyloxypyridin-2-carbonsäure-(D-allothreonylmethylester)amid und 3-Benzyloxypicolinylglycin ethyl ester ausgenommen sind.

8. Verbindungen der Formel I gemäß den Ansprüchen 1 bis 3, 6 und 7, in der
Q O,
X O,
Y CR³ und zusätzlich N, falls R¹ und R² einen Cyclus bilden,
m 0,
A -CHR⁵-, wobei R⁵ den Substituenten des α-C-Atoms einer α-Aminosäure bedeutet, insbesondere einer natürlichen L-Aminosäure oder ihr D-Isomeres,
B CO₂G, bedeutet, wobei
G für einen verzweigten oder unverzweigten oder cyclischen aliphatischen (C₁-C₁₈)-Alkyl-Rest, einen (C₃-C₈)-Cycloalkyl-(C₁-C₈)-alkylrest,
einen verzweigten oder unverzweigten (C₂-C₁₈)-Alkenylrest, wie z.B. einen Geranyl- oder Farnesyl-Rest oder einen Retinyl-Rest, oder (C₂-C₁₈)-Alkinylrest, einen Phenyl-, Benzyl-, Phenethyl-, Phenylpropyl- oder Phenylbutylrest steht,
wobei die vorstehenden Reste einen Substituenten aus der Reihe Hydroxy, (C₁-C₄)-Alkoxy, Acyloxy, (C₁-C₆)-Alkylcarbonyloxy, (C₃-C₈)-Cycloalkylcarbonyloxy, Benzoyloxy, (C₇-C₁₆)-Phenylalkylcarbonyloxy, (C₃-C₈)-Cycloalkoxycarbonyloxy enthalten,
R² Wasserstoff, Brom, Chlor, Cyano, (C₁-C₁₈)-Alkyl, (C₁-C₈)-Alkoxy, (C₁-C₁₈)-Alkoxymethyl, (C₂-C₁₈)-Alkenyloxymethyl, (C₂-C₁₈)-Alkinyloxymethyl,Carbamoyl, N-(C₁-C₁₀)-Alkylcarbamoyl, N-((C₁-C₁₂)-Alkoxy-(C₁-C₄)-alkyl)carbamoyl, N, N-Di-(C₁-C₈)-alkylcarbamoyl, N-(C₃-C₈)-Cycloalkylcarbamoyl, N-(C₆-C₁₂)-Phenylcarbamoyl, N-(C₇-C₁₂)-Phenylalkylcarbamoyl, N-(C₁-C₆)-Alkyl-N-(C₆-C₁₂)phenylcarbamoyl, N-(C₁-C₆)-Alkyl-N-(C₇-C₁₂)-phenylalkylcarbamoyl, N-((C₁-C₆)-Alkoxy-(C₁-C₆)-alkyl)carbamoyl, Carboxy, (C₁-C₂₀)-Alkoxycarbonyl, (C₂-C₂₀)-Alkenyloxycarbonyl, Retinyloxycarbonyl, (C₃-C₈)-Cycloalkoxycarbonyl, (C₃-C₈)-Cycloalkyl-(C₁-C₆)-alkoxycarbonyl, (C₃-C₈)-Cycloalkoxy-(C₁-C₆)-alkoxycarbonyl, Phenyl-(C₁-C₆)-alkoxycarbonyl, Phenoxy-(C₁-C₆)-alkoxycarbonyl, Benzyloxy-(C₁-C₆)-alkoxycarbonyl, wobei ein Phenylrest in der Weise substituiert ist wie für R¹ und R³ definiert, und einer der Reste
R¹ oder R³ Wasserstoff und der andere einen Rest aus der Reihe Wasserstoff, Fluor, Chlor, (C₁-C₈)-Alkyl, (C₁-C₁₀)-Alkoxy, (C₅-C₆)-Cycloalkyl, (C₅-C₆)-Cycloalkyl-(C₁-C₆)-alkyl, (C₅-C₆)-Cycloalkyloxy, (C₅-C₆)-Cycloalkyl-(C₁-C₆)-alkoxy, (C₅-C₆)-Cycloalkyloxy-(C₁-C₆)-alkyl, (C₅-C₆)-Cycloalkyloxy-(C₁-C₆)-alkoxy, (C₅-C₆)-Cycoalkyl-(C₁-C₄)-alkyl-(C₁-C₄)-alkoxy, (C₅-C₆)-Cycloalkyl-(C₁-C₄)-alkoxy-(C₁-C₂)-alkyl, (C₅-C₆)-Cycloalkoxy-(C₁-C₄)-alkoxy-(C₁-C₂)-alkyl, -O-[CH₂]ₓ-C_{f}H_{(2f+1-g)}F_{g}, (C₁-C₆)-Alkoxy-(C₁-C₆)-alkyl, (C₁-C₆)-Alkoxy-(C₁-C₆)-alkoxy, (C₁-C₆)-Akoxy-(C₁-C₄)-alkoxy-(C₁-C₂)-alkyl, substituiertes (C₆-C₁₂)-Phenoxy, (C₇-C₁₁)-Phenylalkyloxy, (C₆-C₁₂)-Phenoxy-(C₁-C₆)-alkoxy oder (C₇-C₁₁)-Phenylalkoxy-(C₁-C₆)-alkoxy, Phenoxy-(C₁-C₄)-alkyl, (C₇-C₁₁)-Phenylalkyloxy-(C₁-C₄)-alkyl, Phenoxy-(C₁-C₄)-alkoxy-(C₁-C₂)-alkyl, (C₇-C₁₁)-Phenylalkyloxy-(C₁-C₄)-alkoxy-(C₁-C₂)-alkyl bedeutet, wobei ein aromatischer Rest mit 1, 2 oder 3 gleichen oder verschiedenen Substituenten aus der Reihe Fluor, Chlor, Cyano, Trifluormethyl, (C₁-C₁₂)-Alkyl, (C₂-C₁₂)-Alkenyl, (C₂-C₁₂)-Alkenyloxy, (C₁-C₁₂)-Alkoxy, substituiert ist,
R¹ und R² mit dem sie tragenden Pyridin einen 5,6,7,8-Tetrahydroisochinolin-Ring bilden,
R⁴ einen verzweigten oder unverzweigten (C₁-C₁₀)-Alkylrest, (C₁-C₄)-Alkoxy-(C₁-C₄)-alkyl oder einen Rest der Formel Z bedeutet,
-[CH₂]ᵥ-[O]_{w}-[CH₂]ₜ-E (Z),
wobei E einen substituierten Phenylrest der Formel F 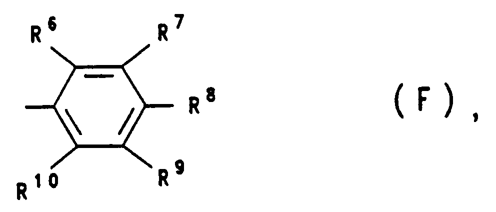 oder einen (C₃-C₈)-Cycloalkylrest bedeutet, wobei
v = 0, 1, 2, 3, w = 0, und t = 0, 1 sein kann und
worin R⁶, R⁷, R⁸, R⁹ und R¹⁰ gleich oder verschieden sind und
Wasserstoff, Fluor, Chlor, Cyano, Trifluormethyl, (C₁-C₆)-Alkyl, (C₁-C₆)-Alkoxy, -O-[CH₂₋]ₓC_{f}H_{(2f+1-g)}F_{g}, N-(C₁-C₈)-Alkylcarbamoyl, N,N-Di-(C₁-C₆)-alkylcarbamoyl, N-(C₃-C₆)-Cycloalkylcarbamoyl, N-(+)-Dehydroabietylaminocarbonyl, gegebenenfalls mit Fluor, Chlor, Trifluormethyl und (C₁-C₆)-Alkoxy substituiertes (C₇-C₁₁)-Phenylalkylcarbamoyl bedeuten, oder wobei
R⁶ und R⁷ oder R⁷ und R⁸ zusammen mit dem sie tragenden Phenylring Naphthalin-Derivate bilden, oder
falls R¹ oder R³ in der Bedeutung von (C₆-C₁₂)-Phenoxy, (C₇-C₁₁)-Phenylalkyloxy, (C₆-C₁₂)-Phenoxy-(C₁-C₆)-alkoxy, (C₇-C₁₁)-Phenylalkoxy-(C₁-C₆)-alkoxy, (C₅-C₆)-Cycloalkyloxy, (C₅-C₆)-Cycloalkyl-(C₁-C₆)-alkoxy, (C₅-C₆)-Cycloalkoxy-(C₁-C₆)-alkoxy oder (C₅-C₆)-Cycloalkyl-(C₁-C₄)-alkyl-(C₁-C₄)-alkoxy steht, so bedeutet dieser Rest im speziellen einen Rest der Formel D
OZ (D), oder
falls R¹ oder R³ in der Bedeutung von Phenyl, Phenoxy-(C₁-C₆)-alkyl, (C₇-C₁₁)-Phenylalkyl, (C₇-C₁₁)-Phenylalkyloxy-(C₁-C₄)-alkyl, (C₅-C₆)-Cycloalkyl, (C₅-C₆)-Cycloalkyl-(C₁-C₆)-alkyl, (C₅-C₆)-Cycloalkoxy-(C₁-C₄)-alkyl, (C₅-C₆)-Cycloalkyl-(C₁-C₄)-alkoxy-(C₁-C₂)-alkyl, (C₅-C₆)-Cycloalkoxy-(C₁-C₄)-alkoxy-(C₁-C₂)-alkyl, steht, so bedeutet dieser Rest im speziellen einen Rest der Formel Z worin in beiden Fällen
v = 1, 2, 3 und 4, w = 0 und t = 0 oder
v = 1,2, 3 und 4, w = 1 und t = 0 oder
v = 1, 2, 3 und 4, w = 1, t = 1 und
f = 1 bis 4
g = 0,1 bis (2f + 1)
x = o und 1 bedeutet,
einschließlich der physiologisch wirksamen Salze,
wobei 3-Benzyloxypyridin-2-carbonsäure-(L-threonylmethylester)amid, 3-Benzyloxypyridin-2-carbonsäure-(L-threonyl(Fmoc-Phg)tert. butylester)amid, 3-Benzyloxypyridin-2-carbonsäure-(L-threonyl-tert. butylester)amid, 3-Benzyloxypyridin-2-carbonsäure-(D-allothreonylmethylester)amid und 3-Benzyloxypicolinylglycin ethyl ester ausgenommen sind.

9. Verbindungen der Formel I gemäß den Ansprüchen 1 bis 3, 6, 7 und 8, in der
Q O,
X O,
Y CR³,
m 0,
A eine -CH₂-Gruppe bedeutet, die mit einer Methylgruppe substituiert sein kann,
B CO₂G, wobei
G für einen verzweigten oder unverzweigten oder cyclischen aliphatischen (C₁-C₁₈)-Alkyl-Rest, einen (C₃-C₈)-Cycloalkyl-(C₁-C₄)-alkylrest, einen verzweigten oder unverzweigten (C₂-C₁₈)-Alkenylrest steht, wobei die vorstehenden Reste einen Substituenten aus der Reihe Hydroxy, (C₁-C₄)-Alkoxy, Acyloxy, (C₁-C₆)-Alkylcarbonyloxy, (C₃-C₈)-Cycloalkylcarbonyloxy, Benzoyloxy, (C₇-C₁₆)-Phenylalkylcarbonyloxy, (C₃-C₈)-Cycloalkoxycarbonyloxy enthalten können, oder
G für einen Phenyl-, Benzyl-, Phenethyl-, Phenylpropyl- oder Phenylbutylrest steht,
R² Wasserstoff, (C₁-C₈)-Alkoxy, (C₁-C₁₆)-Alkoxymethyl, (C₂-C₁₆)-Alkenyloxymethyl, Retinyloxymethyl, N-(C₁-C₁₀)-Alkylcarbamoyl, N-((C₁-C₁₂)-Alkoxy-(C₁-C₃)-alkyl)carbamoyl, N, N-Di-(C₁-C₈)-alkylcarbamoyl, N-(C₅-C₆)-Cycloalkylcarbamoyl, N-Phenylcarbamoyl, N-Phenyl-(C₁-C₄)-alkylcarbamoyl, Carboxy, (C₁-C₁₆)-Alkoxycarbonyl, (C₂-C₁₆)-Alkenyloxycarbonyl, Retinyloxycarbonyl, (C₅-C₆)-Cycloalkoxycarbonyl, (C₅-C₆)-Cycloalkyl-(C₁-C₆)-alkoxycarbonyl, Phenyl-(C₁-C₆)-alkoxycarbonyl, wobei ein Phenylrest in der Weise substituiert ist wie für R¹ und R³ definiert, und einer der Reste
R¹ oder R³ Wasserstoff und der andere Rest einen Rest aus der Reihe Wasserstoff, (C₁-C₁₀)-Alkoxy, (C₅-C₆)-Cycloalkyloxy, (C₅-C₆)-Cycloalkyl-(C₁-C₂)-alkoxy, -O-[CH₂]ₓ-C_{f}H_{(2f+1-g)}F_{g}, (C₁-C₄)-Alkoxy-(C₁-C₄)-alkoxy, substituiertes (C₆-C₁₂)-Phenoxy, (C₇-C₁₁)-Phenylalkyloxy, (C₆-C₁₂)-Phenoxy-(C₁-C₄)-alkoxy oder (C₇-C₁₁)-Phenylalkoxy-(C₁-C₄)-alkoxy bedeutet, wobei ein aromatischer Rest mit 1, 2 oder 3 gleichen oder verschiedenen Substituenten aus der Reihe Fluor, Chlor, Cyano, Trifluormethyl, (C₁-C₁₀)-Alkyl, (C₁-C₁₀)-Alkoxy, (C₁-C₁₀)-Alkenyloxy substituiert ist und
R⁴ einen verzweigten oder unverzweigten (C₁-C₈)-Alkylrest oder einen Rest der Formel Z bedeutet,
-[CH₂]ᵥ-[O]_{w}-[CH₂]ₜ-E (Z),
wobei E einen substituierten Phenylrest der Formel F 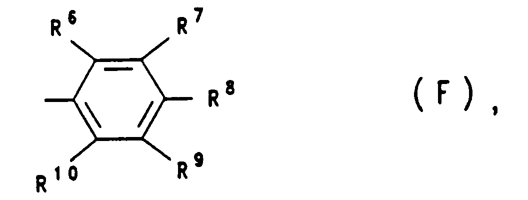 oder einen (C₃-C₈)-Cycloalkylrest bedeutet, wobei
v = 0, 1, 2, 3, w = 0, und t = 0, 1 sein kann und
worin R⁶, R⁷, R⁸, R⁹ und R¹⁰ gleich oder verschieden sind und
Wasserstoff, Fluor, Chlor, Cyano, Trifluormethyl, (C₁-C₆)-Alkyl, (C₁-C₆)-Alkoxy, -O-[CH₂₋]ₓC_{f}H_{(2f+1-g)}F_{g}, N-(C₁-C₈)-Alkylcarbamoyl, N,N-Di-(C₁-C₆)-alkylcarbamoyl, N-(C₃-C₆)-Cycloalkylcarbamoyl, N-(+)-Dehydroabietylaminocarbonyl substituierten Benzylrest und f = 1 bis 4, g = 0,1 bis (2f + 1) und x = 0 und 1 bedeuten einschließlich der physiologisch wirksamen Salze, wobei 3-Benzyloxypicolinylglycin ethyl ester ausgenommen ist.

10. Verbindungen der Formel I gemäß den Ansprüchen 1 bis 3 und 6 bis 9, in der
Q O,
X O,
Y CR³,
m 0,
A eine -CH₂-Gruppe bedeutet,
B CO₂G, wobei
G für einen verzweigten oder unverzweigten aliphatischen (C₁-C₁₆)-Alkyl-Rest, einen 2-Cyclohexylethyl-Rest, einen (C₁-C₄)-Alkoxy-(C₁-C₂)-alkyl-Rest, einen verzweigten oder unverzweigten (C₂-C₁₀)-Alkenylrest, einen Phenyl-, Benzyl-, Phenethyl-, Phenylpropyl- oder Phenylbutylrest steht
R¹ Wasserstoff, (C₁-C₆)-Alkoxy, -O-[CH₂]ₓ-C_{f}H_{(2f+1-g)}F_{g}
R² Wasserstoff, N-(C₁-C₁₀)-Alkylcarbamoyl, N-((C₁-C₁₂)-Alkoxy-(C₁-C₃)-alkyl)carbamoyl, N, N-Di-(C₁-C₈)-alkylcarbamoyl, N-(C₅-C₆)-Cycloalkylcarbamoyl, N-Phenylcarbamoyl, N-Phenyl-(C₁-C₂)-alkylcarbamoyl, Carboxy, (C₁-C₁₆)-Alkoxycarbonyl, (C₂-C₁₆)-Alkenyloxycarbonyl, Retinyloxycarbonyl, (C₅-C₆)-Cycloalkoxycarbonyl, (C₅-C₆)-Cycloalkyl-(C₁-C₆)-alkoxycarbonyl, Phenyl-(C₁-C₆)-alkoxycarbonyl, wobei ein Phenylrest mit 1 oder 2 gleichen oder verschiedenen Substituenten aus der Reihe Fluor, Chlor, Cyano, Trifluormethyl, (C₁-C₁₀)-Alkyl, (C₁-C₁₀)-Alkoxy, (C₁-C₁₀)-Alkenyloxy, substituiert ist und
R³ Wasserstoff, (C₁-C₅)-Alkoxy, (C₅-C₆)-Cycloalkyl-(C₁-C₂)-alkoxy, wobei einer der Substituenten R¹ oder R³ Wasserstoff bedeutet,
R⁴ einen verzweigten oder unverzweigten (C₁-C₆)-Alkylrest, oder einen 2-Phenylethyl-Rest, oder einen mit 1 oder 2 Resten aus der Reihe Fluor, Chlor, Cyano, Trifluormethyl, (C₁-C₆)-Alkyl, (C₁-C₆)-Alkoxy, -O-[CH₂₋]ₓC_{f}H_{(2f+1-g)}F_{g}, N-(C₁-C₈)-Alkylcarbamoyl, N,N-Di-(C₁-C₆)-alkylcarbamoyl, N-(C₃-C₆)-Cycloalkylcarbamoyl, N-(+)-Dehydroabietylaminocarbonyl substituierten Benzylrest bedeutet und f = 1 bis 4, g = 0,1 bis (2f + 1) und x = 1 bedeuten, einschließlich der physiologisch wirksamen Salze.

11. Verbindungen der Formel I gemäß den Ansprüchen 1 bis 3 und 6 bis 10, in der
Q O,
X O,
Y CR³,
m 0,
A eine -CH₂-Gruppe bedeutet,
B CO₂G, wobei
G für einen verzweigten oder unverzweigten aliphatischen (C₁-C₁₆)-Alkyl-Rest oder einen Benzyl-Rest steht,
R¹ Wasserstoff,
R² Wasserstoff, N-(C₁-C₁₀)-Alkylcarbamoyl, N-((C₁-C₁₂)-Alkoxy-(C₁-C₃)-alkyl)carbamoyl, N-Cyclohexylcarbamoyl, N-Phenylcarbamoyl, N-(Phenyl-(C₁-C₂)-alkyl)carbamoyl, wobei in den beiden letzten Fällen der Phenylrest einen Fluor-, einen (C₁-C₁₀)-Alkyl- oder einen (C₁-C₁₀)-Alkoxy-Substituenten tragen kann, Carboxy, (C₁-C₁₆)-Alkoxycarbonyl, (C₂-C₁₆)-Alkenyloxycarbonyl, Retinyloxycarbonyl, (C₅-C₆)-Cycloalkoxycarbonyl, Benzyloxycarbonyl,
R³ Wasserstoff, (C₁-C₅)-Alkoxy, 2-(Cyclohexyl)ethyloxy, wobei einer der Substituenten R² und R³ Wasserstoff bedeutet,
R⁴ einen verzweigten oder unverzweigten (C₁-C₄)-Alkylrest oder einen mit Fluor, Chlor, Trifluormethyl, (C₁-C₄)-Alkyl, (C₁-C₃)-Alkoxy einfach substituierten Benzylrest bedeuten, einschließlich der physiologisch wirksamen Salze.

12. Verbindungen der Formel I gemäß den Ansprüchen 1 bis 3 und 6, in der
Q O,
X O,
Y CR³,
wobei R³ Wasserstoff bedeutet,
m 0,
A eine -CH₂-Gruppe,
B -CO₂G, wobei
G für einen verzweigten oder unverzweigten aliphatischen (C₁-C₁₆)-Alkyl-Rest oder einen Benzyl-Rest steht,
R¹ und R² zusammen mit dem sie tragenden Pyridin einen Isochinolin-Ring mit unsubstituiertem Benzoteil bilden und
R⁴ Methyl bedeutet.

13. Verbindungen der Formel I gemäß den Ansprüchen 1 bis 3 und 6, in der
Q O,
X O,
Y CR³,
m O,
A eine -CH₂-Gruppe,
B -CO₂G, wobei
G für einen verzweigten oder unverzweigten aliphatischen (C₁-C₁₆)-Alkyl-Rest oder einen Benzyl-Rest steht,
R¹ Wasserstoff, und
R² und R³ zusammen mit dem sie tragenden Pyridin einen Chinolin-Ring mit unsubstituiertem Benzoteil bilden und
R⁴ Methyl bedeutet.

14. Verbindungen der Formel I gemäß den Ansprüchen 1, 2, 4 und 5, in denen
Q S,
X O,
Y CR³,
m 0,
A eine -CH₂-Gruppe bedeutet,
B -CO₂G, wobei
G für einen verzweigten oder unverzweigten aliphatischen (C₁-C₁₆)-Alkyl-Rest oder einen Benzyl-Rest steht,
R¹ Wasserstoff,
R² Wasserstoff, N-(C₁-C₁₀)-Alkylcarbamoyl, N-((C₁-C₁₂)-Alkoxy-(C₁-C₃)-alkyl)carbamoyl, N-Cyclohexylcarbamoyl, N-Phenylcarbamoyl, N-(Phenyl-(C₁-C₂)alkyl)carbamoyl, wobei in den beiden letzten Fällen der Phenylrest einen Fluor-, (C₁-C₁₀)-Alkyl- oder (C₁-C₁₀)-Alkoxy-Substituenten tragen kann, Carboxy, (C₁-C₁₆)-Alkoxycarbonyl, (C₂-C₁₆)-Alkenyloxycarbonyl, Retinyloxycarbonyl, (C₅-C₆)-Cycloalkoxycarbonyl, Benzyloxycarbonyl,
R³ Wasserstoff, (C₁-C₅)-Alkoxy, 2-(Cyclohexyl)ethyloxy, wobei einer der Substituenten R² und R³ Wasserstoff bedeutet,
R⁴ einen verzweigten oder unverzweigten (C₁-C₄)-Alkylrest oder einen mit Fluor, Chlor, Trifluormethyl, (C₁-C₄)-Alkyl oder (C₁-C₃)-Alkoxy einfach substituierten Benzylrest bedeuten.

15. Verbindungen der Formel I gemäß den Ansprüchen 1, 2, 4, 5 und 14, in denen
Q S,
X O,
Y CR³,
m 0,
A eine -CH₂-Gruppe bedeutet,
B -CO₂G, wobei
G für einen verzweigten oder unverzweigten aliphatischen (C₁-C₁₆)-Alkyl-Rest, oder einen Benzyl-Rest steht,
R¹ Wasserstoff,
R² Carboxy oder (C₁-C₁₆)-Alkoxycarbonyl
R³ Wasserstoff und
R⁴ einen unverzweigten oder unverzweigten (C₁-C₄)-Alkylrest bedeuten.

16. Verbindungen der Formel I gemäß den Ansprüchen 1 bis 15 zuzüglich 3-Benzyloxypyridin-2-carbonsäure-(L-threonylmethylester)amid, 3-Benzyloxypyridin-2-carbonsäure-(L-threonyl(Fmoc-Phg)tert. butylester)amid, 3-Benzyloxypyridin-2-carbonsäure-(L-threonyl-tert. butylester)amid, 3-Benzyoxypyridin-2-carbonsäure-(D-allothreonylmethylester)amid und 3-Benzyloxypicolinylglycin ethyl ester zur Anwendung als Arzneimittel.

17. Verbindungen gemäß den Ansprüchen 1 bis 16 für die Anwendung zur Hemmung der Kollagenbiosynthese.

18. Verbindungen gemäß den Ansprüchen 1 bis 16 als Ester-Prodrugs der Prolylhydroxylase-Inhibitoren der Formel I, in denen B Carboxyl oder dessen Salz bedeutet.

19. Verbindungen gemäß den Ansprüchen 1 bis 16 zur Anwendung als Fibrosupressiva.

20. Verbindungen gemäß den Ansprüchen 1 bis 16 zur Herstellung eines Arzneimittels gegen fibrotische Erkrankungen.

21. Verbindungen gemäß den Ansprüchen 1 bis 16 zur Herstellung eines Arzneimittels gegen fibrotische Erkrankungen der Leber.

22. Verbindungen gemäß den Ansprüchen 1 bis 16 zur Herstellung eines Arzneimittels gegen fibrotische Erkrankungen der Lunge.

23. Verbindungen gemäß den Ansprüchen 1 bis 16 zur Herstellung eines Arzneimittels gegen fibrotische Erkrankungen der Haut.

24. Verfahren zur Herstellung von Verbindungen nach Formel I gemäß den Ansprüchen 1 bis 15, bei der
A einen substituierten Alkylen-Teil,
B CO₂G
Y CR³ und
m 0 oder 1 bedeuten,
in dem
i1.) Pyridin-2-carbonsäuren der Formel II (R²³ = H) mit den Aminoestern der Formel III zu den Amidestern der Formel I umgesetzt werden, oder
i2.) Pyridin-2-carbonsäureester der Formel II (R²³ = niedrig Alkyl) unter den Bedingungen der Aminolyse zu den Verbindungen der Formel I umgesetzt werden; 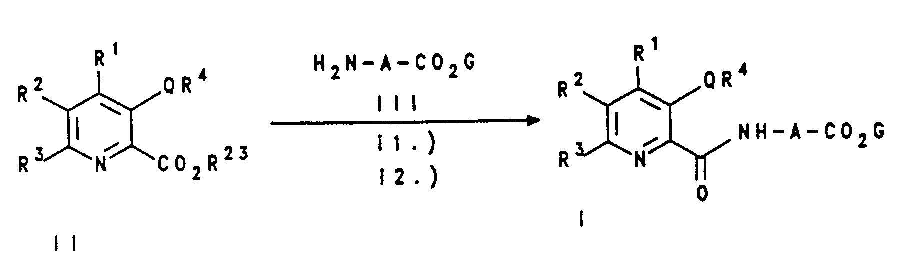 oder
ii) die Verbindungen der Formel IV mit dem Alkohol GOH zu Verbindungen der Formel I verestert werden; 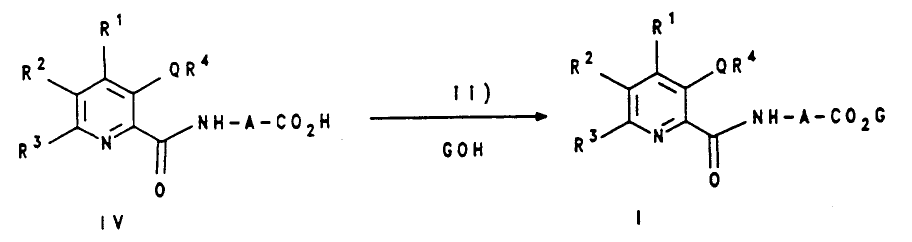 oder
iii) die Verbindungen der Formel V mit R⁴X zu Verbindungen der Formel I alkyliert werden ; 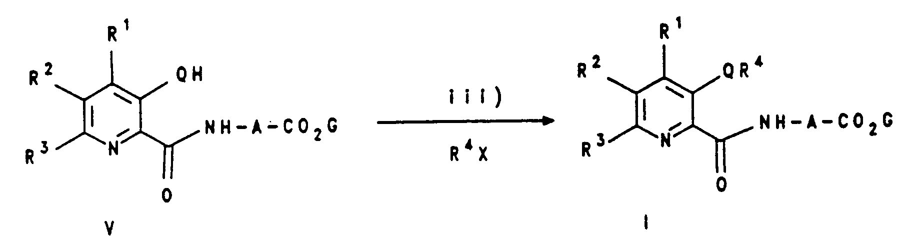 wobei X für eine Abgangsgruppe, insbesondere für Halogen, OSO₂Me, OSO₂ Phenyl steht, und ggf.
iv) die Verbindungen der Formel I, sofern Q = O und NR' gilt, in ihre Pyridin-N-oxide (I') 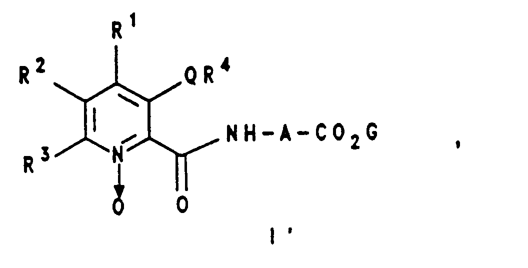 übergeführt werden.

## Claims

1. A compound of the formula I 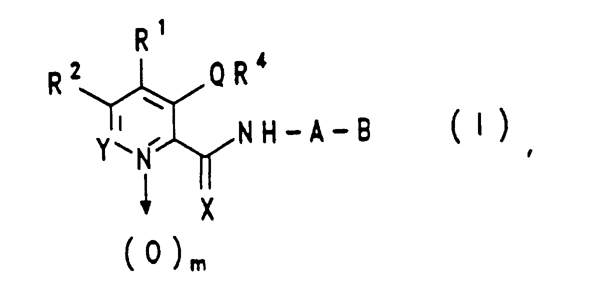 in which
Q is O, S, NR' or a bond,
X is O or S,
Y is C-R³ or,
if R¹ and R² form a cycle,
Y is N or CR³,
m is 0 or 1,
A is (C₁-C₄)-alkylene, which is optionally substituted by one or two substituents from the group halogen, cyano, nitro, trifluoromethyl, (C₁-C₆)-alkyl, (C₁-C₆)-hydroxyalkyl, (C₁-C₆)-alkoxy, -O-[CH₂]ₓ-C_{f}H_{(2f+1-g)}Hal_{g}, preferably (C₁-C₈)-fluoroalkoxy, (C₁-C₈)-fluoroalkenyloxy, (C₁-C₈)-fluoroalkynyloxy, -OCF₂Cl or -O-CF₂-CHFCl, (C₁-C₆)-alkylmercapto, (C₁-C₆) -a kylsulfinyl, (C₁-C₆)-alkylsulfonyl, (C₁-C₆)-alkylcarbonyl, (C₁-C₆)-alkoxycarbonyl, carbamoyl, N-(C₁-C₄)-alkylcarbamoyl, N,N-di-(C₁-C₄)-alkylcarbamoyl, (C₁-C₆)-alkylcarbonyloxy, (C₃-C₈)-cycloalkyl, phenyl, benzyl, phenoxy, benzyloxy, anilino, N-methylanilino, phenylmercapto, phenylsulfonyl, phenylsulfinyl, sulfamoyl, N-(C₁-C₄)-alkylsulfamoyl or N,N-di-(C₁-C₄)-alkylsulfamoyl, or
by a substituted (C₆-C₁₂)-aryloxy, (C₇-C₁₁)-aralkyloxy, (C₆-C₁₂)-aryl or (C₇-C₁₁)-aralkyl radical which carries in the aryl moiety 1, 2, 3, 4 or 5 identical or different substituents from the group halogen, cyano, nitro, trifluoromethyl, (C₁-C₆)-alkyl, (C₁-C₆)-alkoxy, -O-[CH₂]ₓ-C_{f}H_{(2f+1g)}Hal_{g}, -OCF₂Cl, -O-CF₂-CHFCl, (C₁-C₆)-alkylmercapto, (C₁-C₆)-alkylsulfinyl, (C₁-C₆)-alkylsulfonyl, (C₁-C₆)-alkylcarbonyl, (C₁-C₆)-alkoxycarbonyl, carbamoyl, N-(C₁-C₄)-alkylcarbamoyl, N,N-di-(C₁-C₄)-alkylcarbamoyl, (C₁-C₆)-alkylcarbonyloxy, (C₃-C₈)-cycloalkyl, sulfamoyl, N-(C₁-C₄)-alkylsulfamoyl or N,N-di-(C₁-C₄)-alkylsulfamoyl, or
by the substituents R⁵ of the α-C atom of an α-amino acid, it being possible to use the natural L-amino acids and their D-isomers;
B is -CO₂G, where G is the radical of an alcohol GOH,
R¹, R² and R³ are identical or different and are hydrogen, hydroxyl, halogen, cyano, trifluoromethyl, nitro, carboxyl, (C₁-C₂₀)-alkyl, (C₃-C₈)-cycloalkyl, (C₃-C₈)cycloalkyl-(C₁-C₁₂)-alkyl, (C₃-C₈)-cycloalkoxy, (C₃-C₈)-cycloalkyl-(C₁-C₁₂)-alkoxy, (C₃-C₈)-cycloalkyloxy-(C₁-C₁₂)-alkyl, (C₃-C₈)-cycloalkyloxy-(C₁-C₁₂)-alkoxy, (C₃-C₈)-cycloalkyl-(C₁-C₈)-alkyl-(C₁-C₆)-alkoxy, (C₃-C₈)-cycloalkyl-(C₁-C₈)-alkoxy-(C₁-C₆)-alkyl, (C₃-C₈)-cycloalkyloxy-(C₁-C₈)-alkoxy-(C₁-C₆)-alkyl, (C₃-C₈)-cycloalkoxy-(C₁-C₈)-alkoxy-(C₁-C₈)-alkoxy, (C₆-C₁₂)-aryl,(C₇-C₁₆)-aralkyl,(C₇-C₁₆)-aralkenyl, (C₇-C₁₆)-aralkynyl, (C₂-C₂₀)-alkenyl, (C₂-C₂₀)-alkynyl, (C₁-C₂₀)-alkoxy, (C₂-C₂₀)-alkenyloxy, (C₂-C₂₀)-alkynyloxy, retinyloxy, (C₁-C₂₀)-alkoxy-(C₁-C₁₂)-alkyl, (C₁-C₁₂)-alkoxy-(C₁-C₁₂)-alkoxy, (C₁-C₁₂)-alkoxy-(C₁-C₈)-alkoxy-(C₁-C₈)-alkyl, (C₆-C₁₂)-aryloxy, (C₇-C₁₆)-aralkyloxy,(C₆-C₁₂)-aryloxy-(C₁-C₆)-alkoxy, (C₇-C₁₆)-aralkoxy-(C₁-C₆)-alkoxy, (C₁-C₁₆)-hydroxyalkyl, (C₆-C₁₆)-aryloxy-(C₁-C₈)-alkyl, (C₇-C₁₆)-aralkoxy-(C₁-C₈)-alkyl, (C₆-C₁₂)-aryloxy-(C₁-C₈)-alkoxy-(C₁-C₆)-alkyl, (C₇-C₁₂)-aralkyloxy-(C₁-C₈)-alkoxy-(C₁-C₆)-alkyl, (C₂-C₂₀)-alkenyloxy-(C₁-C₆)-alkyl, (C₂-C₂₀)-alkynyloxy-(C₁-C₆)-alkyl, retinyloxy-(C₁-C₆)-alkyl, -O-[CH₂₋]ₓC_{f}H_{(2f+1-g)}F_{g}, -OCF₂Cl, -OCF₂-CHFCl,
(C₁-C₂₀)-alkylcarbonyl, (C₃-C₈)-cycloalkylcarbonyl, (C₆-C₁₂)-arylcarbonyl, (C₇-C₁₆)-aralkylcarbonyl, cinnamoyl, (C₂-C₂₀)-alkenylcarbonyl, (C₂-C₂₀)-alkynylcarbonyl,
(C₁-C₂₀)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₂-C₂₀)-alkenyloxycarbonyl, retinyloxycarbonyl, (C₂-C₂₀)-alkynyloxycarbonyl, (C₆-C₁₂)-aryloxy-(C₁-C₆)-alkoxycarbonyl, (C₇-C₁₆)-aralkoxy-(C₁-C₆)-alkoxycarbonyl, (C₃-C₈)-cycloalkyl-(C₁-C₆)-alkoxycarbonyl, (C₃-C₈)-cycloalkoxy-(C₁-C₆)-alkoxycarbonyl,
(C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy, (C₂-C₁₂)-alkenylcarbonyloxy, (C₂-C₁₂)-alkynylcarbonyloxy,
(C₁-C₁₂)-alkoxycarbonyloxy, (C₁-C₁₂)-alkoxy-(C₁-C₁₂)-alkoxycarbonyloxy, (C₆-C₁₂)-aryloxycarbonyloxy, (C₇-C₁₆)-aralkyloxycarbonyloxy, (C₃-C₈)-cycloalkoxycarbonyloxy, (C₂-C₁₂)-alkenyloxycarbonyloxy, (C₂-C₁₂)-alkynyloxycarbonyloxy,
carbamoyl, N-(C₁-C₁₂)-alkylcarbamoyl, N,N-di-(C₁-C₁₂)-alkylcarbamoyl, N-(C₃-C₈)-cycloalkylcarbamoyl, N,N-dicyclo-(C₃-C₈)-alkylcarbamoyl, N-(C₁-C₁₀)-alkyl-N-(C₃-C₈)-cycloalkylcarbamoyl, N-((C₃-C₈)-cycloalkyl-(C₁-C₆)-alkyl)carbamoyl, N-(C₁-C₆)-alkyl-N-((C₃-C₈)-cycloalkyl-(C₁-C₆)-alkyl)carbamoyl, N-(+)-dehydroabietylcarbamoyl, N-(C₁-C₆)-alkyl-N-(+)-dehydroabietylcarbamoyl,N-(C₆-C₁₂)-arylcarbamoyl, N-(C₇-C₁₆)-aralkylcarbamoyl, N-(C₁-C₁₀)-alkyl-N-(C₆-C₁₆)-arylcarbamoyl,N-(C₁-C₁₀)-alkyl-N-(C₇-C₁₆)-aralkylcarbamoyl, N-((C₁-C₁₈)-alkoxy-(C₁-C₁₀)alkyl)carbamoyl, N-((C₆-C₁₆)-aryloxy-(C₁-C₁₀)-alkyl)carbamoyl, N-((C₇-C₁₆)-aralkyloxy-(C₁-C₁₀)-alkyl)carbamoyl, N-(C₁-C₁₀)-alkyl-N-((C₁-C₁₀)-alkoxy-(C₁-C₁₀)-alkyl)carbamoyl, N-(C₁-C₁₀)-alkyl-N-((C₆-C₁₂)-aryloxy-(C₁-C₁₀)-alkyl)carbamoyl, N-(C₁-C₁₀)-alkyl-N-((C₇-C₁₆)-aralkyloxy-(C₁-C₁₀)-alkyl)carbamoyl, or CON(CH₂)ₕ, in which a CH₂ group can be replaced by O, S, N-(C₁-C₈)-alkylimino, N-(C₃-C₈)-cycloalkylimino, N-(C₃-C₈)-cycloalkyl-(C₁-C₄)-alkylimino, N-(C₆-C₁₂)-arylimino, N-(C₇-C₁₆)-aralkylimino or N-(C₁-C₄)-alkoxy-(C₁-C₆)-alkylimino, and h is from 3 to 7,
a carbamoyl radical of the formula II 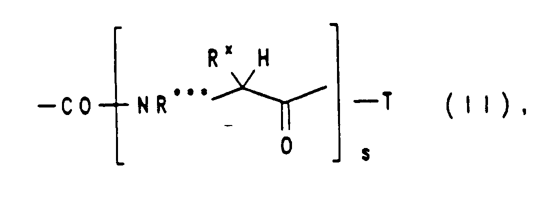 in which
R^{x} is the substituent of an α-amino acid to which the L- and D-amino acids belong,
s is 1, 2, 3, 4 or 5, and
T is OH, OR or NR*R**, where
R*, R** and R*** are identical or different and are hydrogen, (C₆-C₁₂)-aryl, (C₇-C₁₁)-aralkyl, (C₁-C₈)-alkyl, (C₃-C₈)-cycloalkyl, (+)-dehydroabietyl, (C₁-C₈)-alkoxy-(C₁-C₈)-alkyl, (C₇-C₁₂)-aralkoxy-(C₁-C₈)-alkyl,(C₆-C₁₂)-aryloxy-(C₁-C₈)-alkyl, (C₁-C₁₀)-alkanoyl, optionally substituted (C₇-C₁₆)-aralkanoyl or optionally substituted (C₆-C₁₂)-aroyl, or
R* and R** together are -[CH₂]ₕ, in which a CH₂ group can be replaced by O, S, SO, SO₂, N-acylamino, N-(C₁-C₁₀)-alkoxycarbonylimino, N-(C₁-C₈)-alkylimino, N-(C₃-C₈)-cycloalkylimino, N-(C₃-C₈)-cycloalkyl-(C₁-C₄)-alkylimino, N-(C₆-C₁₂)-arylimino, N-(C₇-C₁₆)-aralkylimino or N-(C₁-C₄)-alkoxy-(C₁-C₆)-alkylimino, and h is from 3 to 7,
carbamoyloxy, N-(C₁-C₁₂)-alkylcarbamoyloxy, N,N-di-(C₁-C₁₂)-alkylcarbamoyloxy. N-(C₃-C₈)-cycloalkylcarbamoyloxy, N-(C₆-C₁₂)-arylcarbamoyloxy, N-(C₇-c₁₆)-aralkylcarbamoyloxy, N-(C₁-C₁₀)-alkyl-N-(C₆-C₁₂)-arylcarbamoyloxy, N-(C₁-C₁₀)-alkyl-N-(C₇-C₁₆)-aralkylcarbamoyloxy, N-((C₁-C₁₀)-alkyl)carbamoyloxy, N-((C₆-C₁₂)-aryloxy-(C₁-C₁₀)-alkyl)carbamoyloxy, N-((C₇-C₁₆)-aralkyloxy-(C₁-C₁₀)-alkyl)carbamoyloxy, N-(C₁-C₁₀)-alkyl-N-((C₁-C₁₀)-alkoxy-(C₁-C₁₀)-alkyl)-carbamoyloxy, N-(C₁-C₁₀)-alkyl-N-((C₆-C₁₂)-aryloxy-(C₁-C₁₀)-alkyl) arbamoyloxy, N-(C₁-C₁₀)-alkyl-N-((C₇-C₁₆)-aralkyloxy-(C₁-C₁₀)-alkyl)carbamoyloxy,
amino, (C₁-C₁₂)-alkylamino, di-(C₁-C₁₂)-alkylamino, (C₃-C₈)-cycloalkylamino, (C₃-C₁₂)-alkenylamino, (C₃-C₁₂)-alkynylamino, N-(C₆-C₁₂)-arylamino, N-(C₇-C₁₁)-aralkylamino, N-alkyl-aralkylamino, N-alkyl-arylamino, (C₁-C₁₂)-alkoxyamino, (C₁-C₁₂)-alkoxy-N-(C₁-C₁₀)-alkylamino,
(C₁-C₁₂)-alkanoylamino, (C₃-C₈)-cycloalkanoylamino, (C₆-C₁₂)-aroylamino, (C₇-C₁₆)-aralkanoylamino, (C₁-C₁₂)-alkanoyl-N-(C₁-C₁₀)-alkylamino, (C₃-C₈)-cycloalkanoyl-N-(C₁-C₁₀)-alkylamino, (C₆-C₁₂)-aroyl-N-(C₁-C₁₀)-alkylamino, (C₇-C₁₁)-aralkanoyl-N-(C₁-C₁₀)-alkylamino,
(C₁-C₁₂)-alkanoylamino-(C₁-C₈)-alkyl, (C₃-C₈)-cycloalkanoylamino-(C₁-C₈)-alkyl, (C₆-C₁₂)-aroylamino-(C₁-C₈)-alkyl, (C₇-C₁₆)-aralkanoylamino-(C₁-C₈)-alkyl, amino-(C₁-C₁₀)-alkyl, N-(C₁-C₁₀)-alkylamino-(C₁-C₁₀)-alkyl, N,N-di(C₁-C₁₀)-alkylamino-(C₁-C₁₀)-alkyl, (C₃-C₈)-cycloalkylamino(C₁-C₁₀)-alkyl, (C₁-C₂₀)-alkylmercapto, (C₁-C₂₀)-alkylsulfinyl, (C₁-C₂₀)-alkylsulfonyl, (C₆-C₁₂)-arylmercapto, (C₆-C₁₂)-arylsulfinyl, (C₆-C₁₂)-arylsulfonyl, (C₇-C₁₆)-aralkylmercapto, (C₇-C₁₆)-aralkylsulfinyl, (C₇-C₁₆)-aralkylsulfonyl, (C₁-C₁₂)-alkylmercapto-(C₁-C₆)-alkyl, (C₁-C₁₂)-alkylsulfinyl-(C₁-C₆)-alkyl, (C₁-C₁₂)-alkylsulfonyl-(C₁-C₆)-alkyl, (C₆-C₁₂)-arylmercapto-(C₁-C₆)-alkyl, (C₆-C₁₂)-arylsulfinyl-(C₁-C₆)-alkyl, (C₆-C₁₂)-arylsulfonyl-(C₁-C₆)-alkyl, (C₇-C₁₆)-aralkylmercapto-(C₁-C₆)-alkyl, (C₇-C₁₆)-aralkylsulfinyl-(C₁-C₆)-alkyl, (C₇-C₁₆)-aralkylsulfonyl-(C₁-C₆)-alkyl,
sulfamoyl, N-(C₁-C₁₀)-alkylsulfamoyl, N,N-di-(C₁-C₁₀)-alkylsulfamoyl, (C₃-C₈)-cycloalkylsulfamoyl,
N-(C₆-C₁₂)-arylsulfamoyl,
N-(C₇-C₁₆)-aralkylsulfamoyl,
N-(C₁-C₁₀)-alkyl-N-(C₆-C₁₂)-arylsulfamoyl,
N-(C₁-C₁₀)-alkyl-N-(C₇-C₁₆)-aralkylsulfamoyl,
(C₁-C₁₀)-alkylsulfonamido,
N-((C₁-C₁₀)-alkyl)-(C₁-C₁₀)-alkylsulfonamido,
(C₇-C₁₆)-aralkylsulfonamido or N-((C₁-C₁₀)-alkyl-(C₇-C₁₆)-aralkylsulfonamido,
where the radicals which contain an aryl radical can, for their part, be substituted on the aryl by from 1 to 5 identical or different radicals from the group:
hydroxyl, halogen, cyano, trifluoromethyl, nitro, carboxyl, (C₂-C₁₆)-alkyl, (C₃-C₈)-cycloalkyl, (C₃-C₈)-cycloalkyl-(C₁-C₁₂)-alkyl, (C₃-C₈)-cycloalkoxy, (C₃-C₈)-cycloalkyl-(C₁-C₁₂)-alkoxy, (C₃₋C₈)-cycloalkyloxy-(C₁-C₁₂)-alkyl, (C₃-C₈)-cycloalkyloxy-(C₁-C₁₂)-alkoxy, (C₃-C₈)-cycloalkyl-(C₁-C₈)-alkyl-(C₁-C₆)-alkoxy, (C₃-C₈)-cycloalkyl(C₁-C₈)-alkoxy-(C₁-C₆)-alkyl, (C₃-C₈)-cycloalkyloxy-(C₁-C₈)-alkoxy-(C₁-C₆)-alkyl, (C₃-C₈)-cycloalkoxy-(C₁-C₈)-alkoxy-(C₁-C₈)-alkoxy, (C₆-C₁₂)-aryl, (C₇-C₁₆)-aralkyl, (C₂-C₁₆)-alkenyl, (C₂-C₁₂)-alkynyl, (C₁-C₁₆)-alkoxy, (C₁-C₁₆)-alkenyloxy, (C₁-C₁₂)-alkoxy-(C₁-C₁₂)-alkyl, (C₁-C₁₂)-alkoxy-(C₁-C₁₂)-alkoxy, (C₁-C₁₂)-alkoxy(C₁-C₈)-alkoxy-(C₁-C₈)-alkyl, (C₆-C₁₂)-aryloxy, (C₇-C₁₆)-aralkyloxy, (C₆-C₁₂)-aryloxy-(C₁-C₆)-alkoxy, (C₇-C₁₆)-aralkoxy-(C₁-C₆)-alkoxy, (C₁-C₈)-hydroxyalkyl, (C₆-C₁₆)-aryloxy-(C₁-C₈)-alkyl, (C₇-C₁₆)-aralkoxy-(C₁-C₈)-alkyl, (C₆-C₁₂)-aryloxy-(C₁-C₈)-alkoxy-(C₁-C₆)-alkyl, (C₇-C₁₂)-aralkyloxy-(C₁-C₈)-alkoxy-(C₁-C₆)-alkyl, -O-[CH₂₋]ₓC_{f}H_{(2f+1-g)}F_{g}, -OCF₂Cl, -OCF₂-CHFCl,
(C₁-C₁₂)-alkylcarbonyl, (C₃-C₈)-cycloalkylcarbonyl, (C₆-C₁₂)-arylcarbonyl, (C₇-C₁₆)-aralkylcarbonyl,
(C₁-C₁₂)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₂-C₁₂)-alkenyloxycarbonyl, (C₂-C₁₂)-alkynyloxycarbonyl, (C₆-C₁₂)-aryloxy-(C₁-C₆)-alkoxycarbonyl, (C₇-C₁₆)-aralkoxy-(C₁-C₆)-alkoxycarbonyl, (C₃-C₈)-cycloalkyl-(C₁-C₆)-alkoxycarbonyl, (C₃-C₈)-cycloalkoxy-(C₁-C₆)-alkoxycarbonyl,
(C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy, (C₂-C₁₂)-alkenylcarbonyloxy, (C₂-C₁₂)-alkynylcarbonyloxy,
(C₁-C₁₂)-alkoxycarbonyloxy, (C₁-C₁₂)-alkoxy-(C₁-C₁₂)-alkoxycarbonyloxy, (C₆-C₁₂)-aryloxycarbonyloxy, (C₇-C₁₆)-aralkyloxycarbonyloxy, (C₃-C₈)-cycloalkoxycarbonyloxy, (C₂-C₁₂)-alkenyloxycarbonyloxy, (C₂-C₁₂)-alkynyloxycarbonyloxy,
carbamoyl, N-(C₁-C₁₂)-alkylcarbamoyl, N,N-di(C₁-C₁₂)-alkylcarbamoyl, N-(C₃-C₈)-cycloalkylcarbamoyl, N,N-dicyclo-(C₃-C₈)-alkylcarbamoyl, N-(C₁-C₁₀)-alkyl-N-(C₃-C₈)-cycloalkylcarbamoyl, N-((C₃-C₈)-cycloalkyl-(C₁-C₆)-alkyl)carbamoyl, N-(C₁-C₆)-alkyl-N-((C₃-C₈)-cycloalkyl-(C₁-C₆)-alkyl)carbamoyl, N-(+)-dehydroabietylcarbamoyl, N-(C₁-C₆)-alkyl-N-(+)-dehydroabietylcarbamoyl, N-(C₆-C₁₂)-arylcarbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl, N-((C₁-C₁₆)-alkoxy-(C₁-C₁₀)-alkyl)carbamoyl, N-((C₆-C₁₆)-aryloxy-(C₁-C₁₀)-alkyl)carbamoyl, N-((C₇-C₁₆)-aralkyloxy-(C₁-C₁₀)-alkyl)carbamoyl,
N-(C₁-C₁₀)-alkyl-N-((C₁-C₁₀)-alkoxy-(C₁-C₁₀)-alkyl)carbamoyl, N-(C₁-C₁₀)-alkyl-N-((C₆-C₁₂)-aryloxy-(C₁-C₁₀)-alkyl)carbamoyl, N-(C₁-C₁₀)-alkyl-N-((C₇-C₁₆)-aralkyloxy-(C₁-C₁₀)-alkyl)-carbamoyl, or CON(CH₂)ₕ, in which a CH₂ group can be replaced by O, S, N-(C₁-C₈)-alkylimino, N-(C₃-C₈)-cycloalkylimino, N-(C₃-C₈)-cycloalkyl-(C₁-C₄)-alkylimino, N-(C₆-C₁₂)-arylimino, N-(C₇-C₁₆)-aralkylimino or N-(C₁-C₄)-alkoxy-(C₁-C₆)-alkylimino, and h is from 3 to 7,
carbamoyloxy, N-(C₁-C₁₂)-alkylcarbamoyloxy, N,N-di-(C₁-C₁₂)-alkylcarbamoyloxy, N-(C₃-C₈)-cycloalkylcarbamoyloxy, N-(C₆-C₁₆)-arylcarbamoyloxy, N-(C₇-C₁₆)-aralkylcarbamoyloxy, N-(C₁-C₁₀)-alkyl-N-(C₆-C₁₂)-arylcarbamoyloxy, N-(C₁-C₁₀)-alkyl-N-(C₇-C₁₆)-aralkylcarbamoyloxy, N-((C₁-C₁₀)-alkyl)carbamoyloxy, N-((C₆-C₁₂)-aryloxy-(C₁-C₁₀)-alkyl)carbamoyloxy, N-((C₇-C₁₆)-aralkyloxy-(C₁-C₁₀)-alkyl)carbamoyloxy, N-(C₁-C₁₀)-alkyl-N-((C₁-C₁₀)-alkoxy-(C₁-C₁₀)-alkyl)carbamoyloxy, N-(C₁-C₁₀)-alkyl-N-((C₆-C₁₂)-aryloxy-(C₁-C₁₀)-alkyl)carbamoyloxy, N-(C₁-C₁₀)-alkyl-N-((C₇-C₁₆)-aralkyloxy-(C₁-C₁₀)-alkyl)carbamoyloxy,
amino, (C₁-C₁₂)-alkylamino,di-(C₁-C₁₂)-alkylamino, (C₃-C₈)-cycloalkylamino, (C₃-C₁₂)-alkenylamino, (C₃-C₁₂)-alkynylamino, N-(C₆-C₁₂)-arylamino, N-(C₇-C₁₁)-aralkylamino, N-alkyl-aralkylamino, N-alkyl-arylamino, (C₁-C₁₂)-alkoxyamino, (C₁-C₁₂)-alkoxy-N-(C₁-C₁₀)-alkylamino,
(C₁-C₁₂)-alkanoylamino, (C₃-C₈)-cycloalkanoylamino, (C₆-C₁₂)-aroylamino, (C₇-C₁₆)-aralkanoylamino, (C₁-C₁₂)-alkanoyl-N-(C₁-C₁₀)-alkylamino, (C₃-C₈)-cycloalkanoyl-N-(C₁-C₁₀)-alkylamino, (C₆-C₁₂)-aroyl-N-(C₁-C₁₀)-alkylamino, (C₇-C₁₁)-aralkanoyl-N-(C₁-C₁₀)-alkylamino,
(C₁-C₁₂)-alkanoylamino-(C₁-C₈)-alkyl, (C₃-C₈)-cycloalkanoylamino-(C₁-C₈)-alkyl, (C₆-C₁₂)-aroylamino- (C₁-C₈)-alkyl, (C₇-C₁₆)-aralkanoylamino-(C₁-C₈)-alkyl, amino-(C₁-C₁₀)-alkyl, N-(C₁-C₁₀)-alkylamino-(C₁-C₁₀)-alkyl, N,N-di-(C₁-C₁₀)-alkylamino-(C₁-C₁₀)-alkyl, (C₃-C₈)-cycloalkylamino-(C₁-C₁₀)-alkyl,
(C₁-C₁₂)-alkylmercapto, (C₁-C₁₂)-alkylsulfinyl, (C₁-C₁₂)-alkylsulfonyl, (C₆-C₁₆)-arylmercapto, (C₆-C₁₆)-arylsulfinyl, (C₆-C₁₆)-arylsulfonyl, (C₇-C₁₆)-aralkylmercapto, (C₇-C₁₆)-aralkylsulfinyl or (C₇-C₁₆)-aralkylsulfonyl,
R¹ and R² or R² and R³ form a chain [CH₂]ₒ in which one or two CH₂ groups of the chain, which is saturated or unsaturated by a C=C double bond, are optionally replaced by O, S, SO, SO₂ or NR', and o is 3, 4 or 5, and
R' is hydrogen, (C₆-C₁₂)-aryl, (C₁-C₈)-alkyl, (C₁-C₈)-alkoxy-(C₁-C₈)-alkyl, (C₇-C₁₂)-aralkoxy-(C₁-C₈)-alkyl, (C₆-C₁₂)-aryloxy-(C₁-C₈)-alkyl, (C₁-C₁₀)-alkanoyl, optionally substituted (C₇-C₁₆)-aralkanoyl or optionally substituted (C₆-C₁₂)-aroyl, where
the radicals R¹ and R² or R² and R³, together with the pyridine or pyridazine carrying them, preferably form a 5, 6, 7, 8-tetrahydroisoquinoline ring, a 5, 6, 7, 8-tetrahydroquino-line ring or a 5, 6, 7, 8-tetrahydrocinnoline ring,
or
R¹ and R² or R² and R³ form a carbocyclic or a heterocyclic, 5- or 6-membered aromatic ring, where
the radicals R¹ and R² or R² and R³, together with the pyridine or pyridazine carrying them, preferably form the following optionally substituted heterocyclic ring systems:
thienopyridines,
furanopyridines,
pyridopyridines,
pyrimidinopyridines,
imidazopyridines,
thiazolopyridines,
oxazolopyridines,
quinoline, isoquinoline and
cinnoline,
where quinoline, isoquinoline or cinnoline preferably satisfy the formulae 1a, 1b and 1c 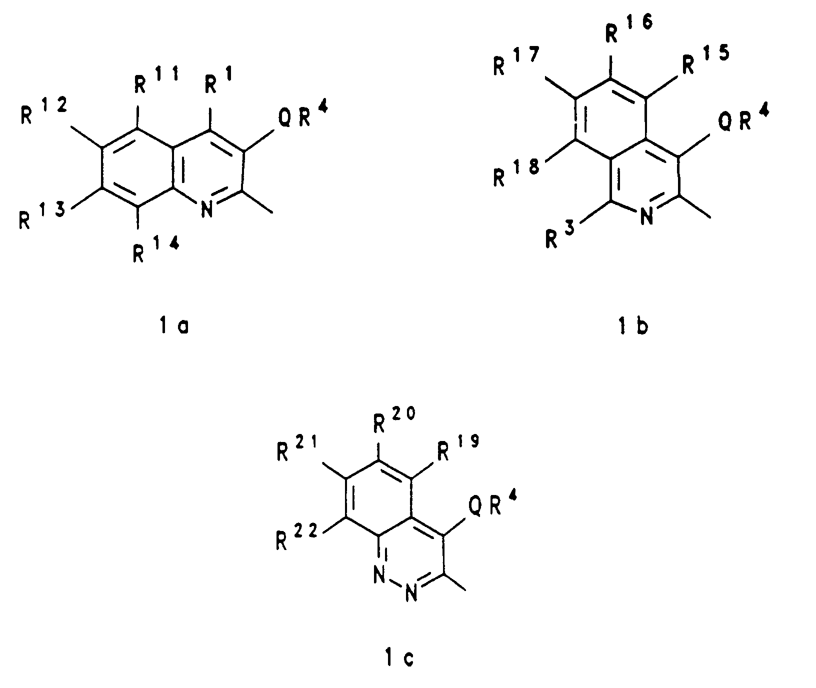 and the substituents R¹¹ to R²², in each case independently of each other, have the meaning of R¹, R² and R³,
R⁴ is, if Q is a bond, halogen, nitrile or trifluoromethyl, or, if Q is O or S, a branched or unbranched (C₁-C₂₀)-alkyl radical, an unsubstituted, saturated fluoroalkyl radical of the formula [CH₂]ₓC_{f}H_{(2f+1-g)}F_{g}, a (C₆-C₁₆)-aryl radical, a (C₇-C₁₆)-aralkyl radical, a heteroaryl radical or a heteroaralkyl radical,
where these radicals are substituted by one or more radicals from the group
hydroxyl, halogen, cyano, trifluoromethyl, nitro, carboxyl, (C₁-C₁₂)-alkyl, (C₃-C₈)-cycloalkyl, (C₃-C₈)-cycloalkyl-(C₁-C₁₂)-alkyl, (C₃-C₈)-cycloalkoxy, (C₃-C₈)-cycloalkyl-(C₁-C₁₂)-alkoxy, (C₃-C₈)-cycloalkyloxy-(C₁-C₁₂)-alkyl, (C₃-C₈)-cycloalkyloxy-(C₁-C₁₂)-alkoxy, (C₃-C₈)-cycloalkyl-(C₁-C₈)-alkyl-(C₁-C₆)-alkoxy, (C₃-C₈)-cycloalkyl-(C₁-C₈)-alkoxy-(C₁-C₆)-alkyl, (C₃-C₈)-cycloalkyloxy-(C₁-C₈)-alkoxy-(C₁-C₆)-alkyl, (C₃-C₈)-cycloalkoxy-(C₁-C₈)-alkoxy-(C₁-C₈)-alkoxy, (C₆-C₁₂)-aryl, (C₇-C₁₆)-aralkyl, (C₂-C₁₂)-alkenyl, (C₂-C₁₂)-alkynyl, (C₁-C₁₂)-alkoxy, (C₁-C₁₂)-alkoxy-(C₁-C₁₂)-alkyl, (C₁-C₁₂)-alkoxy-(C₁-C₁₂)-alkoxy, (C₁-C₁₂)-alkoxy-(C₁-C₈)-alkoxy-(C₁-C₈)-alkyl, (C₆-C₁₂)-aryloxy, (C₇-C₁₆)-aralkyloxy, (C₆-C₁₂)-aryloxy-(C₁-C₆)-alkoxy, (C₇-C₁₆)-aralkoxy-(C₁-C₆)-alkoxy, (C₁-C₈)-hydroxyalkyl, (C₆-C₁₆)-aryloxy-(C₁-C₈)-alkyl, (C₇-C₁₆)-aralkoxy-(C₁-C₈)-alkyl, (C₆-C₁₂)-aryloxy-(C₁-C₈)-alkoxy-(C₁-C₆)-alkyl, (C₇-C₁₂)-aralkyloxy-(C₁-C₈)-alkoxy-(C₁-C₆)-alkyl, -O-[CH₂₋]ₓC_{f}H_{(2f+1-g)}F_{g}, -OCF₂Cl, -OCF₂-CHFCl,
(C₁-C₁₂)-alkylcarbonyl, (C₃-C₈)-cycloalkylcarbonyl, (C₆-C₁₂)-arylcarbonyl, (C₇-C₁₆)-aralkylcarbonyl, cinnamoyl, (C₂-C₁₂)-alkenylcarbonyl, (C₂-C₁₂)-alkynylcarbonyl,
(C₁-C₁₂)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₂-C₁₂)-alkenyloxycarbonyl, (C₂-C₁₂)-alkynyloxycarbonyl, (C₆-C₁₂)-aryloxy-(C₁-C₆)-alkoxycarbonyl, (C₇-C₁₆)-aralkoxy-(C₁-C₆)-alkoxycarbonyl, (C₃-C₈)-cycloalkyl-(C₁-C₆)-alkoxycarbonyl, (C₃-C₈)-cycloalkoxy-(C₁-C₆)-alkoxycarbonyl,
(C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy, (C₂-C₁₂)-alkenylcarbonyloxy, (C₂-C₁₂)-alkynylcarbonyloxy,
(C₁-C₁₂)-alkoxycarbonyloxy, (C₁-C₁₂)-alkoxy(C₁-C₁₂)-alkoxycarbonyloxy, (C₆-C₁₂)-aryloxycarbonyloxy, (C₇-C₁₆)-aralkyloxycarbonyloxy, (C₃-C₈)-cycloalkoxycarbonyloxy, (C₂-C₁₂)-alkenyloxycarbonyloxy, (C₂-C₁₂)-alkynyloxycarbonyloxy,
carbamoyl, N-(C₁-C₁₂)-alkylcarbamoyl, N,N-di-(C₁-C₁₂)-alkylcarbamoyl, N-(C₃-C₈)-cycloalkylcarbamoyl, N,N-dicyclo-(C₃-C₈)-alkylcarbamoyl, N-(C₁-C₁₀)-alkyl-N-(C₃-C₈)-cycloalkylcarbamoyl, N-((C₃-C₈)-cycloalkyl-(C₁-C₆)-alkyl)carbamoyl, N-(C₁-C₆)-alkyl-N-((C₃-C₈)-cycloalkyl-(C₁-C₆)-alkyl)carbamoyl, N-(+)-dehydroabietylcarbamoyl, N-(C₁-C₆)-alkyl-N-(+)-dehydroabietylcarbamoyl, N-(C₆-C₁₂)-arylcarbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl, N((C₁-C₁₀)-alkoxy-(C₁-C₁₀)-alkyl)carbamoyl, N-((C₆-C₁₆)-aryloxy-(C₁-C₁₀)-alkyl)carbamoyl, N-((C₇-C₁₆)-aralkyloxy-(C₁-C₁₀)-alkyl)carbamoyl, N-(C₁-C₁₀)-alkyl-N-((C₁-C₁₀)-alkoxy-(C₁-C₁₀)-alkyl)carbamoyl,N-(C₁-C₁₀)-alkyl-N-((C₆-C₁₂)-aryloxy-(C₁-C₁₀)-alkyl)carbamoyl, N(C₁-C₁₀)-alkyl-N-((C₇-C₁₆)-aralkyloxy-(C₁-C₁₀)-alkyl)carbamoyl, or CON(CH₂)ₕ, in which a CH₂ group can be replaced by O, S, N-(C₁-C₈)-alkylimino, N-(C₃-C₈)-cycloalkylimino, N-(C₃-C₈)-cycloalkyl-(C₁-C₄)-alkylimino, N-(C₆-C₁₂)-arylimino, N-(C₇-C₁₆)-aralkylimino or N-(C₁-C₄)-alkoxy-(C₁-C₆)-alkylimino, and h is from 3 to 7, or by
a carbamoyl radical of the formula II 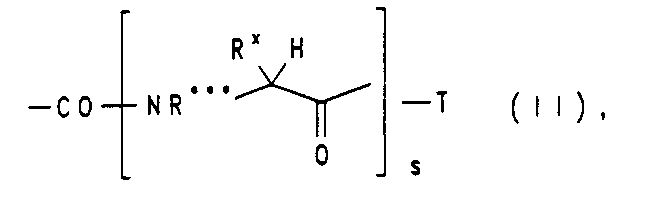 in which
R^{x} is the substituent of an α-amino acid to which the L- and D-amino acids belong,
s is 1, 2, 3, 4 or 5, and
T is OH, OR or NR*R**, where
R*, R** and R*** are identical or different and are hydrogen, (C₆-C₁₂)-aryl, (C₇-C₁₁)-aralkyl, (C₁-C₈)-alkyl, (C₃-C₈)-cycloalkyl, (+)-dehydroabietyl, (C₁-C₈)-alkoxy-(C₁-C₈)-alkyl, (C₇-C₁₂)-aralkoxy-(C₁-C₈)-alkyl, (C₆-C₁₂)-aryloxy-(C₁-C₈)-alkyl, (C₁-C₁₀)-alkanoyl, optionally substituted (C₇-C₁₆)-aralkanoyl or optionally substituted (C₆-C₁₂)-aroyl, or
R* and R** together are - [CH₂]ₕ, in which a CH₂ group can be replaced by O, S, SO, SO₂, N-acylamino, N-(C₁-C₁₀)-alkoxycarbonylimino, N-(C₁-C₈)-alkylimino, N-(C₃-C₈)-cycloalkylimino, N-(C₃-C₈)-cycloalkyl-(C₁-C₄)alkylimino, N-(C₆-C₁₂)-arylimino, N-(C₇-C₁₆)-aralkylimino or N-(C₁-C₄)-alkoxy(C₁-C₆)-alkylimino, and h is from 3 to 7, or by
carbamoyloxy, N-(C₁-C₁₂)-alkylcarbamoyloxy, N,N-di-(C₁-C₁₂)-alkylcarbamoyloxy, N-(C₃-C₈)-cycloalkylcarbamoyloxy, N-(C₆-C₁₂)-arylcarbamoyloxy, N-(C₇-C₁₆)-aralkylcarbamoyloxy, N-(C₁-C₁₀)-alkyl-N-(C₆-C₁₂)-arylcarbamoyloxy, N-(C₁-C₁₀)-alkyl-N-(C₇-C₁₆)-aralkylcarbamoyloxy, N-((C₁-C₁₀)-alkyl)carbamoyloxy, N-((C₆-C₁₂)-aryloxy-(C₁-C₁₀)-alkyl)carbamoyloxy, N-((C₇-C₁₆)-aralkyloxy-(C₁-C₁₀)-alkyl)carbamoyloxy, N-(C₁-C₁₀)-alkyl-N-((C₁-C₁₀)-alkoxy-(C₁-C₁₀)-alkyl)carbamoyloxy, N-(C₁-C₁₀)-alkyl-N-((C₆-C₁₂)-aryloxy-(C₁-C₁₀)-alkyl)carbamoyloxy, N-(C₁-C₁₀)-alkyl-N-((C₇-C₁₆)-aralkyloxy-(C₁-C₁₀)-alkyl)carbamoyloxy,
amino, (C₁-C₁₂)-alkylamino, di-(C₁-C₁₂)-alkylamino, (C₃-C₈)-cycloalkylamino, (C₃-C₁₂)-alkenylamino, (C₃-C₁₂)-alkynylamino, N-(C₆-C₁₂)-arylamino, N-(C₇-C₁₁)-aralkylamino, N-alkyl-aralkylamino, N-alkyl-arylamino, (C₁-C₁₂)-alkoxyamino, (C₁-C₁₂)-alkoxy-N-(C₁-C₁₀)-alkylamino,
(C₁-C₁₂)-alkanoylamino, (C₃-C₈)-cycloalkanoylamino, (C₆-C₁₂)-aroylamino, (C₇-C₁₆)-aralkanoylamino, (C₁-C₁₂)-alkanoyl-N-(C₁-C₁₀)-alkylamino, (C₃-C₈)-cycloalkanoyl-N-(C₁-C₁₀)-alkylamino, (C₆-C₁₂)-aroyl-N-(C₁-C₁₀)-alkylamino, (C₇-C₁₁)-aralkanoyl-N-(C₁-C₁₀)-alkylamino,
(C₁-C₁₂)-alkanoylamino-(C₁-C₈)-alkyl, (C₃-C₈)-cycloalkanoylamino-(C₁-C₈)-alkyl, (C₆-C₁₂)-aroylamino-(C₁-C₈)-alkyl, (C₇-C₁₆)-aralkanoylamino-(C₁-C₈)-alkyl, amino-(C₁-C₁₀)-alkyl, N-(C₁-C₁₀)-alkylamino-(C₁-C₁₀)-alkyl, N,N-di(C₁-C₁₀)-alkylamino-(C₁-C₁₀)-alkyl,(C₃-C₈)-cycloalkylamino-(C₁-C₁₀)-alkyl,
(C₁-C₁₂)-alkylmercapto, (C₁-C₁₂)-alkylsulfinyl, (C₁-C₁₂)-alkylsulfonyl, (C₆-C₁₂)-arylmercapto, (C₆-C₁₂)-arylsulfinyl, (C₆-C₁₂)-arylsulfonyl, (C₇-C₁₆)-aralkylmercapto,(C₇-C₁₆)-aralkylsulfinyl, (C₇-C₁₆)-aralkylsu fonyl,
sulfamoyl, N-(C₁-C₁₀)-alkylsulfamoyl, N,N-di-(C₁-C₁₀)-alkylsulfamoyl, (C₃-C₈)-cycloalkylsulfamoyl,
N-(C₆-C₁₂)-arylsulfamoyl,
N-(C₇-C₁₆)-aralkylsulfamoyl,
N-(C₁-C₁₀)-alkyl-N-(C₆-C₁₂)-arylsulfamoyl, N-(C₁-C₁₀)-alkyl-N-(C₇-C₁₆)-aralkylsulfamoyl, (C₁-C₁₀)-alkylsulfonamido, N-((C₁-C₁₀)-alkyl)-(C₁-C₁₀)-alkylsulfonamido, (C₇-C₁₆)-aralkylsulfonamido or N-((C₁-C₁₀)-alkyl-(C₇-C₁₆)-aralkylsulfonamido,
where the radicals which contain an aryl radical can, for their part, be substituted on the aryl by from 1 to 5 identical or different radicals from the group:
hydroxyl, halogen, cyano, trifluoromethyl, nitro, carboxyl, (C₁-C₁₂)-alkyl, (C₃-C₈)-cycloalkyl, (C₃-C₈)-cycloalkyl-(C₁-C₁₂)-alkyl,(C₃-C₈)-cycloalkoxy, (C₃-C₈)-cycloalkyl-(C₁-C₁₂)-alkoxy, (C₃-C₈)-cycloalkyloxy-(C₁-C₁₂)-alkyl, (C₃-C₈)-cycloalkyloxy-(C₁-C₁₂)-alkoxy, (C₃-C₈)-cycloalkyl-(C₁-C₈)-alkyl-(C₁-C₆)-alkoxy, (C₃-C₈)-cycloalkyl-(C₁-C₈)-alkoxy-(C₁-C₆)-alkyl, (C₃-C₈)-cycloalkyloxy-(C₁-C₈)-alkoxy-(C₁-C₆)-alkyl, (C₃-C₈)-cycloalkoxy-(C₁-C₈)-alkoxy-(C₁-C₈)-alkoxy, (C₆-C₁₂)-aryl, (C₇-C₁₆)-aralkyl, (C₂-C₁₂)-alkenyl, (C₂-C₁₂)-alkynyl, (C₁-C₁₂)-alkoxy, (C₁-C₁₂)-alkoxy-(C₁-C₁₂)-alkyl, (C₁-C₁₂)-alkoxy-(C₁-C₁₂)-alkoxy, (C₁-C₁₂)-alkoxy-(C₁-C₈)-alkoxy-(C₁-C₈)-alkyl, (C₆-C₁₂)-aryloxy, (C₇-C₁₆)-aralkyloxy, (C₆-C₁₂) - aryloxy-(C₁-C₆)-alkoxy, (C₇-C₁₆)-aralkoxy-(C₁-C₆)-alkoxy, (C₁-C₈)-hydroxyalkyl, (C₆-C₁₆)-aryloxy-(C₁-C₈)-alkyl, (C₇-C₁₆)-aralkoxy-(C₁-C₈)-alkyl, (C₆-C₁₂)-aryloxy-(C₁-C₈)-alkoxy-(C₁-C₆)-alkyl, (C₇-C₁₂)-aralkyloxy-(C₁-C₈)-alkoxy-(C₁-C₆)-alkyl, -O-[CH₂₋]ₓC_{f}H_{(2f+1-g)}F_{g}, -OCF₂Cl, -OCF₂-CHFCl,
(C₁-C₁₂)-alkylcarbonyl, (C₃-C₈)-cycloalkylcarbonyl, (C₆-C₁₂)-arylcarbonyl, (C₇-C₁₆)-aralkylcarbonyl,
(C₁-C₁₂)-alkoxycarbonyl, (C₁-C₁₂)-alkoxy-(C₁-C₁₂)-alkoxycarbonyl, (C₆-C₁₂)-aryloxycarbonyl,(C₇-C₁₆)-aralkoxycarbonyl, (C₃-C₈)-cycloalkoxycarbonyl, (C₂-C₁₂)-alkenyloxycarbonyl, (C₂-C₁₂)-alkynyloxycarbonyl, (C₆-C₁₂)-aryloxy-(C₁-C₆)-alkoxycarbonyl, (C₇-C₁₆)-aralkoxy-(C₁-C₆)-alkoxycarbonyl, (C₃-C₈)-cycloalkyl-(C₁-C₆)-alkoxycarbonyl, (C₃-C₈)-cycloalkoxy-(C₁-C₆)-alkoycarbonyl,
(C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy, (C₂-C₁₂) - alkenylcarbonyloxy, (C₂-C₁₂)-alkynylcarbonyloxy,
(C₁-C₁₂)-alkoxycarbonyloxy, (C₁-C₁₂)-alkoxy-(C₁-C₁₂)-alkoxycarbonyloxy, (C₆-C₁₂)-aryloxycarbonyloxy, (C₇-C₁₆)-aralkyloxycarbonyloxy, (C₃-C₈)-cycloalkoxycarbonyloxy, (C₂-C₁₂)-alkenyloxycarbonyloxy, (C₂-C₁₂)-alkynyloxycarbonyloxy,
carbamoyl, N-(C₁-C₁₂)-alkylcarbamoyl, N,N-di-(C₁-C₁₂)-alkylcarbamoyl, N-(C₃-C₈)-cycloalkylcarbamoyl, N,N-dicyclo-(C₃-C₈)-alkylcarbamoyl, N-(C₁-C₁₀)-alkyl-N-(C₃-C₈)-cycloalkylcarbamoyl, N-((C₃-C₈)-cycloalkyl-(C₁-C₆)-alkyl)carbamoyl, N-(C₁-C₆)-alkyl-N-((C₃-C₈)-cycloalkyl-(C₁-C₆)-alkyl)carbamoyl, N-(+)-dehydroabietylcarbamoyl, N-(C₁-C₆)-alkyl-N-(+)-dehydroabietylcarbamoyl, N-(C₆-C₁₂)-arylcarbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl, N-((C₁-C₁₀)-alkoxy-(C₁-C₁₀)-alkyl)carbamoyl, N-((C₆-C₁₆)-aryloxy-(C₁-C₁₀)-alkyl)carbamoyl, N-((C₇-C₁₆)-aralkyloxy-(C₁-C₁₀)-alkyl)carbamoyl, N-(C₁-C₁₀)-alkyl-N-((C₁-C₁₀)-alkoxy-(C₁-C₁₀)-alkyl)carbamoyl, N-(C₁-C₁₀)-alkyl-N-((C₆-C₁₂)-aryloxy-(C₁-C₁₀)-alkyl)carbamoyl, N-(C₁-C₁₀)-alkyl-N-((C₇-C₁₆)-aralkyloxy-(C₁-C₁₀)-alkyl)carbamoyl,or CON(CH₂)ₕ, in which a CH₂ group can be replaced by O,S,N-(C₁-C₈)-alkylimino, N-(C₃-C₈)-cycloalkylimino, N(C₃-C₈)-cycloalkyl-(C₁-C₄)-alkylimino, N-(C₆-C₁₂)-arylimino, N-(C₇-C₁₆)-aralkylimino or N-(C₁-C₄)-alkoxy-(C₁-C₆)-alkylimino, and h is from 3 to 7,
carbamoyloxy, N-(C₁-C₁₂)-alkylcarbamoyloxy, N,N-di-(C₁-C₁₂)-alkylcarbamoyloxy, N-(C₃-C₈)-cycloalkylcarbamoyloxy, N-(C₆-C₁₆)-arylcarbamoyloxy, N-(C₇-C₁₆)-aralkylcarbamoyloxy, N-(C₁-C₁₀)-alkyl-N-(C₆-C₁₂)-arylcarbamoyloxy, N-(C₁-C₁₀)-alkyl-N-(C₇-C₁₆)-aralkylcarbamoyloxy, N-((C₁-C₁₀)-alkyl)carbamoyloxy, N-((C₆-C₁₂)-aryloxy-(C₁-C₁₀)-alkyl)carbamoyloxy, N-((C₇-C₁₆)-aralkyloxy-(C₁-C₁₀)-alkyl)carbamoyloxy, N-(C₁-C₁₀)-alkyl-N-((C₁-C₁₀)-alkoxy-(C₁-C₁₀)-alkyl)carbamoyloxy, N-(C₁-C₁₀)-alkyl-N-((C₆-C₁₂)-aryloxy-(C₁-C₁₀)-alkyl)carbamoyloxy, N-(C₁-C₁₀)-alkyl-N-((C₇-C₁₆)-aralkyloxy-(C₁-C₁₀)-alkyl)carbamoyloxy,
amino, (C₁-C₁₂)-alkylamino, di-(C₁-C₁₂)-alkylamino, (C₃-C₈)-cycloalkylamino, (C₃-C₁₂)-alkenylamino, (C₃-C₁₂)-alkynylamino, N-(C₆-C₁₂)-arylamino, N-(C₇-C₁₁)-aralkylamino, N-alkyl-aralkylamino, N-alkyl-arylamino, (C₁-C₁₂)-alkoxyamino, (C₁-C₁₂)-alkoxy-N-(C₁-C₁₀)-alkylamino,
(C₁-C₁₂)-alkanoylamino, (C₃-C₈)-cycloalkanoylamino, (C₆-C₁₂)-aroylamino, (C₇-C₁₆)-aralkanoylamino, (C₁-C₁₂)-alkanoyl-N-(C₁-C₁₀)-alkylamino, (C₃-C₈)-cycloalkanoyl-N-(C₁-C₁₀)-alkylamino, (C₆-C₁₂)-aroyl-N-(C₁-C₁₀)-alkylamino, (C₇-C₁₁)-aralkanoyl-N-(C₁-C₁₀)-alkylamino,
(C₁-C₁₂)-alkanoylamino-(C₁-C₈)-alkyl, (C₃-C₈)-cycloalkanoylamino-(C₁-C₈)-alkyl, (C₆-C₁₂)-aroylamino-(C₁-C₈)-alkyl, (C₇-C₁₆)-aralkanoylamino-(C₁-C₈)-alkyl, amino-(C₁-C₁₀)-alkyl, N-(C₁-C₁₀)-alkylamino-(C₁-C₁₀)-alkyl, N,N-di-(C₁-C₁₀)-alkylamino-(C₁-C₁₀)-alkyl, (C₃-C₈)-cycloalkylamino-(C₁-C₁₀)-alkyl,
(C₁-C₁₂)-alkylmercapto, (C₁-C₁₂)-alkylsulfinyl, (C₁-C₁₂)-alkylsulfonyl, (C₆-C₁₆)-arylmercapto, (C₆-C₁₆)-arylsulfinyl, (C₆-C₁₆)-arylsulfonyl, (C₇-C₁₆)-aralkylmercapto, (C₇-C₁₆)-aralkylsulfinyl or (C₇-C₁₆)-aralkylsulfonyl, and
R⁴ is R'', provided Q has the meaning of NR', where R' and R'' are identical or different and are hydrogen, (C₆-C₁₂)-aryl, (C₇-C₁₁)-aralkyl, (C₁-C₈)-alkyl, (C₁-C₈)-alkoxy-(C₁-C₈)-alkyl, (C₇-C₁₂)-aralkoxy-(C₁-C₈)-alkyl, (C₆-C₁₂)-aryloxy-(C₁-C₈)-alkyl, (C₁-C₁₀)-alkanoyl, optionally substituted (C₇-C₁₆)-aralkanoyl or optionally substituted (C₆-C₁₂)-aroyl, or
R' and R'' together are -[CH₂]h, in which a CH₂ group can be replaced by O, S, N-acylimino or N-(C₁-C₁₀)-alkoxycarbonylimino, and
f is 1 to 8,
g is 0 or 1 to (2f+1),
x is 0 to 3,
h is 3 to 7,
including the physiologically active salts,
where 3-benzyloxypyridine-2-carboxylic acid (L-threonyl methyl ester) amide, 3-benzyloxypyridine-2-carboxylic acid (L-threonyl(Fmoc-Phg) tert-butyl ester) amide, 3-benzyloxypyridine-2-carboxylic acid (L-threonyl tert-butyl ester) amide, 3-benzyloxypyridine-2-carboxylic acid (D-allothreonyl methyl ester) amide and 3-benzyloxypicolinylglycine ethyl ester are excepted.

2. A compound of the formula I, as claimed in claim 1,
in which
Q is O, S, NR' or a bond,
X is O,
Y is CR³ or,
if R¹ and R² form a cycle,
Y is N or CR³,
m is 0 or 1, and
G is the radical of an alcohol GOH, and 3-benzyloxypicolinylglycine ethyl ester is expected.

3. A compound of the formula I, as claimed in claims 1 and 2, in which
Q is O, NR' or a bond,
X is O and G is the radical of an alcohol GOH, and 3-benzyloxypicolinylglycine ethyl ester is expected.

4. A compound of the formula I, as claimed in claims 1 and 2, in which
Q is S,
X is O,
m is 0 or 1 and G is the radical of an alcohol GOH.

5. A compound of the formula I, as claimed in claims 1, 2 and 4, in which
Q is S
X is O
m is 0 and G is the radical of an alcohol GOH.

6. A compound of the formula I as claimed in claims 1 to 3, in which
Q is O, NR' or a bond,
X is O,
Y is CR³ or, if R¹ and R² form a cycle, N or CR³,
m is 0 or 1,
A is (C₁-C₃)-alkylene which is optionally substituted once by halogen, cyano, trifluoromethyl, (C₁-C₆)-alkyl, (C₁-C₆)-hydroxyalkyl, (C₁-C₆)-alkoxy or -O-[CH₂]ₓ-C_{f}H_{(2f+1-g)}F_{g}, or
A is -CHR⁵-, where R⁵ is one of the substituents of the α-carbon atom of an α-amino acid, in particular of a natural L-amino acid and of its D-isomer,
B is -CO₂G, where
G is the radical of an alcohol GOH, in which G is a branched or unbranched, or cyclic, aliphatic (C₁-C₂₀)-alkyl radical, or a branched or unbranched, optionally cyclic, (C₂-C₂₀)-alkenyl radical, a retinyl radical, a (C₂-C₂₀)-alkynyl radical or a corresponding (C₄-C₂₀)-alkenynyl radical, where the radicals can in each case contain one or more multiple bonds, or a (C₆-C₁₆)-aryl radical, a (C₇-C₁₆)-aralkyl radical or a 5-or 6-membered, preferably nitrogen-containing, heteroaryl radical or a 5- or 6-membered, preferably nitrogen-containing, heteroaralkyl radical, where the above radicals are, in particular, one or more substituents from the group
hydroxyl, halogen, cyano, trifluoromethyl, nitro, carboxyl, (C₁-C₁₂)-alkyl, (C₃-C₈)-cycloalkyl, (C₅-C₈)-cycloalkenyl, (C₆-C₁₂)-aryl, (C₇-C₁₆)-aralkyl, (C₂-C₁₂)-alkenyl, (C₂-C₁₂)-alkynyl, (C₁-C₁₂)-alkoxy, (C₁-C₁₂)-alkoxy-(C₁-C₁₂)alkyl, (C₁-C₁₂)-alkoxy- (C₁-C₁₂)alkoxy, (C₆-C₁₂)-aryloxy, (C₇-C₁₆)-aralkyloxy, (C₁-C₈)-hydroxyalkyl, -O-[CH₂₋]ₓC_{f}H_{(2f+1-g)}F_{g}, -OCF₂Cl, -OCF₂-CHFCl,
(C₁-C₁₂)-alkylcarbonyl, (C₃-C₈)-cycloalkylcarbonyl, (C₆-C₁₂)-arylcarbonyl, (C₇-C₁₆)- ralkylcarbonyl, cinnamoyl, (C₂-C₁₂)-alkenylcarbonyl, (C₂-C₁₂)-alkynylcarbonyl,
(C₁-C₁₂)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₂-C₁₂)-alkenyloxycarbonyl, (C₂-C₁₂)-alkynyloxycarbonyl,
(C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy, (C₂-C₁₂)-alkenylcarbonyloxy, (C₂-C₁₂)-alkynylcarbonyloxy,
(C₁-C₁₂)-alkoxycarbonyloxy, (C₁-C₁₂)-alkoxy(C₁-C₁₂)-alkoxycarbonyloxy, (C₆-C₁₂)-aryloxycarbonyloxy, (C₇-C₁₆)-aralkyloxycarbonyloxy, (C₃-C₈)-cycloalkoxycarbonyloxy, (C₂-C₁₂)-alkenyloxycarbonyloxy, (C₂-C₁₂)-alkynyloxycarbonyloxy,
carbamoyl, N-(C₁-C₁₂)-alkylcarbamoyl, N,N-di-(C₁-C₁₂)-alkylcarbamoyl, N-(C₃-C₈)-cycloalkylcarbamoyl, N-(C₆-C₁₆)-arylcarbamoyl, N-(C₇-C₁₆)-aralkylcarbamoyl, N-(C₁-C₁₀)-alkyl-N-(C₆-C₁₆)arylcarbamoyl, N-(C₁-C₁₀)-alkyl-N-(C₇-C₁₆)-aralkylcarbamoyl,
N-((C₁-C₁₀)-alkoxy-(C₁-C₁₀)alkyl)carbamoyl,
N-((C₆-C₁₂)-aryloxy-(C₁-C₁₀)alkyl)carbamoyl,
N-((C₇-C₁₆)-aralkyloxy-(C₁-C₁₀)alkyl)carbamoyl,
N-(C₁-C₁₀)-alkyl-N-((C₁-C₁₀)-alkoxy-(C₁-C₁₀)-alkyl)carbamoyl,
N-(C₁-C₁₀)-alkyl-N-((C₆-C₁₆)-aryloxy-(C₁-C₁₀)alkyl)-carbamoyl,
N-(C₁-C₁₀)-alkyl-N-((C₇-C₁₆)aralkyloxy-C₁-C₁₀)alkyl)carbamoyl,
carbamoyloxy, N-(C₁-C₁₂)-alkylcarbamoyloxy, N,N-di-(C₁-C₁₂)-alkylcarbamoyloxy, N-(C₃-C₈)-cycloalkylcarbamoyloxy, N-(C₆-C₁₂)-arylcarbamoyloxy, N-(C₇-C₁₆)-aralkylcarbamoyloxy, N-(C₁-C₁₀)-alkyl-N-(C₆-C₁₂)arylcarbamoyloxy, N-(C₁-C₁₀)-alkyl-N-(C₇-C₁₆)-aralkylcarbamoyloxy,
N-((C₁-C₁₀)alkyl)carbamoyloxy,
N-((C₆-C₁₂)-aryloxy-(C₁-C₁₀)alkyl)carbamoyloxy,
N-((C₇-C₁₆)-aralkyloxy-(C₁-C₁₀)alkyl)carbamoyloxy, N-(C₁-C₁₀)-alkyl-N-((C₁-C₁₀)-alkoxy-(C₁-C₁₀)-alkyl)carbamoyloxy,
N-(C₁-C₁₀)-alkyl-N-((C₆-C₁₂)-aryloxy-(C₁-C₁₀)alkyl)-carbamoyloxy,
N-(C₁-C₁₀)-alkyl-N-((C₇-C₁₆)-aralkyloxy-(C₁-C₁₀)-alkyl)carbamoyloxy,
amino, (C₁-C₁₂)-alkylamino, di-(C₁-C₁₂)alkylamino, (C₃-C₈)-cycloalkylamino, (C₂-C₁₂)-alkenylamino, (C₂-C₁₂)-alkynylamino, N-(C₆-C₁₂)-arylamino, N-(C₇-C₁₁)-aralkylamino, N-alkyl-aralkylamino, N-alkyl-arylamino, (C₁-C₁₂)-alkoxyamino, (C₁-C₁₂)-alkoxy-N-(C₁-C₁₀)-alkylamino,
(C₁-C₁₂)-alkanoylamino, (C₃-C₈)-cycloalkanoylamino, (C₆-C₁₂)-aroylamino, (C₇-C₁₆)-aralkanoylamino, (C₁-C₁₂)-alkanoyl-N-(C₁-C₁₀)-alkylamino, (C₃-C₈)-cycloalkanoyl-N-(C₁-C₁₀)-alkylamino, (C₆-C₁₂)-aroyl-N-(C₁-C₁₀)alkylamino, (C₇-C₁₁)-aralkanoyl-N-(C₁-C₁₀)-alkylamino,
(C₁-C₁₂)-alkanoylamino-(C₁-C₈)-alkyl, (C₃-C₈)-cycloalkanoylamino-(C₁-C₈)alkyl, (C₆-C₁₂)-aroylamino-(C₁-C₈)-alkyl, (C₇-C₁₂)-aralkanoylamino-(C₁-C₈)-alkyl, amino-(C₁-C₁₀)-alkyl, N-(C₁-C₁₀)alkylamino-(C₁-C₁₀)alkyl, N,N-di(C₁-C₁₀)-alkylamino-(C₁-C₁₀)alkyl, (C₃-C₈)cycloalkylamino-(C₁-C₁₀)alkyl,
(C₁-C₁₂)-alkylmercapto, (C₁-C₁₂)-alkylsulfinyl, (C₁-C₁₂)-alkylsulfonyl, (C₆-C₁₆)-arylmercapto, (C₆-C₁₆)-arylsulfinyl, (C₆-C₁₂)-arylsulfonyl, (C₇-C₁₆)-aralkylmercapto,(C₇-C₁₆)-aralkylsulfinyl, (C₇-C₁₆)-aralkylsulfonyl,
sulfamoyl, N-(C₁-C₁₀)-alkylsulfamoyl, N,N-di-(C₁-C₁₀)-alkylsulfamoyl, (C₃-C₈)-cycloalkylsulfamoyl,
N-(C₆-C₁₂)-arylsulfamoyl,
N-(C₇-C₁₆)-aralkylsulfamoyl,
N-(C₁-C₁₀)-alkyl-N-(C₆-C₁₂)-arylsulfamoyl,
N-(C₁-C₁₀)-alkyl-N-(C₇-C₁₆)-aralkylsulfamoyl,
(C₁-C₁₀)-alkyl-sulfonamido,
N-((C₁-C₁₀)alkyl)-(C₁-C₁₀)alkylsulfonamido,
(C₇-C₁₆)-aralkylsulfonamido or N-((C₁-C₁₀)alkyl-(C₇-C₁₆)-aralkylsulfonamido, where the radicals which contain an aryl radical can, for their part, be substituted on the aryl by from 1 to 5 identical or different radicals from the group: hydroxyl, halogen, cyano, trifluoromethyl, nitro, carboxyl, (C₁-C₁₂)-alkyl, (C₃-C₈)-cycloalkyl, (C₆-C₁₂)-aryl, (C₇-C₁₆)-aralkyl, (C₁-C₁₂)-alkoxy, (C₁-C₁₂)-alkoxy-(C₁-C₁₂)alkyl, (C₁-C₁₂)-alkoxy-(C₁-C₁₂)alkoxy, (C₆-C₁₂)-aryloxy, (C₇-C₁₆)-aralkyloxy, (C₁-C₈)-hydroxyalkyl,
(C₁-C₁₂)-alkylcarbonyl, (C₃-C₈)-cycloalkylcarbonyl, (C₆-C₁₂)-arylcarbonyl, (C₇-C₁₆)-aralkylcarbonyl,
(C₁-C₁₂)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₂-C₁₂)-alkenyloxycarbonyl, (C₂-C₁₂)-alkynyloxycarbonyl,
(C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy, (C₂-C₁₂)-alkenylcarbonyloxy, (C₂-C₁₂)-alkynylcarbonyloxy,
(C₁-C₁₂)-alkoxycarbonyloxy, (C₁-C₁₂)-alkoxy-(C₁-C₁₂)-alkoxycarbonyloxy, (C₆-C₁₂)-aryloxycarbonyloxy, (C₇-C₁₆)-aralkyloxycarbonyloxy, (C₃-C₈)-cycloalkoxycarbonyloxy, (C₂-C₁₂)-alkenyloxycarbonyloxy, (C₂-C₁₂)-alkynyloxycarbonyloxy,
carbamoyl, N-(C₁-C₁₂)-alkylcarbamoyl, N,N-di-(C₁-C₁₂)-alkylcarbamoyl, N-(C₃-C₈)-cycloalkylcarbamoyl, N-(C₆-C₁₂)-arylcarbamoyl, N-(C₇-C₁₆)-aralkylcarbamoyl, N-(C₁-C₁₀)-alkyl-N-(C₆-C₁₂)-arylcarbamoyl, N-(C₁-C₁₀)-alkyl-N-(C₇-C₁₆)-aralkylcarbamoyl,
N-((C₁-C₁₀)-alkoxy-(C₁-C₁₀)alkyl)carbamoyl,
N-((C₆-C₁₂)-aryloxy-(C₁-C₁₀)alkyl)carbamoyl,
N-((C₇-C₁₆)-aralkyloxy-(C₁-C₁₀)alkyl)carbamoyl,
N-(C₁-C₁₀)-alkyl-N-((C₁-C₁₀)-alkoxy-(C₁-C₁₀)alkyl)-carbamoyl,
N-(C₁-C₁₀)-alkyl-N-((C₆-C₁₂)-aryloxy-(C₁-C₁₀)alkyl)-carbamoyl,
N-(C₁-C₁₀)-alkyl-N-((C₇-C₁₆)aralkyloxy-(C₁-C₁₀)-alkyl)carbamoyl,
carbamoyloxy, N-(C₁-C₁₂)-alkylcarbamoyloxy, N,N-di-(C₁-C₁₂)-alkylcarbamoyloxy, N-(C₃-C₈)-cycloalkylcarbamoyloxy, N-(C₆-C₁₂)-arylcarbamoyloxy, N-(C₇-C₁₆)-aralkylcarbamoyloxy, N-(C₁-C₁₀)-alkyl-N-(C₆-C₁₂)arylcarbamoyloxy, N-(C₁-C₁₀)-alkyl-N-(C₇-C₁₆)-aralkylcarbamoyloxy, N-((C₁-C₁₀)alkyl)carbamoyloxy,
N-((C₆-C₁₂)-aryloxy-(C₁-C₁₀)alkyl)carbamoyloxy,
N-((C₇-C₁₆)-aralkyloxy-(C₁-C₁₀)alkyl)carbamoyloxy, N-(C₁-C₁₀)-alkyl-N-((C₁-C₁₀)-alkoxy-(C₁-C₁₀)alkyl)-carbamoyloxy,
N-(C₁-C₁₀)-alkyl-N-((C₆-C₁₂)-aryloxy-(C₁-C₁₀)alkyl)-carbamoyloxy,
N-(C₁-C₁₀)-alkyl-N-((C₇-C₁₆)-aralkyloxy-(C₁-C₁₀)-alkyl)carbamoyloxy,
amino, (C₁-C₁₂)-alkylamino, di-(C₁-C₁₂)alkylamino, (C₃-C₈)-cycloalkylamino, (C₃-C₁₂)-alkenylamino, (C₃-C₁₂)-alkynylamino, N-(C₆-C₁₂)-arylamino, N-(C₇-C₁₁)-aralkylamino, N-alkyl-aralkylamino, N-alkyl-arylamino, (C₁-C₁₂)-alkoxyamino, (C₁-C₁₂)-alkoxy-N-(C₁-C₁₀)-alkylamino,
(C₁-C₁₂)-alkanoylamino, (C₃-C₈)-cycloalkanoylamino, (C₆-C₁₂)-aroylamino, (C₇-C₁₆)-aralkanoylamino, (C₁-C₁₂)-alkanoyl-N-(C₁-C₁₀)-alkylamino, (C₃-C₈)-cycloalkanoyl-N-(C₁-C₁₀)-alkylamino, (C₆-C₁₂)-aroyl-N-(C₁-C₁₀)alkylamino, (C₇-C₁₁)-aralkanoyl-N-(C₁-C₁₀)-alkylamino,
(C₁-C₁₂)-alkanoylamino-(C₁-C₈)-alkyl, (C₃-C₈)-cycloalkanoylamino-(C₁-C₈)alkyl, (C₆-C₁₂)-aroylamino-(C₁-C₈)-alkyl, (C₇-C₁₆)-aralkanoylamino-(C₁-C₈)-alkyl, amino-(C₁-C₁₀)-alkyl, N-(C₁-C₁₀)alkylamino-(C₁-C₁₀)alkyl, N,N-di(C₁-C₁₀)-alkylamino-(C₁-C₁₀)alkyl, (C₃-C₈)-cycloalkylamino-(C₁-C₁₀)alkyl,
(C₁-C₁₂)-alkylmercapto, (C₁-C₁₂)-alkylsulfinyl, (C₁-C₁₂)-alkylsulfonyl, (C₆-C₁₂)-arylmercapto, (C₆-C₁₂)-arylsulfinyl, (C₆-C₁₂)-arylsulfonyl, (C₇-C₁₆)-aralkylmercapto, (C₇-C₁₆)-aralkylsulfinyl or (C₇-C₁₆)-aralkylsulfonyl,
R² is hydrogen, (C₁-C₂₀)-alkyl, (C₂-C₂₀)-alkenyl, (C₂-C₂₀)-alkynyl, (C₁-C₂₀)-alkoxy, (C₂-C₂₀)-alkenyloxy, (C₂-C₂₀)-alkynyloxy, retinyloxy, (C₁-C₂₀)-alkoxy-(C₁-C₃)-alkyl, (C₂-C₂₀)-alkenyloxy-(C₁-C₃)-alkyl, retinyloxy-(C₁-C₃)-alkyl, (C₂-C₂₀)-alkynyloxy-(C₁-C₃)-alkyl, halogen, cyano, trifluoromethyl, (C₁-C₈)-hydroxyalkyl, (C₁-C₂₀)-alkanoyl, (C₇-C₁₆)-aralkanoyl, (C₆-C₁₂)-aroyl, (C₆-C₁₂)-aryl, (C₇-C₁₆)-aralkyl, -O-[CH₂]ₓC_{f}H_{(2f+1-g)}F_{g}, NR'R'',(C₁-C₁₀)-alkylmercapto, (C₁-C₁₀)-alkylsulfinyl, (C₁-C₁₀)-alkylsulfonyl, (C₆-C₁₂)-arylmercapto, (C₆-C₁₂)-arylsulfinyl, (C₆-C₁₂)-arylsulfonyl, (C₇-C₁₂)-aralkylmercapto, (C₇-C₁₂)-aralkylsulfinyl, (C₇-C₁₂)-aralkylsulfonyl, (C₆-C₁₂)-aryloxy, (C₇-C₁₆)-aralkyloxy, carboxyl, (C₁-C₂₀)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₂-C₂₀)-alkenyloxycarbonyl, retinyloxycarbonyl, (C₂-C₂₀)-alkynyloxycarbonyl, (C₃-C₈)-cycloalkyl-(C₁-C₆)-alkoxycarbonyl, (C₃-C₈)-cycloalkoxy-(C₁-C₆)-alkoxycarbonyl, (C₆-C₁₂)-aryloxy-(C₁-C₆)-alkoxycarbonyl, (C₇-C₁₆)-aralkoxy-(C₁-C₆)-alkoxycarbonyl,
carbamoyl, N-(C₁-C₁₂)-alkylcarbamoyl, N,N-di-(C₁-C₁₂)-alkylcarbamoyl, N-(C₃-C₈)-cycloalkylcarbamoyl, N,N-dicyclo(C₃-C₈)-alkylcarbamoyl, N-(C₁-C₁₀)-alkyl-N-(C₃-C₈)-cycloalkylcarbamoyl, N-((C₃-C₈)-cycloalkyl(C₁-C₆)-alkyl)carbamoyl, N-(C₁-C₆)-alkyl-N-((C₃-C₈)-cycloalkyl-(C₁-C₆)-alkyl)carbamoyl, N-(+)-dehydroabietylcarbamoyl, N-(C₁-C₆)-alkyl-N-(+)-dehydroabietylcarbamoyl,N-(C₆-C₁₂)-arylcarbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl, N-((C₁-C₁₂)-alkoxy-(C₁-C₁₀)-alkyl)carbamoyl, N-((C₆-C₁₆)-aryloxy-(C₁-C₁₀)-alkyl)carbamoyl, N-((C₇-C₁₆)-aralkyloxy-(C₁-C₁₀)-alkyl)carbamoyl, N-(C₁-C₁₀)-alkyl-N-((C₁-C₁₀)-alkoxy-(C₁-C₁₀)-alkyl)carbamoyl, N-(C₁-C₁₀)-alkyl-N-((C₆-C₁₂)-aryloxy-(C₁-C₁₀)-alkyl)carbamoyl, N-(C₁-C₁₀)-alkyl-N-((C₇-C₁₆)-aralkyloxy-(C₁-C₁₀)-alkyl)carbamoyl, or CON(CH₂)ₕ, in which a CH₂ group can be replaced by O, S, N-(C₁-C₈)-alkylimino, N-(C₃-C₈)-cycloalkylimino, N-(C₃-C₈)-cycloalkyl-(C₁-C₄)-alkylimino, N-(C₆-C₁₂)-arylimino, N-(C₇-C₁₆)-aralkylimino or N-(C₁-C₄)-alkoxy-(C₁-C₆)-alkylimino, and h is from 3 to 7,
where aryl is substituted in the manner as defined for R¹ and R³,
R¹ and R³ are identical or different and are hydrogen, halogen, (C₁-C₁₂)-alkyl, (C₁-C₁₂)-alkoxy, -O-[CH₂]ₓ-C_{f}H_{(2f+1-g)}Hal_{g}, (C₁-C₁₂)-alkoxy-(C₁-C₁₂)-alkyl, (C₁-C₈)-alkoxy-(C₁-C₁₂)-alkoxy, (C₁-C₁₂)-alkoxy-(C₁-C₈)-alkoxy-(C₂-C₆)-alkyl, (C₇-C₁₁)-aralkyloxy, (C₃-C₈)-cycloalkyl, (C₃-C₈)-cycloalkyl-(C₁-C₈)-alkyl, (C₃-C₈)-cycloalkyloxy, (C₃-C₈)-cycloalkyl-(C₁-C₈)-alkoxy, (C₃-C₈)-cycloalkyloxy-(C₁-C₈)-alkyl, (C₃-C₈)-cycloalkyloxy-(C₁-C₈)-alkoxy, (C₃-C₈)-cycloalkyl-(C₁-C₆)-alkyl-(C₁-C₆)-alkoxy, (C₃-C₈)-cycloalkyl-(C₁-C₆)-alkoxy-(C₁-C₆)-alkyl, (C₃-C₈)-cycloalkoxy-(C₁-C₆)-alkoxy-(C₁-C₆)-alkyl, NR^{Y}R^{Z}, (C₁-C₈)-alkylmercapto, (C₁-C₈)-alkylsulfinyl or (C₁-C₈)-alkylsulfonyl, (C₆-C₁₂)-arylmercapto, (C₆-C₁₂)-arylsulfinyl, (C₆-C₁₂)-arylsulfonyl, (C₇-C₁₂)-aralkylmercapto, (C₇-C₁₁)-aralkylsulfinyl, (C₇-C₁₁)-aralkylsulfonyl, substituted (C₆-C₁₂)-aryloxy-(C₁-C₆)-alkyl, (C₇-C₁₁)-aralkoxy-(C₁-C₆)-alkyl, (C₆-C₁₂)-aryloxy-(C₁-C₆)-alkoxy-(C₁-C₆)-alkyl, (C₇-C₁₁)-aralkyloxy-(C₁-C₆)-alkoxy-(C₁-C₆)-alkyl, (C₆-C₁₂)-aryloxy, (C₇-C₁₁)-aralkyloxy,(C₆-C₁₂)-aryloxy-(C₁-C₆)-alkoxy or (C₇-C₁₁)-aralkoxy-(C₁-C₆)-alkoxy, where an aromatic radical carries 1, 2, 3, 4 or 5 identical or different substituents from the group hydrogen, halogen, cyano, nitro, trifluoromethyl, (C₁-C₁₆)-alkyl, (C₁-C₁₆)-alkenyl, (C₁-C₆)-hydroxyalkyl, (C₁-C₁₆)-alkoxy, (C₁-C₁₆)-alkenyloxy, -O-[CH₂]ₓC_{f}H_{(2f+1-g)}F_{g}, -OCF₂Cl, -O-CF₂-CHFCl, (C₁-C₆)-alkylmercapto, (C₁-C₆)-alkylsulfinyl, (C₁-C₆)-alkylsulfonyl, (C₁-C₆)-alkylcarbonyl, (C₁-C₆)-alkoxycarbonyl, carbamoyl, N-(C₁-C₄)-alkylcarbamoyl, N,N-di-(C₁-C₄)-alkylcarbamoyl, (C₁-C₆)-alkylcarbonyloxy, (C₃-C₈)-cycloalkylcarbamoyl, phenyl, benzyl, phenoxy, benzyloxy, NR^{Y}R^{Z}, phenylmercapto, phenylsulfonyl, phenylsulfinyl, sulfamoyl, N-(C₁-C₄)-alkylsulfamoyl or N,N-di-(C₁-C₄)-alkylsulfamoyl, or optionally carries up to 3 of the abovementioned identical or different substituents, and two adjacent carbon atoms of the aralkyloxy radical together carry a chain -[CH₂-] and/or -CH=CH-CH=CH-, where a CH₂ group of the chain is optionally replaced by O, S, SO, SO₂, or NR',
R¹ and R² or R² and R³ form a chain [CH₂]ₒ, where o is 3, 4 or 5, or
form, together with the pyridine or pyridazine carrying them, a cinnoline ring, a quinoline ring or an isoquinoline ring,
R⁴ is, if Q is a bond, fluorine, chlorine or bromine, or, if Q is O, a branched or unbranched (C₁-C₂₀)-alkyl radical, which can contain up to 3 C-C multiple bonds, an unsubstituted saturated fluoroalkyl radical of the formula [CH₂]ₓC_{f}H_{(2f+1-g)}F_{g}, a (C₆-C₁₆)-aryl radical or a (C₇-C₁₆)-aralkyl radical, which can contain up to 2 C-C multiple bonds in the alkyl chain, or a heteroaryl radical or a heteroarylalkyl radical, where these radicals are substituted by one or more radicals from the group hydroxyl, fluorine, chlorine, cyano, trifluoromethyl, carboxyl, (C₁-C₁₂)-alkyl, (C₃-C₈)-cycloalkyl, (C₃-C₈)-cycloalkyl-(C₁-C₁₂)-alkyl, (C₃-C₈)-cycloalkoxy, (C₃-C₈)-cycloalkyl-(C₁-C₁₂)-alkoxy,(C₃-C₈)-cycloalkyloxy-(C₁-C₁₂)-alkyl, (C₃-C₈)-cycloalkyloxy-(C₁-C₁₂)-alkoxy, (C₃-C₈)-cycloalkyl-(C₁-C₈)-alkyl-(C₁-C₆)-alkoxy, (C₃-C₈)-cycloalkoxy-(C₁-C₈)-alkoxy-(C₁-C₈)-alkoxy, (C₆-C₁₂)-aryl, (C₇-C₁₆)-aralkyl, (C₂-C₁₂)-alkenyl, (C₂-C₁₂)-alkynyl, (C₁-C₁₂)-alkoxy, (C₁-C₁₂)-alkoxy-(C₁-C₁₂)-alkoxy, (C₁-C₁₂)-alkoxy-(C₁-C₈)-alkoxy-(C₁-C₈)-alkyl, (C₆-C₁₂)-aryloxy, (C₇-C₁₆)-aralkyloxy, (C₆-C₁₂)-aryloxy-(C₁-C₆)-alkoxy, (C₇-C₁₆)-aralkoxy-(C₁-C₆)-alkoxy, (C₁-C₈)-hydroxyalkyl, -O-[CH₂]ₓC_{f}H_{(2f+1-g)}F_{g},
(C₁-C₁₂)-alkylcarbonyl, (C₃-C₈)-cycloalkylcarbonyl, (C₆-C₁₂)-arylcarbonyl, (C₇-C₁₆)-aralkylcarbonyl,
(C₁-C₁₂)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₂-C₁₂)-alkenyloxycarbonyl, (C₂-C₁₂)-alkynyloxycarbonyl, (C₃-C₈)-cycloalkyl-(C₁-C₆)-alkoxycarbonyl,
(C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, carbamoyl, N-(C₁-C₁₂)-alkylcarbamoyl, N,N-di-(C₁-C₁₂)-alkylcarbamoyl, N-(C₃-C₈)-cycloalkylcarbamoyl, N,N-dicyclo-(C₃-C₈)-alkylcarbamoyl, N-(C₁-C₁₀)-alkyl-N-(C₃-C₈)-cycloalkylcarbamoyl, N-((C₃-C₈)-cycloalkyl-(C₁-C₆)-alkyl)carbamoyl, N-(C₁-C₆)-alkyl-N-((C₃-C₈)-cycloalkyl-(C₁-C₆)-alkyl)carbamoyl, N-(+)-dehydroabietylcarbamoyl, N-(C₁-C₆)-alkyl-N-(+)-dehydroabietylcarbamoyl, N-(C₆-C₁₂)-arylcarbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl, N-((C₁-C₁₀)-alkoxy-(C₁-C₁₀)-alkyl)carbamoyl, N-((C₆-C₁₆)-aryloxy-(C₁-C₁₀)-alkyl)carbamoyl, N-((C₇-C₁₆)-aralkyloxy-(C₁-C₁₀)-alkyl)carbamoyl, or CON(CH₂)ₕ, in which a CH₂ group can be replaced by O, N-(C₁-C₈)-alkylimino, N-(C₃-C₈)-cycloalkylimino, N-(C₃-C₈)-cycloalkyl-(C₁-C₄)-alkylimino, N-(C₆-C₁₂)-arylimino or N-(C₇-C₁₆)-aralkylimino, and h is from 3 to 6,
where the radicals which contain an aryl radical can, for their part, be substituted on the aryl by from 1 to 5 identical or different radicals from the group:
hydroxyl, fluorine, chlorine, cyano, trifluoromethyl, carboxyl, (C₁-C₁₂)-alkyl, (C₃-C₈)-cycloalkyl, (C₁-C₆)-alkoxy, (C₃-C₈)-cycloalkoxy, (C₁-C₁₂)-alkoxycarbonyl,
N-(C₁-C₆)-alkylcarbamoyl, N,N-di-(C₁-C₆)-alkylcarbamoyl or N-(C₃-C₈)-cycloalkylcarbamoyl, and
R⁴ is R'', provided Q has the meaning of NR', where R' and R'' are identical or different and are hydrogen, (C₁-C₈)-alkyl, or (C₇-C₁₁)-aralkyl which is optionally substituted once by fluorine, chlorine or (C₁-C₄)-alkoxy,
R^{Y} and R^{Z} are identical or different and are hydrogen, (C₆-C₁₂)-aryl, (C₁-C₁₀)-alkyl, (C₃-C₁₀)-cycloalkyl, (C₁-C₈)-alkoxy-(C₁-C₈)-alkyl, (C₇-C₁₂)-aralkoxy-(C₁-C₈)-alkyl, (C₆-C₁₂)-aryloxy-(C₁-C₈)-alkyl, (C₁-C₁₀)-alkanoyl, optionally substituted (C₇-C₁₆)-aralkanoyl or optionally substituted (C₆-C₁₂)-aroyl, or
R^{Y} and R^{Z} together are -[CH₂]ₕ₋, in which a CH₂ group can be replaced by O, S, N-(C₁-C₄)-alkanoylimino or N-(C₁-C₄)-alkoxycarbonylimino, and
f is 1 to 8,
g is 0 or 1 to (2f+1),
h is 3 to 6,
x is 0 to 3, and
n is 3 or 4, including the physiologically active salts, where 3-benzyloxypyridine-2-carboxylic acid (L-threonyl methyl ester) amide, 3-benzyloxypyridine-2-carboxylic acid (L-threonyl(Fmoc-Phg) tert-butyl ester) amide, 3-benzyloxypyridine-2-carboxylic acid (L-threonyl tert-butyl ester) amide, 3-benzyloxypyridine-2-carboxylic acid (D-allothreonyl methyl ester) amide and 3-benzyloxypicolinylglycine ethyl ester are excepted.

7. A compound of the formula I as claimed in claims 1 to 3 and 6, in which
Q is O, NR' or a bond,
X is O,
Y is CR³ or, if R¹ and R² form a cycle, N or CR³,
m is 0,
A is (C₁-C₃)-alkylene which is optionally substituted once by halogen, cyano, trifluoromethyl, (C₁-C₆)-alkyl, (C₁-C₆)-hydroxyalkyl, (C₁-C₆)-alkoxy or -O-[CH₂]ₓ-C_{f}H_{(2f+1-g)}F_{g} or
A is -CHR⁵-, where R⁵ is one of the substituents of the α-carbon atom of an α-amino acid, in particular of a natural L-amino acid and of its D-isomer,
B is -CO₂G, where
G is a branched or unbranched, or cyclic, aliphatic (C₁-C₂₀)-alkyl radical, a retinyl radical or a branched or unbranched (C₂-C₂₀)-alkenyl radical or a (C₂-C₂₀)-alkynyl radical which can in each case contain one or more C-C multiple bonds, or a (C₆-C₁₂)-aryl radical, a (C₇-C₁₁)-aralkyl radical or a heteroaryl or heteroaralkyl radical, where the above radicals can carry one or two substituents from the group (C₁-C₈)-alkyl, (C₃-C₈)-cycloalkyl, fluorine, chlorine, hydroxyl, (C₁-C₆)-alkoxy, (C₁-C₆)-alkoxy-(C₁-C₆)-alkoxy, (C₆-C₁₂)-aryloxy, (C₇-C₁₂)-aralkyloxy,
(C₁-C₈)-alkylcarbonyl, (C₃-C₈)-cycloalkylcarbonyl, (C₆-C₁₂)-arylcarbonyl, (C₇-C₁₂)-aralkylcarbonyl,
(C₁-C₈)-alkoxycarbonyl, (C₁-C₆)-alkoxy-(C₁-C₆)-alkoxycarbonyl, (C₆-C₁₂)-aryloxycarbonyl, (C₇-C₁₂)-aralkoxycarbonyl, (C₃-C₈)-cycloalkoxycarbonyl,
(C₁-C₂₀)-alkylcarbonyloxy, (C₃-C₈)cycloalkylcarbonyloxy, (C₆-C₁₂)-arylcarbonyloxy, (C₇-C₁₂)-aralkylcarbonyloxy,
(C₁-C₈)-alkoxycarbonyloxy, (C₁-C₆)-alkoxy-(C₁-C₆)-alkoxycarbonyloxy, (C₆-C₁₂)-aryloxycarbonyloxy, (C₇-C₁₂)-aralkyloxycarbonyloxy, (C₃-C₈)-cycloalkoxycarbonyloxy,
carbamoyl, N-(C₁-C₈)-alkylcarbamoyl, N,N-di-(C₁-C₈)-alkylcarbamoyl,N-(C₃-C₈)-cycloalkylcarbamoyl,
N-((C₁-C₆)-alkoxy-(C₁-C₆)alkyl)carbamoyl,
amino, (C₁-C₆)-alkylamino, di-(C₁-C₆)alkylamino, (C₃-C₈)-cycloalkylamino, N-(C₆-C₁₂)-arylamino, N-(C₇-C₁₁)-aralkylamino, N-(C₁-C₅)-alkyl-(C₆-C₁₂)-arylamino,
(C₁-C₈)-alkanoylamino, (C₃-C₈)-cycloalkanoylamino, (C₆-C₁₂)-aroylamino, (C₇-C₁₂)-aralkanoylamino, (C₁-C₈)-alkanoyl-N-(C₁-C₆)-alkylamino, (C₃-C₈)-cycloalkanoyl-N-(C₁-C₆)-alkylamino, (C₆-C₁₂)-aroyl-N-(C₁-C₆)alkylamino or (C₇-C₁₁)-aralkanoyl-N-(C₁-C₆)-alkylamino, and
where the radicals which contain an aryl radical are substituted, in particular, by up to 3 substituents from the group
hydroxyl, fluorine, chlorine, cyano, trifluoromethyl, (C₁-C₆)-alkyl, (C₃-C₈)-cycloalkyl, (C₁-C₈)-alkoxy,
(C₁-C₆)-alkylcarbonyl, (C₃-C₈)-cycloalkylcarbonyl,
(C₁-C₆)-alkoxycarbonyl, (C₃-C₈)-cycloalkoxycarbonyl,
(C₁-C₆)-alkylcarbonyloxy, (C₃-C₈)-cycloalkylcarbonyloxy,
(C₁-C₆)-alkoxycarbonyloxy, (C₃-C₈)-cycloalkoxycarbamoyloxy,
carbamoyl, N-(C₁-C₆)-alkylcarbamoyl, N,N-di-(C₁-C₆)-alkylcarbamoyl, N-(C₃-C₈)-cycloalkylcarbamoyl,
N-((C₁-C₆)-alkoxy-(C₁-C₆)alkyl)carbamoyl,
N-(C₁-C₆)-alkyl-N-((C₁-C₆)-alkoxy-(C₁-C₆)alkyl)carbamoyl,
carbamoyloxy, N-(C₁-C₆)-alkylcarbamoyloxy, N,N-di-(C₁-C₆)-alkylcarbamoyloxy, N-(C₃-C₈)-cycloalkylcarbamoyloxy,
(C₁-C₆)-alkanoylamino, (C₃-C₈)-cycloalkanoylamino,
(C₁-C₆)alkylmercapto, (C₁-C₆)-alkylsulfinyl, (C₁-C₆)-alkylsulfonyl, and
R² is hydrogen, (C₁-C₂₀)-alkyl, (C₂-C₂₀)-alkenyl, (C₂-C₂₀)-alkenyloxy, (C₂-C₂₀)-alkynyloxy, retinyloxy, (C₁-C₂₀)-alkoxy-(C₁-C₃)-alkyl, (C₁-C₂₀)-alkoxy-(C₁-C₃)-alkyl, (C₂-C₂₀)-alkenyloxy-(C₁-C₃)-alkyl, retinyloxy-(C₁-C₃)-alkyl, (C₂-C₂₀)-alkynyloxy-(C₁-C₃)-alkyl, (C₁-C₂₀)-alkoxy, halogen, cyano, trifluoromethyl, (C₁-C₁₆)-hydroxyalkyl, (C₁-C₂₀)-alkanoyl, (C₇-C₁₂)-aralkanoyl, (C₆-C₁₂)-aroyl, -O-[CH₂]ₓC_{f}H_{(2f+1-g)}F_{g}, NR' R'', (C₁-C₁₀)-alkylmercapto, (C₁-C₁₀)-alkylsulfinyl, (C₁-C₁₀)-alkylsulfonyl, (C₆-C₁₂)-arylmercapto, (C₆-C₁₂)-arylsulfinyl, (C₆-C₁₂)-arylsulfonyl, (C₇-C₁₂)-aralkylmercapto, (C₇-C₁₂)-aralkylsulfinyl, (C₇-C₁₂)-aralkylsulfonyl, (C₆-C₁₂)-aryloxy, (C₇-C₁₆)-aralkyloxy, carboxyl, (C₁-C₂₀)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₂-C₂₀)-alkenyloxycarbonyl, retinyloxycarbonyl, (C₂-C₂₀)-alkynyloxycarbonyl, (C₃-C₈)-cycloalkyl-(C₁-C₆)-alkoxycarbonyl, (C₃-C₈)-cycloalkoxy-(C₁-C₆)-alkoxycarbonyl, (C₆-C₁₂)-aryloxy-(C₁-C₆)-alkoxycarbonyl, (C₇-C₁₆)-aralkoxy-(C₁-C₆)-alkoxycarbonyl, carbamoyl, N-(C₁-C₁₂)-alkylcarbamoyl, N,N-di-(C₁-C₁₂)-alkylcarbamoyl, N-(C₃-C₈)-cycloalkylcarbamoyl, N,N-dicyclo(C₃-C₈)-alkylcarbamoyl, N-(C₁-C₁₀)-alkyl-N-(C₃-C₈)-cycloalkylcarbamoyl, N-((C₃-C₈)-cycloalkyl-(C₁-C₆)-alkyl)carbamoyl, N-(C₁-C₆)-alkyl-N-((C₃-C₈)-cycloalkyl-(C₁-C₆)-alkyl)carbamoyl, N-(+)-dehydroabietylcarbamoyl, N-(C₁-C₆)-alkyl-N-(+)-dehydroabietylcarbamoyl, N-(C₆-C₁₂)-arylcarbamoyl, N-(C₇-C₁₆)-aralkylcarbamoyl, N-(C₁-C₁₀)-alkyl-N-(C₆-C₁₆)-arylcarbamoyl,N-(C₁-C₁₀)-alkyl-N-(C₇-C₁₂)-aralkylcarbamoyl, N-((C₁-C₁₂)-alkoxy-(C₁-C₁₀)-alkyl)carbamoyl, N-((C₆-C₁₆)-aryloxy-(C₁-C₁₀)-alkyl)carbamoyl,
N-((C₇-C₁₆)-aralkyloxy-(C₁-C₁₀)-alkyl)carbamoyl, N-(C₁-C₁₀)-alkyl-N-((C₁-C₁₀)-alkoxy-(C₁-C₁₀)-alkyl)carbamoyl, N-(C₁-C₁₀)-alkyl-N-((C₆-C₁₂)-aryloxy-(C₁-C₁₀)-alkyl)carbamoyl,N-(C₁-C₁₀)-alkyl-N-((C₇-C₁₆)-aralkyloxy-(C₁-C₁₀)-alkyl)-carbamoyl, or CON(CH₂)ₕ, in which a CH₂ group can be replaced by O, S, N- (C₁-C₈)-alkylimino, N-(C₃-C₈)-cycloalkylimino, N-(C₃-C₈)-cycloalkyl-(C₁-C₄)-alkylimino, N-(C₆-C₁₂)-arylimino, N-(C₇-C₁₆)-aralkylimino or N-(C₁-C₄)-alkoxy-(C₁-C₆)-alkylimino, and h is from 3 to 6,
where aryl is substituted in the manner as defined for R¹ and R³,
R¹ and R³ are identical or different and are hydrogen, halogen, (C₁-C₁₂)-alkyl, (C₁-C₁₂)-alkoxy, -O-[CH₂]ₓ-C_{f}H_{(2f+1-g)}Hal_{g}, (C₁-C₁₂)-alkoxy-(C₁-C₁₂)-alkyl, (C₁-C₈)-alkoxy-(C₁-C₁₂)-alkoxy,(C₁-C₁₂)-alkoxy-(C₁-C₈)-alkoxy-(C₂-C₆)-alkyl, (C₇-C₁₁)-aralkyloxy, (C₃-C₈)-cycloalkyl, (C₃-C₈)-cycloalkyl-(C₁-C₈) alkyl, (C₃-C₈)-cycloalkyloxy, (C₃-C₈)-cycloalkyl-(C₁-C₈)-alkoxy, (C₃-C₈)-cycloalkyloxy-(C₁-C₈)-alkyl, (C₃-C₈)cycloalkyloxy-(C₁-C₈)-alkoxy, (C₃-C₈)-cycloalkyl-(C₁-C₆)-alkyl-(C₁-C₆)-alkoxy, (C₃-C₈)-cycloalkyl-(C₁-C₆)-alkoxy-(C₁-C₆)-alkyl, (C₃-C₈)-cycloalkoxy-(C₁-C₆)-alkoxy-(C₁-C₆)-alkyl, NR^{Y}R^{Z}, (C₁-C₈)-alkylmercapto, (C₁-C₈)-alkylsulfinyl or (C₁-C₈)-alkylsulfonyl, (C₆-C₁₂)-arylmercapto, (C₆-C₁₂)-arylsulfinyl, (C₆-C₁₂)-arylsulfonyl, (C₇-C₁₂)-aralkylmercapto, (C₇-C₁₁)-aralkylsulfinyl, (C₇-C₁₁)-aralkylsulfonyl, substituted (C₆-C₁₂)-aryloxy-(C₁-C₆)-alkyl, (C₇-C₁₁)-aralkoxy-(C₁-C₆)-alkyl, (C₆-C₁₂)-aryloxy-(C₁-C₆)-alkoxy-(C₁-C₆)-alkyl, (C₇-C₁₁)-aralkyloxy-(C₁-C₆)-alkoxy-(C₁-C₆)-alkyl, (C₆-C₁₂)-aryloxy, (C₇-C₁₁)-aralkyloxy,(C₆-C₁₂)-aryloxy-(C₁-C₆)-alkoxy or (C₇-C₁₁)-aralkoxy-(C₁-C₆)-alkoxy, where an aromatic radical carries 1, 2, 3, 4 or 5 identical or different substituents from the group hydrogen, halogen, cyano, nitro, trifluoromethyl, (C₁-C₁₂)-alkyl, (C₁-C₁₂)-alkenyl, (C₁-C₆)-hydroxyalkyl, (C₁-C₁₂)-alkoxy, (C₁-C₁₂)-alkenyloxy, -O-[CH₂]ₓC_{f}H_{(2f+1-g)}F_{g}, -OCF₂Cl, -O-CF₂-CHFCl, (C₁-C₆)-alkylmercapto, (C₁-C₆)-alkylsulfinyl, (C₁-C₆)-alkylsulfonyl, (C₁-C₆)-alkylcarbonyl, (C₁-C₆)-alkoxycarbonyl, carbamoyl, N-(C₁-C₄)-alkylcarbamoyl, N,N-di-(C₁-C₄)-alkylcarbamoyl, (C₁-C₆)-alkylcarbonyloxy, (C₃-C₈)-cycloalkylcarbamoyl, phenyl, benzyl, phenoxy, benzyloxy, NR^{Y}R^{Z}, phenylmercapto, phenylsulfonyl, phenylsulfinyl, sulfamoyl, N-(C₁-C₄)-alkylsulfamoyl or N,N-di-(C₁-C₄)-alkylsulfamoyl, or optionally carries up to 3 of the abovementioned identical or different substituents, and two adjacent carbon atoms of the aralkyloxy radical together carry a chain -[CH₂-] and/or -CH=CH-CH=CH-, where a CH₂ group of the chain is optionally replaced by O, S, SO, SO₂ or NR^{Y},
R¹ and R² or R² and R³ can form a chain [CH₂]ₒ, where o is 3, 4 or 5, and
R⁴ is, provided Q is a bond, chlorine or, if Q is O, is a branched or unbranched (C₁-C₁₀)-alkyl radical, which can contain one or two C-C multiple bonds, or an unsubstituted fluoroalkyl radical of the formula -[CH₂]ₓC_{f}H_{(2f+1-g)}F_{g} or (C₁-C₈)-alkoxy-(C₁-C₆)-alkyl, (C₁-C₆)-alkoxy-(C₁-C₄)-alkoxy-(C₁-C₄)-alkyl or a radical of the formula Z,
-[CH₂]ᵥ-[O]_{w}-[CH₂]ₜ-E (Z),
where E is a substituted phenyl radical of the formula F 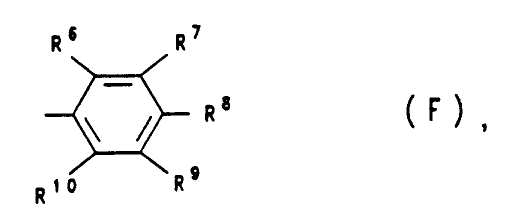 or a (C₃-C₈)-cycloalkyl radical, where
v is 0, 1, 2, 3, 4, 5 or 6, w is 0 or 1, and t is 0, 1, 2 or 3, with the restriction that v is not equal to 0 if w is 1, and R⁶, R⁷, R⁸, R⁹ and R¹⁰ are identical or different and are hydrogen, halogen, cyano, nitro, trifluoromethyl, (C₁-C₆)-alkyl, (C₃-C₈)-cycloalkyl, (C₁-C₆)-alkoxy, -O-[CH₂₋]ₓC_{f}H_{(2f+1-g)}F_{g}, -OCF₂Cl, -O-CF₂-CHFCl, (C₁-C₆)-alkylmercapto, (C₁-C₆)-hydroxyalkyl, (C₁-C₆)-alkoxy-(C₁-C₆)-alkoxy, (C₁-C₆)-alkoxy-(C₁-C₆)-alkyl, (C₁-C₆)-alkylsulfinyl, (C₁-C₆)-alkylsulfonyl, (C₁-C₆)-alkylcarbonyl, (C₁-C₈)-alkoxycarbonyl, carbamoyl, N-(C₁-C₈)-alkylcarbamoyl, N,N-di-(C₁-C₈)-alkylcarbamoyl, (C₇-C₁₁)-aralkylcarbamoyl which is optionally substituted by fluorine, chlorine, bromine, trifluoromethyl or (C₁-C₆)-alkoxy, N-(C₃-C₈)-cycloalkylcarbamoyl, N-(C₃-C₈)-cycloalkyl-(C₁-C₄)-alkylcarbamoyl, (C₁-C₆)-alkylcarbonyloxy, phenyl, benzyl, phenoxy, benzyloxy, NR^{Y}R^{Z}, such as amino, anilino, N-methylanilino, phenylmercapto, phenylsulfonyl, phenylsulfinyl, sulfamoyl, N-(C₁-C₈)-alkylsulfamoyl or N,N-di-(C₁-C₈)-alkylsulfamoyl, or two adjacent substituents together are a chain -[CH₂₋]ₙ or -CH=CH-CH=CH-, where a CH₂ group of the chain is optionally replaced by O, S, SO, SO₂ or NR^{Y}, and where a heteroaryl radical can carry 1, 2 or 3 substituents, and a cycloalkyl radical one substituent, from the above group, and
R⁴ is R'', provided Q has the meaning of NR', where
R' is hydrogen or methyl, and
R'' is benzyl, and
provided R¹ and/or R³ have the meaning of (C₆-C₁₂)-aryloxy, (C₇-C₁₁)-aralkyloxy, (C₆-C₁₂)-aryloxy-(C₁-C₆)-alkoxy, (C₇-C₁₁)-aralkoxy-(C₁-C₆)-alkoxy or a corresponding radical containing terminal cycloalkyl groups, this radical is preferably a radical of the formula D
OZ (D), or
provided R¹ and/or R³ have the meaning of (C₇-C₁₁)-aralkyl, (C₆-C₁₂)-aryloxy-(C₁-C₆)-alkyl, (C₇-C₁₁)-aralkoxy-(C₁-C₆)-alkyl or a corresponding radical containing terminal cycloalkyl groups, this radical is preferably a radical of the formula Z,
R^{Y} and R^{Z} are identical or different and are hydrogen, (C₆-C₁₂)-aryl, (C₁-C₁₀)-alkyl, (C₃-C₁₀)-cycloalkyl, (C₁-C₈)-alkoxy-(C₁-C₈)-alkyl, (C₇-C₁₂)-aralkoxy-(C₁-C₈)-alkyl,(C₆-C₁₂)-aryloxy-(C₁-C₈)-alkyl, (C₁-C₁₀)-alkanoyl, optionally substituted (C₇-C₁₆)-aralkanoyl or optionally substituted (C₆-C₁₂)-aroyl, or
R^{Y} and R^{Z} are together -[CH₂]ₕ₋, in which a CH₂ group can be replaced by O, S, N-(C₁-C₄)-alkanoylimino or N-(C₁-C₄)-alkoxycarbonylimino, and
f is 1 to 8,
g is 0 or 1 to (2f+1),
h is 3 to 6,
x is 0 to 3, and
n is 3 or 4,
including the physiologically active salts, where 3-benzyloxypyridine-2-carboxylic acid (L-threonyl methyl ester) amide, 3-benzyloxypyridine-2-carboxylic acid (L-threonyl(Fmoc-Phg) tert-butyl ester) amide, 3-benzyloxypyridine-2-carboxylic acid (L-threonyl tert-butyl ester) amide, 3-benzyloxypyridine-2-carboxylic acid (D-allothreonyl methyl ester) amide and 3-benzyloxypicolinylglycine ethyl ester are excepted.

8. A compound of the formula I as claimed in claims 1 to 3, 6 and 7, in which
Q is O,
X is O,
Y is CR³ and, additionally, N if R¹ and R² form a cycle,
m is 0,
A is -CHR⁵-, where R⁵ is the substituent of the α-carbon atom of an α-amino acid, in particular of a natural L-amino acid or its D-isomer,
B is -CO₂G, where
G is a branched or unbranched, or cyclic, aliphatic (C₁-C₁₈)-alkyl radical, a (C₃-C₈)-cycloalkyl-(C₁-C₈)-alkyl radical,
a branched or unbranched (C₂-C₁₈)-alkenyl radical, such as, for example, a geranyl or farnesyl radical, or a retinyl radical, or (C₂-C₁₈)-alkynyl radical, a phenyl radical, benzyl radical, phenethyl radical, phenylpropyl radical or phenylbutyl radical,
where the above radicals contain a substituent from the group hydroxyl, (C₁-C₄)-alkoxy, acyloxy, (C₁-C₆)-alkylcarbonyloxy, (C₃-C₈)-cycloalkylcarbonyloxy, benzoyloxy, (C₇-C₁₆)-phenylalkylcarbonyloxy or (C₃-C₈)-cycloalkoxycarbonyloxy,
R² is hydrogen, bromine, chlorine, cyano, (C₁-C₁₈)-alkyl, (C₁-C₈)-alkoxy, (C₁-C₁₈)-alkoxymethyl, (C₂-C₁₈)-alkenyloxymethyl, (C₂-C₁₈)-alkynyloxymeth yl, carbamoyl, N-(C₁-C₁₀)-alkylcarbamoyl, N-((C₁-C₁₂)-alkoxy-(C₁-C₄)-alkyl)carbamoyl, N,N-di-(C₁-C₈)-alkylcarbamoyl, N-(C₃-C₈)-cycloalkylcarbamoyl, N-(C₆-C₁₂)-phenylcarbamoyl, N-(C₇-C₁₂)-phenylalkylcarbamoyl, N-(C₁-C₆)-alkyl-N-(C₆-C₁₂)phenylcarbamoyl,N-(C₁-C₆)-alkyl-N-(C₇-C₁₂)-phenylalkylcarbamoyl, N-((C₁-C₆)-alkoxy-(C₁-C₆)-alkyl)carbamoyl, carboxyl, (C₁-C₂₀)-alkoxycarbonyl, (C₂-C₂₀)-alkenyloxycarbonyl, retinyloxycarbonyl, (C₃-C₈)-cycloalkoxycarbonyl, (C₃-C₈)-cycloalkyl-(C₁-C₆)-alkoxycarbonyl, (C₃-C₈)-cycloalkoxy-(C₁-C₆)-alkoxycarbonyl, phenyl-(C₁-C₆)-alkoxycarbonyl, phenoxy-(C₁-C₆)-alkoxycarbonyl or benzyloxy-(C₁-C₆)-alkoxycarbonyl, where a phenyl radical is substituted in the manner as defined for R¹ and R³, and one of the radicals
R¹ or R³ is hydrogen and the other a radical from the group hydrogen, fluorine, chlorine, (C₁-C₈)-alkyl, (C₁-C₁₀)-alkoxy, (C₅-C₆)-cycloalkyl, (C₅-C₆)-cycloalkyl-(C₁-C₆)-alkyl, (C₅-C₆)-cycloalkyloxy, (C₅-C₆)-cycloalkyl-(C₁-C₆)-alkoxy, (C₅-C₆)-cycloalkyloxy-(C₁-C₆)-alkyl, (C₅-C₆)-cycloalkyloxy (C₁-C₆)-alkoxy, (C₅-C₆)-cycloalkyl-(C₁-C₄)-alkyl-(C₁-C₄)-alkoxy, (C₅-C₆)-cycloalkyl-(C₁-C₄)-alkoxy-(C₁-C₂)-alkyl, (C₅-C₆)-cycloalkoxy-(C₁-C₄)-alkoxy-(C₁-C₂)-alkyl, -O-[CH₂]ₓC_{f}H_{(2f+1-g)}F_{g}, (C₁-C₆)-alkoxy-(C₁-C₆)-alkyl, (C₁-C₆)-alkoxy-(C₁-C₆)-alkoxy, (C₁-C₆)-alkoxy-(C₁-C₄)-alkoxy-(C₁-C₂)-alkyl, substituted (C₆-C₁₂)-phenoxy, (C₇-C₁₁)-phenylalkyloxy, (C₆-C₁₂)-phenoxy-(C₁-C₆)-alkoxy or (C₇-C₁₁)-phenylalkoxy-(C₁-C₆)-alkoxy, phenoxy-(C₁-C₄)-alkyl, (C₇-C₁₁)-phenylalkyloxy-(C₁-C₄)-alkyl,phenoxy-(C₁-C₄)-alkoxy-(C₁-C₂)-alkyl or (C₇-C₁₁)-phenylalkyloxy-(C₁-C₄)-alkoxy-(C₁-C₂)-alkyl, where an aromatic radical is substituted by 1, 2 or 3 identical or different substituents from the group fluorine, chlorine, cyano, trifluoromethyl, (C₁-C₁₂)-alkyl, (C₂-C₁₂)-alkenyl, (C₂-C₁₂)-alkenyloxy or (C₁-C₁₂)-alkoxy,
R¹ and R², with the pyridine carrying them, form a 5,6,7,8-tetrahydroisoquinoline ring,
R⁴ is a branched or unbranched (C₁-C₁₀)-alkyl radical, (C₁-C₄)-alkoxy-(C₁-C₄)-alkyl or a radical of the formula Z,
-[CH₂]ᵥ-[O]_{w}-[CH₂]ₜ-E (Z),
where E is a substituted phenyl radical of the formula F 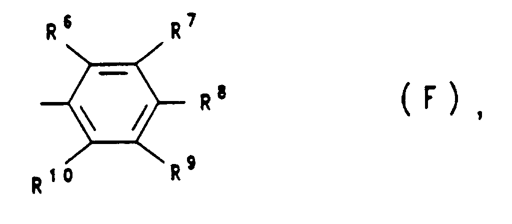 or a (C₃-C₈)-cycloalkyl radical, where
v is 0, 1, 2 or 3, w is 0, and t can be 0 or 1, and in which R⁶, R⁷, R⁸, R⁹ and R¹⁰ are identical or different and are hydrogen, fluorine, chlorine, cyano, trifluoromethyl, (C₁-C₆)-alkyl, (C₁-C₆)-alkoxy, -O-[CH₂]ₓC_{f}H_{(2f+1-g)}F_{g}, N-(C₁-C₈)-alkylcarbamoyl, N,N-di-(C₁-C₆)-alkylcarbamoyl, N-(C₃-C₆)-cycloalkylcarbamoyl, N-(+)-dehydroabietylaminocarbonyl, or (C₇-C₁₁)-phenylalkylcarbamoyl, which is optionally substituted by fluorine, chlorine, trifluoromethyl or (C₁-C₆)-alkoxy, or where R⁶ and R⁷ or R⁷ and R⁸, together with the phenyl ring carrying them, form naphthalene derivatives, or
if R¹ or R³ has the meaning of (C₆-C₁₂)-phenoxy, (C₇-C₁₁)-phenylalkyloxy, (C₆-C₁₂)-phenoxy-(C₁-C₆)-alkoxy, (C₇-C₁₁)-phenylalkoxy-(C₁-C₆)-alkoxy, (C₅-C₆)-cycloalkyloxy, (C₅-C₆)-cycloalkyl-(C₁-C₆)-alkoxy, (C₅-C₆)-cycloalkoxy-(C₁-C₆)-alkoxy or (C₅-C₆)-cycloalkyl-(C₁-C₄)-alkyl-(C₁-C₄)-alkoxy, this radical is then, especially, a radical of the formula D
OZ (D), or
if R¹ or R³ has the meaning of phenyl, phenoxy-(C₁-C₆)-alkyl, (C₇-C₁₁)-phenylalkyl, (C₇-C₁₁)-phenylalkyloxy-(C₁-C₄)-alkyl, (C₅-C₆)-cycloalkyl, (C₅-C₆)-cycloalkyl-(C₁-C₆)-alkyl, (C₅-C₆)-cycloalkoxy-(C₁-C₄)-alkyl, (C₅-C₆)-cycloalkyl-(C₁-C₄)-alkoxy-(C₁-C₂)-alkyl or (C₅-C₆)-cycloalkoxy-(C₁-C₄)-alkoxy-(C₁-C₂)-alkyl, this radical is then, especially, a radical of the formula Z in which, in both cases,
v is 1, 2, 3 or 4, w is 0 and t is 0, or
v is 1, 2, 3 or 4, w is 1 and t is 0, or
v is 1, 2, 3 or 4, w is 1, t is 1, and
f is 1 to 4,
g is 0 or 1 to (2f+1),
x is 0 or 1,
including the physiologically active salts, where 3-benzyloxypyridine-2-carboxylic acid (L-threonyl methyl ester) amide, 3-bezyloxypyridine-2-carboxylic acid (L-threonyl(Fmoc-Phg) tert-butyl ester) amide, 3-benzyloxypyridine-2-carboxylic acid (L-threonyl tert-butyl ester) amide, 3-benzyloxypyridine-2-carboxylic acid (D-allothreonyl methyl ester) amide and 3-benzyloxypicolinylglycine ethyl ester are excepted.

9. A compound of the formula I as claimed in claims 1 to 3, 6, 7 and 8, in which
Q is O,
X is O,
Y is CR³,
m is 0,
A is a -CH₂- group which can be substituted by a methyl group,
B is -CO₂G, where
G is a branched or unbranched, or cyclic, aliphatic (C₁-C₁₈)-alkyl radical, a (C₃-C₈)-cycloalkyl-(C₁-C₄)-alkyl radical or a branched or unbranched (C₂-C₁₈)-alkenyl radical, where the above radicals can contain a substituent from the group hydroxyl, (C₁-C₄)-alkoxy, acyloxy, (C₁-C₆)-alkylcarbonyloxy, (C₃-C₈)-cycloalkylcarbonyloxy, benzoyloxy, (C₇-C₁₆)-phenylalkylcarbonyloxy or (C₃-C₈)-cycloalkoxycarbonyloxy, or
G is a phenyl radical, benzyl radical, phenethyl radical, phenylpropyl radical or phenylbutyl radical,
R² ishydrogen, (C₁-C₈)-alkoxy, (C₁-C₁₆)-alkoxymethyl, (C₂-C₁₆)-alkenyloxymethyl, retinyloxymethyl, N-(C₁-C₁₀)-alkylcarbamoyl,N-((C₁-C₁₂)-alkoxy-(C₁-C₃)-alkyl)carbamoyl, N,N-di-(C₁-C₈)-alkylcarbamoyl, N-(C₅-C₆)-cycloalkylcarbamoyl, N-phenylcarbamoyl, N-phenyl-(C₁-C₄)-alkylcarbamoyl, carboxyl, (C₁-C₁₆)-alkoxycarbonyl, (C₂-C₁₆)-alkenyloxycarbonyl, retinyloxy-carbonyl, (C₅-C₆)-cycloalkoxycarbonyl, (C₅-C₆)-cycloalkyl-(C₁-C₆)-alkoxycarbonyl or phenyl-(C₁-C₆)-alkoxycarbonyl, where a phenyl radical is substituted in the manner as defined for R¹ and R³, and one of the radicals
R¹ or R³ is hydrogen and the other radical is a radical from the group hydrogen, (C₁-C₁₀)-alkoxy, (C₅-C₆)-cycloalkyloxy, (C₅-C₆)-cycloalkyl-(C₁-C₂)-alkoxy, -O-[CH₂]ₓ-C_{f}H_{(2f+1-g)}F_{g}, (C₁-C₄)-alkoxy-(C₁-C₄)-alkoxy, substituted (C₆-C₁₂)-phenoxy, (C₇-C₁₁)-phenylalkyloxy, (C₆-C₁₂)-phenoxy-(C₁-C₄)-alkoxy or (C₇-C₁₁)-phenylalkoxy-(C₁-C₄)-alkoxy, where an aromatic radical is substituted by 1, 2 or 3 identical or different substituents from the group fluorine, chlorine, cyano, trifluoromethyl, (C₁-C₁₀)-alkyl, (C₁-C₁₀)-alkoxy or (C₁-C₁₀)-alkenyloxy, and
R⁴ is a branched or unbranched (C₁-C₈)-alkyl radical or a radical of the formula Z,
-[CH₂]ᵥ-[O]_{w}-[CH₂]ₜ-E (Z),
where E is a substituted phenyl radical of the formula F 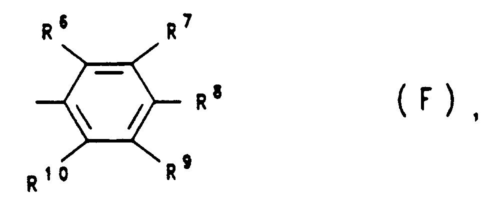 or a (C₃-C₈)-cycloalkyl radical, where
v is 0, 1, 2 or 3, w is 0, and t can be 0 or 1, and in which R⁶, R⁷, R⁸, R⁹ and R¹⁰ are identical or different and are hydrogen, fluorine, chlorine, cyano, trifluoromethyl, (C₁-C₆)-alkyl, (C₁-C₆)-alkoxy, -O-[CH₂₋]ₓC_{f}H_{(2f+1-g)}F_{g}, N-(C₁-C₈)-alkylcarbamoyl, N,N-di-(C₁-C₆)-alkylcarbamoyl, N-(C₃-C₆)-cycloalkylcarbamoyl, N-(+)-dehydroabietylaminocarbonyl substituted benzyl radical, and f is 1 to 4, g is 0 or 1 to (2f+1) and x is 0 or 1, including the physiologically active salts, where 3-benzyloxypicolinylglycine ethyl ester is expected.

10. A compound of the formula I as claimed in claims 1 to 3 and 6 to 9, in which
Q is O,
X is O,
Y is CR³,
m is 0,
A is a -CH₂- group,
B is -CO₂G, where
G is a branched or unbranched aliphatic (C₁-C₁₆)-alkyl radical, a 2-cyclohexylethyl radical, a (C₁-C₄)-alkoxy-(C₁-C₂)-alkyl radical, a branched or unbranched (C₂-C₁₀)-alkenyl radical, a phenyl radical, benzyl radical, phenethyl radical, phenylpropyl radical or phenylbutyl radical,
R¹ is hydrogen, (C₁-C₆)-alkoxy or -O-[CH₂]ₓC_{f}H_{(2f+1-g)}F_{g},
R² is hydrogen, N-(C₁-C₁₀)-alkylcarbamoyl, N-((C₁-C₁₂)-alkoxy-(C₁-C₃)-alkyl) carbamoyl, N,N-di-(C₁-C₈)-alkylcarbamoyl, N-(C₅-C₆)-cycloalkylcarbamoyl, N-phenylcarbamoyl, N-phenyl-(C₁-C₂)-alkylcarbamoyl, carboxyl, (C₁-C₁₆)-alkoxycarbonyl, (C₂-C₁₆)-alkenyloxycarbonyl, retinyloxycarbonyl, (C₅-C₆)-cycloalkoxycarbonyl, (C₅-C₆)-cycloalkyl-(C₁-C₆)-alkoxycarbonyl or phenyl-(C₁-C₆)-alkoxycarbonyl, where a phenyl radical is substituted by 1 or 2 identical or different substituents from the group fluorine, chlorine, cyano, trifluoromethyl, (C₁-C₁₀)-alkyl, (C₁-C₁₀)-alkoxy or (C₁-C₁₀)-alkenyloxy, and
R³ is hydrogen, (C₁-C₅)-alkoxy or (C₅-C₆)-cycloalkyl-(C₁-C₂)-alkoxy, where one of the substituents R¹ and R³ is hydrogen,
R⁴ is a branched or unbranched (C₁-C₆)-alkyl radical, or a 2-phenylethyl radical, or a benzyl radical substituted by 1 or 2 radicals from the group fluorine, chlorine, cyano, trifluoromethyl, (C₁-C₆)-alkyl, (C₁-C₆)-alkoxy, -O-[CH₂₋]ₓC_{f}H_{(2f+1-g)}F_{g}, N-(C₁-C₈)-alkylcarbamoyl, N,N-di-(C₁-C₆)-alkylcarbamoyl, N-(C₃-C₆)-cycloalkylcarbamoyl or N-(+)-dehydroabietylaminocarbonyl, and f is 1 to 4, g is 0 or 1 to (2f+1) and x is 1, including the physiologically active salts.

11. A compound of the formula I as claimed in claims 1 to 3 and 6 to 10, in which
Q is O,
X is O,
Y is CR³,
m is 0,
A is a -CH₂- group,
B is -CO₂G, where
G is a branched or unbranched aliphatic (C₁-C₁₆)-alkyl radical or a benzyl radical,
R¹ is hydrogen,
R² is hydrogen, N-(C₁-C₁₀)-alkylcarbamoyl, N-((C₁-C₁₂)-alkoxy-(C₁-C₃)-alkyl)carbamoyl, N-cyclohexylcarbamoyl, N-phenylcarbamoyl, N-(phenyl-(C₁-C₂)-alkyl)carbamoyl, where, in the last two cases, the phenyl radical can carry a fluorine substituent, a (C₁-C₁₀)-alkyl substituent or a (C₁-C₁₀)-alkoxy substituent, carboxyl, (C₁-C₁₆)-alkoxycarbonyl, (C₂-C₁₆)-alkenyloxycarbonyl, retinyloxycarbonyl, (C₅-C₆)-cycloalkoxycarbonyl or benzyloxycarbonyl,
R³ is hydrogen, (C₁-C₅)-alkoxy or 2-(cyclohexyl)-ethyloxy, where one of the substituents R² and R³ is hydrogen,
R⁴ is a branched or unbranched (C₁-C₄)-alkyl radical or a benzyl radical which is substituted once by fluorine, chlorine, trifluoromethyl, (C₁-C₄)-alkyl or (C₁-C₃)-alkoxy, including the physiologically active salts.

12. A compound of the formula I as claimed in claims 1 to 3 and 6, in which
Q is O,
X is O,
Y is CR³,
where R³ is hydrogen,
m is 0,
A is a -CH₂- group,
B is -CO₂G, where
G is a branched or unbranched aliphatic (C₁-C₁₆)-alkyl radical or a benzyl radical,
R¹ and R², together with the pyridine carrying them, form an isoquinoline ring with an unsubstituted benzo moiety, and
R⁴ is methyl.

13. A compound of the formula I as claimed in claims 1 to 3 and 6, in which
Q is O,
X is O,
Y is CR³,
m is 0,
A is a -CH₂- group,
B is -CO₂G, where
G is a branched or unbranched aliphatic (C₁-C₁₆)-alkyl radical or a benzyl radical,
R¹ is hydrogen, and
R² and R³, together with the pyridine carrying them, form a quinoline ring with an unsubstituted benzo moiety, and
R⁴ is methyl.

14. A compound of the formula I as claimed in claims 1, 2, 4 and 5, in which
Q is S,
X is O,
Y is CR³,
m 0,
A is a -CH₂- group,
B is -CO₂G, where
G is a branched or unbranched aliphatic (C₁-C₁₆)-alkyl radical or a benzyl radical,
R¹ is hydrogen,
R² is hydrogen, N-(C₁-C₁₀)-alkylcarbamoyl, N-((C₁-C₁₂)-alkoxy-(C₁-C₃)-alkyl)carbamoyl, N-cyclohexylcarbamoyl, N-phenylcarbamoyl, N-(phenyl-(C₁-C₂)alkyl)carbamoyl, where, in the last two cases, the phenyl radical can carry a fluorine substituent, (C₁-C₁₀)-alkyl substituent or (C₁-C₁₀)-alkoxy substituent, carboxyl, (C₁-C₁₆)-alkoxycarbonyl, (C₂-C₁₆)-alkenyloxycarbonyl, retinyloxycarbonyl, (C₅-C₆)-cycloalkoxycarbonyl or benzyloxycarbonyl,
R³ is hydrogen, (C₁-C₅)-alkoxy or 2-(cyclohexyl)-ethyloxy, where one of the substituents R² and R³ is hydrogen,
R⁴ is a branched or unbranched (C₁-C₄)-alkyl radical or a benzyl radical which is substituted once by fluorine, chlorine, trifluoromethyl, (C₁-C₄)-alkyl or (C₁-C₃)-alkoxy.

15. A compound of the formula I as claimed in claims 1, 2, 4, 5 and 14, in which
Q is S,
X is O,
Y is CR³,
m is 0,
A is a -CH₂- group,
B is -CO₂G, where
G is a branched or unbranched aliphatic (C₁-C₁₆)-alkyl radical or a benzyl radical,
R¹ is hydrogen,
R² is carboxyl or (C₁-C₁₆)-alkoxycarbonyl,
R³ is hydrogen, and
R⁴ is a branched or unbranched (C₁-C₄)-alkyl radical.

16. A compound of the formula I as claimed in claims 1 to 15, together with 3-benzyloxypyridine-2-carboxylic acid (L-threonyl methyl ester) amide, 3-benzyloxypyridine-2-carboxylic acid (L-threonyl-(Fmoc-Phg) tert-butyl ester) amide, 3-benzyloxypyridine-2-carboxylic acid (L-threonyl tert-butyl ester) amide, 3-benzyloxypyridine-2-carboxylic acid (D-allothreonyl methyl ester) amide and 3-benzyloxypicolinylglycine ethyl ester for use as a pharmaceutical.

17. A compound as claimed in claims 1 to 16 to be used for inhibiting collagen biosynthesis.

18. A compound as claimed in claims 1 to 16 as an ester prodrug of the prolyl hydroxylase inhibitors of the formula I in which B is carboxyl or its salt.

19. A compound as claimed in claims 1 to 16 for use as a fibrosuppressive agent.

20. A compound as claimed in claims 1 to 16 for the preparation of a pharmaceutical against fibrotic diseases.

21. A compound as claimed in claims 1 to 16 for the preparation of a pharmaceutical against fibrotic diseases of the liver.

22. A compound as claimed in claims 1 to 16 for the preparation of a pharmaceutical against fibrotic diseases of the lung.

23. A compound as claimed in claims 1 to 16 for the preparation of a pharmaceutical against fibrotic diseases of the skin.

24. A process for the preparation of compounds according to formula I as claimed in claims 1 to 15, in which
A is a substituted alkylene moiety,
B is -CO₂G,
Y is CR³, and
m is 0 or 1,
by
i1.) reacting pyridine-2-carboxylic acids of the formula II (R²³ = H) with the amino esters of the formula III to form the amide eaters of the formula I, or
i2.) reacting pyridine-2-carboxylic eaters of the formula II (R²³ = lower alkyl), under the conditions of aminolysis, to form the compounds of the formula I; 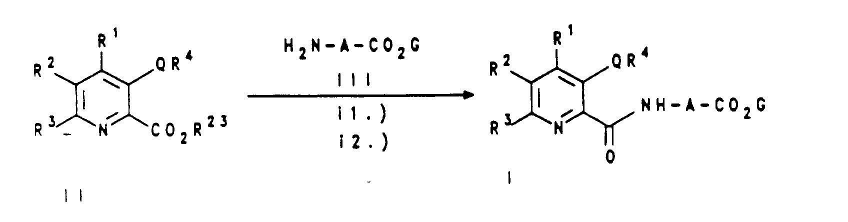
ii) esterifying the compounds of the formula IV with the alcohol GOH to form compounds of the formula I; 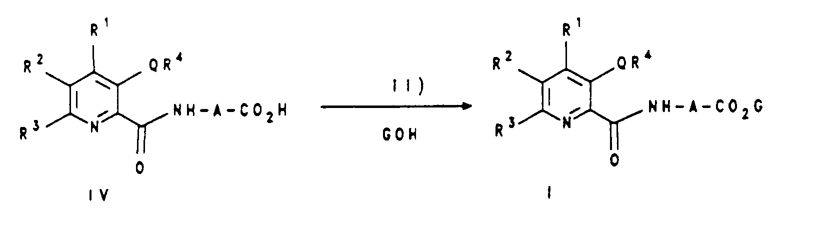 or
iii) alkylating the compounds of the formula V with R⁴X to form compounds of the formula I; 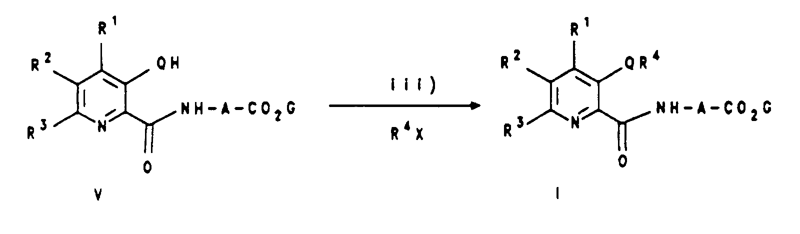 where X is a leaving group, in particular halogen, OSO₂Me or OSO₂ phenyl, and, where appropriate,
iv) converting the compounds of the formula I, provided Q is O or NR', into their pyridine N-oxides (I').

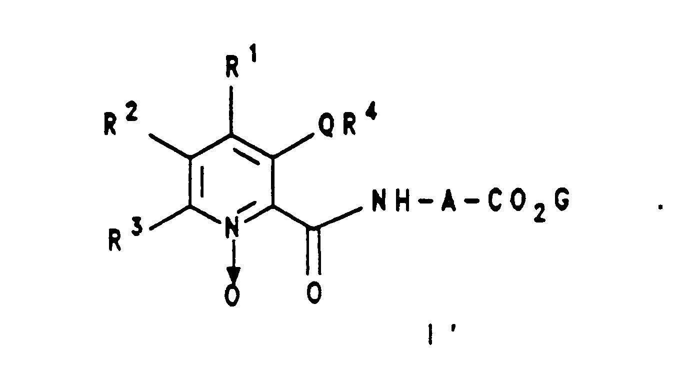

## Revendications

1. Composés de formule générale I 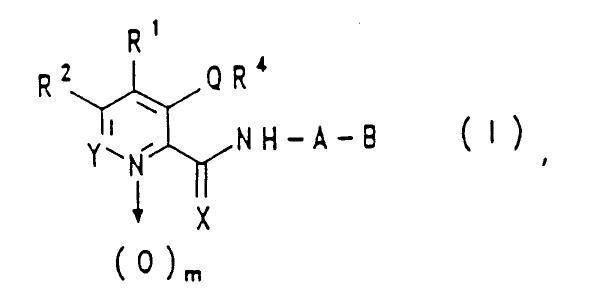 dans laquelle
Q représente O, S, NR' ou une liaison,
X représente O ou S,
Y représente C-R³ ou,
lorsque R¹ et R² forment un cycle,
Y représente N ou CR³,
m est égal à 0 ou 1,
A représente un radical alkylène en C₁-C₄, qui est éventuellement substitué par un ou deux substituants choisis parmi un ou des atomes d'halogène ou groupes cyano, nitro, trifluorométhyle, alkyle en C₁-C₆, hydroxyalkyle en C₁-C₆, alcoxy en C₁-C₆, -O-[CH₂]ₓ-C_{f}H_{(2f+1-g)}Hal_{g}, de préférence fluoroalcoxy en C₁-C₈, fluoroalcényloxy en C₁-C₈, fluoroalcynyloxy en C₁-C₈, -OCF₂Cl ou -O-CF₂-CHFCl, alkyl(C₁-C₆)mercapto, alkyl(C₁-C₆)-sulfinyle, alkyl(C₁-C₆)sulfonyle, alkyl(C₁-C₆)carbonyle, alcoxy(C₁-C₆)carbonyle, carbamoyle, N-alkyl(C₁-C₄)-carbamoyle, N,N-dialkyl(C₁-C₄)carbamoyle, alkyl(C₁-C₆)-carbonyloxy, cycloalkyle en C₃-C₈, phényle, benzyle, phénoxy, benzyloxy, anilino, N-méthylanilino, phénylmercapto, phénylsulfonyle, phénylsulfinyle, sulfamoyle, N-alkyl(C₁-C₄)sulfamoyle, N,N-dialkyl(C₁-C₄)sulfamoyle, ou par un radical aryloxy en C₆-C₁₂, aralkyloxy en C₇-C₁₁, aryle en C₆-C₁₂ ou aralkyle en C₇-C₁₁ substitué, qui porte sur le fragment aryle 1, 2, 3, 4 ou 5 substituants, identiques ou différents, choisis parmi des atomes d'halogène et des groupes cyano, nitro, trifluorométhyle, alkyle en C₁-C₆, alcoxy en C₁-C₆, -O-[CH₂]ₓ-C_{f}H_{(2f+1-g)}F_{g},Hal_{g}, -OCF₂Cl, -O-CF₂-CHFCl, alkyl(C₁-C₆)mercapto, alkyl(C₁-C₆)sulfinyle, alkyl(C₁-C₆)-sulfonyle, alkyl(C₁-C₆)carbonyle, alcoxy(C₁-C₆)-carbonyle, carbamoyle, N-alkyl(C₁-C₄)carbamoyle, N,N-dialkyl(C₁-C₄)carbamoyle, alkyl(C₁-C₆)carbonyloxy, cycloalkyle en C₃-C₈, sulfamoyle, N-alkyl(C₁-C₄)-sulfamoyle ou N,N-dialkyl(C₁-C₄)sulfamoyle, ou par les substituants R⁵ de l'atome de carbone α d'un α-aminoacide, les L-aminoacides naturels et leurs isomères D pouvant être utilisés;
B représente un groupe -CO₂G, G représentant le reste d'un alcool GOH,
R¹, R² et R³ sont identiques ou différents et représentent un atome d'hydrogène ou d'halogène ou un groupe hydroxy, cyano, trifluorométhyle, nitro, carboxy, alkyle en C₁-C₂₀, cycloalkyle en C₃-C₈, cycloalkyl(C₃-C₈)-alkyle(C₁-C₁₂), cycloalcoxy en C₃-C₈, cycloalkyl(C₃-C₈)-alcoxy(C₁-C₁₂), cycloalkyloxy(C₃-C₈)-alkyle(C₁-C₁₂), cycloalkyloxy(C₃-C₈)-alcoxy(C₁-C₁₂), cycloalkyl(C₃-C₈)-alkyl(C₁-C₈)-alcoxy(C₁-C₆), Cycloalkyl(C₃-C₈)-alcoxy(C₁-C₈)-alkyle(C₁-C₆), cycloalkyloxy(C₃-C₈)-alcoxy(C₁-C₈)-alkyle(C₁-C₆), cycloalcoxy(C₃-C₈)-alcoxy(C₁-C₈)-alcoxy(C₁-C₈), aryle en C₆-C₁₂, aralkyle en C₇-C₁₆, aralcényle en C₇-C₁₆, aralcynyle en C₇-C₁₆, alcényle en C₂-C₂₀, alcynyle en C₂-C₂₀, alcoxy en C₁-C₂₀, alcényloxy en C₂-C₂₀, alcynyloxy en C₂-C₂₀, rétinyloxy, alcoxy(C₁-C₂₀)-alkyle(C₁-C₁₂), alcoxy(C₁-C₁₂)-alcoxy(C₁-C₁₂), alcoxy(C₁-C₁₂)-alcoxy(C₁-C₈)-alkyle(C₁-C₈), aryloxy en C₆-C₁₂, aralkyloxy en C₇-C₁₆, aryloxy(C₆-C₁₂)-alcoxy(C₁-C₆), aralcoxy(C₇-C₁₆)-alcoxy(C₁-C₆), hydroxyalkyle en C₁-C₁₆, aryloxy(C₆-C₁₆)-alkyle(C₁-C₈), aralcoxy(C₇-C₁₆)-alkyle(C₁-C₈), aryloxy(C₆-C₁₂)-alcoxy(C₁-C₈)-alkyle(C₁-C₆), aralkyloxy(C₇-C₁₂)-alcoxy(C₁-C₈)-alkyle(C₁-C₆), alcényloxy(C₂-C₂₀)-alkyle(C₁-C₆), alcynyloxy(C₂-C₂₀)-alkyle(C₁-C₆), rétinyloxy-alkyle(C₁-C₆), -O-[CH₂]ₓC_{f}H_{(2f+1-g)}F_{g}, -OCF₂Cl, -OCF₂-CHFCl,
un groupe alkyl(C₁-C₂₀)-carbonyle, cycloalkyl(C₃-C₈)-carbonyle, aryl(C₆-C₁₂)-carbonyle, aralkyl(C₇-C₁₆)-carbonyle, cinnamoyle, alcényl(C₂-C₂₀)-carbonyle, alcynyl(C₂-C₂₀)-carbonyle,
un groupe alcoxy(C₁-C₂₀)-carbonyle, alcoxy(C₁-C₁₂)-alcoxy(C₁-C₁₂)-carbonyle, aryloxy(C₆-C₁₂)-carbonyle, aralcoxy(C₇-C₁₆)-carbonyle, cycloalcoxy(C₃-C₈)-carbonyle, alcényloxy(C₂-C₂₀)-carbonyle, rétinyloxycarbonyle, alcynyloxy(C₂-C₂₀)-carbonyle, aryloxy(C₆-C₁₂)-alcoxy(C₁-C₆)-carbonyle, aralcoxy(C₇-C₁₆)-alcoxy(C₁-C₆)-carbonyle, cycloalkyl(C₃-C₈)-alcoxy(C₁-C₆)-carbonyle, cycloalcoxy(C₃-C₈)-alcoxy(C₁-C₆)-carbonyle,
un groupe alkyl(C₁-C₁₂)-carbonyloxy, cycloalkyl(C₃-C₈)-carbonyloxy, aryl(C₆-C₁₂)-carbonyloxy, aralkyl(C₇-C₁₆)-carbonyloxy, cinnamoyloxy, alcényl(C₂-C₁₂)-carbonyloxy, alcynyl(C₂-C₁₂)-carbonyloxy,
un groupe alcoxy(C₁-C₁₂)-carbonyloxy, alcoxy(C₁-C₁₂)-alcoxy(C₁-C₁₂)-carbonyloxy, aryloxy(C₆-C₁₂)-carbonyloxy, aralkyloxy(C₇-C₁₆)-carbonyloxy, cycloalcoxy(C₃-C₈)-carbonyloxy, alcényloxy(C₂-C₁₂)-carbonyloxy, alcynyloxy(C₂-C₁₂)-carbonyloxy,
un groupe carbamoyle, N-alkyl(C₁-C₁₂)-carbamoyle, N,N-dialkyl(C₁-C₁₂)-carbamoyle, N-cycloalkyl(C₃-C₈)-carbamoyle, N,N-dicycloalkyl(C₃-C₈)-carbamoyle, N-alkyl(C₁-C₁₀)-N-cycloalkyl(C₃-C₈)-carbamoyle, N-[cycloalkyl(C₃-C₈)-alkyl(C₁-C₆)]carbamoyle, N-alkyl(C₁-C₆)-N-[cycloalkyl(C₃-C₈)-alkyl(C₁-C₆)]carbamoyle, N-(+)-déhydroabiétylcarbamoyle, N-alkyl(C₁-C₆)-N-(+)-déhydroabiétylcarbamoyle, N-aryl(C₆-C₁₂)-carbamoyle, N-aralkyl(C₇-C₁₆)-carbamoyle, N-alkyl(C₁-C₁₀)-N-aryl(C₆-C₁₆)-carbamoyle, N-alkyl(C₁-C₁₀)-N-aralkyl(C₇-C₁₆)-carbamoyle, N-[alcoxy(C₁-C₁₈)-alkyl(C₁-C₁₀)]carbamoyle, N-[aryloxy(C₆-C₁₆)-alkyl(C₁-C₁₀)]carbamoyle, N-[aralkyloxy(C₇-C₁₆)-alkyl(C₁-C₁₀)]carbamoyle, N-alkyl(C₁-C₁₀)-N-[alcoxy(C₁-C₁₀)-alkyl(C₁-C₁₀)]carbamoyle, N-alkyl(C₁-C₁₀)-N-[aryloxy(C₆-C₁₂)-alkyl(C₁-C₁₀)]carbamoyle, N-alkyl(C₁-C₁₀)-N-[aralkyloxy(C₇-C₁₆)-alkyl(C₁-C₁₀)]carbamoyle,
un groupe CON(CH₂)ₕ dans lequel un groupe CH₂ peut être remplacé par O, S ou par un groupe N-alkyl(C₁-C₈)imino, N-cycloalkyl(C₃-C₈)imino, N-cycloalkyl(C₃-C₈)-alkyl(C₁-C₄)imino, N-aryl(C₆-C₁₂)imino, N-aralkyl(C₇-C₁₆)imino ou N-alcoxy(C₁-C₄)-alkyl(C₁-C₆)imino, et h va de 3 à 7,
un radical carbamoyle de formule générale II 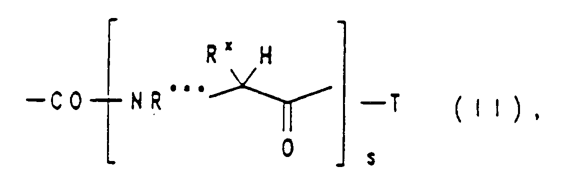 dans laquelle
R^{x} représente les substituants d'un α-aminoacide, comprenant les L-aminoacides et D-aminoacides,
s est égal à 1, 2, 3, 4 ou 5, et
T représente OH, OR ou NR*R**,
R* , R** et R*** étant identiques ou différents et représentant un atome d'hydrogène ou un groupe aryle en C₆-C₁₂, aralkyle en C₇-C₁₁, alkyle en C₁-C₈, cycloalkyle en C₃-C₈, (+)-déhydroabiétyle, alcoxy(C₁-C₈)-alkyle(C₁-C₈), aralcoxy(C₇-C₁₂)-alkyle(C₁-C₈), aryloxy(C₆-C₁₂)-alkyle(C₁-C₈), alcanoyle en C₁-C₁₀, un groupe aralcanoyle en C₇-C₁₆ éventuellement subtitué, un groupe aroyle en C₆-C₁₂ éventuellement substitué, ou
R* et R** représentent ensemble un groupement -[CH₂]ₕ dans lequel un groupe CH₂ peut être remplacé par O, S, SO, SO₂, ou par un groupe N-acylamino, N-alcoxy(C₁-C₁₀)-carbonylimino, N-alkyl(C₁-C₈)imino, N-cycloalkyl(C₃-C₈)imino, N-cycloalkyl(C₃-C₈)-alkyl(C₁-C₄)-imino, N-aryl(C₆-C₁₂)imino, N-aralkyl(C₇-C₁₆)imino ou N-alcoxy(C₁-C₄)-alkyl(C₁-C₆)imino, et h va de 3 à 7,
un groupe carbamoyloxy, N-alkyl(C₁-C₁₂)-carbamoyloxy, N,N-dialkyl(C₁-C₁₂)-carbamoyloxy, N-cycloalkyl(C₃-C₈)-carbamoyloxy, N-aryl(C₆-C₁₂)-carbamoyloxy, N-aralkyl(C₇-C₁₆)-carbamoyloxy, N-alkyl(C₁-C₁₀)-N-aryl(C₆-C₁₂)-carbamoyloxy, N-alkyl(C₁-C₁₀)-N-aralkyl(C₇-C₁₆)-carbamoyloxy, N-[alkyl(C₁-C₁₀)]carbamoyloxy, N-[aryloxy(C₆-C₁₂)-alkyl(C₁-C₁₀)]carbamoyloxy, N-[aralkyloxy(C₇-C₁₆)-alkyl(C₁-C₁₀)]carbamoyloxy, N-alkyl(C₁-C₁₀)-N-[alcoxy(C₁-C₁₀)-alkyl(C₁-C₁₀)]carbamoyloxy, N-alkyl(C₁-C₁₀)-N-[aryloxy(C₆-C₁₂)-alkyl(C₁-C₁₀)]carbamoyloxy, N-alkyl(C₁-C₁₀)-N-[aralkyloxy(C₇-C₁₆)-alkyl(C₁-C₁₀)]-carbamoyloxy,
un groupe amino, alkyl(C₁-C₁₂)amino, dialkyl(C₁-C₁₂)-amino, cycloalkyl(C₃-C₈)amino, alcényl(C₃-C₁₂)amino, alcynyl(C₃-C₁₂)amino, N-aryl(C₆-C₁₂)amino, N-aralkyl(C₇-C₁₁)amino, N-alkyl-aralkylamino, N-alkyl-arylamino, alcoxy(C₁-C₁₂)amino, alcoxy(C₁-C₁₂)-N-alkyl(C₁-C₁₀)-amino,
un groupe alcanoyl(C₁-C₁₂)amino, cycloalcanoyl(C₃-C₈)-amino, aroyl(C₆-C₁₂)amino, aralcanoyl(C₇-C₁₆)amino, alcanoyl(C₁-C₁₂)-N-alkyl(C₁-C₁₀)amino, cycloalcanoyl(C₃-C₈)-N-alkyl(C₁-C₁₀)amino, aroyl(C₆-C₁₂)-N-alkyl(C₁-C₁₀)amino, aralcanoyl(C₇-C₁₁)-N-alkyl(C₁-C₁₀)amino,
un groupe alcanoyl(C₁-C₁₂)amino-alkyle(C₁-C₈), cycloalcanoyl(C₃-C₈)amino-alkyle(C₁-C₈), aroyl(C₆-C₁₂)amino-alkyle(C₁-C₈), aralcanoyl(C₇-C₁₆)amino-alkyle(C₁-C₈), amino-alkyle(C₁-C₁₀), N-alkyl(C₁-C₁₀)amino-alkyle(C₁-C₁₀), N,N-dialkyl(C₁-C₁₀)amino-alkyle(C₁-C₁₀), cycloalkyl(C₃-C₈)amino-alkyle(C₁-C₁₀), alkyl(C₁-C₂₀)mercapto, alkyl(C₁-C₂₀)sulfinyle, alkyl(C₁-C₂₀)sulfonyle, aryl(C₆-C₁₂)mercapto, aryl(C₆-C₁₂)sulfinyle, aryl(C₆-C₁₂)sulfonyle, aralkyl(C₇-C₁₆)mercapto, aralkyl(C₇-C₁₆)sulfinyle, aralkyl (C₇-C₁₆)sulfonyle, alkyl(C₁-C₁₂)mercapto-alkyle(C₁-C₆), alkyl(C₁-C₁₂)sulfinyl-alkyle(C₁-C₆), alkyl(C₁-C₁₂)sulfonyl-alkyle(C₁-C₆), aryl(C₆-C₁₂)mercapto-alkyle(C₁-C₆), aryl(C₆-C₁₂)sulfinyl-alkyle(C₁-C₆), aryl(C₆-C₁₂)sulfonyl-alkyle(C₁-C₆), aralkyl(C₇-C₁₆)mercapto-alkyle(C₁-C₆), aralkyl(C₇-C₁₆)sulfinyl-alkyle(C₁-C₆), aralkyl(C₇-C₁₆)sulfonyl-alkyle(C₁-C₆),
un groupe sulfamoyle, N-alkyl(C₁-C₁₀)sulfamoyle, N,N-dialkyl (C₁-C₁₀) sulfamoyle,
cycloalkyl (C₃-C₈)sulfamoyle,
N-aryl(C₆-C₁₂)sulfamoyle,
N-aralkyl(C₇-C₁₆)sulfamoyle,
N-alkyl(C₁-C₁₀)-N-aryl(C₆-C₁₂)sulfamoyle,
N-alkyl(C₁-C₁₀)-N-aralkyl(C₇-C₁₆)sulfamoyle,
alkyl(C₁-C₁₀)sulfonamido,
N-[alkyl(C₁-C₁₀)-alkyl(C₁-C₁₀)]sulfonamido, aralkyl-(C₇-C₁₆)sulfonamido, N-[alkyl(C₁-C₁₀)-aralkyl(C₇-C₁₆)]sulfonamido,
les radicaux qui contiennent un fragment aryle pouvant pour leur part être substitués sur le fragment aryle par 1 à 5 substituants, identiques ou différents, choisis parmi:
des atomes d'halogène ou des groupes hydroxy, cyano, trifluorométhyle, nitro, carboxy, alkyle en C₁-C₁₆, cycloalkyle en C₃-C₈, cycloalkyl(C₃-C₈)-alkyle(C₁-C₁₂), cycloalkyloxy en C₃-C₈, cycloalkyl(C₃-C₈)-alcoxy(C₁-C₁₂), cycloalkyloxy(C₃-C₈)-alkyle(C₁-C₁₂), cycloalkyloxy(C₃-C₈)-alcoxy(C₁-C₁₂), cycloalkyl(C₃-C₈)-alkyl(C₁-C₈)-alcoxy(C₁-C₆), cycloalkyl(C₃-C₈)-alcoxy(C₁-C₈)-alkyle(C₁-C₆), cycloalkyloxy(C₃-C₈)-alcoxy(C₁-C₈)-alkyle(C₁-C₆), cycloalcoxy(C₃-C₈)-alcoxy(C₁-C₈)-alcoxy(C₁-C₈), aryle en C₆-C₁₂, aralkyle en C₇-C₁₆, alcényle en C₂-C₁₆, alcynyle en C₂-C₁₂, alcoxy en C₁-C₁₆, alcényloxy en C₁-C₁₆, alcoxy(C₁-C₁₂)alkyle(C₁-C₁₂), alcoxy(C₁-C₁₂)-alcoxy(C₁-C₁₂), alcoxy(C₁-C₁₂)-alcoxy(C₁-C₈)-alkyle(C₁-C₈), aryloxy en C₆-C₁₂, aralkyloxy en C₇-C₁₆, aryloxy(C₆-C₁₂)-alcoxy(C₁-C₆), aralcoxy(C₇-C₁₆)-alcoxy(C₁-C₆), hydroxyalkyle en C₁-C₈, aryloxy(C₆-C₁₆)-alkyle(C₁-C₈), aralcoxy(C₇-C₁₆)-alkyle(C₁-C₈), aryloxy(C₆-C₁₂)-alcoxy(C₁-C₈)-alkyle(C₁-C₆), aralkyloxy(C₇-C₁₂)-alcoxy(C₁-C₈)-alkyle(C₁-C₆), -O-[CH₂]ₓC_{f}H_{(2f+1-g)}F_{g}, -OCF₂Cl, -OCF₂-CHFCl, alkyl(C₁-C₁₂)carbonyle, cycloalkyle(C₃-C₈)carbonyle, aryl(C₆-C₁₂)carbonyle, aralkyl(C₇-C₁₆)carbonyle, alcoxy(C₁-C₁₂)carbonyle, alcoxy(C₁-C₁₂)-alcoxy(C₁-C₁₂)-carbonyle, aryloxy(C₆-C₁₂)carbonyle, aralcoxy(C₇-C₁₆)-carbonyle, cycloalkyloxy(C₃-C₈)carbonyle, alcényloxy(C₂-C₁₂)carbonyle, alcynyloxy(C₂-C₁₂)carbonyle, aryloxy(C₆-C₁₂)-alcoxy(C₁-C₆)carbonyle, aralcoxy(C₇-C₁₆)-alcoxy(C₁-C₆)carbonyle, cycloalkyl(C₃-C₈)-alcoxy(C₁-C₆)-carbonyle, cycloalkyloxy(C₃-C₈)-alcoxy(C₁-C₆)carbonyle,
un groupe alkyl(C₁-C₁₂)-carbonyloxy, cycloalkyl(C₃-C₈)-carbonyloxy, aryl(C₆-C₁₂)-carbonyloxy, aralkyl(C₇-C₁₆)-carbonyloxy, cinnamoyloxy, alcényl(C₂-C₁₂)-carbonyloxy, alcynyl(C₂-C₁₂)-carbonyloxy,
un groupe alcoxy(C₁-C₁₂)-carbonyloxy, alcoxy(C₁-C₁₂)-alcoxy(C₁-C₁₂)-carbonyloxy, aryloxy(C₆-C₁₂)-carbonyloxy, aralkyloxy(C₇-C₁₆)-carbonyloxy, cycloalkyloxy(C₃-C₈)-carbonyloxy, alcényloxy(C₂-C₁₂)-carbonyloxy, alcynyloxy(C₂-C₁₂)-carbonyloxy,
un groupe carbamoyle, N-alkyl(C₁-C₁₂)-carbamoyle, N,N-dialkyl(C₁-C₁₂)-carbamoyle, N-cycloalkyl(C₃-C₈)-carbamoyle, N,N-dicycloalkyl(C₃-C₈)-carbamoyle, N-alkyl(C₁-C₁₀)-N-cycloalkyl(C₃-C₈)-carbamoyle, N-[cycloalkyl(C₃-C₈)-alkyl(C₁-C₆)]carbamoyle, N-alkyl(C₁-C₆)-N-[cycloalkyl(C₃-C₈)-alkyl(C₁-C₆)]carbamoyle, N-(+)-déhydroabiétylcarbamoyle, N-alkyl(C₁-C₆)-N-(+)-déhydroabiétylcarbamoyle, N-aryl(C₆-C₁₂)-carbamoyle, N-aralkyl(C₇-C₁₆)-carbamoyle, N-alkyl(C₁-C₁₀)-N-aryl(C₆-C₁₆)-carbamoyle, N-alkyl(C₁-C₁₀)-N-aralkyl(C₇-C₁₆)-carbamoyle, N-[alcoxy(C₁-C₆)-alkyl(C₁-C₁₀)]carbamoyle, N-[aryloxy(C₆-C₁₆)-alkyl(C₁-C₁₀)]carbamoyle, N-[aralkyloxy(C₇-C₁₆)alkyl(C₁-C₁₀)]carbamoyle, N-alkyl(C₁-C₁₀)-N-[alcoxy(C₁-C₁₀)-alkyl(C₁-C₁₀)]carbamoyle, N-alkyl(C₁-C₁₀)-N-[aryloxy(C₆-C₁₂)-alkyl(C₁-C₁₀)]carbamoyle, N-alkyl(C₁-C₁₀)-N-[aralkyloxy(C₇-C₁₆)-alkyl(C₁-C₁₀)]carbamoyle,
un groupe CON(CH₂)ₕ dans lequel un groupe CH₂ peut être remplacé par O, S ou par un groupe N-alkyl(C₁-C₈)imino, N-cycloalkyl(C₃-C₈)imino, N-cycloalkyl(C₃-C₈)-alkyl(C₁-C₄)imino, N-aryl(C₆-C₁₂)imino, N-aralkyl(C₇-C₁₆)imino ou N-alcoxy(C₁-C₄)-alkyl(C₁-C₆)imino, et h va de 3 à 7, des groupes carbamoyloxy, N-alkyl(C₁-C₁₂)-carbamoyloxy, N,N-dialkyl(C₁-C₁₂)-carbamoyloxy, N-cycloalkyl(C₃-C₈)-carbamoyloxy, N-aryl(C₆-C₁₆)-carbamoyloxy, N-aralkyl(C₇-C₁₆)-carbamoyloxy, N-alkyl(C₁-C₁₀)-N-aryl(C₆-C₁₂)-carbamoyloxy, N-alkyl(C₁-C₁₀)-N-aralkyl(C₇-C₁₆)-carbamoyloxy, N-[alkyl(C₁-C₁₀)]carbamoyloxy, N-[aryloxyC₆-C₁₂)-alkyl(C₁-C₁₀)]carbamoyloxy, N-[aralkyloxy(C₇-C₁₆)-alkyl(C₁-C₁₀)]carbamoyloxy, N-alkyl(C₁-C₁₀)-N-[alcoxy(C₁-C₁₀)-alkyl(C₁-C₁₀)]carbamoyloxy, N-alkyl(C₁-C₁₀)-N-[aryloxy(C₆-C₁₂)-alkyl(C₁-C₁₀)]carbamoyloxy, N-alkyl(C₁-C₁₀)-N-[aralkyloxy(C₇-C₁₆)-alkyl(C₁-C₁₀)]-carbamoyloxy,
des groupes amino, alkyl(C₁-C₁₂)amino, dialkyl(C₁-C₁₂)-amino, cycloalkyl(C₃-C₈)amino, alcényl(C₃-C₁₂)amino, alcynyl(C₃-C₁₂)amino, N-aryl(C₆-C₁₂)amino, N-aralkyl(C₇-C₁₁)amino, N-alkyl-aralkylamino, N-alkyl-arylamino, alcoxy(C₁-C₁₂)amino, alcoxy(C₁-C₁₂)-N-alkyl(C₁-C₁₀)-amino,
des groupes alcanoyl(C₁-C₁₂)amino, cycloalcanoyl(C₃-C₈)-amino, aroyl(C₆-C₁₂)amino, aralcanoyl(C₇-C₁₆)amino, alcanoyl(C₁-C₁₂)-N-alkyl(C₁-C₁₀)amino, cycloalcanoyl(C₃-C₈)-N-alkyl(C₁-C₁₀)amino, aroyl(C₆-C₁₂)-N-alkyl(C₁-C₁₀)amino, aralcanoyl(C₇-C₁₁)-N-alkyl(C₁-C₁₀)amino, des groupes alcanoyl(C₁-C₁₂)amino-alkyle(C₁-C₈), cycloalcanoyl(C₃-C₈)amino-alkyle(C₁-C₈), aroyl(C₆-C₁₂)amino-alkyle(C₁-C₈), aralcanoyl(C₇-C₁₆)amino-alkyle(C₁-C₈), aminoalkyl en C₁-C₁₀, N-alkyl(C₁-C₁₀)amino-alkyle(C₁-C₁₀), N,N-dialkyl(C₁-C₁₀)amino-alkyle(C₁-C₁₀), cycloalkyl(C₃-C₈)amino-alkyle(C₁-C₁₀),
des groupes alkyl(C₁-C₁₂)mercapto, alkyl(C₁-C₁₂)-sulfinyle, alkyl(C₁-C₁₂)sulfonyle, aryl(C₆-C₁₆)mercapto, aryl(C₆-C₁₆)sulfinyle, aryl(C₆-C₁₆)sulfonyle, aralkyl(C₇-C₁₆)mercapto, aralkyl(C₇-C₁₆)sulfinyle, aralkyl(C₇-C₁₆)sulfonyle,
R¹ et R² ou R² et R³ forment une chaîne [CH₂]ₒ, dans laquelle un ou deux groupes CH₂ de la chaîne saturée ou insaturée comportant une double liaison C=C sont éventuellement remplacés par O, S, SO, SO₂ ou NR', o = 3, 4 ou 5, et
R' représente un atome d'hydrogène ou un groupe aryle en C₆-C₁₂, alkyle en C₁-C₁₈, alcoxy(C₁-C₈)-alkyle(C₁-C₈), aralcoxy(C₇-C₁₂)-alkyle(C₁-C₈), aryloxy(C₆-C₁₂)-alkyle(C₁-C₈), alcanoyle en C₁-C₁₀, un groupe aralcanoyle en C₇-C₁₆ éventuellement substitué, un groupe aroyle en C₆-C₁₂ éventuellement substitué,
les radicaux R¹ et R² ou R² et R³ formant de préférence, conjointement avec le reste pyridine ou pyridazine qui les porte, le cycle 5,6,7,8-tétrahydro-isoquinoléine, 5,6,7,8-tétrahydroquinoléine ou 5,6,7,8-tétrahydrocinnoline, ou
R¹ et R² ou R² et R³ forment un cycle aromatique à 5 ou 6 chaînons, carbocyclique ou hétérocyclique,
les radicaux R¹ et R² ou R² et R³ formant de préférence, conjointement avec le reste pyridine ou pyridazine qui les porte, les systèmes cycliques hétérocycliques, éventuellement substitués, suivants:
thiénopyridines,
furannopyridines,
pyridopyridines,
pyrimidinopyridines,
imidazopyridines,
thiazolopyridines,
oxazolopyridines,
quinoléine,
isoquinoléine et
cinnoline,
les cycles quinoléine, isoquinoléine et cinnoline correspondant de préférence aux formules 1a, 1b et 1c 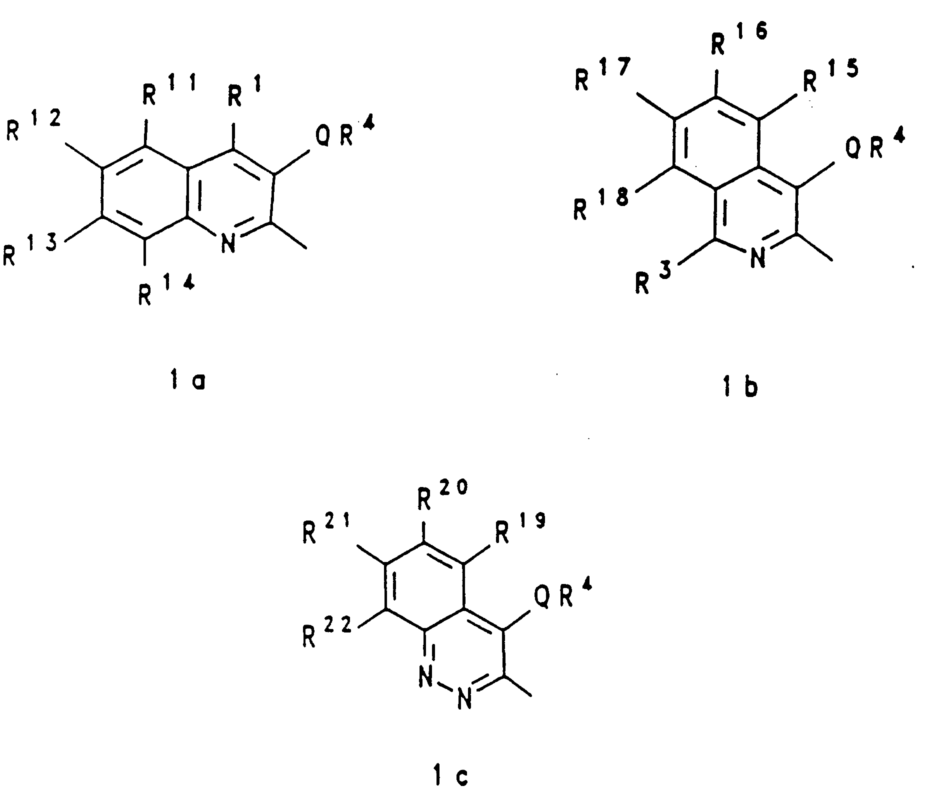 et les substituants R¹¹ à R²² ayant chacun, indépendamment les uns des autres, les significations de R¹, R² et R³,
R⁴, lorsque Q est une liaison, représente un atome d'halogène ou le groupe nitrile ou trifluorométhyle ou, lorsque Q est O ou S, représente un radical alkyle en C₁-C₂₀ ramifié ou non ramifié, un radical fluoroalkyle saturé, non substitué, de formule [CH₂]ₓC_{f}H_{(2f+1-g)}F_{g}, un radical aryle en C₆-C₁₆, un radical aralkyle en C₇-C₁₆, un radical hétéroaryle ou un radical hétéroaralkyle,
ces radicaux étant substitués par un ou plusieurs substituants choisis parmi
des atomes d'halogène et des groupes hydroxy, cyano, trifluorométhyle, nitro, carboxy, alkyle en C₁-C₁₂, cycloalkyle en C₃-C₈, cycloalkyl(C₃-C₈)-alkyle(C₁-C₁₂), cycloalcoxy en C₃-C₈, cycloalkyl(C₃-C₈)-alcoxy(C₁-C₁₂), cycloalkyloxy(C₃-C₈)-alkyle(C₁-C₁₂), cycloalkyloxy(C₃-C₈)-alcoxy(C₁-C₁₂), cycloalkyl(C₃-C₈)-alkyl(C₁-C₈)-alcoxy(C₁-C₆), cycloalkyl(C₃-C₈)-alcoxy(C₁-C₈)-alkyle(C₁-C₆), cycloalkyloxy(C₃-C₈)-alcoxy(C₁-C₈)-alkyle(C₁-C₆), cycloalcoxy(C₃-C₈)-alcoxy(C₁-C₈)-alcoxy(C₁-C₈), aryle en C₆-C₁₂, aralkyle en C₇-c₁₆, alcényle en C₂-C₁₂, alcynyle en C₂-C₁₂, alcoxy en C₁-C₁₂, alcoxy(C₁-C₁₂)alkyle(C₁-C₁₂), alcoxy(C₁-C₁₂)-alcoxy(C₁-C₁₂), alcoxy(C₁-C₁₂)-alcoxy(C₁-C₈)-alkyle(C₁-C₈), aryloxy en C₆-c₁₂, aralkyloxy en C₇-C₁₆, aryloxy(C₆-C₁₂)-alcoxy(C₁-C₆), aralcoxy(C₇-C₁₆)-alcoxy(C₁-C₆), hydroxyalkyle en C₁-C₈, aryloxy(C₆-C₁₆)-alkyle(C₁-C₈), aralcoxy(C₇-C₁₆)-alkyle(C₁-C₈), aryloxy(C₆-C₁₂)-alcoxy(C₁-C₈)-alkyle(C₁-C₆), aralkyloxy(C₇-C₁₂)-alcoxy(C₁-C₈)-alkyle(C₁-C₆), -O-[CH₂]ₓC_{f}H_{(2f+1-g)}F_{g}, -OCF₂Cl, -OCF₂-CHFCl, des groupes alkyl(C₁-C₁₂)carbonyle, cycloalkyl(C₃-C₈)-carbonyle, alkyl(C₆-C₁₂)carbonyle, aralkyl(C₇-C₁₆)-carbonyle, cinnamoyle, alcényl(C₂-C₁₂)carbonyle, alcynyl(C₂-C₁₂)carbonyle,
alcoxy(C₁-C₁₂)carbonyle, alcoxy(C₁-C₁₂)-alcoxy(C₁-C₁₂)carbonyle, aryloxy(C₆-C₁₂)carbonyle, aralcoxy(C₇-C₁₆)carbonyle, cycloalcoxy(C₃-C₈)carbonyle, alcényloxy(C₂-C₁₂)carbonyle, alcynyloxy(C₂-C₁₂)carbonyle, aryloxy(C₆-C₁₂)-alcoxy(C₁-C₆)carbonyle, aralcoxy(C₇-C₁₆)-alcoxy(C₁-C₆)carbonyle, cycloalkyl(C₃-C₈)-alcoxy(C₁-C₆)carbonyle, cycloalcoxy(C₃-C₈)-alcoxy(C₁-C₆)carbonyle, un groupe alkyl(C₁-C₁₂)-carbonyloxy, cycloalkyl(C₃-C₈)-carbonyloxy, aryl(C₆-C₁₂)-carbonyloxy, aralkyl(C₇-C₁₆)-carbonyloxy, cinnamoyloxy, alcényl(C₂-C₁₂)-carbonyloxy, alcynyl(C₂-C₁₂)-carbonyloxy,
un groupe alcoxy(C₁-C₁₂)-carbonyloxy, alcoxy(C₁-C₁₂)-alcoxy(C₁-C₁₂)-carbonyloxy, aryloxy(C₆-C₁₂)-carbonyloxy, aralkyloxy(C₇-C₁₆)-carbonyloxy, cycloalkyloxy(C₃-C₈)-carbonyloxy, alcényloxy(C₂-C₁₂)-carbonyloxy, alcynyloxy(C₂-C₁₂)-carbonyloxy,
un groupe carbamoyle, N-alkyl(C₁-C₁₂)-carbamoyle, N,N-dialkyl(C₁-C₁₂)-carbamoyle, N-cycloalkyl(C₃-C₈)-carbamoyle, N,N-dicycloalkyl(C₃-C₈)-carbamoyle, N-alkyl(C₁-C₁₀)-N-cycloalkyl(C₃-C₈)-carbamoyle, N-[cycloalkyl(C₃-C₈)-alkyl(C₁-C₆)]carbamoyle, N-alkyl(C₁-C₆)-N-[cycloalkyl(C₃-C₈)-alkyl(C₁-C₆)]carbamoyle, N-(+)-déhydroabiétylcarbamoyle, N-alkyl(C₁-C₆)-N-(+)-déhydroabiétylcarbamoyle, N-aryl(C₆-C₁₂)-carbamoyle, N-aralkyl(C₇-C₁₆)-carbamoyle, N-alkyl(C₁-C₁₀)-N-aryl(c₆-C₁₆)-carbamoyle, N-alkyl(C₁-C₁₀)-N-aralkyl(C₇-C₁₆)-carbamoyle, N-[alcoxy(C₁-C₁₀)-alkyl(C₁-C₁₀)]carbamoyle, N-[aryloxy(C₆-c₁₆)-alkyl(C₁-C₁₀)]carbamoyle, N-[aralkyloxy(C₇-C₁₆)-alkyl-(C₁-C₁₀)]carbamoyle, N-alkyl(C₁-C₁₀)-N-[alcoxy(C₁-C₁₀)alkyl(C₁-C₁₀)]carbamoyle, N-alkyl(C₁-C₁₀)-N-[aryloxy(C₆-C₁₂)-alkyl(C₁-C₁₀)]carbamoyle, N-alkyl(C₁-C₁₀)-N-[aralkyloxy(C₇-C₁₆)-alkyl(C₁-C₁₀)]carbamoyle,
un groupe CON(CH₂)ₕ dans lequel un groupe CH₂ peut être remplacé par O, S ou par un groupe N-alkyl(C₁-C₈)imino, N-cycloalkyl(C₃-C₈)imino, N-cycloalkyl(C₃-C₈)-alkyl(C₁-C₄)imino, N-aryl(C₆-C₁₂)imino, N-aralkyl(C₇-C₁₆)imino ou N-alcoxy(C₁-C₄)-alkyl(C₁-C₆)imino, et h va de 3 à 7,
ou par
un groupe carbamoyle de formule générale II 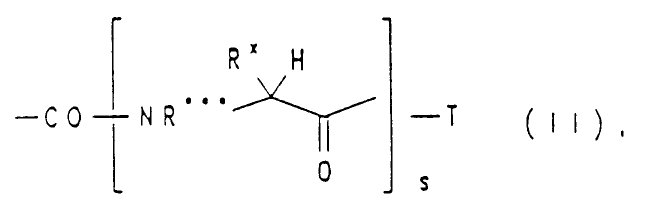 dans laquelle
R^{x} représente le substituant d'un α-aminoacide, comprenant les L-aminoacides et D-aminoacides,
s est égal à 1, 2, 3, 4 ou 5, et
T représente OH, OR ou NR*R**,
R*, R** et R*** étant identiques ou différents et représentant un atome d'hydrogène ou un groupe aryle en C₆-C₁₂, aralkyle en C₇-C₁₁, alkyle en C₁-C₈, cycloalkyle en C₃-C₈, (+)-déhydroabiétyle, alcoxy(C₁-C₈)-alkyl(C₁-C₈), aralcoxy(C₇-C₁₂)-alkyle(C₁-C₈), aryloxy(C₆-C₁₂)-alkyle(C₁-C₈), alcanoyle en C₁-C₁₀, un groupe aralcanoyle en C₇-C₁₆ éventuellement subtitué, un groupe aroyle en C₆-C₁₂ éventuellement substitué, ou
R* et R** représentent ensemble un groupement -[CH₂]ₕ dans lequel un groupe CH₂ peut être remplacé par O, S, SO, SO₂, ou par un groupe N-acylamino, N-alcoxy(C₁-C₁₀)-carbonylimino, N-alkyl(C₁-C₈)imino, N-cycloalkyl(C₃-C₈)imino, N-cycloalkyl(C₃-C₈)-alkyl(C₁-C₄)imino, N-aryl(C₆-C₁₂)imino, N-aralkyl(C₇-C₁₆)imino ou N-alcoxy(C₁-C₄)-alkyl(C₁-C₆)imino, et h va de 3 à 7, ou par
un groupe carbamoyloxy, N-alkyl (C₁-C₁₂)-carbamoyloxy, N,N-dialkyl(C₁-C₁₂)-carbamoyloxy, N-cycloalkyl(C₃-C₈)-carbamoyloxy, N-aryl(C₆-C₁₂)-carbamoyloxy, N-aralkyl(C₇-C₁₆)-carbamoyloxy, N-alkyl(C₁-C₁₀)-N-aryl(C₆-C₁₂)-carbamoyloxy, N-alkyl(C₁-C₁₀)-N-aralkyl(C₇-C₁₆)-carbamoyloxy, N-[alkyl(C₁-C₁₀)]carbamoyloxy, N-[aryloxy(C₆-C₁₂)-alkyl(C₁-C₁₀)]carbamoyloxy, N-[aralkyloxy-(C₇-C₁₆)-alkyl(C₁-C₁₀)]carbamoyloxy, N-alkyl(C₁-C₁₀)-N-[alcoxy(C₁-C₁₀)-alkyl(C₁-C₁₀)]carbamoyloxy, N-alkyl-(C₁-C₁₀)-N-[aryloxy(C₆-C₁₂)-alkyl(C₁-C₁₀)]carbamoyloxy, N-alkyl(C₁-C₁₀)-N-[aralkyloxy(C₇-C₁₆)-alkyl(C₁-C₁₀)]-carbamoyloxy,
un groupe amino, alkyl(C₁-C₁₂)amino, dialkyl(C₁-C₁₂)-amino, cycloalkyl(C₃-C₈)amino, alcényl(C₃-C₁₂)amino, alcynyl(C₃-C₁₂)amino, N-aryl(C₆-C₁₂)amino, N-aralkyl-(C₇-C₁₁)amino, N-alkyl-aralkylamino, N-alkyl-arylamino, alcoxy(C₁-C₁₂)amino, alcoxy(C₁-C₁₂)-N-alkyl(C₁-C₁₀)amino,
un groupe alcanoyl(C₁-C₁₂)amino, cycloalcanoyl(C₃-C₈)amino, aroyl(C₆-C₁₂)amino, aralcanoyl(C₇-C₁₆)amino, alcanoyl(C₁-C₁₂)-N-alkyl(C₁-C₁₀)amino, cycloalcanoyl(C₃-C₈)-N-alkyl(C₁-C₁₀)amino, aroyl(C₆-C₁₂)-N-alkyl(C₁-C₁₀)amino, aralcanoyl(C₇-C₁₁)-N-alkyl(C₁-C₁₀)amino, un groupe alcanoyl(C₁-C₁₂)amino-alkyle(C₁-C₈), cycloalcanoyl(C₃-C₈)amino-alkyle(C₁-C₈), aroyl(C₆-C₁₂)amino-alkyle(C₁-C₈), aralcanoyl(C₇-C₁₆)amino-alkyle(C₁-C₈), amino-alkyle(C₁-C₁₀), N-alkyl(C₁-C₁₀)amino-alkyle(C₁-C₁₀), N,N-dialkyl(C₁-C₁₀)amino-alkyle(C₁-C₁₀), cycloalkyl (C₃-C₈)amino-alkyle(C₁-C₁₀),
alkyl(C₁-C₁₂)mercapto, alkyl(C₁-C₁₂)sulfinyle, alkyl(C₁-C₁₂)sulfonyle, aryl(C₆-C₁₂)mercapto, aryl(C₆-C₁₂)sulfinyle, aryl(C₆-C₁₂)sulfonyle, aralkyl(C₇-c₁₆)mercapto, aralkyl(C₇-C₁₆)sulfinyle, aralkyl (C₇-C₁₆)sulfonyle,
un groupe sulfamoyle, N-alkyl(C₁-C₁₀)sulfamoyle, N,N-dialkyl (C₁-C₁₀) sulfamoyle,
cycloalkyl(C₃-C₈)sulfamoyle,
N-aryl(C₆-C₁₂)sulfamoyle,
N-aralkyl(C₇-C₁₆)sulfamoyle,
N-alkyl(C₁-C₁₀)-N-aryl(C₆-C₁₂)sulfamoyle,
N-alkyl(C₁-C₁₀)-N-aralkyl(C₇-C₁₆)sulfamoyle,
alkyl(C₁-C₁₀)sulfonamido,
N-[alkyl(C₁-C₁₀)-alkyl(C₁-C₁₀)]sulfonamido, aralkyl(C₇-C₁₆)sulfonamido, N-[alkyl(C₁-C₁₀)-aralkyl(C₇-C₁₆)]sulfonamido,
les radicaux qui contiennent un fragment aryle pouvant pour leur part être substitués sur le fragment aryle par 1 à 5 substituants, identiques ou différents, choisis parmi:
des atomes d'halogène ou des groupes hydroxy, cyano, trifluorométhyle, nitro, carboxy, alkyle en C₁-C₁₂, cycloalkyle en C₃-C₈, cycloalkyl(C₃-C₈)-alkyle(C₁-C₁₂), cycloalcoxy en C₃-C₈, cycloalkyl(C₃-C₈)-alcoxy(C₁-C₁₂), cycloalkyloxy(C₃-C₈)-alkyle(C₁-C₁₂), cycloalkyloxy(C₃-C₈)-alcoxy(C₁-C₁₂), cycloalkyl(C₃-C₈)-alkyl(C₁-C₈)-alcoxy-(C₁-C₆), cycloalkyl(C₃-C₈)-alcoxy(C₁-C₈)-alkyle(C₁-C₆), cycloalkyloxy(C₃-C₈)-alcoxy(C₁-C₈)-alkyle(C₁-C₆), cycloalcoxy(C₃-C₈)-alcoxy(C₁-C₈)-alcoxy(C₁-C₈), aryle en C₆-C₁₂, aralkyle en C₇-C₁₆, alcényle en C₂-C₁₂, alcynyle en C₂-C₁₂, alcoxy en C₁-C₁₂, alcoxy(C₁-C₁₂)alkyle(C₁-C₁₂), alcoxy(C₁-C₁₂)-alcoxy(C₁-C₁₂), alcoxy(C₁-C₁₂)alcoxy(C₁-C₈)-alkyle(C₁-C₈), aryloxy en C₆-C₁₂, aralkyloxy en C₇-C₁₆, aryloxy(C₆-C₁₂)-alcoxy(C₁-C₆), aralcoxy(C₇-C₁₆)-alcoxy(C₁-C₆), hydroxyalkyle en C₁-C₈, aryloxy(C₆-C₁₆)-alkyle(C₁-C₈), aralcoxy(C₇-C₁₆)-alkyle(C₁-C₈), aryloxy(C₆-C₁₂)-alcoxy(C₁-C₈)-alkyle(C₁-C₆), aralkyloxy(C₇-C₁₂)-alcoxy(C₁-C₈)-alkyle(C₁-C₆),
-O-[CH₂]ₓC_{f}H_{(2f+1-g)}F_{g}, -OCF₂Cl, -OCF₂-CHFCl, alkyl(C₁-C₁₂)carbonyle, cycloalkyle(C₃-C₈)carbonyle, aryl(C₆-C₁₂)carbonyle, aralkyl(C₇-C₁₆)carbonyle, alcoxy(C₁-C₁₂)carbonyle, alcoxy(C₁-C₁₂)-alcoxy(C₁-C₁₂)carbonyle, aryloxy(C₆-C₁₂)carbonyle, aralcoxy(C₇-C₁₆)carbonyle, cycloalcoxy(C₃-C₈)carbonyle, alcényloxy(C₂-C₁₂)carbonyle, alcynyloxy(C₂-C₁₂)carbonyle, aryloxy(C₆-C₁₂)-alcoxy(C₁-C₆)carbonyle, aralcoxy(C₇-C₁₆)-alcoxy(C₁-C₆)carbonyle, cycloalkyl(C₃-C₈)-alcoxy(C₁-C₆)carbonyle, cycloalkyloxy(C₃-C₈)-alcoxy(C₁-C₆)carbonyle, alkyl(C₁-C₁₂)-carbonyloxy, cycloalkyl(C₃-C₈)-carbonyloxy, aryl(C₆-C₁₂)-carbonyloxy, aralkyl(C₇-C₁₆)-carbonyloxy, cinnamoyloxy, alcényl(C₂-C₁₂)-carbonyloxy, alcynyl(C₂-C₁₂)-carbonyloxy,
un groupe alcoxy(C₁-C₁₂)-carbonyloxy, alcoxy(C₁-C₁₂)-alcoxy(C₁-C₁₂)-carbonyloxy, aryloxy(C₆-C₁₂)-carbonyloxy, aralkyloxy(C₇-C₁₆)-carbonyloxy, cycloalcoxy(C₃-C₈)-carbonyloxy, alcényloxy(C₂-C₁₂)-carbonyloxy, alcynyloxy(C₂-C₁₂)-carbonyloxy,
un groupe carbamoyle, N-alkyl(C₁-C₁₂)-carbamoyle, N,N-dialkyl(C₁-C₁₂)-carbamoyle, N-cycloalkyl(C₃-C₈)-carbamoyle, N,N-dicycloalkyl(C₃-C₈)-carbamoyle, N-alkyl(C₁-C₁₀)-N-cycloalkyl(C₃-C₈)-carbamoyle, N-[cycloalkyl(C₃-C₈)-alkyl(C₁-C₆)]carbamoyle, N-alkyl(C₁-C₆)-N-[cycloalkyl(C₃-C₈)-alkyl(C₁-C₆)]carbamoyle, N-(+)-déhydroabiétylcarbamoyle, N-alkyl(C₁-C₆)-N-(+)-déhydroabiétylcarbamoyle, N-aryl(C₆-C₁₂)-carbamoyle, N-aralkyl(C₇-C₁₆)carbamoyle, N-alkyl(C₁-C₁₀)-N-aryl(C₆-C₁₆)-carbamoyle, N-alkyl(C₁-C₁₀)-N-aralkyl(C₇-C₁₆)-carbamoyle, N-[alcoxy(C₁-C₁₀)-alkyl(C₁-C₁₀)]carbamoyle, N-[aryloxy(C₆-C₁₆)-alkyl(C₁-C₁₀)]carbamoyle, N-[aralkyloxy(C₇-C₁₆)-alkyl(C₁-C₁₀)]carbamoyle, N-alkyl(C₁-C₁₀)-N-[alcoxy(C₁-C₁₀)-alkyl(C₁-C₁₀)]carbamoyle, N-alkyl(C₁-C₁₀)-N-[aryloxy(C₆-C₁₂)-alkyl(C₁-C₁₀)]carbamoyle, N-alkyl(C₁-C₁₀)-N-[aralkyloxy(C₇-C₁₆)-alkyl(C₁-C₁₀)]carbamoyle,
un groupe CON(CH₂)ₕ dans lequel un groupe CH₂ peut être remplacé par O, S ou par un groupe N-alkyl(C₁-C₈)imino, N-cycloalkyl(C₃-C₈)imino, N-cycloalkyl(C₃-C₈)-alkyl(C₁-C₄)imino, N-aryl(C₆-C₁₂)imino, N-aralkyl(C₇-C₁₆)imino ou N-alcoxy(C₁-C₄)-alkyl(C₁-C₆)imino, et h va de 3 à 7, des groupes carbamoyloxy, N-alkyl(C₁-C₁₂)-carbamoyloxy, N,N-dialkyl(C₁-C₁₂)-carbamoyloxy, N-cycloalkyl(C₃-C₈)-carbamoyloxy, N-aryl(C₆-C₁₆)-carbamoyloxy, N-aralkyl(C₇-C₁₆)-carbamoyloxy, N-alkyl(C₁-C₁₀)-N-aryl(C₆-C₁₂)-carbamoyloxy, N-alkyl(C₁-C₁₀)-N-aralkyl(C₇-C₁₆)carbamoyloxy, N-[alkyl(C₁-C₁₀)]carbamoyloxy, N-[aryloxyC₆-C₁₂)-alkyl(C₁-C₁₀)]carbamoyloxy, N-[aralkyloxy(C₇-C₁₆)-alkyl(C₁-C₁₀)]carbamoyloxy, N-alkyl(C₁-C₁₀)-N-[alcoxy(C₁-C₁₀)-alkyl(C₁-C₁₀)]carbamoyloxy, N-alkyl(C₁-C₁₀)-N-[aryloxy(C₆-C₁₂)-alkyl(C₁-C₁₀)]carbamoyloxy, N-alkyl(C₁-C₁₀)-N-[aralkyloxy(C₇-C₁₆)-alkyl(C₁-C₁₀)]carbamoyloxy,
des groupes amino, alkyl(C₁-C₁₂)amino, dialkyl(C₁-C₁₂)amino, cycloalkyl(C₃-C₈)amino, alcényl(C₃-C₁₂)amino, alcynyl(C₃-C₁₂)amino, N-aryl(C₆-C₁₂)amino, N-aralkyl(C₇-C₁₁)amino, N-alkyl-aralkylamino, N-alkyl-arylamino, alcoxy(C₁-C₁₂)amino, alcoxy(C₁-C₁₂)-N-alkyl(C₁-C₁₀)amino,
des groupes alcanoyl(C₁-C₁₂)amino, cycloalcanoyl(C₃-C₈)amino, aroyl(C₆-C₁₂)amino, aralcanoyl(C₇-C₁₆)amino, alcanoyl(C₁-C₁₂)-N-alkyl(C₁-C₁₀)amino, cycloalcanoyl(C₃-C₈)-N-alkyl(C₁-C₁₀)amino, aroyl(C₆-C₁₂)-N-alkyl(C₁-C₁₀)amino, aralcanoyl(C₇-C₁₁)-N-alkyl(C₁-C₁₀)amino, des groupes alcanoyl(C₁-C₁₂)amino-alkyle(C₁-C₈), cycloalcanoyl(C₃-C₈)amino-alkyle(C₁-C₈), aroyl(C₆-C₁₂)aminoalkyle(C₁-C₈), aralcanoyl(C₇-C₁₆)amino-alkyle(C₁-C₈), aminoalkyl en C₁-C₁₀, N-alkyl(C₁-C₁₀)amino-alkyle(C₁-C₁₀), N,N-dialkyl(C₁-C₁₀)amino-alkyle(C₁-C₁₀), cycloalkyl(C₃-C₈)amino-alkyl(C₁-C₁₀),
des groupes alkyl(C₁-C₁₂)mercapto, alkyl(C₁-C₁₂)sulfinyle, alkyl(C₁-C₁₂)sulfonyle, aryl(C₆-C₁₆)mercapto, aryl(C₆-C₁₆)sulfinyle, aryl(C₆-C₁₆)sulfonyle, aralkyl(C₇-C₁₆)mercapto, aralkyl(C₇-C₁₆)sulfinyle, aralkyl(C₇-C₁₆)sulfonyle, et
R⁴ représente R'', lorsque Q a la signification de NR', R' et R'' étant identiques ou différents et représentant un atome d'hydrogène ou un groupe aryle en C₆-C₁₂, aralkyle en C₇-C₁₁, alkyle en C₁-C₈, alcoxy(C₁-C₈)-alkyle(C₁-C₈), aralcoxy(C₇-C₁₂)-alkyle(C₁-C₈), aryloxy(C₆-C₁₂)-alkyle(C₁-C₈), alcanoyle en C₁-C₁₀, un groupe aralcanoyle en C₇-C₁₆ éventuellement substitué, ou un groupe aroyle en C₆-C₁₂ éventuellement substitué, ou
R' et R'' forment ensemble un groupement -[CH₂]ₕ, un groupe CH₂ pouvant être remplacé par O, S, ou par un radical N-acylimino ou N-alcoxy(C₁-C₁₀)-carbonylimino, et
f va de 1 à 8,
g est égal à 0 ou va de 1 à (2f + 1),
x va de 0 à 3,
h va de 3 à 7,
y compris les sels physiologiquement actifs,
à l'exclusion du 3-benzyloxypyridine-2-(L-thréonylméthylester)carboxamide, du 3-benzyloxyridine-2-[L-thréonyl(Fmoc-Phg)-tert-butylester]carboxamide, du 3-benzyloxypyridine-2-(L-thréonyl-tert-butylester)carboxamide, du 3-benzyloxypyridine-2-(D-allothréonylméthylester)carboxamide et de l'ester éthylique de 3-benzyloxypicolinylglycine.

2. Composés de formule I selon la revendication 1,
dans lesquels
Q représente O, S, NR' ou une liaison,
X représente O,
Y représente CR³ ou,
lorsque R¹ et R² représentent un cycle,
Y représente N ou CR³,
m est 0 ou 1 et
G représente le reste d'un alcool GOH,
et à l'exclusion de l'ester éthylique de 3-benzyloxypicolinylglycine.

3. Composés de formule I selon les revendications 1 et 2, dans lesquels
Q représente O, NR' ou une liaison,
X représente O et
G représente le reste d'un alcool GOH,
et à l'exclusion de l'ester éthylique de 3-benzyloxypicolinylglycine.

4. Composés de formule I selon les revendications 1 et 2, dans lesquels
Q représente S,
X représente O,
m est 0 ou 1 et
G représente le reste d'un alcool GOH.

5. Composés de formule I selon les revendications 1, 2 et 4, dans lesquels
Q représente S,
X représente O,
m est 0 et
G représente le reste d'un alcool GOH

6. Composés de formule I selon les revendications 1 à 3, dans lesquels
Q représente O, NR' ou une liaison,
X représente O,
Y représente CR³ ou, lorsque R¹ et R² forment un cycle,
représente N ou CR³,
m représente 0 ou 1,
A représente un groupe alkylène en C₁-C₃ qui est éventuellement monosubstitué par un atome d'halogène ou par un groupe cyano, trifluorométhyle, alkyle en C₁-C₆, hydroxyalkyle en C₁-C₆, alcoxy en C₁-C₆, -O-[CH₂]ₓ-C_{f}H_{(2f+1-g)}F_{g} ou
A représente -CHR⁵-, R⁵ représentant l'un des substituants de l'atome de carbone α d'un α-aminoacide, en particulier d'un L-aminoacide naturel et de son isomère D,
B représente -CO₂G,
G représente le reste d'un alcool GOH dans lequel G représente un radical alkyle en C₁-C₂₀ aliphatique ramifié ou non ramifié ou cyclique, ou un radical alcényle en C₂-C₂₀ ramifié ou non ramifié, éventuellement cyclique, le radical rétinyle, un radical alcynyle en C₂-C₂₀ ou un radical alcénynyle en C₄-C₂₀ correspondant, les radicaux pouvant contenir chacun une ou plusieurs liaisons multiples, ou un radical aryle en C₆-C₁₆, un radical aralkyle en C₇-C₁₆ ou un radical hétéroaryle à 5 ou 6 chaînons, de préférence azoté, ou un radical hétéroalkyle à 5 ou 6 chaînons, de préférence azoté, les radicaux précédents pouvant porter un ou plusieurs substituants choisis parmi: des atomes d'halogène et des groupes hydroxy, cyano, trifluorométhyle, nitro, carboxy, alkyle en C₁-C₁₂, cycloalkyle en C₃-C₈, cycloalcényle en C₅-C₈, aryle en C₆-C₁₂, aralkyle en C₇-C₁₆, alcényle en C₂-C₁₂, alcynyle en C₂-C₁₂, alcoxy en C₁-C₁₂, alcoxy(C₁-C₁₂)-alkyle(C₁-C₁₂), alcoxy(C₁-C₁₂)-alcoxy(C₁-C₁₂), aryloxy en C₆-C₁₂, aralkyloxy en C₇-C₁₆, hydroxyalkyle en C₁-C₈, -O-[CH₂]ₓC_{f}H_{(2f+1-g)}F_{g}, -OCF₂Cl, -OCF₂-CHFCl, alkyl(C₁-C₁₂)-carbonyle, cycloalkyl(C₃-C₈)-carbonyle, aryl(C₆-C₁₂)-carbonyle, aralkyl(C₇-C₁₆)-carbonyle, cinnamoyle, alcényl(C₂-C₁₂)-carbonyle, alcynyl(C₂-C₁₂)-carbonyle,
alcoxy(C₁-C₁₂)-carbonyle, alcoxy(C₁-C₁₂)-alcoxy(C₁-C₁₂)-carbonyle, aryloxy(C₆-C₁₂)-carbonyle, aralcoxy(C₇-C₁₆)-carbonyle, cycloalcoxy(C₃-C₈)-carbonyle, alcényloxy-(C₃-C₁₂)-carbonyle, alcynyloxy(C₂-C₁₂)-carbonyle,
alkyl(C₁-C₁₂)-carbonyloxy, cycloalkyl(C₃-C₈)-carbonyloxy, aryl(C₆-C₁₂)-carbonyloxy, aralkyl(C₇-C₁₆)-carbonyloxy, cinnamoyloxy, alcényl(C₂-C₁₂)-carbonyloxy, alcynyl(C₂-C₁₂)-carbonyloxy,
alcoxy(C₁-C₁₂)-carbonyloxy, alcoxy(C₁-C₁₂)-alcoxy(C₁-C₁₂)carbonyloxy, aryloxy(C₆-C₁₂)-carbonyloxy, aralkyloxy(C₇-C₁₆)-carbonyloxy, cycloalcoxy(C₃-C₈)-carbonyloxy, alcényloxy(C₂-C₁₂)-carbonyloxy, alcynyloxy(C₂-C₁₂)carbonyloxy,
carbamoyle, N-alkyl(C₁-C₁₂)-carbamoyle, N,N-dialkyl(C₁-C₁₂)-carbamoyle, N-cycloalkyl(C₃-C₈)-carbamoyle, N-aryl(C₆-C₁₆)-carbamoyle, N-aralkyl(C₇-C₁₆)-carbamoyle, N-alkyl(C₁-C₁₀)-N-aryl(C₆-C₁₆)-carbamoyle, N-alkyl(C₁-C₁₀)-N-aralkyl(C₇-C₁₆)-carbamoyle, N-[alcoxy(C₁-C₁₀)alkyl(C₁-C₁₀)]carbamoyle, N-[aryloxy(C₆-C₁₂)-alkyl-(C₁-C₁₀)]carbamoyle, N-[aralkyloxy(C₇-C₁₆)-alkyl-(C₁-C₁₀)]carbamoyle, N-alkyl(C₁-C₁₀)-N-[alcoxy(C₁-C₁₀)-alkyl(C₁-C₁₀)]carbamoyle, N-alkyl(C₁-C₁₀)-N-[aryloxy-(C₆-C₁₆)alkyl(C₁-C₁₀)]carbamoyle, N-alkyl(C₁-C₁₀)-N-[aralkyloxy(C₇-C₁₆)-alkyl(C₁-C₁₀)]carbamoyle, carbamoyloxy, N-alkyl(C₁-C₁₂)-carbamoyloxy, N,N-dialkyl(C₁-C₁₂)-carbamoyloxy, N-cycloalkyl(C₃-C₈)-carbamoyloxy, N-aryl(C₆-C₁₂)-carbamoyloxy, N-aralkyl(C₇-C₁₆)-carbamoyloxy, N-alkyl(C₁-C₁₀)-N-aryl(C₆-C₁₂)-carbamoyloxy, N-alkyl(C₁-C₁₀)-N-aralkyl(C₇-C₁₆)-carbamoyloxy, N-[alkyl(C₁-C₁₀)]carbamoyloxy, N-[aryloxy(C₆-C₁₂)-alkyl(C₁-C₁₀)]carbamoyloxy, N-[aralkyloxy(C₇-C₁₆)-alkyl(C₁-C₁₀)]carbamoyloxy, N-alkyl(C₁-C₁₀)-N-[alcoxy(C₁-C₁₀)-alkyl(C₁-C₁₀)]carbamoyloxy, N-alkyl(C₁-C₁₀)-N-[aryloxy(C₆-C₁₂)alkyl(C₁-C₁₀)]carbamoyloxy, N-alkyl(C₁-C₁₀)-N-[aralkyloxy(C₇-C₁₆)-alkyl(C₁-C₁₀)]carbamoyloxy,
amino, alkyl(C₁-C₁₂)amino, dialkyl(C₁-C₁₂)amino, cycloalkyl(C₃-C₈)amino, alcényl(C₂-C₁₂)amino, alcynyl(C₂-C₁₂)amino, N-aryl(C₆-C₁₂)amino, N-aralkyl(C₇-C₁₁)amino, N-alkyl-aralkylamino, N-alkyl-arylamino, alcoxy(C₁-C₁₂)amino, alcoxy(C₁-C₁₂)-N-alkyl(C₁-C₁₀)amino, alcanoyl(C₁-C₁₂)amino, cycloalcanoyl(C₃-C₈)amino, aroyl(C₆-C₁₂)amino, aralcanoyl(C₇-C₁₆)amino, alcanoyl(C₁-C₁₂)N-alkyl(C₁-C₁₀)amino, cycloalcanoyl(C₃-C₈)-N-alkyl(C₁-C₁₀)amino, aroyl(C₆-C₁₂)-N-alkyl(C₁-C₁₀)amino,
aralcanoyl(C₇-C₁₁)-N-alkyl(C₁-C₁₀)amino,
alcanoyl(C₁-C₁₂)amino-alkyle(C₁-C₈), cycloalcanoyl(C₃-C₈)amino-alkyle(C₁-C₈), aroyl(C₆-C₁₂)amino-alkyle(C₁-C₈), aralcanoyl(C₇-C₁₂)amino-alkyle(C₁-C₈), amino-alkyle en C₁-C₁₀, N-alkyl(C₁-C₁₀)amino-alkyle(C₁-C₁₀), N,N-dialkyl(C₁-C₁₀)amino-alkyle(C₁-C₁₀), cycloalkyl(C₃-C₈)amino-alkyle(C₁-C₁₀),
alkyl(C₁-C₁₂)mercapto, alkyl(C₁-C₁₂)sulfinyle, alkyl(C₁-C₁₂)sulfonyle, aryl(C₆-C₁₆)mercapto, aryl(C₆-C₁₆)sulfinyle, aryl(C₆-C₁₂)sulfonyle, aralkyl(C₇-C₁₆)mercapto, aralkyl(C₇-C₁₆)sulfinyle, aralkyl(C₇-C₁₆)sulfonyle, sulfamoyle, N-alkyl(C₁-C₁₀)sulfamoyle, N,N-dialkyl(C₁-C₁₀)sulfamoyle,
cycloalkyl(C₃-C₈)sulfamoyle,
N-aryl(C₆-C₁₂)sulfamoyle,
N-aralkyl(C₇-C₁₆)sulfamoyle,
N-alkyl(C₁-C₁₀)-N-aryl(C₆-C₁₂)sulfamoyle,
N-alkyl(C₁-C₁₀)-N-aralkyl(C₇-C₁₆)sulfamoyle,
alkyl(C₁-C₁₀)sulfonamido,
N-[alkyl(C₁-C₁₀)]-alkyl(C₁-C₁₀) sulfonamido, aralkyl(C₇-C₁₆)sulfonamido, N-[alkyl(C₁-C₁₀)]-aralkyl(C₇-C₁₆)sulfonamido,
les radicaux contenant un fragment aryle pouvant pour leur part être substitués sur le fragment aryle par 1 à 5 radicaux, identiques ou différents, choisis parmi: des atomes d'halogène et des groupes hydroxy, cyano, trifluorométhyle, nitro, carboxy, alkyle en C₁-C₁₂, cycloalkyle en C₃-C₈, aryle en C₆-C₁₂, aralkyle en C₇-C₁₆, alcoxy en C₁-C₁₂, alcoxy(C₁-C₁₂)-alkyle(C₁-C₁₂), alcoxy(C₁-C₁₂)-alcoxy(C₁-C₁₂), aryloxy en C₆-C₁₂, aralkyloxy en C₇-C₁₆, hydroxyalkyle en C₁-C₈, alkyl(C₁-C₁₂)-carbonyle, cycloalkyl(C₃-C₈)-carbonyle, aryl(C₆-C₁₂)-carbonyle, aralkyloxy(C₇-C₁₆)-carbonyle,
alcoxy(C₁-C₁₂)-carbonyle, alcoxy(C₁-C₁₂)-alcoxy(C₁-C₁₂)carbonyle, aryloxy(C₆-C₁₂)-carbonyle, aralcoxy(C₇-C₁₆)carbonyle, cycloalkyloxy(C₃-C₈)-carbonyle, alcényloxy(C₂-C₁₂)-carbonyle, alcynyloxy(C₂-C₁₂)-carbonyle,
alkyl(C₁-C₁₂)-carbonyloxy, cycloalkyl(C₃-C₈)-carbonyloxy, aryl(C₆-C₁₂)-carbonyloxy, aralkyl(C₇-C₁₆)-carbonyloxy, cinnamoyloxy, alcényl(C₂-C₁₂)-carbonyloxy, alcynyl(C₂-C₁₂)-carbonyloxy,
alcoxy(C₁-C₁₂)-carbonyloxy, alcoxy(C₁-C₁₂)-alcoxy(C₁-C₁₂)carbonyloxy, aryloxy(C₆-C₁₂)-carbonyloxy, aralkyloxy(C₇-C₁₆)-carbonyloxy, cycloalkyloxy(C₃-C₈)-carbonyloxy, alcényloxy(C₂-C₁₂)-carbonyloxy, alcynyloxy(C₂-C₁₂)carbonyloxy,
carbamoyle, N-alkyl(C₁-C₁₂)-carbamoyle, N,N-dialkyl(C₁-C₁₂)-carbamoyle, N-cycloalkyl(C₃-C₈)-carbamoyle, N-aryl(C₆-C₁₂)-carbamoyle, N-aralkyl(C₇-C₁₆)-carbamoyle, N-alkyl(C₁-C₁₀)-N-aryl(C₆-C₁₂)carbamoyle, N-alkyl(C₁-C₁₀)-N-aralkyl(C₇-C₁₆)-carbamoyle, N-[alcoxy(C₁-C₁₀)alkyl(C₁-C₁₀)]carbamoyle, N-[aryloxy(C₆-C₁₂)-alkyl(C₁-C₁₀)]carbamoyle, N-[aralkyloxy(C₇-C₁₆)-alkyl(C₁-C₁₀)]carbamoyle, N-alkyl(C₁-C₁₀)-N-[alcoxy(C₁-C₁₀)alkyl(C₁-C₁₀)]carbamoyle, N-alkyl(C₁-C₁₀)-N-[aryloxy(C₆-C₁₂)-alkyl(C₁-C₁₀)]carbamoyle, N-alkyl(C₁-C₁₀)-N-[aralkyloxy(C₇-C₁₆)-alkyl(C₁-C₁₀)]carbamoyle,
carbamoyloxy, N-alkyl(C₁-C₁₂)-carbamoyloxy, N,N-dialkyl(C₁-C₁₂)-carbamoyloxy, N-cycloalkyl(C₃-C₈)carbamoyloxy, N-aryl(C₆-C₁₂)-carbamoyloxy, N-aralkyl(C₇-C₁₆)-carbamoyloxy, N-alkyl(C₁-C₁₀)-N-aryl(C₆-C₁₂)-carbamoyloxy,
N-alkyl(C₁-C₁₀)-N-aralkyl(C₇-C₁₆)-carbamoyloxy, N-[alkyl(C₁-C₁₀)]carbamoyloxy, N-[aryloxy(C₆-C₁₂)-alkyl(C₁-C₁₀)]carbamoyloxy, N-[aralkyloxy(C₇-C₁₆)-alkyl(C₁-C₁₀)]carbamoyloxy, N-alkyl(C₁-C₁₀)-N-[alcoxy(C₁-C₁₀)-alkyl(C₁-C₁₀)]carbamoyloxy, N-alkyl(C₁-C₁₀)-N-[aryloxy(C₆-C₁₂)-alkyl(C₁-C₁₀)]carbamoyloxy, N-alkyl(C₁-C₁₀)-N-[aralkyloxy(C₇-C₁₆)-alkyl(C₁-C₁₀)]carbamoyloxy,
amino, alkyl(C₁-C₁₂)amino, dialkyl(C₁-C₁₂)amino, cycloalkyl(C₃-C₈)amino, alcényl(C₃-C₁₂)amino, alcynyl(C₃-C₁₂)amino, N-aryl(C₆-C₁₂)amino, N-aralkyl(C₇-C₁₁)amino, N-alkyl-aralkylamino, N-alkyl-arylamino, alcoxy(C₁-C₁₂)amino, alcoxy(C₁-C₁₂)-N-alkyl(C₁-C₁₀)amino,
alcanoyl(C₁-C₁₂)amino, cycloalcanoyl(C₃-C₈)amino, aroyl(C₆-C₁₂)amino, aralcanoyl(C₇-C₁₆)amino, alcanoyl(C₁-C₁₂)-N-alkyl(C₁-C₁₀)amino, cycloalcanoyl(C₃-C₈)-N-alkyl(C₁-C₁₀)amino, aroyl(C₆-C₁₂)-N-alkyl(C₁-C₁₀)amino,
aralcanoyl(C₇-C₁₁)-N-alkyl(C₁-C₁₀)amino,
alcanoyl(C₁-C₁₂)amino-alkyle(C₁-C₈), cycloalcanoyl(C₃-C₈)amino-alkyle(C₁-C₈), aroyl(C₆-C₁₂)amino-alkyle(C₁-C₈), aralcanoyl(C₇-C₁₆)amino-alkyle(C₁-C₈), amino-alkyle en C₁-C₁₀, N-alkyl(C₁-C₁₀)amino-alkyle(C₁-C₁₀), N,N-dialkyl(C₁-C₁₀)amino-alkyle(C₁-C₁₀), cycloalkyl(C₃-C₈)amino-alkyle(C₁-C₁₀),
alkyl(C₁-C₁₂)mercapto, alkyl(C₁-C₁₂)sulfinyle, alkyl(C₁-C₁₂)sulfonyle, aryl(C₆-C₁₂)mercapto, aryl(C₆-C₁₂)sulfinyle, aryl(C₆-C₁₂)sulfonyle, aralkyl(C₇-C₁₆)mercapto, aralkyl(C₇-C₁₆)sulfinyle, aralkyl(C₇-C₁₆)sulfonyle,
R² représente un atome d'hydrogène ou d'halogène ou un groupe alkyle en C₁-C₂₀, alcényle en C₂-C₂₀, alcynyle en C₂-C₂₀, alcoxy en C₁-C₂₀, alcényloxy en C₂-C₂₀, alcynyloxy en C₂-C₂₀, rétinyloxy, alcoxy(C₁-C₂₀)-alkyle(C₁-C₃), alcényloxy(C₂-C₂₀)-alkyle(C₁-C₃), rétinyloxy-alkyle(C₁-C₃), alcynyloxy(C₂-C₂₀)-alkyle(C₁-C₃), cyano, trifluorométhyle, hydroxyalkyle en C₁-C₈, alcanoyle en C₁-C₂₀, aralcanoyle en C₇-C₁₆, aroyle en C₆-C₁₂, aryle en C₆-C₁₂, aralkyle en C₇-C₁₆, -O-[CH₂]ₓC_{f}H_{(2f+1-g)}F_{g}, NR'R'', alkyl(C₁-C₁₀)mercapto, alkyl(C₁-C₁₀)sulfinyle, alkyl(C₁-C₁₀)sulfonyle, aryl(C₆-C₁₂)mercapto, aryl(C₆-C₁₂)sulfinyle, aryl(C₆-C₁₂)sulfonyle, aralkyl(C₇-C₁₂)mercapto, aralkyl(C₇-C₁₂)sulfinyle, aralkyl(C₇-C₁₂)sulfonyle, aryloxy en C₆-C₁₂, aralkyloxy en C₇-C₁₆, carboxy, alcoxy(C₁-C₂₀)carbonyle, alcoxy(C₁-C₁₂)alcoxy(C₁-C₁₂)carbonyle, aryloxy(C₆-C₁₂)carbonyle, aralcoxy(C₇-C₁₆)-carbonyle, cycloalkyloxy(C₃-C₈)-carbonyle, alcényloxy(C₂-C₂₀)-carbonyle, rétinyloxycarbonyle, alcynyloxy(C₂-C₂₀)-carbonyle, cycloalkyl(C₃-C₈)-alcoxy(C₁-C₆)carbonyle, cycloalkyloxy(C₃-C₈)-alcoxy(C₁-C₆)carbonyle, aryloxy(C₆-C₁₂)-alcoxy(C₁-C₆)carbonyle,
aralcoxy(C₇-C₁₆)-alcoxy(C₁-C₆)carbonyle, carbamoyle, N-alkyl(C₁-C₁₂)-carbamoyle, N,N-dialkyl(C₁-C₁₂)carbamoyle, N-cycloalkyl(C₃-C₈)-carbamoyle, N,N-dicycloalkyl(C₃-C₈)-carbamoyle, N-alkyl(C₁-C₁₀)-N-cycloalkyl(C₃-C₈)-carbamoyle, N-[cycloalkyl(C₃-C₈)-alkyl(C₁-C₆)]-carbamoyle, N-alkyl(C₁-C₆)-N-[cycloalkyl(C₃-C₈)-alkyl(C₁-C₆)]carbamoyle, N-(+)-déhydroabiétylcarbamoyle, N-alkyl(C₁-C₆)-N-(+)-déhydroabiétylcarbamoyie, N-aryl(C₆-C₁₂)-carbamoyle, N-aralkyl(C₇-C₁₆)-carbamoyle, N-alkyl(C₁-C₁₀)-N-aryl(C₆-C₁₆)-carbamoyle, N-alkyl(C₁-C₁₀)-N-aralkyl(C₇-C₁₆)-carbamoyle, N-[alcoxy(C₁-C₁₂)alkyl(C₁-C₁₀)]carbamoyle, N-[aryloxy(C₆-C₁₆)-alkyl(C₁-C₁₀)]carbamoyle, N-[aralkyloxy(C₇-C₁₆)-alkyl(C₁-C₁₀)]carbamoyle, N-alkyl(C₁-C₁₀)-N-[alcoxy(C₁-C₁₀)alkyl(C₁-C₁₀)]carbamoyle, N-alkyl(C₁-C₁₀)-N-[aryloxy(C₆-C₁₂)-alkyl(C₁-C₁₀)]carbamoyle, N-alkyl(C₁-c₁₀)-N-[aralkyloxy(C₇-C₁₆)-alkyl(C₁-C₁₀)]carbamoyle, un groupe CON(CH₂)ₕ dans lequel un groupe CH₂ peut être remplacé par O, S ou par un groupe N-alkyl(C₁-C₈)imino, N-cycloalkyl(C₃-C₈)imino, N-cycloalkyl(C₃-C₈)-alkyl(C₁-C₄)imino, N-aryl(C₆-C₁₂)imino, N-aralkyl(C₇-C₁₆)imino ou N-alcoxy(C₁-C₄)-alkyl(C₁-C₆)imino, et h va de 3 à 7,
le fragment aryle étant substitué comme défini pour R¹ et R³,
R¹ et R³ sont identiques ou différents et représentent un atome d'hydrogène ou d'halogène ou un groupe alkyle en C₁-C₁₂, alcoxy en C₁-C₁₂, -O-[CH₂]ₓC_{f}H_{(2f+1-g)}Hal_{g}, alcoxy(C₁-C₁₂)-alkyle(C₁-C₁₂), alcoxy(C₁-C₈)-alcoxy(C₁-C₁₂), alcoxy(C₁-C₁₂)-alcoxy(C₁-C₈)-alkyle(C₂-C₆), aralkyloxy en C₇-C₁₁, cycloalkyle en C₃-C₈, cycloalkyl(C₃-C₈)-alkyle(C₁-C₈), cycloalkyloxy en C₃-C₈, cycloalkyl(C₃-C₈)-alcoxy(C₁-C₈), cycloalkyloxy(C₃-C₈)-alkyle(C₁-C₈), cycloalkyloxy(C₃-C₈)-alcoxy(C₁-C₈), cycloalkyl(C₃-C₈)-alkyl(C₁-C₆)-alcoxy(C₁-C₆), cycloalkyl(C₃-C₈)alcoxy(C₁-C₆)-alkyle(C₁-C₆), cycloalkyloxy(C₃-C₈)-alcoxy(C₁-C₆)-alkyle(C₁-C₆), NR^{Y}R^{Z}, alkyl(C₁-C₈)mercapto, alkyl(C₁-C₈)sulfinyle ou alkyl(C₁-C₈)sulfonyle, aryl(C₆-C₁₂)mercapto, aryl(C₆-C₁₂)sulfinyle, aryl(C₆-C₁₂)sulfonyle, aralkyl(C₇-C₁₂)mercapto, aralkyl(C₇-C₁₁)sulfinyle, aralkyl(C₇-C₁₁)sulfonyle, aryloxy(C₆-C₁₂)alkyle(C₁-C₆) substitué, aralcoxy(C₇-C₁₁)-alkyle(C₁-C₆), aryloxy(C₆-C₁₂)-alcoxy(C₁-C₆)-alkyle(C₁-C₆), aralkyloxy(C₇-C₁₁)-alcoxy(C₁-C₆)-alkyle(C₁-C₆), aryloxy en C₆-C₁₂, aralkyloxy en C₇-C₁₁, aryloxy(C₆-C₁₂)-alcoxy(C₁-C₆) ou aralcoxy(C₇-C₁₁)-alcoxy(C₁-C₆), un radical aromatique portant 1, 2, 3, 4 ou 5 substituants, identiques ou différents, choisis parmi des atomes d'hydrogène ou d'halogène et des groupes cyano, nitro, trifluorométhyle, alkyle en C₁-C₁₆, alcényle en C₁-C₁₆, hydroxyalkyle en C₁-C₆, alcoxy en C₁-C₁₆, alcényloxy en C₁-C₁₆,
-O-[CH₂]ₓ-C_{f}H_{(2f+1-g)}F_{g}, -OCF₂Cl, -O-CF₂-CHFCl, alkyl(C₁-C₆)mercapto, alkyl(C₁-C₆)sulfinyle, alkyl(C₁-C₆)sulfonyle, alkyl(C₁-C₆)carbonyle, alcoxy(C₁-C₆)carbonyle, carbamoyle, N-alkyl(C₁-C₄)carbamoyle, N,N-dialkyl(C₁-C₄)carbamoyle, alkyl(C₁-C₆)-carbonyloxy, cycloalkyl(C₃-C₈)carbamoyle, phényle, benzyle, phénoxy, benzyloxy, NR^{Y}R^{Z}, phénylmercapto, phénylsulfonyle, phénylsulfinyle, sulfamoyle, N-alkyl(C₁-C₄)-sulfamoyle ou N,N-dialkyl(C₁-C₄)sulfamoyle, ou portant éventuellement jusqu'à trois des substituants mentionnés précédemment, identiques ou différents, et deux atomes de carbone contigus du radical aralkyloxy portant ensemble une chaîne -[CH₂-] et/ou -CH=CH-CH=CH-, un groupe CH₂ de la chaîne étant éventuellement remplacé par O, S, SO, SO₂ ou NR',
R¹ et R² ou R² et R³ forment une chaîne [CH₂]ₒ, o étant égal à 3, 4 ou 5, ou
conjointement avec le reste pyridine ou pyridazine qui les porte, forment un cycle cinnoline, quinoléine ou isoquinoléine,
R⁴, lorsque Q est une liaison, représente un atome de fluor, de chlore ou de brome, ou, lorque Q est O, représente un radical alkyle en C₁-C₂₀ ramifié ou non ramifié, qui peut comporter jusqu'à trois liaisons multiples C-C, un radical fluoroalkyle saturé non substitué, de formule [CH₂]ₓC_{f}H_{(2f+1-g)}F_{g}, un radical aryle en C₆-C₁₆ ou un radical aralkyle en C₆-C₁₆, qui peut comporter jusqu'à deux liaisons multiples C-C dans la chaîne alkyle, ou un radical hétéroaryle ou un radical hétéroarylalkyle, ces radicaux étant substitues par un ou plusieurs substituants choisis parmi des atomes de fluor et de chlore et des groupes hydroxy, cyano, trifluorométhyle, carboxy, alkyle en C₁-C₁₂, cycloalkyle en C₃-C₈, cycloalkyl-(C₃-C₈)-alkyle(C₁-C₁₂), cycloalcoxy en C₃-C₈, cycloalkyl-(C₃-C₈)-alcoxy(C₁-C₁₂), cycloalkyloxy (C₃-C₈)-alkyle-(C₁-C₁₂), cycloalkyloxy(C₃-C₈)-alcoxy (C₁-C₁₂), cycloalkyl(C₃-C₈)-alkyl-(C₁-C₈)-alcoxy (C₁-C₆), cycloalkyloxy(C₃-C₈)-alcoxy(C₁-C₈)alcoxy(C₁-C₈), aryle en C₆-C₁₂, aralkyle en C₇-C₁₆, alcényle en C₂-C₁₂, alcynyle en C₂-C₁₂, alcoxy en C₁-C₁₂, alcoxy(C₁-C₁₂)-alcoxy(C₁-C₁₂), alcoxy(C₁-C₁₂)-alcoxy(C₁-C₈)-alkyle(C₁-C₈), aryloxy en C₆-C₁₂, aralkyloxy en C₇-C₁₆, aryloxy(C₆-C₁₂)-alcoxy(C₁-C₆), aralcoxy(C₇-C₁₆)alcoxy(C₁-C₆), hydroxyalkyle en C₁-C₈,
-O-[CH₂]ₓC_{f}H_{(2f+1-g)}F_{g}, alkyl(C₁-C₁₂)-carbonyle, cycloalkyl(C₃-C₈)-carbonyle, aryl(C₆-C₁₂)-carbonyle, aralkyl(C₇-C₁₆)-carbonyle,
alcoxy(C₁-C₁₂)-carbonyle, alcoxy(C₁-C₁₂)-alcoxy(C₁-C₁₂)carbonyle, aryloxy(C₆-C₁₂)-carbonyle, aralcoxy(C₇-C₁₆)carbonyle, cycloalkyloxy(C₃-C₈)-carbonyle, alcényloxy(C₂-C₁₂)-carbonyle, alcynyloxy(C₂-C₁₂)-carbonyle, cycloalkyl(C₃-C₈)-alcoxy(C₁-C₆)-carbonyle,
alkyl(C₁-C₁₂)-carbonyloxy, cycloalkyl(C₃-C₈)-carbonyloxy, aryl(C₆-C₁₂)-carbonyloxy, aralkyl(C₇-C₁₆)-carbonyloxy,
carbamoyle, N-alkyl(C₁-C₁₂)-carbamoyle, N,N-dialkyl(C₁-C₁₂)-carbamoyle, N-cycloalkyl(C₃-C₈)-carbamoyle, N,N-dicycloalkyl(C₃-C₈)-carbamoyle, N-alkyl(C₁-C₁₀)-N-cycloalkyl(C₃-C₈)-carbamoyle, N-[cycloalkyl(C₃-C₈)-alkyl(C₁-C₆)]carbamoyle, N-alkyl(C₁-C₆)-N-[cycloalkyl(C₃-C₈)alkyl(C₁-C₆)]carbamoyle, N-(+)-déhydroabiétylcarbamoyle, N-alkyl(C₁-C₆)-N-(+)-déhydroabiétylcarbamoyle, N-aryl(C₆-C₁₂)-carbamoyle, N-aralkyl(C₇-C₁₆)-carbamoyle, N-alkyl(C₁-C₁₀)-N-aryl(C₆-C₁₆)-carbamoyle, N-alkyl(C₁-C₁₀)-N-aralkyl(C₇-C₁₆)-carbamoyle, N-[alcoxy(C₁-C₁₀)alkyl(C₁-C₁₀)]carbamoyle, N-[aryloxy(C₆-C₁₆)-alkyl(C₁-C₁₀)]carbamoyle, N-[aralkyloxy(C₇-C₁₆)-alkyl(C₁-C₁₀)]carbamoyle, CON(CH₂)ₕ, dans lequel un groupe CH₂ peut être remplacé par O ou par un groupe N-alkyl(C₁-C₈)imino, N-cycloalkyl(C₃-C₈)imino, N-cycloalkyl(C₃-C₈)alkyl(C₁-C₄)imino, N-aryl(C₆-C₁₂)imino, ou N-aralkyl(C₇-C₁₆)imino, et h va de 3 à 6,
les radicaux qui contiennent un fragment aryle pouvant pour leur part être substitues sur le fragment aryle par 1 à 5 substituants, identiques ou différents, choisis parmi:
des atomes de fluor et de chlore et des groupes hydroxy, cyano, trifluorométhyle, carboxy, alkyle en C₁-C₁₂, cycloalkyle en C₃-C₈, alcoxy en C₁-C₆, cycloalkyloxy en C₃-C₈, alcoxy(C₁-C₁₂)-carbonyle,
N-alkyl(C₁-C₆)-carbamoyle, N,N-dialkyl(C₁-C₆)-carbamoyle, N-cycloalkyl(C₃-C₈)-carbamoyle et
R⁴ représente R'', lorsque Q a la signification de NR', R' et R'' étant identiques ou différents et représentant un atome d'hydrogène ou un radical alkyle en C₁-C₈ ou un radical aralkyle en C₇-C₁₁ éventuellement monosubstitué par un atome de fluor ou de chlore ou par un groupe alcoxy en C₁-C₄,
R^{Y} et R^{Z} sont identiques ou différents et représentent un atome d'hydrogène ou un groupe aryle en C₆-C₁₂, alkyle en C₁-C₁₀, cycloalkyle en C₃-C₁₀, alcoxy(C₁-C₈)-alkyle(C₁-C₈), aralcoxy(C₇-C₁₂)-alkyle(C₁-C₈), aryloxy(C₆-C₁₂)alkyle(C₁-C₈), alcanoyle en C₁-C₁₀, un groupe aralcanoyle en C₇-C₁₆ éventuellement substitué ou un groupe aroyle en C₆-C₁₂ éventuellement substitué, ou
R^{Y} et R^{Z} représentent ensemble un groupement -[CH₂]ₕ, dans lequel un groupe CH₂ peut être remplacé par O, S ou par un groupe N-alcanoyl(C₁-C₄)imino ou N-alcoxy(C₁-C₄)carbonylimino, et
f va de 1 à 8,
g est égal à O ou va de 1 à (2f + 1),
h va de 3 à 6,
x va de 0 à 3 et
n est 3 ou 4,
y compris les sels physiologiquement actifs,
à l'exclusion du 3-benzyloxypyridine-2-(L-thréonylméthylester)carboxamide, du 3-benzyloxypyridine-2-[L-thréonyl-(Fmoc-Phg)-tert-butylester]carboxamide, du 3-benzyloxypyridine-2-(L-thréonyl-tert-butylester)carboxamide, du 3-benzyloxypyridine-2-(D-allothréonylméthylester)carboxamide et de l'ester éthylique de 3-benzyloxypicolinylglycine.

7. Composés de formule I selon les revendications 1 à 3 et 6, dans lesquels
Q représente O, NR' ou une liaison,
X représente O,
Y représente CR³ ou, lorsque R¹ et R² forment un cycle,
représente N ou CR³,
m est égal à 0,
A représente un radical alkylène en C₁-C₃ qui est éventuellement monosubstitué par un atome d'halogène ou par un groupe cyano, trifluorométhyle, alkyle en C₁-C₆, hydroxyalkyle en C₁-C₆, alcoxy en C₁-C₆, -O-[CH₂]ₓ-C_{f}H_{(2f+1-g)}F_{g} ou
A représente -CHR⁵-, R⁵ représentant l'un des substituants de l'atome de carbone α d'un α-aminoacide, en particulier d'un L-aminoacide naturel et de son isomère D,
B représente -CO₂G,
G représentant un radical alkyle en C₁-C₂₀ aliphatique ramifié ou non ramifié ou cyclique, le radical rétinyle ou
un radical alcényle en C₂-C₂₀ ramifié ou non ramifié ou un radical alcynyle en C₂-C₂₀, pouvant comporter chacun une ou plusieurs liaisons multiples C-C, ou
un radical aryle en C₆-C₁₂, un radical aralkyle en C₇-C₁₁ ou un radical hétéroaryle ou hétéroaralkyle,
les radicaux précédents pouvant porter un ou deux substituants choisis parmi des atomes de fluor ou de chlore et des groupes alkyle en C₁-C₈, cycloalkyle en C₃-C₈, hydroxy, alcoxy en C₁-C₆, alcoxy(C₁-C₆)-alcoxy(C₁-C₆), aryloxy en C₆-C₁₂, aralkyloxy en C₇-C₁₂,
alkyl(C₁-C₈)-carbonyle, cycloalkyl(C₃-C₈)-carbonyle, aryl(C₆-C₁₂)-carbonyle, aralkyl(C₇-C₁₂)-carbonyle, alcoxy(C₁-C₈)-carbonyle, alcoxy(C₁-C₆)-alcoxy(C₁-C₆)-carbonyle, aryloxy(C₆-C₁₂)-carbonyle, aralcoxy(C₇-C₁₂)carbonyle, cycloalkyloxy(C₃-C₈)-carbonyle,
alkyl(C₁-C₂₀)-carbonyloxy, cycloalkyl(C₃-C₈)-carbonyloxy, aryl(C₆-C₁₂)-carbonyloxy, aralkyl(C₇-C₁₂)-carbonyloxy, alcoxy(C₁-C₈)-carbonyloxy, alcoxy(C₁-C₆)-alcoxy(C₁-C₆)carbonyloxy, aryloxy(C₆-C₁₂)-carbonyloxy, aralkyloxy(C₇-C₁₂)-carbonyloxy, cycloalkyloxy(C₃-C₈)-carbonyloxy, carbamoyle, N-alkyl(C₁-C₈)-carbamoyle, N,N-dialkyl(C₁-C₈)-carbamoyle, N-cycloalkyl(C₃-C₈)-carbamoyle, N-[alcoxy(C₁-C₆)-alkyl(C₁-C₆)]carbamoyle,
amino, alkyl(C₁-C₆)amino, dialkyl(C₁-C₆)amino, cycloalkyl(C₃-C₈)amino, N-aryl(C₆-C₁₂)amino, N-aralkyl(C₇-C₁₁)amino, N-alkyl(C₁-C₅)-aryl(C₆-C₁₂)amino,
alcanoyl(C₁-C₈)amino, cycloalcanoyl(C₃-C₈)amino, aroyl(C₆-C₁₂)amino, aralcanoyl(C₇-C₁₂)amino, alcanoyl(C₁-C₈)N-alkyl(C₁-C₆)amino, cycloalcanoyl(C₃-C₈)-N-alkyl(C₁-C₆)amino, aroyl(C₆-C₁₂)-N-alkyl(C₁-C₆)amino, aralcanoyl(C₇-C₁₁)-N-alkyl(C₁-C₆)amino, et
les radicaux qui comportent un fragment aryle étant en particulier substitués par jusqu'à 3 substituants choisis parmi
des atomes de fluor ou de chlore et des groupes hydroxy, cyano, trifluorométhyle, alkyle en C₁-C₆, cycloalkyle en C₃-C₈, alcoxy en C₁-C₈,
alkyl(C₁-C₆)-carbonyle, cycloalkyl(C₃-C₈)-carbonyle, alcoxy(C₁-C₆)-carbonyle,cycloalkyloxy (C₃-C₈)-carbonyle, alkyl(C₁-C₆)-carbonyloxy, cycloalkyl(C₃-C₈)-carbonyloxy, alcoxy(C₁-C₆)-carbonyloxy, cycloalkyloxy(C₃-C₈)-carbamoyloxy,
carbamoyle, N-alkyl(C₁-C₆)-carbamoyle, N,N-dialkyl(C₁-C₆)-carbamoyle, N-cycloalkyl(C₃-C₈)-carbamoyle, N-[alcoxy(C₁-C₆)-alkyl(C₁-C₆)]carbamoyle,
N-alkyl(C₁-C₆)-N-[alcoxy(C₁-C₆)-alkyl(C₁-C₆)]carbamoyle, carbamoyloxy, N-alkyl(C₁-C₆)-carbamoyloxy, N,N-dialkyl(C₁-C₆)-carbamoyloxy, N-cycloalkyl(C₃-C₈)-carbamoyloxy, alcanoyl(C₁-C₆)amino, cycloalcanoyl(C₃-C₈)amino, alkyl(C₁-C₆)mercapto, alkyl(C₁-C₆)sulfinyle, alkyl(C₁-C₆)sulfonyle, et
R² représente un atome d'hydrogène ou un groupe alkyle en C₁-C₂₀, alcényle en C₂-C₂₀, alcényloxy en C₂-C₂₀, alcynyloxy en C₂-C₂₀, rétinyloxy, alcoxy(C₁-C₂₀)-alkyle(C₁-C₃), alcoxy(C₁-C₂₀)-alkyle(C₁-C₃), alcényloxy(C₂-C₂₀)-alkyle(C₁-C₃), rétinyloxy-alkyle(C₁-C₃), alcynyloxy(C₂-C₂₀)-alkyle(C₁-C₃), alcoxy en C₁-C₂₀, un atome d'halogène, un groupe cyano, trifluorométhyle, hydroxyalkyle en C₁-C₁₆, alcanoyle en C₁-C₂₀, aralcanoyle en C₇-C₁₂, aroyle en C₆-C₁₂, -O-[CH₂]ₓC_{f}H_{(2f+1-g)}F_{g}, NR'R'', alkyl(C₁-C₁₀)mercapto, alkyl(C₁-C₁₀)sulfinyle, alkyl(C₁-C₁₀)sulfonyle, aryl(C₆-C₁₂)mercapto, aryl(C₆-C₁₂)sulfinyle, aryl(C₆-C₁₂)sulfonyle, aralkyl(C₇-C₁₂)mercapto, aralkyl(C₇-C₁₂)sulfinyle, aralkyl(C₇-C₁₂)sulfonyle, aryloxy en C₆-C₁₂, aralkyloxy en C₇-C₁₆, carboxy, alcoxy(C₁-C₂₀)-carbonyle, alcoxy(C₁-C₁₂)-alcoxy(C₁-C₁₂)-carbonyle, aryloxy(C₆-C₁₂)carbonyle, aralcoxy(C₇-C₁₆)-carbonyle, cycloalkyloxy(C₃-C₈)-carbonyle, alcényloxy(C₂-C₂₀)-carbonyle, rétinyloxycarbonyle, alcynyloxy(C₂-C₂₀)-carbonyle, cycloalkyl(C₃-C₈)-alcoxy(C₁-C₆)-carbonyle, cycloalcoxy(C₃-C₈)-alcoxy(C₁-C₆)-carbonyle, aryloxy(C₆-C₁₂)-alcoxy(C₁-C₆)-carbonyle, aralcoxy(C₇-C₁₆)-alcoxy(C₁-C₆)carbonyle, carbamoyle, N-alkyl(C₁-C₁₂)-carbamoyle, N,N-dialkyl(C₁-C₁₂)-carbamoyle, N-cycloalkyl(C₃-C₈)carbamoyle, N,N-dicycloalkyl(C₃-C₈)-carbamoyle, N-alkyl(C₁-C₁₀)-N-cycloalkyl(C₃-C₈)-carbamoyle, N-[cycloalkyl(C₃-C₈)-alkyl(C₁-C₆)]carbamoyle, N-alkyl(C₁-C₆)-N-[cycloalkyl(C₃-C₈)-alkyl(C₁-C₆)]carbamoyle, N-(+)-déhydroabiétylcarbamoyle, N-alkyl(C₁-C₆)-N-(+)-déhydroabiétylcarbamoyle, N-aryl(C₆-C₁₂)-carbamoyle, N-aralkyl(C₇-C₁₆)-carbamoyle, N-alkyl(C₁-C₁₀)-N-aryl(C₆-C₁₆)carbamoyle, N-alkyl(C₁-C₁₀)-N-aralkyl(C₇-C₁₂)-carbamoyle, N-[alcoxy(C₁-C₁₂)-alkyl(C₁-C₁₀)]carbamoyle, N-[aryloxy(C₆-C₁₆)-alkyl(C₁-C₁₀)]carbamoyle, N-[aralkyloxy(C₇-C₁₆)alkyl(C₁-C₁₀)]carbamoyle, N-alkyl(C₁-C₁₀)-N-[alcoxy(C₁-C₁₀)-alkyl(C₁-C₁₀)]carbamoyle, N-alkyl(C₁-C₁₀)-N[aryloxy(C₆-C₁₂)-alkyl(C₁-C₁₀)]carbamoyle, N-alkyl(C₁-C₁₀)-N-[aralkyloxy(C₇-C₁₆)-alkyl(C₁-C₁₀)]carbamoyle, CON(CH₂)ₕ dans lequel un groupe CH₂ peut être remplacé par O, S ou par un groupe N-alkyl(C₁-C₈)imino, N-cycloalkyl(C₃-C₈)imino, N-cycloalkyl(C₃-C₈)-alkyl(C₁-C₄)imino, N-aryl(C₆-C₁₂)imino, N-aralkyl(C₇-C₁₆)imino ou N-alcoxy(C₁-C₄)-alkyl(C₁-C₆)imino, et h va de 3 à 6,
le fragment aryle étant substitué comme défini pour R¹ et R³,
R¹ et R³ sont identiques ou différents et représentent un atome d'hydrogène ou d'halogène ou un groupe alkyle en C₁-C₁₂, alcoxy en C₁-C₁₂, -O-[CH₂]ₓC_{f}H_{(2f+1-g)}Hal_{g}, alcoxy(C₁-C₁₂)-alkyle-C₁-C₁₂, alcoxy(C₁-C₈)-alcoxy(C₁-C₁₂), alcoxy(C₁-C₁₂)-alcoxy(C₁-C₈)-alkyle(C₂-C₆), aralkyloxy en C₇-C₁₁, cycloalkyle en C₃-C₈, cycloalkyl(C₃-C₈)-alkyle(C₁-C₈), cycloalkyloxy en C₃-C₈, cycloalkyl(C₃-C₈)-alcoxy(C₁-C₈), cycloalkyloxy(C₃-C₈)-alkyle(C₁-C₈), cycloalkyloxy(C₃-C₈)-alcoxy(C₁-C₈), cycloalkyl(C₃-C₈)-alkyl(C₁-C₆)-alcoxy(C₁-C₆), cycloalkyl(C₃-C₈)alcoxy(C₁-C₆)-alkyle(C₁-C₆), cycloalkyloxy (C₃-C₈)-alcoxy(C₁-C₆)-alkyle(C₁-C₆), NR^{Y}R^{Z}, alkyl(C₁-C₈)mercapto, alkyl(C₁-C₈)sulfinyle ou alkyl(C₁-C₈)sulfonyle, aryl(C₆-C₁₂)mercapto, aryl(C₆-C₁₂)sulfinyle, aryl(C₆-C₁₂)sulfonyle, aralkyl(C₇-C₁₂)mercapto, aralkyl(C₇-C₁₁)sulfinyle, aralkyl(C₇-C₁₁)sulfonyle, aryloxy(C₆-C₁₂)alkyle(C₁-C₆) substitué, aralcoxy(C₇-C₁₁)-alkyle(C₁-C₆), aryloxy(C₆-C₁₂)-alcoxy(C₁-C₆)-alkyle(C₁-C₆), aralkyloxy(C₇-C₁₁)-alcoxy(C₁-C₆)-alkyle(C₁-C₆), aryloxy en C₆-C₁₂, aralkyloxy en C₇-C₁₁, aryloxy(C₆-C₁₂)-alcoxy(C₁-C₆) ou aralcoxy(C₇-C₁₁)-alcoxy(C₁-C₆), un radical aromatique portant 1, 2, 3, 4 ou 5 substituants, identiques ou différents, choisis parmi des atomes d'hydrogène ou d'halogène et des groupes cyano, nitro, trifluorométhyle, alkyle en C₁-C₁₂, alcényle en C₁-C₁₂, hydroxyalkyle en C₁-C₆, alcoxy en C₁-C₁₂, alcényloxy en C₁-C₁₂,
-O-[CH₂]ₓC_{f}H_{(2f+1-g)}F_{g}, -OCF₂Cl, -O-CF₂-CHFCl, alkyl(C₁-C₆)mercapto, alkyl(C₁-C₆)sulfinyle, alkyl(C₁-C₆)sulfonyle, alkyl(C₁-C₆)carbonyle, alcoxy(C₁-C₆)carbonyle, carbamoyle, N-alkyl(C₁-C₄)carbamoyle, N,N-dialkyl(C₁-C₄)carbamoyle, alkyl(C₁-C₆)carbonyloxy, cycloalkyl(C₃-C₈)carbamoyle, phényle, benzyle, phénoxy, benzyloxy, NR^{Y}R^{Z}, phénylmercapto, phénylsulfonyle, phénylsulfinyle, sulfamoyle, N-alkyl(C₁-C₄)-sulfamoyle, N,N-dialkyl(C₁-C₄)sulfamoyle, ou portant éventuellement jusqu'à trois des substituants mentionnés précédemment, identiques ou différents, et deux atomes de carbone contigus du radical aralkyloxy portant ensemble une chaîne -[CH₂-] et/ou -CH=CH-CH=CH-, un groupe CH₂ de la chaîne étant éventuellement remplacé par O, S, SO, SO₂ ou NR^{Y},
R¹ et R² ou R² et R³ peuvent former une chaîne [CH₂]ₒ, o étant égal à 3, 4 ou 5, et
R⁴, lorsque Q est une liaison, représente un atome de chlore ou
lorsque Q est O, représente un radical alkyle en C₁-C₁₀ ramifié ou non ramifié, qui peut comporter une ou deux liaisons multiples C-C, ou un radical fluoroalkyle non substitué de formule -[CH₂]ₓC_{f}H_{(2f+1-g)}F_{g}, ou un radical alcoxy(C₁-C₈)-alkyle(C₁-C₆), alcoxy(C₁-C₆)-alcoxy(C₁-C₄)alkyle(C₁-C₄) ou un radical de formule Z,
-[CH₂]ᵥ-[O]_{w}-[CE₂]ₜ-E (Z)
dans laquelle E représente un radical phényle substitué de formule F 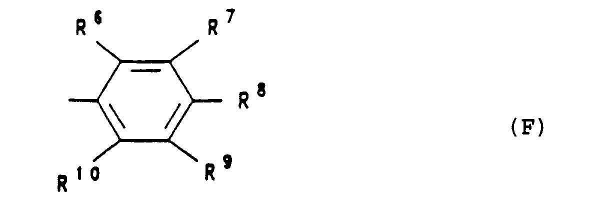 ou un radical cycloalkyle en C₃-C₈,
v représentant 0, 1, 2, 3, 4, 5, 6, w représentant 0 ou 1 et t représentant 0, 1, 2, 3, avec la restriction que v est différent de 0 lorsque w = 1, et R⁶, R⁷, R⁸ , R⁹ et R¹⁰ étant identiques ou différents et représentant un atome d'hydrogène ou d'halogène ou un groupe cyano, nitro, trifluorométhyle, alkyle en C₁-C₆, cycloalkyle en C₃-C₈, alcoxy en C₁-C₆, -O-[CH₂]ₓC_{f}H_{(2f+1-g)}F_{g}, -OCF₂Cl, -O-CF₂-CHFCl, alkylmercapto en C₁-C₆, hydroxyalkyle en C₁-C₆, alcoxy(C₁-C₆)-alcoxy(C₁-C₆), alcoxy(C₁-C₆)-alkyle(C₁-C₆), alkyl(C₁-C₆)sulfinyle, alkyl(C₁-C₆)sulfonyle, alkyl(C₁-C₆)-carbonyle, alcoxy(C₁-C₈)-carbonyle, carbamoyle, N-alkyl(C₁-C₈)-carbamoyle, N,N-dialkyl(C₁-C₈)carbamoyle, un groupe aralkyl (C₇-C₁₁)-carbamoyle éventuellement substitué par un atome de fluor, de chlore ou de brome ou par un radical trifluorométhyle ou alcoxy en C₁-C₆, un groupe cycloalkyl(C₃-C₈)-carbamoyle, N-cycloalkyl(C₃-C₈)-alkyl(C₁-C₄)-carbamoyle, alkyl(C₁-C₆)carbonyloxy, phényle, benzyle, phénoxy, benzyloxy, un groupe NR^{Y}R^{Z}, tel qu'amino, anilino, N-méthylanilino, phénylmercapto, phénylsulfonyle, phénylsulfinyle, sulfamoyle, N-alkyl(C₁-C₈)sulfamoyle ou N,N-dialkyl(C₁-C₈)sulfamoyle, ou deux substituants contigus représentant ensemble une chaîne -[CH₂]ₙ ou -CH=CH-CH=CH-, un groupe CH₂ de la chaîne étant éventuellement remplacé par O, S, SO, SO₂ ou NR^{Y}, et un radical hétéroaryle pouvant porter 1, 2 ou 3 substituants, et un radical cycloalkyle pouvant porter un substituant choisi dans la série précédente, et
R⁴ représente R'', lorsque Q a la signification de NR',
R' représentant un atome d'hydrogène ou le groupe méthyle et
R'' représentant le groupe benzyle, et
lorsque R¹ et/ou R³ représente(nt) un groupe aryloxy en C₆-C₁₂, aralkyloxy en C₇-C₁₁, aryloxy(C₆-C₁₂)-alcoxy(C₁-C₆), aralcoxy(C₇-C₁₁)-alcoxy(C₁-C₆) ou un radical correspondant contenant des groupes cycloalkyle en bout de chaîne, ce radical représente de préférence un radical de formule D
OZ (D), ou
lorsque R¹ et/ou R³ représente(nt) un groupe aralkyle en C₇-C₁₁, aryloxy(C₆-C₁₂)-alkyle(C₁-C₆), aralcoxy(C₇-C₁₁)alkyle(C₁-C₆) ou un radical correspondant contenant des groupes cycloalkyle en bout de chaîne, ce radical représente de préférence un radical de formule Z,
R^{Y} et R^{Z} sont identiques ou différents et représentent un atome d'hydrogène ou un groupe aryle en C₆-C₁₂, alkyle en C₁-C₁₀, cycloalkyle en C₃-C_{10,} alcoxy(C₁ C₈)-alkyle(C₁-C₈), aralcoxy(C₇-C₁₂)-alkyle(C₁-C₈), aryloxy(C₆-C₁₂)alkyle(C₁-C₈), alcanoyle en C₁-C₁₀, un groupe aralcanoyle en C₇-C₁₆ éventuellement substitué ou un groupe aroyle en C₆-C₁₂ éventuellement substitué, ou
R^{Y} et R^{Z} représentent ensemble un groupement -[CH₂]ₕ-, dans lequel un groupe CH₂ peut être remplacé par O, S ou par un groupe N-alcanoyl(C₁-C₄)imino ou N-alcoxy(C₁-C₄)carbonylimino, et
f va de 1 à 8,
g est 0, 1 à (2f + 1),
h va de 3 à 6,
x va de 0 à 3 et
n est 3 ou 4,
y compris les sels physiologiquement actifs,
à l'exclusion du 3-benzyloxypyridine-2-(L-thréonylméthylester)carboxamide, du 3-benzyloxypyridine-2-[L-thréonyl-(Fmoc-Phg)-tert-butylester]carboxamide, du 3-benzyloxypyridine-2-(L-thréonyl-tert-butylester)carboxamide, du 3-benzyloxypyridine-2-(D-allothréonylméthylester)carboxamide et de l'ester éthylique de 3-benzyloxypicolinylglycine.

8. Composés de formule I selon les revendications 1 à 3, 6 et 7, dans lesquels
Q représente O,
X représente O,
Y représente CR³ et en outre N, lorsque R¹ et R² forment un cycle,
m est égal à 0,
A représente un groupe -CHR⁵- dans lequel R⁵ représente le substituant de l'atome de carbone α d'un α-aminoacide, en particulier d'un L-aminoacide naturel ou de son isomère D,
B représente -CO₂G,
G représentant un radical alkyle en C₁-C₁₈ aliphatique ramifié ou non ramifié ou cyclique, un radical cycloalkyl(C₃-C₈)-alkyle(C₁-C₈),
un radical alcényle en C₂-C₁₈ ramifié ou non ramifié, comme par exemple le radical géranyle ou farnésyle, ou le radical rétinyle, ou un radical alcynyle en C₂-C₁₈, le radical phényle, benzyle, phénéthyle, phénylpropyle, ou phénylbutyle,
les radicaux précédents comportant un substituant choisi parmi des groupes hydroxy, alcoxy en C₁-C₄, acyloxy, alkyl(C₁-C₆)-carbonyloxy, cycloalkyl(C₃-C₈)-carbonyloxy, benzoyloxy, phénylalkyl(C₇-C₁₆)-carbonyloxy, cycloalkyloxy(C₃-C₈)-carbonyloxy,
R² représente un atome d'hydrogène, de brome ou de chlore, ou un groupe cyano, alkyle en C₁-C₁₈, alcoxy en C₁-C₈, alcoxy(C₁-C₁₈)-méthyle, alcényloxy(C₂-C₁₈)-méthyle, alcynyloxy(C₂-C₁₈)-méthyle, carbamoyle, N-alkyl(C₁-C₁₀)carbamoyle, N-[alcoxy(C₁-C₁₂)-alkyl(C₁-C₄)]carbamoyle, N,N-dialkyl(C₁-C₈)-carbamoyle, N-cycloalkyl(C₃-C₈)carbamoyle, N-phénylcarbamoyle, N-phénylalkyl(C₇-C₁₂)carbamoyle, N-alkyl(C₁-C₆)-N-phénylcarbamoyle, N-alkyl(C₁-C₆)-N-phénylalkyl(C₇-C₁₂)-carbamoyle, N-[alcoxy(C₁-C₆)-alkyl(C₁-C₆)]carbamoyle, carboxy, alcoxy(C₁-C₂₀)carbonyle, alcényloxy(C₂-C₂₀)-carbonyle, rétinyloxycarbonyle, cycloalkloxy(C₃-C₈)-carbonyle, cycloalkyl(C₃-C₈)-alcoxy(C₁-C₆)-carbonyle, cycloalkyloxy(C₃-C₈)alcoxy(C₁-C₆)-carbonyle, phénylalcoxy(C₁-C₆)-carbonyle, phénoxyalcoxy(C₁-C₆)-carbonyle, benzyloxyalcoxy(C₁-C₆)carbonyle, un radical phényle étant substitué comme défini pour R¹ et R³, et
l'un des radicaux R¹ et R³ représente un atome d'hydrogène et l'autre représente un atome d'hydrogène, de fluor ou de chlore ou un groupe alkyle en C₁-C₈, alcoxy en C₁-C₁₀, cycloalkyle en C₅-C₆, cycloalkyl(C₅-C₆)-alkyle(C₁-C₆), cycloalkyloxy en C₅-C₆, cycloalkyl(C₅-C₆)-alcoxy(C₁-C₆), cycloalkyloxy(C₅-C₆)-alkyle(C₁-C₆), cycloalkyloxy(C₅-C₆)alcoxy(C₁-C₆), cycloalkyl(C₅-C₆)-alkyle(C₁-C₄)-alcoxy(C₁-C₄), cycloalkyl(C₅-C₆)-alcoxy(C₁-C₄)-alkyle(C₁-C₂), cycloalkyloxy (C₅-C₆)-alcoxy(C₁-C₄)-alkyle(C₁-C₂), -O-[CH₂]ₓC_{f}H_{(2f+1-g)}F_{g}, alcoxy(C₁-C₆)-alkyle(C₁-C₆), alcoxy(C₁-C₆)-alcoxy(C₁-C₆), alcoxy(C₁-C₆)-alcoxy(C₁-C₄)alkyle(C₁-C₂), un groupe phénoxy en C₆-C₁₂ substitué, un groupe phénylalkyloxy en C₇-C₁₁, phénoxy(C₆-C₁₂)-alcoxy(C₁-C₆) ou phénylalcoxy(C₇-C₁₁)-alcoxy(C₁-C₆), phénoxyalkyle(C₁-C₄), phénylalkyloxy(C₇-C₁₁)-alkyle(C₁-C₄), phénoxyalcoxy(C₁-C₄)-alkyle(C₁-C₂), phénylalkyloxy(C₇-C₁₁)-alcoxy(C₁-C₄)-alkyle(C₁-C₂), un radical aromatique étant substitué par 1, 2 ou 3 substituants identiques ou différents, choisis parmi des atomes de fluor et de chlore et des groupes cyano, trifluorométhyle, alkyle en C₁-C₁₂, alcényle en C₂-C₁₂, alcényloxy en C₂-C₁₂, alcoxy en C₁-C₁₂,
R¹ et R² forment, avec le reste pyridine qui les porte, un cycle 5,6,7,8-tétrahydroisoquinoléine,
R⁴ représente un radical alkyle en C₁-C₁₀ ramifié ou non ramifié, alcoxy(C₁-C₄)-alkyle(C₁-C₄) ou un radical de formule Z,
-[CH₂]ᵥ-[O]_{w}-[CH₂]ₜ-E (Z)
dans laquelle E représente un radical phényle substitué de formule F 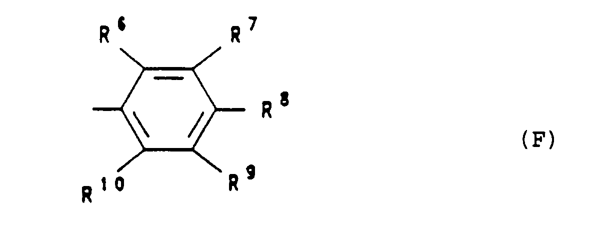 ou un radical cycloalkyle en C₃-C₈,
v pouvant être 0, 1, 2, 3, w étant égal à 0 et t étant égal à 0 ou 1 et,
R⁶, R⁷, R⁸, R⁹ et R¹⁰ étant identiques ou différents et représentant un atome d'hydrogène, de fluor ou de chlore, ou un groupe cyano, trifluorométhyle, alkyle en C₁-C₆, alcoxy en C₁-C₆, -O-[CH₂]ₓC_{f}H_{(2f+1-g)}F_{g}, N-alkyl(C₁-C₈)carbamoyle, N,N-dialkyl(C₁-C₆)-carbamoyle, N-cycloalkyl(C₃-C₆)-carbamoyle, N-(+)-déhydroabiétylaminocarbonyle, un groupephénylalkyl(C₇-C₁₁)-carbamoyle éventuellement substitué par un atome de fluor ou de chlore ou par un groupe trifluorométhyle ou alcoxy en C₁-C₆, ou
R⁶ et R⁷ ou R⁷ et R⁸ formant ensemble, avec le cycle phényle qui les porte, des dérivés de naphtalène, ou
lorsque R¹ ou R³ représente un groupe phénoxy en C₆-C₁₂, phénylalkyloxy en C₇-C₁₁, phénoxy(C₆-C₁₂)-alcoxy(C₁-C₆), phénylalcoxy(C₇-C₁₁)-alcoxy(C₁-C₆), cycloalkyloxy en C₅-C₆, cycloalkyl(C₅-C₆)-alcoxy(C₁-C₆), cycloalcoxy(C₅-C₆)-alcoxy(C₁-C₆) ou cycloalkyl(C₅-C₆)-alkyl(C₁-C₄)alcoxy(C₁-C₄), ce radical représente en particulier un radical de formule D
OZ (D), ou
lorsque R₁ ou R³ représente un groupe phényle, phénoxyalkyle(C₁-C₆), phénylalkyle en C₇-C₁₁, phénylalcoxy(C₇-C₁₁)-alkyle(C₁-C₄), cycloalkyle en C₅-C₆, cycloalkyl(C₅-C₆)-alkyle(C₁-C₆), cycloalkyloxy(C₅-C₆)-alkyle(C₁-C₄), cycloalkyl(C₅-C₆)-alcoxy(C₁-C₄)-alkyle(C₁-C₂), cycloalkyloxy(C₅-C₆)-alcoxy(C₁-C₄)-alkyle(C₁-C₂), ce radical représente en particulier un radical de formule Z,
dans les deux cas,
v représentant 1, 2, 3 ou 4, w étant égal à O et t étant égal à O ou
v représentant 1, 2, 3 ou 4, w étant égal à 1 et t étant égal à 0 ou
v représentant 1, 2, 3 ou 4, w étant égal à 1 et t étant égal à 1, et
f allant de 1 à 4,
g représentant 0 ou 1 à (2f + 1),
x étant égal à 0 ou 1,
y compris les sels physiologiquement actifs,
à l'exclusion du 3-benzyloxypyridine-2-(L-thréonylméthylester)carboxamide, du 3-benzyloxypyridine-2-[L-thréonyl(Fmoc-Phg)-tert-butylester]carboxamide, du 3-benzyloxypyridine-2-(L-thréonyl-tert-butylester)carboxamide, du 3-benzyloxypyridine-2-(D-allothréonylméthylester)carboxamide et de l'ester éthylique de 3-benzyloxypicolinylglycine.

9. Composés de formule I selon les revendications 1 à 3, 6, 7 et 8, dans lesquels
Q représente O,
X représente O,
Y représente CR³,
m est égal à 0,
A représente un groupe -CH₂- qui peut être substitué par un groupe méthyle,
B représente -CO₂G,
G représentant un radical alkyle en C₁-C₁₈ aliphatique ramifié ou non ramifié ou cyclique, un radical cycloalkyl(C₃-C₈)-alkyle(C₁-C₄), un radical alcényle en C₂-C₁₈ ramifié ou non ramifié, les radicaux précédents pouvant comporter un substituant choisi parmi des groupes hydroxy, alcoxy en C₁-C₄, acyloxy, alkyl(C₁-C₆)-carbonyloxy, cycloalkyl(C₃-C₈)-carbonyloxy, benzoyloxy, phénylalkyl (C₇-C₁₆)-carbonyloxy, cycloalkyloxy (C₃-C₈)-carbonyloxy, ou
G représente un radical phényle, benzyle, phénéthyle, phénylpropyle ou phénylbutyle,
R² représente un atome d'hydrogène ou un groupe alcoxy en C₁-C₈, alcoxy(C₁-C₁₆)-méthyle, alcényloxy(C₂-C₁₆)-méthyle, rétinyloxyméthyle, N-alkyl(C₁-C₁₀)-carbamoyle, N-[alcoxy(C₁-C₁₂)-alkyl(C₁-C₃)]carbamoyle, N,N-dialkyl(C₁-C₈)-carbamoyle, N-cycloalkyl(C₅-C₆)-carbamoyle, N-phénylcarbamoyle, N-phényl-alkyl(C₁-C₄)-carbamoyle, carboxy, alcoxy(C₁-C₁₆)-carbonyle, alcényloxy(C₂-C₁₆)-carbonyle, rétinyloxycarbonyle, cycloalkyloxy (C₅-C₆)-carbonyle, cycloalkyl(C₅-C₆)-alcoxy(C₁-C₆)-carbonyle, phénylalcoxy(C₁-C₆)-carbonyle, un radical phényle étant substitué comme défini pour R¹ et R³, et
l'un des radicaux R¹ et R³ représente un atome d'hydrogène et l'autre représente un atome d'hydrogène ou un groupe alcoxy en C₁-C₁₀, cycloalkyloxy en C₅-C₆, cycloalkyl(C₅-C₆)-alcoxy(C₁-C₂), -O-[CH₂]ₓC_{f}H_{(2f+1-g)}F_{g}, alcoxy(C₁-C₄)-alcoxy(C₁-C₄), phénoxy en C₆-C₁₂ substitué, phénylalkyloxy en C₇-C₁₁, phénoxy(C₆-C₁₂)-alcoxy(C₁-C₄) ou phénylalcoxy(C₇-C₁₁)-alcoxy(C₁-C₄), un radical aromatique étant substitué par 1, 2 ou 3 substituants, identiques ou différents, choisis parmi des atomes de fluor et de chlore et des groupes cyano, trifluorométhyle, alkyle en C₁-C₁₀, alcoxy en C₁-C₁₀, alcényloxy en C₁-C₁₀ et
R⁴ représente un radical alkyle en C₁-C₈ ramifié ou non ramifié ou un radical de formule Z
-[CH₂]ᵥ-[O]_{w}-[CH₂]ₜ-E (Z)
E représentant un radical phényle substitué de formule F 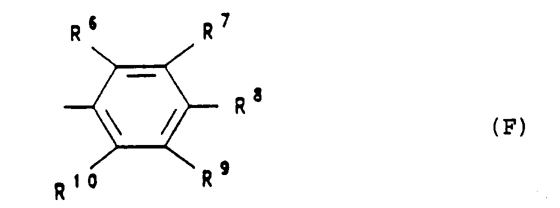 ou un radical cycloalkyle en C₃-C₈,
v représentant 0, 1, 2, 3, w étant égal à 0 et t pouvant être 0 ou 1, et
R⁶, R⁷, R⁸, R⁹ et R¹⁰ étant identiques ou différents et représentant un radical benzyle substitué par un atome d'hydrogène, de fluor ou de chlore, ou par un groupe cyano, trifluorométhyle, alkyle en C₁-C₆, alcoxy en C₁-C₆, -O-[CH₂]ₓC_{f}H_{(2f+1-g)}F_{g}, N-alkyl(C₁-C₈)-carbamoyle, N,N-dialkyl(C₁-C₆)-carbamoyle, N-cycloalkyl(C₃-C₆)-carbamoyle, N-(+)-déhydroabiétylaminocarbonyle, et f allant de 1 à 4, g représentant 0 ou 1 à (2f + 1) et x étant égal à 0 ou 1,
y compris les sels physiologiquement actifs,
à l'exclusion de l'ester éthylique de 3-benzyloxypicolinylglycine.

10. Composés de formule I selon les revendications 1 à 3 et 6 à 9, dans lesquels
Q représente O,
X représente O,
Y représente CR³,
m est égal à 0,
A représente un groupe -CH₂-,
B représente -CO₂G,
G représentant un radical alkyle en C₁-C₁₆ aliphatique ramifié ou non ramifié, un radical 2-cyclohexyléthyle, un radical alcoxy(C₁-C₄)-alkyle(C₁-C₂), un radical alcényle en C₂-C₁₀ ramifié ou non ramifié, le radical phényle, benzyle, phénéthyle, phénylpropyle ou phénylbutyle,
R¹ représente un atome d'hydrogène ou un groupe alcoxy en C₁-C₆, -O-[CH₂]ₓC_{f}H_{(2f+1-g)}F_{g},
R² représente un atome d'hydrogène ou un groupe N-[alkyl(C₁-C₁₀)-carbamoyle, N-[alcoxy(C₁-C₁₂)-alkyl(C₁-C₃)]-carbamoyle, N,N-dialkyl(C₁-C₈)-carbamoyle, N-cycloalkyl(C₅-C₆)-carbamoyle, N-phénylcarbamoyle, N-phénylalkyl(C₁-C₂)-carbamoyle, carboxy, alcoxy(C₁-C₁₆)-carbonyle, alcényloxy(C₂-C₁₆)-carbonyle, rétinyloxycarbonyle, cycloalkyloxy (C₅-C₆)-carbonyle, cycloalkyl-(C₅-C₆)-alcoxy(C₁-C₆)-carbonyle, phénylalcoxy(C₁-C₆)-carbonyle, un radical phényle étant substitué par un ou deux substituants identiques ou différents choisis parmi des atomes de fluor et de chlore et des groupes cyano, trifluorométhyle, alkyle en C₁-C₁₀, alcoxy en C₁-C₁₀, alcényloxy en C₁-C₁₀, et
R³ représente un atome d'hydrogène ou un groupe alcoxy en C₁-C₅, cycloalkyl(C₅-C₆)-alcoxy(C₁-C₂), l'un des substituants R¹ et R³ représentant un atome d'hydrogène,
R⁴ représente le radical alkyle en C₁-C₆ ramifié ou non ramifié, ou le radical 2-phényléthyle, ou un radical benzyle substitué par un ou deux substituants choisis parmi des atomes de fluor et de chlore et des groupes cyano, trifluorométhyle, alkyle en C₁-C₆, alcoxy en C₁-C₆, -O-[CH₂]ₓC_{f}H_{(2f+1-g)}F_{g}, N-alkyl(C₁-C₈)-carbamoyle, N,N-dialkyl(C₁-C₆)-carbamoyle, N-cycloalkyl(C₃-C₆)-carbamoyle, N-(+)-déhydroabiétylaminocarbonyle, et f représente 1 à 4, g représente 0, 1 à (2f + 1) et x est égal à 1,
y compris les sels physiologiquement actifs.

11. Composés de formule I selon les revendications 1 à 3 et 6 à 10, dans lesquels
Q représente O,
X représente O,
Y représente CR³,
m est égal à 0,
A représente un groupe -CH₂-,
B représente -CO₂G,
G représentant un radical alkyle en C₁-C₁₆ aliphatique ramifié ou non ramifié, ou le radical benzyle,
R¹ représente un atome d'hydrogène,
R² représente un atome d'hydrogène ou un groupe N-alkyl-(C₁-C₁₀)-carbamoyle, N-[alcoxy(C₁-C₁₂)-alkyl(C₁-C₃)]-carbamoyle, N-cyclohexylcarbamoyle, N-phénylcarbamoyle, N-[phénylalkyl(C₁-C₂)]carbamoyle, dans les deux derniers cas le radical phényle pouvant porter un substituant fluoro, alkyle en C₁-C₁₀ ou alcoxy en C₁-C₁₀, ou représente un groupe carboxy, alcoxy(C₁-C₁₆)-carbonyle, alcényloxy(C₂-C₁₆)-carbonyle, rétinyloxycarbonyle, cycloalkyloxy (C₅-C₆)-carbonyle, benzyloxycarbonyle,
R³ représente un atome d'hydrogène ou un groupe alcoxy en C₁-C₅, 2-(cyclohexyl)éthyloxy, l'un des substituants R² et R³ représentant un atome d'hydrogène,
R⁴ représente un radical alkyle en C₁-C₄ ramifié ou non ramifié, ou un radical benzyle monosubstitué par un atome de fluor ou de chlore ou par un groupe trifluorométhyle, alkyle en C₁-C₄ ou alcoxy en C₁-C₃,
y compris les sels physiologiquement actifs.

12. Composés de formule I selon les revendications 1 à 3 et 6, dans lesquels
Q représente O,
X représente O,
Y représente CR³,
R³ représentant un atome d'hydrogène,
m est égal à 0,
A représente un groupe -CH₂-,
B représente -CO₂G,
G représentant un radical alkyle en C₁-C₁₆ aliphatique ramifié ou non ramifié ou le radical benzyle,
R¹ et R² forment ensemble, avec le reste pyridine qui les porte, un cycle isoquinoléine à fragment benzénique non substitué, et
R⁴ représente le groupe méthyle.

13. Composés de formule I selon les revendications 1 à 3 et 6, dans lesquels
Q représente O,
X représente O,
Y représente CR³,
m est égal à 0,
A représente un groupe -CH₂-,
B représente -CO₂G,
G représentant un radical alkyle en C₁-C₁₆ aliphatique ramifié ou non ramifié ou le radical benzyle,
R¹ représente un atome d'hydrogène, et
R² et R³ forment, conjointement avec le reste pyridine qui les porte, un cycle quinoléine à fragment benzénique non substitué, et
R⁴ représente le groupe méthyle.

14. Composés de formule I selon les revendications 1, 2, 4 et 5, dans lesquels
Q représente S,
X représente O,
Y représente CR³,
m est égal à 0,
A représente un groupe -CH₂-,
B représente -CO₂G,
G représentant un radical alkyle en C₁-C₁₆ aliphatique ramifié ou non ramifié ou le radical benzyle,
R¹ représente un atome d'hydrogène,
R² représente un atome d'hydrogène ou un groupe N-alkyl-(C₁-C₁₀)-carbamoyle, N-[alcoxy(C₁-C₁₂)-alkyl(C₁-C₃)]-carbamoyle, N-cyclohexylcarbamoyle, N-phénylcarbamoyle, N-[phényl-alkyl(C₁-C₂)]carbamoyle, dans les: deux derniers cas le radical phényle pouvant porter un substituant fluoro, alkyle en C₁-C₁₀ ou alcoxy en C₁-C₁₀, ou représente un groupe carboxy, alcoxy(C₁-C₁₆)-carbonyle, alcényloxy(C₂-C₁₆)-carbonyle, rétinyloxycarbonyle, cycloalkyloxy(C₅-C₆)-carbonyle, benzyloxycarbonyle,
R³ représente un atome d'hydrogène ou un groupe alcoxy en C₁-C₅, 2-(cyclohexyl)éthyloxy, l'un des substituants R² et R³ représentant un atome d'hydrogène,
R⁴ représente un radical alkyle en C₁-C₄ ramifié ou non ramifié ou un radical benzyle monosubstitué par un atome de fluor ou de chlore ou par un groupe trifluorométhyle, alkyle en C₁-C₄ ou alcoxy en C₁-C₃.

15. Composés de formule I selon les revendications 1, 2, 4, 5 et 14, dans lesquels
Q représente S,
X représente O,
Y représente CR³,
m est égal à 0,
A représente un groupe -CH₂-,
B représente -CO₂G,
G représentant un radical alkyle en C₁-C₁₆ aliphatique ramifié ou non ramifié ou le radical benzyle,
R¹ représente un atome d'hydrogène,
R² représente un groupe carboxy ou alcoxy(C₁-C₁₆)-carbonyle,
R³ représente un atome d'hydrogène et
R⁴ représente un radical alkyle en C₁-C₄ ramifié ou non ramifié.

16. Composés de formule I selon les revendications 1 à 15, y compris le 3-benzyloxypyridine-2-(L-thréonylméthylester)carboxamide, le 3-benzyloxypyridine-2-[L-thréonyl-(Fmoc-Phg)-tert-butylester]carboxamide, le 3-benzyloxypyridine-2-(L-thréonyl-tert-butylester)carboxamide, le 3-benzyloxypyridine-2-(D-allothréonylméthylester)carboxamide et l'ester éthylique de 3-benzyloxypicolinylglycine en tant que médicaments.

17. Composés selon les revendications 1 à 16, pour utilisation pour l'inhibition de la biosynthèse du collagène.

18. Composés selon les revendications 1 à 16, en tant que précurseurs ester des inhibiteurs de prolylhydroxylase de formule I, dans lesquels B représente le groupe carboxy ou un sel de celui-ci.

19. Composés selon les revendications 1 à 16, pour utilisation en tant que fibrosuppresseurs.

20. Composés selon les revendications 1 à 16, pour la fabrication d'un médicament contre des maladies fibreuses.

21. Composés selon les revendications 1 à 16, pour la fabrication d'un médicament contre des maladies fibreuses du foie.

22. Composés selon les revendications 1 à 16, pour la fabrication d'un médicament contre des maladies fibreuses du poumon.

23. Composés selon les revendications 1 à 16, pour la fabrication d'un médicament contre des maladies fibreuses de la peau.

24. Procédé pour la préparation des composés de formule I selon les revendications 1 à 15, dans lesquels
A représente un fragment alkylène substitué,
B représente -CO₂G,
Y représente CR³ et
m représente 0 ou 1,
dans lequel
i.1) on fait réagir des acides pyridine-2-carboxyliques de formule II (R²³ = H) avec les aminoesters de formule III, pour aboutir aux amidoesters de formule I, ou
i.2) on fait réagir des esters d'acides pyridine-2-carboxyliques de formule II (R²³ représente un groupe alkyle inférieur) dans les conditions de l'aminolyse, pour aboutir aux composés de formule I; 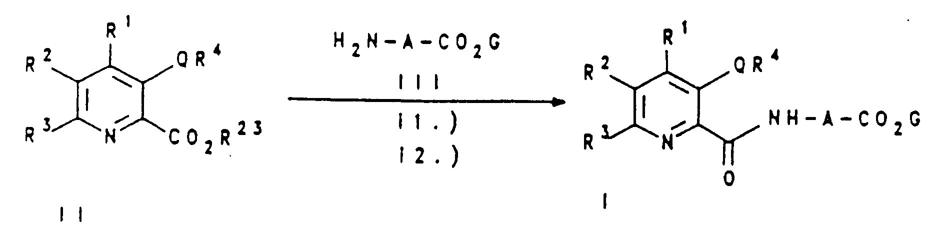 ou
ii) on estérifie les composés de formule IV par l'alcool GOH, pour aboutir aux composés de formule I; 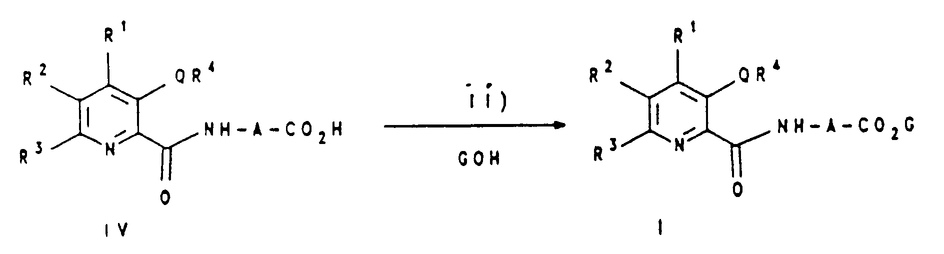 ou
iii) on soumet les composés de formule V à une alkylation avec R⁴X, pour aboutir aux composés de formule I; 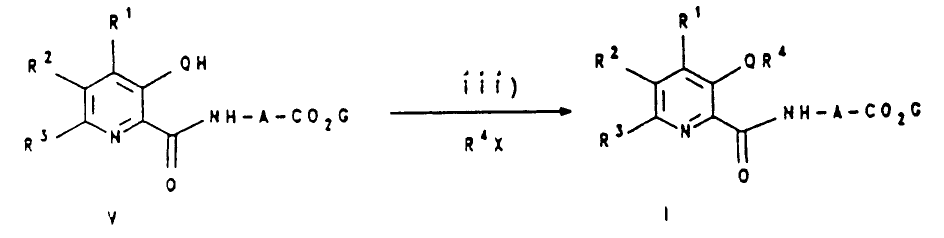 X représentant un groupe séparable, en particulier un atome d'halogène ou un groupe OSO₂Me ou OSO₂-phényle, et éventuellement
iv) on convertit les composés de formule I, lorsque Q représente O ou NR', en leurs N-oxydes de pyridine (I').
